(19) Europäisches Patentamt
European Patent Office
Office européen des brevets

(11) EP 2 516 008 B1

(12) **EUROPEAN PATENT SPECIFICATION**

(45) Date of publication and mention of the grant of the patent:
**16.04.2014 Bulletin 2014/16**

(21) Application number: **10801407.7**

(22) Date of filing: **20.12.2010**

(51) Int Cl.:
*A61P 31/18* (2006.01) *A61K 31/519* (2006.01)
*C07D 487/04* (2006.01)

(86) International application number:
**PCT/EP2010/070306**

(87) International publication number:
**WO 2011/076765 (30.06.2011 Gazette 2011/26)**

# (54) NOVEL ANTIVIRAL COMPOUNDS

NEUE ANTIVIRALE VERBINDUNGEN

NOUVEAUX COMPOSÉS ANTIVIRAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2009 GB 0922520**
**26.01.2010 GB 201001204**

(43) Date of publication of application:
**31.10.2012 Bulletin 2012/44**

(73) Proprietor: **Katholieke Universiteit Leuven**
**3000 Leuven (BE)**

(72) Inventors:
- **CARLENS, Gunter**
  **B-3401 Landen (BE)**
- **CHALTIN, Patrick**
  **B-1370 Zetrud-lumay (BE)**
- **CHRIST, Frauke**
  **B-3001 Heverlee (BE)**
- **DEBYSER, Zeger**
  **B-3001 Heverlee (BE)**
- **MARCHAND, Arnaud**
  **B-3360 Korbeek-lo (BE)**
- **MARCHAND, Damien**
  **2000 Antwerp (BE)**
- **VOET, Arnout**
  **B-8550 Zwevegem (BE)**
- **DE MAEYER, Marc**
  **B-3054 Vaalbeek (BE)**

(74) Representative: **Cabinet Plasseraud**
**96/98 Boulevard Carnot**
**BP 105**
**59027 Lille Cedex (FR)**

(56) References cited:
**EP-A1- 1 375 486** **US-A1- 2004 147 547**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 516 008 B1

**Description**

## FIELD OF THE INVENTION

The present invention relates to a series of novel compounds having antiviral activity, more specifically HIV (Human Immunodeficiency Virus) replication inhibiting activity. The invention also relates to methods for the preparation of such compounds, as well as to novel intermediates useful in one or more steps of such syntheses. The invention also relates to pharmaceutical compositions comprising an effective amount of such compounds as active ingredients. This invention further relates to the compounds for use as a medicine and to use of such compounds in the manufacture of a medicament, more in particular useful for the prevention or treatment of subjects suffering from viral infections, in particular HIV infection. Also described are methods for the prevention or treatment of viral infections in animals by the administration a therapeutically effective amount of such compounds, optionally combined with one or more other drugs having antiviral activity.

## BACKGROUND OF THE INVENTION

A retrovirus designated human immunodeficiency virus (HIV) is the etiological agent of the complex disease that includes progressive destruction of the immune system (acquired immune deficiency syndrome, hereinafter AIDS) and degeneration of the central and peripheral nervous system. There are two types of HIV, HIV-1 and HIV-2, the latter producing a less severe disease than the former. Being a retrovirus, its genetic material is in the form of RNA (ribonucleic acid) consisting of two single RNA strands. Coexisting with RNA are reverse transcriptase (having polymerase and ribonuclease activity), integrase, a protease and other proteins.

It is known in the art that some antiviral compounds which act as inhibitors of HIV replication are effective agents in the treatment of AIDS and similar diseases. Drugs that are known and approved for the treatment of HIV-infected patients belong to one of the following classes:

- nucleoside reverse transcriptase (RT) inhibitors such as, but not limited to, azidothymidine (AZT), and lamivudine (3TC),
- nucleotide reverse transcriptase inhibitors such as, but not limited to, tenofovir (R-PMPA),
- non-nucleoside reverse transcriptase inhibitors such as, but not limited to, nevirapine, efavirenz, etravirine and lersivirine,
- protease inhibitors such as, but not limited to, nelfinavir, saquinavir, ritonavir, atazanavir, darunavir and amprenavir,
- fusion inhibitors such as enfuvirtide,
- CCR5 antagonists such as maraviroc, and
- integrase inhibitors such as raltegravir or elvitegravir.

Replication of the human immunodeficiency virus type 1 (hereinafter referred as HIV-1) can be drastically reduced in infected patients by combining potent antiviral drugs targeted at multiple viral targets, as reviewed by Vandamme et al. in Antiviral Chem. Chemother. (1998) 9:187-203.

Multiple-drug combination regimes can reduce viral load below the detection limit of the most sensitive tests. Nevertheless low level ongoing replication has been shown to occur, possibly in sanctuary sites, leading to the emergence of drug-resistant strains, according to Perelson et al. in Nature (1997) 387:123-124. Furthermore the selectivity of many antiviral agents is rather low, possibly making them responsible for side-effects and toxicity. Moreover, HIV can develop resistance to most, if not all, currently approved antiviral drugs, according to Schmit et al. in J. Infect. Dis. (1996) 174:962-968. It is well documented that the ability of HIV to rapidly evolve drug resistance, together with toxicity problems resulting from known drugs, requires the development of additional classes of antiviral drugs.

Thus, there is still a stringent need in the art for potent inhibitors of HIV. Therefore a goal of the present invention is to satisfy this urgent need by identifying efficient pharmaceutically active ingredients that are active against HIV, less toxic, more stable (i.e. chemically stable, metabolically stable), effective against viruses resistant to currently available drugs and/or which are more resistant to virus mutations than existing antiviral drugs and that can be useful, either alone or in combination with other active ingredients, for the treatment of retroviral infections, in particular lentiviral infections, and more particularly HIV infections, in mammals and more specifically in humans. It is also known to the skilled in the art that the physicochemical properties of known drugs as well as their ADME-Tox (administration, distribution, metabolism, excretion and toxicology) properties may limit or prohibit their use in the treatment of diseases. Therefore, a problem of existing drugs that can be overcome with the compounds of the invention can be selected from a poor or inadequate physicochemical or ADME-Tox properties such as solubility, LogP, CYP inhibition, hepatic stability, plasma stability, among others have been taken into account in the design and the synthesis of the compounds of the present invention. Furthermore, another goal of the present invention is to complement existing antiviral drugs in such a way

that the resulting drug combination has improved activity or improved resistance to virus mutation than each of the individual compounds.

## SUMMARY OF THE INVENTION

The present invention is based on the unexpected finding that at least one of the above-mentioned problems can be solved by a novel class of compounds.

The present invention provides new antiviral agents, especially anti-retroviral agents, and more particularly anti-HIV compounds. These compounds have a structure as described further herein and we show that they possess antiviral activity, more specifically against HIV. The present invention demonstrates that these compounds efficiently inhibit the replication of HIV. Therefore, these compounds constitute a useful class of new potent antiviral compounds that can be used in the treatment and/or prevention of viral infections in animals, mammals and humans, more specifically for the treatment and/or prevention of HIV in humans.

The present invention furthermore relates to the use of such compounds as medicines, more specifically as antiviral agents, and to their use for the manufacture of medicaments for treating and/or preventing viral infections, in particular retroviral infections such as, but not limited to HIV in a subject such as humans. The invention also relates to methods for the preparation of all such compounds and to pharmaceutical compositions comprising them in an antiviral effective amount.

Also described is a method of treatment or prevention of viral infections, in particular retroviral infections such as, but not limited to HIV in humans or animals by the administration of one or more such compounds, optionally in combination with one or more other antiviral agents, to a patient in need thereof.

One aspect of the present invention is the provision of novel compounds, said compounds having a structure according to the formula (A):

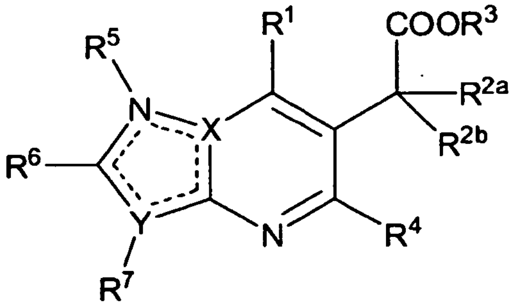

(A)

wherein,

- each dotted line represents an optional double bond whereby two dotted lines of the 5 dotted lines constitute a double bond and these 2 double bonds are non-adjacent;
- each of X and Y are independently selected from C or N, whereby at least one of X and Y is N;
- $R^1$ is independently selected from cycloalkyl; cycloalkenyl; cycloalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl or heterocycle-heteroalkynyl;
  and wherein said cycloalkyl, cycloalkenyl, cycloalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more $R^{10}$;
- each of $R^{2a}$ and $R^{2b}$ is independently selected from hydrogen; cyano; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl, heterocycle-heteroalkenyl, or heterocycle-heteroalkynyl; or $R^{2a}$ and $R^{2b}$ can be taken together to form vinyl or vinylalkyl;
  and wherein said alkyl, alkenyl, alkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- $R^3$ is independently selected from hydrogen; alkyl; alkenyl; alkynyl; heteroalkyl; heteroalkenyl; heteroalkynyl; aryl;

heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl;

wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, - C(O)OH or $NH_2$;

- $R^4$ is independently selected from hydrogen; alkyl; alkenyl or alkynyl; wherein said alkyl, alkenyl or alkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- $R^5$ is not present or is selected from hydrogen; alkyl; alkenyl; alkynyl; heteroalkyl; heteroalkenyl; and heteroalkynyl; wherein said alkyl, alkenyl, alkynyl, heteroalkyl, heteroalkenyl, and heteroalkynyl can be unsubstituted or substituted with one or more one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- $R^6$ is selected from hydrogen; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- $R^7$ is selected from being not present; hydrogen; halogen; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- each $R^{10}$ is independently selected from the group consisting of halogen; $-OR^{11}$; =O; $-SR^{11}$; =S; $-S(O)R^{12}$; $-S(O)_2R^{12}$; $-S(O)_2NR^{13}R^{14}$; trifluoromethyl; nitro; - $NR^{13}R^{14}$; $-NR^{11}S(O)_2R^{12}$; cyano; $-C(O)OR^{11}$; $-C(O)NR^{13}R^{14}$; $-C(O)R^{12}$; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl; and wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- each $R^{11}$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl; heterocycle-heteroalkenyl; and heterocycle-heteroalkynyl;
wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocycle-alkynyl, heterocycle-heteroalkyl, heterocycle-heteroalkenyl, and heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- each $R^{12}$ is independently selected from hydrogen; hydroxyl; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl; heterocycle-heteroalkenyl; and heterocycle-heteroalkynyl;
wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocycle-alkynyl, heterocycle-heteroalkyl, heterocycle-heteroalkenyl, and heterocycle-heteroalkynyl can be unsubstituted

or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;

- each $R^{13}$ and $R^{14}$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl; heterocycle-heteroalkenyl; and heterocycle-heteroalkynyl;
  and wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocycle-alkynyl, heterocycle-heteroalkyl, heterocycle-heteroalkenyl, and heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
  and wherein $R^{13}$ and $R^{14}$ can be taken together with the N to which they are attached in order to form a (5-, 6-, or 7-membered) heterocycle which can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, - C(O)OH or $NH_2$;

and pharmaceutically acceptable salts thereof.

In a particular embodiment, $R^1$ is selected from aryl or heterocycle, and yet in a more particular embodiment is selected from phenyl or heteroaryl, wherein said aryl, heterocycle, heteroaryl or phenyl can be unsubstituted or substituted, in a particular embodiment substituted with one or more $R^{10}$.

In yet another particular embodiment, one of $R^{2a}$ and $R^{2b}$ is not hydrogen. In another particular embodiment, one of $R^{2a}$ and $R^{2b}$ is hydrogen and the other of $R^{2a}$ and $R^{2b}$ is selected from alkyl, alkenyl, alkynyl, heteroalkyl, heteroalkenyl or heteroalkynyl. In a yet more particular embodiment, one of $R^{2a}$ and $R^{2b}$ is hydrogen and the other of $R^{2a}$ and $R^{2b}$ is selected from alkyl and heteroalkyl.

In yet another particular embodiment, $R^3$ is H.

In yet another particular embodiment, $R^4$ is selected from hydrogen and alkyl, more in particular is methyl.

In yet another particular embodiment, $R^5$ is selected from being not present, hydrogen, and alkyl.

In still another particular embodiment, $R^6$ is selected from hydrogen, alkyl, aryl, and heterocycle, wherein said alkyl, aryl, and heterocycle can be unsubstituted or substituted.

In yet another particular embodiment, $R^7$ is selected from being not present, hydrogen, halogen, alkyl, aryl, and heterocycle, wherein said alkyl, aryl, and heterocycle can be unsubstituted or substituted.

In another embodiment, the compounds of the invention have a structure according to formula (B),

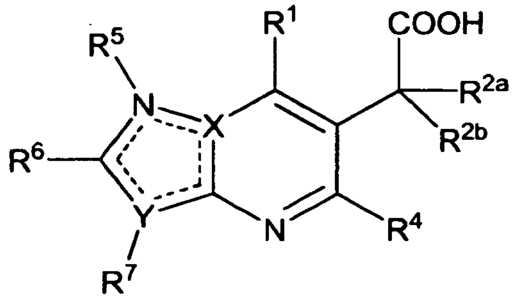

(B)

wherein each of X, Y, the dotted lines, $R^1$, $R^{2a}$, $R^{2b}$, $R^4$, $R^5$, $R^6$, and $R^7$ are as in formula (A) and the embodiments described herein.

In another embodiment, the compounds of the invention have a structure according to formula (C-I) or (C-II),

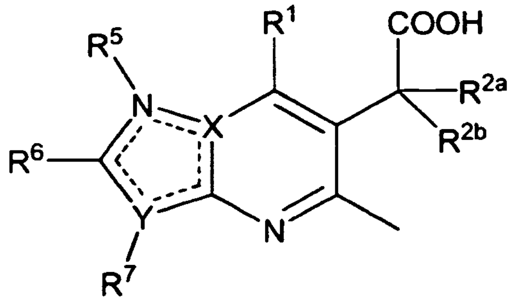

(C-I)

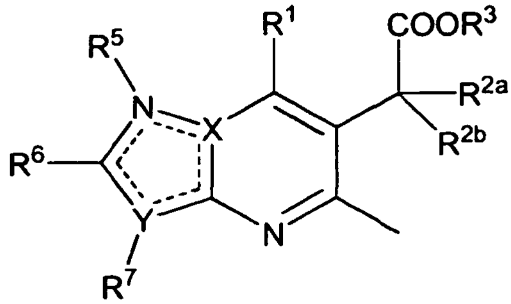

(C-II)

wherein each of X, Y, the dotted lines, $R^1$, $R^{2a}$, $R^{2b}$, $R^3$, $R^5$, $R^6$, and $R^7$ are as in formula (A) and the embodiments described herein. In another embodiment, the compounds of the invention have a structure according to formula (D),

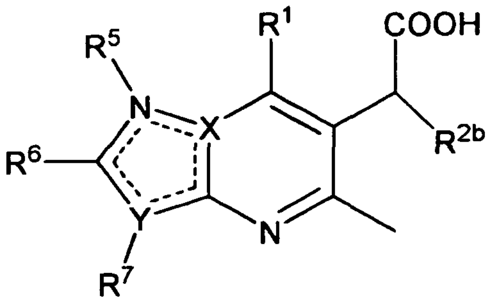

(D)

wherein each of X, Y, the dotted lines, $R^1$, $R^{2b}$, $R^5$, $R^6$, and $R^7$ are as in formula (A) and the embodiments described herein.

In another embodiment, the compounds of the invention have a structure according to formula (E), which consist of formulas (E-I), (E-II), (E-III), or (E-IV),

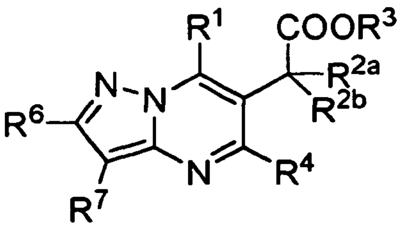

(E-I)

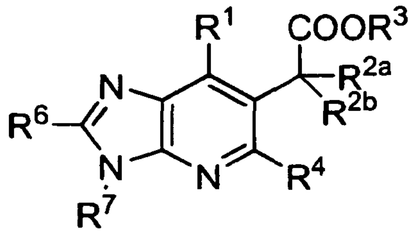

(E-II)

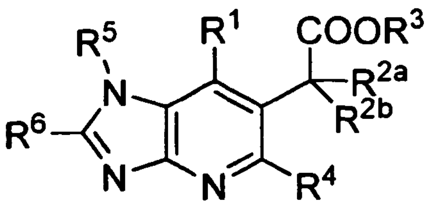

(E-III)

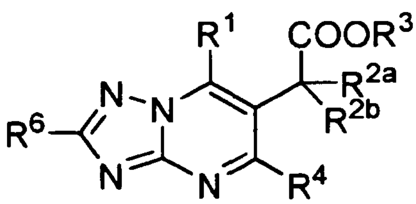

(E-IV)

wherein each of $R^1$, $R^{2a}$, $R^{2b}$, $R^3$, $R^4$, $R^5$, $R^6$, and $R^7$ are as in formula (A) and the embodiments described herein.

Particular embodiments of this aspect are described in the claims and relate to subtypes of the compounds of the invention. In particular embodiments, the terms alkyl, alkenyl or alkynyl can be restricted to refer to their cyclic or acyclic subgroups (such as the acyclic alkyl or cycloalkyl for alkyl).

In a particular embodiment, the compounds of the present invention are selected from the list of:

Ethyl 2-(7-((R)-3-(4-chlorophenylsulfonamido)piperidin-1-yl)-5-methylpyrazolo[1,5-a]pyrimidin-6-yl)pentanoate;
Ethyl 2-(7-((S)-3-(4-chlorophenylsulfonamido)piperidin-1-yl)-5-methylpyrazolo[1,5-a]pyrimidin-6-yl)pentanoate;
Ethyl 2-(7-((R)-3-(4-chlorophenylsulfonamido)pyrrolidin-1-yl)-5-methylpyrazolo[1,5-a]pyrimidin-6-yl)pentanoate;

Ethyl 2-(7-((S)-3-(4-chlorophenylsulfonamido)pyrrolidin-1-yl)-5-methylpyrazolo[1,5-a]pyrimidin-6-yl)pentanoate;
Methyl 2-(5-methyl-7-phenylpyrazolo[1,5-a]pyrimidin-6-yl)pentanoate;
Methyl 2-(5-methyl-7-p-tolylpyrazolo[1,5-a]pyrimidin-6-yl)pentanoate;
Methyl 2-(2-tert-butyl-5-methyl-7-phenylpyrazolo[1,5-a]pyrimidin-6-yl)pentanoate;
Methyl 2-(2-tert-butyl-5-methyl-7-p-tolylpyrazolo[1,5-a]pyrimidin-6-yl)pentanoate;
Methyl 2-(5-methyl-2-phenyl-7-p-tolylpyrazolo[1,5-a]pyrimidin-6-yl)pentanoate;
Methyl 2-(2-tert-butyl-7-(3-hydroxyphenyl)-5-methylpyrazolo[1,5-a]pyrimidin-6-yl)pentanoate;
Methyl 2-(2-tert-butyl-7-(2-naphthyl)-5-methylpyrazolo[1,5-a]pyrimidin-6-yl)pentanoate;
Methyl 2-(2-tert-butyl-7-(1H-indol-5-yl)-5-methylpyrazolo[1,5-a]pyrimidin-6-yl)pentanoate;
Methyl 2-(2-tert-butyl-7-(1H-indol-6-yl)-5-methylpyrazolo[1,5-a]pyrimidin-6-yl)pentanoate;
Methyl 2-(2-tert-butyl)-7-(1-benzofuran-5-yl)-5-methylpyrazolo[1,5-a]pyrimidin-6-yl)pentanoate;
Methyl 2-(2-tert-butyl-7-(1-benzothiophen-5-yl)-5-methylpyrazolo[1,5-a]pyrimidin-6-yl)pentanoate;
Methyl 2-(2-tert-butyl-7-(2,3-dihydrobenzofuran-5-yl)-5-methylpyrazolo[1,5-a]pyrimidin-6-yl)pentanoate;
Methyl 2-(2-tert-butyl-7-(4-chlorophenyl)-5-methylpyrazolo[1,5-a]pyrimidin-6-yl)pentanoate;
Methyl 2-(2-tert-butyl-7-(3,4-dimethylphenyl)-5-methylpyrazolo[1,5-a]pyrimidin-6-yl)pentanoate;
Methyl 2-(2-tert-butyl-7-(4-ethylphenyl)-5-methylpyrazolo[1,5-a]pyrimidin-6-yl)pentanoate;
Methyl 2-(2-tert-butyl-7-(3,4-dihydro-2H-benzo[b][1,4]dioxepin-7-yl)-5-methylpyrazolo[1,5-a]pyrimidin-6-yl)pentanoate;
Methyl 2-(2-tert-butyl-7-(2-(7-(4-methyl-3,4-dihydro-2H-benzo[b][1,4]oxazin-7-yl)-5-methylpyrazolo[1,5-a]pyrimidin-6-yl)pentanoate;
Ethyl 2-(3-bromo-5-methyl-7-p-tolylpyrazolo[1,5-a]pyrimidin-6-yl)pentanoate;
Ethyl 2-(5-methyl-3-phenyl-7-p-tolylpyrazolo[1,5-a]pyrimidin-6-yl)pentanoate;
Ethyl 2-(5-methyl-3,7-di-p-tolylpyrazolo[1,5-a]pyrimidin-6-yl)pentanoate;
Methyl 2-(3-bromo-2-tert-butyl-5-methyl-7-p-tolylpyrazolo[1,5-a]pyrimidin-6-yl)pentanoate;
Methyl 2-(2-tert-butyl-5-methyl-3,7-di-p-tolylpyrazolo[1,5-a]pyrimidin-6-yl)pentanoate;
Methyl 2-(5-methyl-2-propyl-7-p-tolylpyrazolo[1,5-a]pyrimidin-6-yl)pentanoate;
Methyl 2-(2-(furan-2-yl)-5-methyl-7-p-tolylpyrazolo[1,5-a]pyrimidin-6-yl)pentanoate;
Methyl 2-(2-tert-butyl-7-(4-chloro-2-fluorophenyl)-5-methylpyrazolo[1,5-a]pyrimidin-6-yl)pentanoate;
Methyl 2-(2-tert-butyl-7-(2-fluoro-4-methylphenyl)-5-methylpyrazolo[1,5-a]pyrimidin-6-yl)pentanoate;
Methyl 2-(2-tert-butyl-7-p-tolyl-5-methylpyrazolo[1,5-a]pyrimidin-6-yl)-6,6,6-trifluorohexanoate;
Methyl 2-(2-tert-butyl-7-p-tolyl-5-methylpyrazolo[1,5-a]pyrimidin-6-yl)-3-phenylpropanoate;
Methyl 2-(2-tert-butyl-7-(1-methylindol-5-yl)-5-methylpyrazolo[1,5-a]pyrimidin-6-yl)-3-phenylpropanoate;
Methyl 2-(2-tert-butyl-5-methyl-7-(1-methylindolin-5-yl)pyrazolo[1,5-a]pyrimidin-6-yl)pentanoate;
Methyl 2-(2-tert-butyl-5-methyl-7-(1-methyl-1H-indol-6-yl)pyrazolo[1,5-a]pyrimidin-6-yl)pentanoate;
Methyl 2-(2-tert-butyl-7-(chroman-6-yl)-5-methylpyrazolo[1,5-a]pyrimidin-6-yl)pentanoate;
Methyl 2-(2-tert-butyl-7-p-tolyl-5-methylpyrazolo[1,5-a]pyrimidin-6-yl)-3-methylpentanoate;
Methyl 2-(2-tert-butyl-3-chloro-7-p-tolyl-5-methylpyrazolo[1,5-a]pyrimidin-6-yl)pentanoate;
Methyl 2-(2-tert-butyl-7-p-tolyl-5-methylpyrazolo[1,5-a]pyrimidin-6-yl)-4-methoxybutanoate;
Methyl 2-(2-tert-butyl-5-methyl-7-(4-iso-propylphenyl)pyrazolo[1,5-a]pyrimidin-6-yl)pentanoate;
Methyl 2-(2-tert-butyl-5-methyl-7-(4-trifluoromethylphenyl)pyrazolo[1,5-a]pyrimidin-6-yl)pentanoate;
Methyl 2-(2-tert-butyl-7-(2,4-difluorophenyl)-5-methylpyrazolo[1,5-a]pyrimidin-6-yl)pentanoate;
Methyl 2-(2-tert-butyl-7-(2-chloro-4-methylphenyl)-5-methylpyrazolo[1,5-a]pyrimidin-6-yl)pentanoate;
Methyl 2-(7-(2-amino-4-methylphenyl)-2-tert-butyl-5-methylpyrazolo[1,5-a]pyrimidin-6-yl)pentanoate;
Methyl 2-(2-tert-butyl-7-(2-methoxy-4-chlorophenyl)-5-methylpyrazolo[1,5-a]pyrimidin-6-yl)pentanoate;
Methyl 2-(2-tert-butyl-7-(2-fluoro-4-methoxyphenyl)-5-methylpyrazolo[1,5-a]pyrimidin-6-yl)pentanoate;
Methyl 2-(2-tert-butyl-5-methyl-7-(5-quinoline)pyrazolo[1,5-a]pyrimidin-6-yl)pentanoate;
Methyl 2-(2-tert-butyl-5-methyl-7-(8-quinoline)pyrazolo[1,5-a]pyrimidin-6-yl)pentanoate;
Methyl 2-(2-tert-butyl-7-(2,4-dimethylphenyl)-5-methylpyrazolo[1,5-a]pyrimidin-6-yl)pentanoate;
Methyl 2-(2-tert-butyl-3,5-dimethyl-7-p-tolylpyrazolo[1,5-a]pyrimidin-6-yl)pentanoate;
Methyl 2-(2-tert-butyl-5-methyl-3-phenyl-7-p-tolylpyrazolo[1,5-a]pyrimidin-6-yl)pentanoate;
Methyl 2-(2,3,5-trimethyl-7-p-tolyl-3H-imidazo[4,5-b]pyridin-6-yl)acetate;
Methyl 2-(2,3,5-trimethyl-7-p-tolyl-3H-imidazo[4,5-b]pyridin-6-yl)pentanoate;
Ethyl 2-(1,2,5-trimethyl-7-p-tolyl-1H-imidazo[4,5-b]pyridin-6-yl)pentanoate;
Ethyl 2-(3,5-dimethyl-2-propyl-7-p-tolyl-3H-imidazo[4,5-b]pyridin-6-yl)pentanoate;
Ethyl 2-(3,5-dimethyl-2-isopropyl-7-p-tolyl-3H-imidazo[4,5-b]pyridin-6-yl)pentanoate;
2-((7-((R)-3-(4-chlorophenylsulfonamido)piperidin-1-yl)-5-methylpyrazolo[1,5-a]pyrimidin-6-yl)pentanoic acid;
2-((7-((S)-3-(4-chlorophenylsulfonamido)piperidin-1-yl)-5-methylpyrazolo[1,5-a]pyrimidin-6-yl)pentanoic acid;
2-((7-((R)-3-(4-chlorophenylsulfonamido)pyrrolidin-1-yl)-5-methylpyrazolo[1,5-a]pyrimidin-6-yl)pentanoic acid;

2-((7-((S)-3-(4-chlorophenylsulfonamido)pyrrolidin-1-yl)-5-methylpyrazolo[1,5-a]pyrimidin-6-yl)pentanoic acid;
2-((5-methyl-7-phenylpyrazolo[1,5-a]pyrimidin-6-yl)pentanoic acid;
2-((5-methyl-7-p-tolylpyrazolo[1,5-a]pyrimidin-6-yl)pentanoic acid;
2-((2-tert-butyl-5-methyl-7-phenylpyrazolo[1,5-a]pyrimidin-6-yl)pentanoic acid;
2-((2-tert-butyl-5-methyl-7-p-tolylpyrazolo[1,5-a]pyrimidin-6-yl)pentanoic acid;
2-((3-bromo-2-tert-butyl-5-methyl-7-p-tolylpyrazolo[1,5-a]pyrimidin-6-yl)pentanoic acid;
2-((5-methyl-2-phenyl-7-p-tolylpyrazolo[1,5-a]pyrimidin-6-yl)pentanoic acid;
2-((2-tert-butyl-7-(3-hydroxyphenyl)-5-methylpyrazolo[1,5-a]pyrimidin-6-yl)pentanoic acid;
2-((2-tert-butyl-5-methyl-7-(2-naphthyl)pyrazolo[1,5-a]pyrimidin-6-yl)pentanoic acid;
2-((2-tert-butyl-7-(1H-indol-5-yl)-5-methylpyrazolo[1,5-a]pyrimidin-6-yl)pentanoic acid;
2-((2-tert-butyl-7-(1H-indol-6-yl)-5-methylpyrazolo[1,5-a]pyrimidin-6-yl)pentanoic acid;
2-((7-(benzofuran-5-yl)-2-tert-butyl-5-methylpyrazolo[1,5-a]pyrimidin-6-yl)pentanoic acid;
2-((7-(benzo[b]thiophen-5-yl)-2-tert-butyl-5-methylpyrazolo[1,5-a]pyrimidin-6-yl)pentanoic acid;
2-((2-tert-butyl-7-(2,3-dihydrobenzofuran-5-yl)-5-methylpyrazolo[1,5-a]pyrimidin-6-yl)pentanoic acid;
2-((2-tert-butyl-7-(4-chlorophenyl)-5-methylpyrazolo[1,5-a]pyrimidin-6-yl)pentanoic acid;
2-((2-tert-butyl-7-(3,4-dimethylphenyl)-5-methylpyrazolo[1,5-a]pyrimidin-6-yl)pentanoic acid;
2-((2-tert-butyl-7-(4-ethylphenyl)-5-methylpyrazolo[1,5-a]pyrimidin-6-yl)pentanoic acid;
2-((2-tert-butyl-7-(3,4-dihydro-2H-benzo[b][1,4]dioxepin-7-yl)-5-methylpyrazolo[1,5-a]pyrimidin-6-yl)pentanoic acid;
2-((2-tert-butyl-7-(4-methyl-3,4-dihydro-2H-benzo[b][1,4]oxazin-7-yl)-5-methylpyrazolo[1,5-a]pyrimidin-6-yl)pentanoic acid;
2-((5-methyl-3-phenyl-7-p-tolylpyrazolo[1,5-a]pyrimidin-6-yl)pentanoic acid;
2-((3,7-di-p-tolyl-5-methylpyrazolo[1,5-a]pyrimidin-6-yl)pentanoic acid;
2-((2-tert-butyl-3,7-di-p-tolyl-5-methylpyrazolo[1,5-a]pyrimidin-6-yl)pentanoic acid;
2-((5-methyl-2-propyl-7-p-tolylpyrazolo[1,5-a]pyrimidin-6-yl)pentanoic acid;
2-((2-(furan-2-yl)-5-methyl-7-p-tolylpyrazolo[1,5-a]-pyrimidin-6-yl)pentanoic acid;
2-((2-tert-butyl-7-(4-chloro-2-fluorophenyl)-5-methylpyrazolo[1,5-a]pyrimidin-6-yl)pentanoic acid;
2-((2-tert-butyl-7-(2-fluoro-4-methylphenyl)-5-methylpyrazolo[1,5-a]pyrimidin-6-yl)pentanoic acid;
2-((2-tert-butyl-7-p-tolyl-5-methylpyrazolo[1,5-a]pyrimidin-6-yl)-6,6,6-trifluorohexanoic acid;
2-((2-tert-butyl-7-p-tolyl-5-methylpyrazolo[1,5-a]pyrimidin-6-yl)-3-phenylpropanoic acid;
2-((2-tert-butyl-5-methyl-7-(1-methylindolin-5-yl)pyrazolo[1,5-a]pyrimidin-6-yl)pentanoic acid;
2-((2-tert-butyl-5-methyl-7-(1-methyl-1H-indol-6-yl)pyrazolo[1,5-a]pyrimidin-6-yl)pentanoic acid;
2-((2-tert-butyl-7-(chroman-6-yl)-5-methylpyrazolo[1,5-a]pyrimidin-6-yl)pentanoic acid;
2-((2-tert-butyl-7-p-tolyl-5-methylpyrazolo[1,5-a]pyrimidin-6-yl)-3-methylpentanoic acid;
2-((2-tert-butyl-3-chloro-7-p-tolyl-5-methylpyrazolo[1,5-a]pyrimidin-6-yl)pentanoic acid;
2-((2-tert-butyl-7-p-tolyl-5-methylpyrazolo[1,5-a]pyrimidin-6-yl)-4-methoxybutanoic acid;
2-((2-tert-butyl-5-methyl-7-(4-iso-propylphenyl)pyrazolo[1,5-a]pyrimidin-6-yl)pentanoic acid;
2-((2-tert-butyl-5-methyl-7-(4-trifluoromethylphenyl)pyrazolo[1,5-a]pyrimidin-6-yl)pentanoic acid;
2-((2-tert-butyl-7-(2,4-difluorophenyl)-5-methylpyrazolo[1,5-a]pyrimidin-6-yl)pentanoic acid;
2-((2-tert-butyl-7-(2-chloro-4-methylphenyl)-5-methylpyrazolo[1,5-a]pyrimidin-6-yl)pentanoic acid;
2-((7-(2-amino-4-methylphenyl)-2-tert-butyl-5-methylpyrazolo[1,5-a]pyrimidin-6-yl)pentanoic acid;
2-((2-tert-butyl-7-(2-methoxy-4-chlorophenyl)-5-methylpyrazolo[1,5-a]pyrimidin-6-yl)pentanoic acid;
2-((2-tert-butyl-7-(2-fluoro-4-methoxyphenyl)-5-methylpyrazolo[1,5-a]pyrimidin-6-yl)pentanoic acid;
2-((2-tert-butyl-5-methyl-7-(5-quinoline)pyrazolo[1,5-a]pyrimidin-6-yl)pentanoic acid;
2-((2-tert-butyl-5-methyl-7-(8-quinoline)pyrazolo[1,5-a]pyrimidin-6-yl)pentanoic acid;
2-((2-tert-butyl-7-(2,4-dimethylphenyl)-5-methylpyrazolo[1,5-a]pyrimidin-6-yl)pentanoic acid;
2-((2-tert-butyl-3,5-dimethyl-7-p-tolylpyrazolo[1,5-a]pyrimidin-6-yl)pentanoic acid;
2-((2-tert-butyl-5-methyl-3-phenyl-7-p-tolylpyrazolo[1,5-a]pyrimidin-6-yl)pentanoic acid;
2-((2,3,5-Trimethyl-7-p-tolyl-3H-imidazo[4,5-b]pyridin-6-yl)pentanoic acid;
2-((1,2,5-trimethyl-7-p-tolyl-1H-imidazo[4,5-b]pyridin-6-yl)pentanoic acid;
2-((2-propyl-3,5-dimethyl-7-p-tolyl-3H-imidazo[4,5-b]pyridin-6-yl)pentanoic acid;
2-((2-isopropyl-3,5-dimethyl-7-p-tolyl-3H-imidazo[4,5-b]pyridin-6-yl)pentanoate;
Methyl 2-(2-isopropyl-5-methyl-7-*p*-tolyl-[1,2,4]-triazolo[1,5-a]pyrimidin-6-yl)acetate;
2-((2-isopropyl-5-methyl-7-*p*-tolyl-[1,2,4]-triazolo[1,5-a]pyrimidin-6-yl)acetic acid;
Methyl 2-[2-*tert*-butyl-7-(1,2-dihydroacenaphthylen-5-yl)-5-methylpyrazolo[1,5-a]pyrimidin-6-yl]acetate;
2-[2-*tert*-butyl-7-(1,2-dihydroacenaphthylen-5-yl)-5-methylpyrazolo[1,5-a]pyrimidin-6-yl]acetic acid;
Methyl 2-[2-*tert*-butyl-7-(2-methoxy-4-methylphenyl)-5-methylpyrazolo[1,5-a]pyrimidin-6-yl]acetate;
2-[2-*tert*-butyl-7-(2-methoxy-4-methylphenyl)-5-methylpyrazolo[1,5-a]pyrimidin-6-yl]acetic acid;

2-[2-*tert*-butyl-7-(2-hydroxy-4-methylphenyl)-5-methylpyrazolo[1,5-a]pyrimidin-6-yl]acetic acid;
Methyl 2-(2-isopropyl-5-methyl-7-*p*-tolyl-[1,2,4]-triazolo[1,5-*a*]pyrimidin-6-yl)pentanoate;
2-((2-isopropyl-5-methyl-7-*p*-tolyl-[1,2,4]-triazolo[1,5-a]pyrimidin-6-yl)pentanoic acid;
2-((2-benzyl-5-methyl-7-*p*-tolyl-[1,2,4]-triazolo[1,5-*a*]pyrimidin-6-yl)pentanoic acid; and
Ethyl 2-(2-benzyl-5-methyl-7-p-tolyl-[1,2,4]-triazolo]1,5-*a*]pyrimidin-6-yl)pentanoate;

and the pharmaceutically acceptable salts thereof.

According to a second aspect, the invention relates to the compounds as described herein (more in particular of the formulae (A), (B), (C), (D), and (E), embodiments thereof and claims herein) for use as a medicament or a medicine, more in particular for use as an antiviral medicament and for the use in the prevention or treatment of a viral infection in a subject (animal, mammal or human).

The present invention also relates to the use of compounds of the formulate (A), (B), (C), (D), and (E) embodiments thereof and claims as antiviral compounds, more particularly as compounds active against retroviruses, yet more in particular against HIV. The invention also relates to the use of the compounds of the invention for the manufacture of a medicament or as a pharmaceutically active ingredient, especially as a virus replication inhibitor, for instance for the manufacture of a medicament or pharmaceutical composition having antiviral activity for the prevention and/or treatment of viral infections in humans, mammals and animals in general. Also described is a method of prevention or treatment of a viral infection, preferably a retroviral infection in an animal, including mammals, including a human, comprising administering to the animal in need of such treatment a therapeutically effective amount of a compound of the invention as an active ingredient, preferably in admixture with at least a pharmaceutically acceptable carrier.

Another aspect of the invention further relates to methods for the preparation of compounds of formulae and claims herein. Also the intermediates used in the preparation methods described herein are aspects of the present invention.

One embodiment relates to a method for the preparation of the compounds according to the invention comprising the steps of:

- Preparing a substituted or non-substituted alkyl 2-(7-hydroxypyrazolo[1,5-a]pyrimidin-6-yl)acetate derivative or a substituted or non-substituted alkyl 2-(7-hydroxy[1,2,4]triazolo[1,5-a]pyrimidin-6-yl)acetate from a substituted or non-substituted 3-amino pyrazole or 3-amino 1,2,4-triazole and a 2-substituted succinate derivative;
- Converting the 7-hydroxy group from the previous intermediate in an halogen such as Chloro, bromo or iodo;
- Optionally, reacting the compound obtained in the previous step with a compound having a structure of the formula R2a-leaving group and/or R2b-leaving group through a nucleophilic substitution;
- Substituting the 7-halogen atom from the previously obtained compound in a specific manner (amination, alkylation, arylation) with suitable chemical reagents to obtain the desired compounds;
- Hydrolyzing the ester compounds obtained in the previous step to obtain the desired free carboxylic acid derivatives.

Alternatively, the method for preparation of the compounds comprises the following steps:

- Converting a substituted or non substituted 5-amino-1H-imidazole-4-carbonitrile or 4-amino-1H-imidazole-5-carbonitrile derivative in a substituted or non substituted 1-(5-amino-1H-imidazol-4-yl)ketone or 1-(4-amino-1H-imidazol-5-yl)ketone;
- Reacting the previously obtained intermediate with a compound of formula $R^4C(O)CH_2CH_2COOR^3$ or $R^4C(O)CH_2CR^{2a}R^{2b}COOR^3$ in the presence of trimethyl chlorosilane in a polar aprotic solvent at a temperature between 50°C and 200°C;
- Optionally, reacting the compound obtained in the previous step with a compound having a structure of the formula $R^{2a}$-leaving group and/or $R^{2b}$-leaving group through a nucleophilic substitution;
- Hydrolyzing the ester compounds obtained in the previous step to obtain the desired free carboxylic acid derivatives.

Yet another aspect of the present invention relates to pharmaceutical compositions comprising the compounds of the invention according to formulae, embodiments thereof and claims herein in admixture with at least a pharmaceutically acceptable carrier, the active ingredient preferably being in a concentration range of about 0.1 to 100% by weight, and to the use of these derivatives namely as drugs useful for the treatment of subjects suffering from a viral infection, in particular a retroviral infection.

The invention further relates to the use of a composition comprising (a) one or more compounds of the invention (of formulae and claims herein), and (b) one or more viral inhibitors as biologically active agents in respective proportions such as to provide a synergistic effect against a viral infection in a subject, for instance in the form of a combined preparation for simultaneous, separate or sequential use in viral infection therapy. Within the framework of this embodiment of the invention, the viral enzyme inhibitors used as a therapeutically active ingredients (b) may belong to categories

already known in the art. In a particular embodiment, the compounds of the present invention can be combined with the following compounds:

- nucleoside reverse transcriptase (RT) inhibitors such as, but not limited to, azidothymidine (AZT), and lamivudine (3TC),
- nucleotide reverse transcriptase inhibitors such as, but not limited to, tenofovir (R-PMPA),
- non-nucleoside reverse transcriptase inhibitors such as, but not limited to, nevirapine, efavirenz, etravirine and lersivirine,
- protease inhibitors such as, but not limited to, nelfinavir, saquinavir, ritonavir, atazanavir, darunavir and amprenavir,
- fusion inhibitors such as enfuvirtide,
- CCR5 antagonists such as maraviroc, or
- integrase inhibitors such as raltegravir or elvitegravir.

More generally, the invention relates to the compounds of formulae, embodiments and claims herein being useful as agents having biological activity or as diagnostic agents. Any of the uses mentioned with respect to the present invention may be restricted to a non-medical use, a non-therapeutic use, a non-diagnostic use, or exclusively an in vitro use, or a use related to cells remote from an animal.

## DETAILED DESCRIPTION OF THE INVENTION

The present invention will be described with respect to particular embodiments but the invention is not limited thereto.

It is to be noticed that the term "comprising", used in the claims, should not be interpreted as being restricted to the means listed thereafter; it does not exclude other elements or steps.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments. Where an indefinite or definite article is used when referring to a singular noun e.g. "a" or "an", "the", this includes a plural of that noun unless something else is specifically stated.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects.

In each of the following definitions, the number of carbon atoms represents the maximum number of carbon atoms generally optimally present in the substituent or linker; it is understood that where otherwise indicated in the present application, the number of carbon atoms represents the optimal maximum number of carbon atoms for that particular substituent or linker.

The term "leaving group" or "LG" as used herein means a chemical group which is susceptible to be displaced by a nucleophile or cleaved off or hydrolyzed in basic or acidic conditions. In a particular embodiment, a leaving group is selected from a halogen atom (e.g., Cl, Br, I) or a sulfonate (e.g., mesylate, tosylate, triflate).

The term "protecting group" refers to a moiety of a compound that masks or alters the properties of a functional group or the properties of the compound as a whole. The chemical substructure of a protecting group varies widely. One function of a protecting group is to serve as intermediates in the synthesis of the parental drug substance. Chemical protecting groups and strategies for protection/deprotection are well known in the art. See: "Protective Groups in Organic Chemistry", Theodora W. Greene (John Wiley & Sons, Inc., New York, 1991. Protecting groups are often utilized to mask the reactivity of certain functional groups, to assist in the efficiency of desired chemical reactions, e.g. making and breaking chemical bonds in an ordered and planned fashion. Protection of functional groups of a compound alters other physical properties besides the reactivity of the protected functional group, such as the polarity, lipophilicity (hydrophobicity), and other properties which can be measured by common analytical tools. Chemically protected intermediates may themselves be biologically active or inactive. Protected compounds may also exhibit altered, and in some cases, optimized properties in vitro and in vivo, such as passage through cellular membranes and resistance to enzymatic degradation or sequestration. In this role, protected compounds with intended therapeutic effects may be referred to as prodrugs. Another function of a protecting group is to convert the parental drug into a prodrug, whereby the parental drug is released upon conversion of the prodrug in vivo. Because active prodrugs may be absorbed more effectively than the parental drug, prodrugs may possess greater potency in vivo than the parental drug. Protecting groups are removed either in vitro, in the instance of chemical intermediates, or in vivo, in the case of prodrugs. With chemical

intermediates, it is not particularly important that the resulting products after deprotection, e.g. alcohols, be physiologically acceptable, although in general it is more desirable if the products are pharmacologically innocuous.

The term "hydrocarbyl", "$C_{1-18}$ hydrocarbyl", "hydrocarbyl group" or "$C_{1-18}$ hydrocarbyl group" as used herein refers to $C_1$-$C_{18}$ normal, secondary, tertiary, unsaturated or saturated, non-aromatic, acyclic or cyclic, hydrocarbons and combinations thereof. This term therefore comprises alkyl, alkenyl, alkynyl, cycloalkyl, cycloalkenyl and cycloalkynyl.

The terminology "heterohydrocarbyl", "hetero $C_{1-18}$ hydrocarbyl", "heterohydrocarbyl group", "hetero $C_{1-18}$ hydrocarbyl group" or "hydrocarbyl group which optionally includes one or more heteroatoms, said heteroatoms being selected from the atoms consisting of O, S, and N" as used herein, refers to a hyrdocarbyl group where one or more carbon atoms are replaced by an oxygen, nitrogen or sulphur atom(s) and thus includes heteroalkyl, heteroalkenyl, heteroalkynyl and non-aromatic heterocycle. This term therefore comprises as an example alkoxy, alkenyloxy, $C_w$alkyl-O-$C_{18-w}$alkyl, $C_w$ alkenyl-O-alkyl, $C_w$alkyl-NH-$C_{18-w}$alkenyl, among others, wherein w is selected from any number between 1 and 18.

The term "alkyl" or "$C_{1-18}$ alkyl" as used herein means $C_1$-$C_{18}$ normal, secondary, or tertiary, linear or cyclic, branched or straight hydrocarbon with no site of unsaturation. Examples are methyl, ethyl, 1-propyl (n-propyl), 2-propyl (iPr), 1-butyl, 2-methyl-1-propyl(i-Bu), 2-butyl (s-Bu), 2-dimethyl-2-propyl (t-Bu), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, cyclopropylethylene, methylcyclopropylene, 2,3-dimethyl-2-butyl, 3,3-dimethyl-2-butyl, *n*-heptyl, *n*-octyl, *n*-nonyl, *n*-decyl, *n*-undecyl, *n*-dodecyl, *n*-tridecyl, *n*-tetradecyl, *n*-pentadecyl, *n*-hexadecyl, *n*-heptadecyl, *n*-octadecyl, *n*-nonadecyl, *n*-icosyl, cyclopropyl, cyclobutyl, cyclopentyl and cyclohexyl. In a particular embodiment, the term alkyl refers to $C_{1-12}$ hydrocarbons, yet more in particular to $C_{1-6}$ hydrocarbons as further defined herein above.

The term "acyclic alkyl" as used herein means $C_1$-$C_{18}$ normal, secondary, or tertiary, linear, branched or straight, hydrocarbon with no site of unsaturation. Examples are methyl, ethyl, 1-propyl, 2-propyl (iPr), 1-butyl, 2-methyl-1-propyl(i-Bu), 2-butyl (s-Bu), 2-methyl-2-propyl (t-Bu), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl *n*-heptyl, *n*-octyl, *n*-nonyl, *n*-decyl, *n*-undecyl, *n*-dodecyl, *n*-tridecyl, *n*-tetradecyl, *n*-pentadecyl, *n*-hexadecyl, *n*-heptadecyl, *n*-octadecyl, *n*-nonadecyl and *n*-icosyl.

The term "cycloalkyl" or "$C_{3-18}$ cycloalkyl" as used herein and unless otherwise stated means a saturated hydrocarbon monovalent radical having from 3 to 18 carbon atoms consisting of or comprising a $C_{3-10}$ monocyclic or $C_{7-18}$ polycyclic saturated hydrocarbon, such as for instance cyclopropyl, cyclobutyl, cyclopentyl, cyclopropylethylene, methylcyclopropylene, cyclohexyl, cycloheptyl, cyclooctyl, cyclooctylmethylene, norbornyl, fenchyl, trimethyltricycloheptyl, decalinyl, adamantyl and the like.

The term "alkenyl" or "$C_{2-18}$alkenyl" as used herein is $C_2$-$C_{18}$ normal, secondary or tertiary, linear or cyclic, branched or straight hydrocarbon with at least one site (usually 1 to 3, preferably 1) of unsaturation, namely a carbon-carbon, sp2 double bond. Examples include, but are not limited to: ethylene or vinyl ($-CH=CH_2$), allyl ($-CH_2CH=CH_2$), cyclopentenyl ($-C_5H_7$), cyclohexenyl ($-C_6H_9$), cyclopentenylpropylene, methylcyclohexenylene and 5-hexenyl ($-CH_2CH_2CH_2CH_2CH=CH_2$). The double bond may be in the cis or trans configuration. In a particular embodiment, the term alkenyl refers to $C_{1-12}$ hydrocarbons, yet more in particular to $C_{1-6}$ hydrocarbons as further defined herein above.

The term "acyclic alkenyl" as used herein refers to $C_2$-$C_{18}$ normal, secondary or tertiary, linear, branched or straight hydrocarbon with at least one site (usually 1 to 3, preferably 1) of unsaturation, namely a carbon-carbon, sp2 double bond. Examples include, but are not limited to: ethylene or vinyl ($-CH=CH_2$), allyl ($-CH_2CH=CH_2$) and 5-hexenyl ($-CH_2CH_2CH_2CH_2CH=CH_2$). The double bond may be in the cis or trans configuration.

The term "cycloalkenyl" as used herein refers to a non-aromatic hydrocarbon radical having from 4 to 18 carbon atoms with at least one site (usually 1 to 3, preferably 1) of unsaturation, namely a carbon-carbon, sp2 double bond and consisting of or comprising a $C_{4-10}$ monocyclic or $C_{7-18}$ polycyclic hydrocarbon. Examples include, but are not limited to: cyclopentenyl ($-C_5H_7$), cyclopentenylpropylene, methylcyclohexenylene and cyclohexenyl ($-C_6H_9$). The double bond may be in the cis or trans configuration.

The term "alkynyl" or "$C_{2-18}$alkynyl" as used herein refers to $C_2$-$C_{18}$ normal, secondary, tertiary, linear or cyclic, branched or straight hydrocarbon with at least one site (usually 1 to 3, preferably 1) of unsaturation, namely a carbon-carbon, sp triple bond. Examples include, but are not limited to: ethynyl ($-C\equiv CH$), 3-ethyl-cyclohept-1-ynylene, 4-cyclohept-1-yn-methylene and 1-propynyl (propargyl, $-CH_2C\equiv CH$). In a particular embodiment, the term alkenyl refers to $C_{1-12}$ hydrocarbons, yet more in particular to $C_{1-6}$ hydrocarbons as further defined herein above.

The term "acyclic alkynyl" as used herein refers to $C_2$-$C_{18}$ normal, secondary, tertiary, linear, branched or straight hydrocarbon with at least one site (usually 1 to 3, preferably 1) of unsaturation, namely a carbon-carbon, sp triple bond. Examples include, but are not limited to: ethynyl ($-C\equiv CH$) and 1-propynyl (propargyl, $-CH_2C\equiv CH$).

The term "cycloalkynyl" as used herein refers to a non-aromatic hydrocarbon radical having from 5 to 18 carbon atoms with at least one site (usually 1 to 3, preferably 1) of unsaturation, namely a carbon-carbon, sp triple bond and

consisting of or comprising a $C_{5-10}$ monocyclic or $C_{7-18}$ polycyclic hydrocarbon. Examples include, but are not limited to: cyclohept-1-yne, 3-ethyl-cyclohept-1-ynylene, 4-cyclohept-1-yn-methylene and ethylene-cyclohept-1-yne.

The term "alkylene" as used herein each refer to a saturated, branched or straight chain hydrocarbon radical of 1-18 carbon atoms (more in particular $C_{1-12}$ or $C_{1-6}$ carbon atoms), and having two monovalent radical centers derived by the removal of two hydrogen atoms from the same or two different carbon atoms of a parent alkane. Typical alkylene radicals include, but are not limited to: methylene ($-CH_2-$) 1,2-ethyl ($-CH_2CH_2-$), 1,3-propyl ($-CH_2CH_2CH_2-$), 1,4-butyl ($-CH_2CH_2CH_2CH_2-$), and the like.

The term "alkenylene" as used herein each refer to a branched or straight chain hydrocarbon radical of 2-18 carbon atoms (more in particular $C_{2-12}$ or $C_{2-6}$ carbon atoms) with at least one site (usually 1 to 3, preferably 1) of unsaturation, namely a carbon-carbon, sp2 double bond, and having two monovalent radical centers derived by the removal of two hydrogen atoms from the same or two different carbon atoms of a parent alkene.

The term "alkynylene" as used herein each refer to a branched or straight chain hydrocarbon radical of 2-18 carbon atoms (more in particular $C_{2-12}$ or $C_{2-6}$ carbon atoms) with at least one site (usually 1 to 3, preferably 1) of unsaturation, namely a carbon-carbon, sp triple bond, and having two monovalent radical centers derived by the removal of two hydrogen atoms from the same or two different carbon atoms of a parent alkyne.

The term "heteroalkyl" as used herein refers to an acyclic alkyl wherein one or more carbon atoms are replaced by an oxygen, nitrogen or sulphur atom.

The term "heteroalkenyl" as used herein refers to an acyclic alkenyl wherein one or more carbon atoms are replaced by an oxygen, nitrogen or sulphur atom.

The term "heteroalkynyl" as used herein refers to an acyclic alkynyl wherein one or more carbon atoms are replaced by an oxygen, nitrogen or sulphur atom.

The term "heteroalkylene" as used herein refers to an alkylene wherein one or more carbon atoms are replaced by an oxygen, nitrogen or sulphur atom.

The term "heteroalkenylene" as used herein refers to an alkenylene wherein one or more carbon atoms are replaced by an oxygen, nitrogen or sulphur atom.

The term "heteroalkynylene" as used herein refers to an alkynylene wherein one or more carbon atoms are replaced by an oxygen, nitrogen or sulphur atom.

The term "aryl" as used herein means an aromatic hydrocarbon radical of 6-20 carbon atoms derived by the removal of hydrogen from a carbon atom of a parent aromatic ring system. A "parent aromatic ring system" means a monocyclic aromatic ring system or a bi- or tricyclic ring system of which at least one ring is aromatic. Typical aryl groups include, but are not limited to 1 ring, or 2 or 3 rings fused together and includes radicals derived from benzene, naphthalene, anthracene, biphenyl, 2,3-dihydro-1H-indene, and the like. Phenyl is a particular example of an aryl group.

The term "arylalkyl" or "arylalkyl-" as used herein refers to an acyclic alkyl radical in which one of the hydrogen atoms bonded to a carbon atom, typically a terminal or sp3 carbon atom, is replaced with an aryl radical. Typical arylalkyl groups include, but are not limited to, benzyl, 2-phenylethan-1-yl, 2-phenylethen-1-yl, naphthylmethyl, 2-naphthylethyl, and the like. The arylalkyl group comprises 6 to 20 carbon atoms, e.g. the alkyl moiety of the arylalkyl group is 1 to 6 carbon atoms and the aryl moiety is 6 to 14 carbon atoms.

The term "arylalkenyl" or "arylalkenyl-" as used herein refers to an acyclic alkenyl radical in which one of the hydrogen atoms bonded to a carbon atom, is replaced with an aryl radical. The arylalkenyl group comprises 6 to 20 carbon atoms, e.g. the alkenyl moiety of the arylalkenyl group is 1 to 6 carbon atoms and the aryl moiety is 6 to 14 carbon atoms.

The term "arylalkynyl" or "arylalkynyl-" as used herein refers to an acyclic alkynyl radical in which one of the hydrogen atoms bonded to a carbon atom, is replaced with an aryl radical. The arylalkynyl group comprises 6 to 20 carbon atoms, e.g. the alkynyl moiety of the arylalkynyl group is 1 to 6 carbon atoms and the aryl moiety is 6 to 14 carbon atoms.

The term "arylheteroalkyl" or "arylheteroalkyl-" as used herein refers to a heteroalkyl radical in which one of the hydrogen atoms bonded to a carbon atom, typically a terminal or sp3 carbon atom, is replaced with an aryl radical. The arylheteroalkyl group comprises 6 to 20 carbon atoms, e.g. the heteroalkyl moiety of the arylheteroalkyl group is 1 to 6 carbon atoms and the aryl moiety is 6 to 14 carbon atoms.

The term "arylheteroalkenyl" or "arylheteroalkenyl-" as used herein refers to a heteroalkenyl radical in which one of the hydrogen atoms bonded to a carbon atom, is replaced with an aryl radical. The arylheteroalkenyl group comprises 6 to 20 carbon atoms, e.g. the heteroalkenyl moiety of the arylheteroalkenyl group is 1 to 6 carbon atoms and the aryl moiety is 6 to 14 carbon atoms.

The term "arylheteroalkynyl" or "arylheteroalkynyl-" as used herein refers to a heteroalkynyl radical in which one of the hydrogen atoms bonded to a carbon atom, is replaced with an aryl radical. The arylheteroalkynyl group comprises 6 to 20 carbon atoms, e.g. the heteroalkynyl moiety of the arylheteroalkynyl group is 1 to 6 carbon atoms and the aryl moiety is 6 to 14 carbon atoms.

The term "heterocycle" as used herein means a saturated, unsaturated or aromatic ring system of 3 to 18 atoms

including at least one N, O, S, or P. Heterocycle thus include heteroaryl groups. Heterocycle as used herein includes by way of example and not limitation these heterocycles described in Paquette, Leo A. "Principles of Modem Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; Katritzky, Alan R., Rees, C.W. and Scriven, E. "Comprehensive Heterocyclic Chemistry" (Pergamon Press, 1996); and J. Am. Chem. Soc. (1960) 82:5566. In a particular embodiment, the term means pyridyl, dihydroypyridyl, tetrahydropyridyl (piperidyl), thiazolyl, tetrahydrothiophenyl, sulfur oxidized tetrahydrothiophenyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, bis-tetrahydrofuranyl, tetrahydropyranyl, bis-tetrahydropyranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1 H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, ß-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, benzothienyl, benzothiazolyl and isatinoyl.

The term "heteroaryl" means an aromatic ring system of 5 to 18 atoms including at least one N, O, S, or P and thus refers to aromatic heterocycles. Examples of heteroaryl include but are not limited to pyridyl, dihydropyridyl, pyridazinyl, pyrimidinyl, pyrazinyl, s-triazinyl, oxazolyl, imidazolyl, thiazolyl, isoxazolyl, pyrazolyl, isothiazolyl, furyl, thienyl, and pyrrolyl.

The term "non-aromatic heterocycle" as used herein means a saturated or unsaturated non-aromatic ring system of 3 to 18 atoms including at least one N, O, S, or P.

The term "heterocycle-alkyl" or "heterocycle-alkyl-" as used herein refers to an acyclic alkyl radical in which one of the hydrogen atoms bonded to a carbon atom, typically a terminal or sp3 carbon atom, is replaced with a heterocyle radical. An example of a heterocycle-alkyl group is 2-pyridyl-methylene. The heterocycle-alkyl group comprises 6 to 20 atoms, e.g. the alkyl moiety of the heterocycle-alkyl group is 1 to 6 carbon atoms and the heterocycle moiety is 3 to 14 atoms.

The term "heterocycle-alkenyl" or "heterocycle-alkenyl-" as used herein refers to an acyclic alkenyl radical in which one of the hydrogen atoms bonded to a carbon atom, is replaced with an heterocycle radical. The heterocycle-alkenyl group comprises 6 to 20 atoms, e.g. the alkenyl moiety of the heterocycle-alkenyl group is 1 to 6 carbon atoms and the heterocycle moiety is 3 to 14 atoms.

The term "heterocycle-alkynyl" or "heterocycle-alkynyl-" as used herein refers to an acyclic alkynyl radical in which one of the hydrogen atoms bonded to a carbon atom, is replaced with a heterocycle radical. The heterocycle-alkynyl group comprises 6 to 20 atoms, e.g. the alkynyl moiety of the heterocycle-alkynyl group is 1 to 6 carbon atoms and the heterocycle moiety is 3 to 14 atoms.

The term "heterocycle-heteroalkyl" or "heterocycle-heteroalkyl-" as used herein refers to a heteroalkyl radical in which one of the hydrogen atoms bonded to a carbon atom, typically a terminal or sp3 carbon atom, is replaced with a heterocyle radical. The heterocycle-heteroalkyl group comprises 6 to 20 atoms, e.g. the heteroalkyl moiety of the heterocycle-heteroalkyl group is 1 to 6 carbon atoms and the heterocycle moiety is 3 to 14 atoms.

The term "heterocycle-heteroalkenyl" or "heterocycle-heteroalkenyl-" as used herein refers to a heteroalkenyl radical in which one of the hydrogen atoms bonded to a carbon atom, is replaced with an heterocycle radical. The heterocycle-heteroalkenyl group comprises 6 to 20 atoms, e.g. the heteroalkenyl moiety of the heterocycle-heteroalkenyl group is 1 to 6 carbon atoms and the heterocycle moiety is 3 to 14 atoms.

The term "heterocycle-heteroalkynyl" or "heterocycle-heteroalkynyl-" as used herein refers to a heteroalkynyl radical in which one of the hydrogen atoms bonded to a carbon atom, is replaced with a heterocycle radical. The heterocycle-heteroalkynyl group comprises 6 to 20 atoms, e.g. the heteroalkynyl moiety of the heterocycle-heteroalkynyl group is 1 to 6 carbon atoms and the heterocycle moiety is 3 to 14 atoms.

The term "heteroaryl-alkyl" or "heteroaryl-alkyl-" as used herein refers to an acyclic alkyl radical in which one of the hydrogen atoms bonded to a carbon atom, typically a terminal or sp3 carbon atom, is replaced with a heteraryl radical. An example of a heteroaryl-alkyl group is 2-pyridyl-methylene. The heteroaryl-alkyl group comprises 6 to 20 atoms, e.g. the alkyl moiety of the heteroaryl-alkyl group is 1 to 6 carbon atoms and the heteroaryl moiety is 5 to 14 atoms.

The term "heteroaryl-alkenyl" or "heteroaryl-alkenyl-" as used herein refers to an acyclic alkenyl radical in which one of the hydrogen atoms bonded to a carbon atom, is replaced with an heteroaryl radical. The heteroaryl-alkenyl group comprises 6 to 20 atoms, e.g. the alkenyl moiety of the heteroaryl-alkenyl group is 1 to 6 carbon atoms and the heteroaryl moiety is 5 to 14 atoms.

The term "heteroaryl-alkynyl" or "heteroaryl-alkynyl-" as used herein refers to an acyclic alkynyl radical in which one of the hydrogen atoms bonded to a carbon atom, is replaced with a heteroaryl radical. The heteroaryl-alkynyl group

comprises 6 to 20 atoms, e.g. the alkynyl moiety of the heteroaryl-alkynyl group is 1 to 6 carbon atoms and the heteroaryl moiety is 5 to 14 atoms.

The term "heteroaryl-heteroalkyl" or "heteroaryl-heteroalkyl-" as used herein refers to a heteroalkyl radical in which one of the hydrogen atoms bonded to a carbon atom, typically a terminal or sp3 carbon atom, is replaced with a heterocyle radical. The heteroaryl-heteroalkyl group comprises 6 to 20 atoms, e.g. the heteroalkyl moiety of the heteroaryl-heteroalkyl group is 1 to 6 carbon atoms and the heteroaryl moiety is 5 to 14 atoms.

The term "heteroaryl-heteroalkenyl" or "heteroaryl-heteroalkenyl-" as used herein refers to a heteroalkenyl radical in which one of the hydrogen atoms bonded to a carbon atom, is replaced with an heteroaryl radical. The heteroaryl-heteroalkenyl group comprises 6 to 20 atoms, e.g. the heteroalkenyl moiety of the heteroaryl-heteroalkenyl group is 1 to 6 carbon atoms and the heteroaryl moiety is 5 to 14 atoms.

The term "heteroaryl-heteroalkynyl" or "heteroaryl-heteroalkynyl-" as used herein refers to a heteroalkynyl radical in which one of the hydrogen atoms bonded to a carbon atom, is replaced with a heteroaryl radical. The heteroaryl-heteroalkynyl group comprises 6 to 20 atoms, e.g. the heteroalkynyl moiety of the heteroaryl-heteroalkynyl group is 1 to 6 carbon atoms and the heteroaryl moiety is 5 to 14 atoms.

The term "non-aromatic heterocycle-alkyl" or "non-aromatic heterocycle-alkyl-" as used herein refers to an acyclic alkyl radical in which one of the hydrogen atoms bonded to a carbon atom, typically a terminal or sp3 carbon atom, is replaced with a non-aromatic heterocycle radical. The non-aromatic heterocycle-alkyl group comprises 6 to 20 atoms, e.g. the alkyl moiety of the non-aromatic heterocycle-alkyl group is 1 to 6 carbon atoms and the non-aromatic heterocycle moiety is 3 to 14 atoms.

The term "non-aromatic heterocycle-alkenyl" or "non-aromatic heterocycle-alkenyl-" as used herein refers to an acyclic alkenyl radical in which one of the hydrogen atoms bonded to a carbon atom, is replaced with an non-aromatic heterocycle radical. The non-aromatic heterocycle-alkenyl group comprises 6 to 20 atoms, e.g. the alkenyl moiety of the non-aromatic heterocycle-alkenyl group is 1 to 6 carbon atoms and the non-aromatic heterocycle moiety is 3 to 14 atoms.

The term "non-aromatic heterocycle-alkynyl" or "non-aromatic heterocycle-alkynyl-" as used herein refers to an acyclic alkynyl radical in which one of the hydrogen atoms bonded to a carbon atom, is replaced with a non-aromatic heterocycle radical. The non-aromatic heterocycle-alkynyl group comprises 6 to 20 atoms, e.g. the alkynyl moiety of the non-aromatic heterocycle-alkynyl group is 1 to 6 carbon atoms and the non-aromatic heterocycle moiety is 3 to 14 atoms.

The term "non-aromatic heterocycle-heteroalkyl" or "non-aromatic heterocycle-heteroalkyl-" as used herein refers to a heteroalkyl radical in which one of the hydrogen atoms bonded to a carbon atom, typically a terminal or sp3 carbon atom, is replaced with a heterocyle radical. The non-aromatic heterocycle-heteroalkyl group comprises 6 to 20 atoms, e.g. the heteroalkyl moiety of the non-aromatic heterocycle-heteroalkyl group is 1 to 6 carbon atoms and the non-aromatic heterocycle moiety is 3 to 14 atoms.

The term "non-aromatic heterocycle-heteroalkenyl" or "non-aromatic heterocycle-heteroalkenyl-" as used herein refers to a heteroalkenyl radical in which one of the hydrogen atoms bonded to a carbon atom, is replaced with an non-aromatic heterocycle radical. The non-aromatic heterocycle-heteroalkenyl group comprises 6 to 20 atoms, e.g. the heteroalkenyl moiety of the non-aromatic heterocycle-heteroalkenyl group is 1 to 6 carbon atoms and the non-aromatic heterocycle moiety is 3 to 14 atoms.

The term "non-aromatic heterocycle-heteroalkynyl" or "non-aromatic heterocycle-heteroalkynyl-" as used herein refers to a heteroalkynyl radical in which one of the hydrogen atoms bonded to a carbon atom, is replaced with a non-aromatic heterocycle radical. The non-aromatic heterocycle-heteroalkynyl group comprises 6 to 20 atoms, e.g. the heteroalkynyl moiety of the non-aromatic heterocycle-heteroalkynyl group is 1 to 6 carbon atoms and the non-aromatic heterocycle moiety is 3 to 14 atoms.

By way of example, carbon bonded heterocyclic rings are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl. By way of example, nitrogen bonded heterocyclic rings are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or ß-carboline. Still more typically, nitrogen bonded heterocycles include 1-aziridyl, 1-azetedyl, 1-pyrrolyl, 1-imidazolyl, 1-pyrazolyl, and 1-piperidinyl.

As used herein and unless otherwise stated, the terms "alkoxy", "cyclo-alkoxy", "aryloxy", "arylalkyloxy", "heterocycleoxy", "alkylthio", "cycloalkylthio", "arylthio", "arylalkylthio" and "heterocyclethio" refer to substituents wherein an alkyl group, respectively a cycloalkyl, aryl, arylalkyl or heterocycle (each of them such as defined herein), are attached

to an oxygen atom or a sulfur atom through a single bond, such as but not limited to methoxy, ethoxy, propoxy, butoxy, thioethyl, thiomethyl, phenyloxy, benzyloxy, mercaptobenzyl and the like. The same definitions will apply for alkenyl and alkynyl radicals in stead of alkyl.

As used herein and unless otherwise stated, the term "halogen" means any atom selected from the group consisting of fluorine (F), chlorine (Cl), bromine (Br) and iodine (I).

The terminology regarding a chemical group "which optionally includes one or more heteroatoms, said heteroatoms being selected from the atoms consisting of O, S, and N" as used herein, refers to a group where one or more carbon atoms are replaced by an oxygen, nitrogen or sulphur atom and thus includes, depending on the group to which is referred, heteroalkyl, heteroalkenyl, heteroalkynyl, heteroalkyl, heteroalkenyl, heteroalkynyl, cycloheteroalkyl, cycloheteroalkenyl, cycloheteroalkynyl, heteroaryl, arylheteroalkyl, heteroarylalkyl, heteroarylheteroalkyl, arylheteroalkenyl, heteroarylalkenyl, heteroarylheteroalkenyl, heteroarylheteroalkenyl, arylheteroalkynyl, heteroarylalkynyl, heteroarylheteroalkynyl, among others. This term therefore comprises, depending on the group to which is referred, as an example alkoxy, alkenyloxy, alkynyloxy, alkyl-O-alkylene, alkenyl-O-alkylene, arylalkoxy, benzyloxy, heterocycle-heteroalkyl, heterocycle-alkoxy, among others. As an example, the terminology "alkyl which optionally includes one or more heteroatoms, said heteroatoms being selected from the atoms consisting of O, S, and N" therefore refers to heteroalkyl, meaning an alkyl which comprises one or more heteroatoms in the hydrocarbon chain, whereas the heteroatoms may be positioned at the beginning of the hydrocarbon chain, in the hydrocarbon chain or at the end of the hydrocarbon chain. Examples of heteroalkyl include methoxy, methylthio, ethoxy, propoxy, $CH_3$-O-$CH_2$, $CH_3$-S-$CH_2$-, $CH_3$-$CH_2$-O-$CH_2$-, $CH_3$-NH-, $(CH_3)_2$-N-, $(CH_3)_2$-$CH_2$NH-$CH_2CH_2$-, among many other examples. As an example, the terminology "arylalkylene which optionally includes one or more heteroatoms in the alkylene chain, said heteroatoms being selected from the atoms consisting of O, S, and N" therefore refers to arylheteroalkylene, meaning an arylalkylene which comprises one or more heteroatoms in the hydrocarbon chain, whereas the heteroatoms may be positioned at the beginning of the hydrocarbon chain, in the hydrocarbon chain or at the end of the hydrocarbon chain. "Arylheteroalkylene" thus includes aryloxy, arylalkoxy, aryl-alkyl-NH- and the like and examples are phenyloxy, benzyloxy, aryl-$CH_2$S-$CH_2$, aryl-$CH_2$-O-$CH_2$-, aryl-NH-$CH_2$- among many other examples. The same counts for "heteroalkenylene", "heteroalkynylene", and other terms used herein when referred to "which optionally includes one or more heteroatoms, said heteroatoms being selected from the atoms consisting of O, S, and N".

The terminology regarding a chemical group "wherein optionally two or more hydrogen atoms on a carbon atom or heteroatom of said group can be taken together to form a =O or =S" as used herein, refers to a group where two or more hydrogen atoms on a carbon atom or heteroatom of said group are taken together to form =O or =S. As an example, the terminology refers to "an alkyl wherein optionally two or more hydrogen atoms on a carbon atom or heteroatom of said alkyl can be taken together to form a =O or =S", includes among other examples $CH_3$-C(O)-$CH_2$-, $CH_3$-C(O)-, $CH_3$-C(S)-$CH_2$-, $CH_3$-$S(O)_2$-$CH_2$- and $(CH_3)_2$-$CH_2$-C(O)-$CH_2$-$CH_2$-.

The combination for a group "which optionally includes one or more heteroatoms, said heteroatoms being selected from the atoms consisting of O, S, and N" and "wherein optionally two or more hydrogen atoms on a carbon atom or heteroatom of said group can be taken together to form a =O or =S" can combine the two aspects described herein above and includes, if the group referred to is alkyl, among other examples $CH_3$-C(O)O-, $CH_3$-C(O)O-$CH_2$-, $CH_3$-NH-C(O)-, $CH_3$-C(O)-NH- $CH_3$-NH-C(O)$CH_2$-, $CH_3$-NH-C(S)-$CH_2$-, $CH_3$-NH-C(S)-NH-$CH_2$-, $CH_3$-NH-$S(O)_2$- and $CH_3$-NH-$S(O)_2$NH-$CH_2$-.

As used herein with respect to a substituting group, and unless otherwise stated, the terms "substituted" such as in "substituted alkyl", "substituted alkenyl", substituted alkynyl", "substituted aryl", "substituted heterocycle", "substituted arylalkyl", "substituted heterocycle-alkyl" and the like refer to the chemical structures defined herein, and wherein the said hydrocarbyl, heterohydrocarbyl group and/or the said aryl or heterocycle may be optionally substituted with one or more substituents (preferable 1, 2, 3, 4, 5 or 6), meaning that one or more hydrogen atoms are each independently replaced with a substituent. Typical substituents include, but are not limited to and in a particular embodiment said substituents are being independently selected from the group consisting of halogen, amino, hydroxyl, sulfhydryl, alkyl, alkoxy, alkenyl, alkenyloxy, alkynyl, alkynyloxy,cycloalkyl, cycloalkenyl, cycloalkynyl, heteroalkyl, heteroalkenyl, heteroalkynyl, aryl, heterocycle, arylalkyl, arylalkenyl, arylalkynyl, heterocycle-alkyl, heterocycle-alkenyl and heterocycle-alkynyl, -X, -Z, -O-, -OZ, =O, -SZ, $-S^-$, =S, $-NZ_2$, $-N^+Z_3$, =NZ, =N-OZ, $-CX_3$ (e.g. trifluoromethyl), -CN, -OCN, -SCN, - N=C=O, -N=C=S, -NO, $-NO_2$, $=N_2$, $-N_3$, -NZC(O)Z, -NZC(S)Z, $-NZC(O)O^-$,-NZC(O)OZ, -NZC(S)OZ, -NZC(O)NZZ, NZC(NZ)Z, NZC(NZ)NZZ, -C(O)NZZ,-C(NZ)Z, $-S(O)_2O$ , $-S(O)_2OZ$, $-S(O)_2Z$, $-OS(O)_2OZ$, $-OS(O)_2Z$, $-OS(O)_2O^-$,$-S(O)_2NZ$, -S(O)Z, $-OP(O)(OZ)_2$, $-P(O)(OZ)_2$, $-P(O)(O^-)_2$, $-P(O)(OZ)(O^-)$,$-P(O)(OH)_2$, -C(O)Z, -C(O)X, -C(S)Z, -C(O)OZ, $-C(O)O^-$, -C(S)OZ, -C(O)SZ,-C(S)SZ, -C(O)NZZ, -C(S)NZZ, -C(NZ)NZZ, -OC(O)Z, -OC(S)Z, $-OC(O)O^-$,-OC(O)OZ, -OC(S)OZ, wherein each X is independently a halogen selected from F, Cl, Br, or I; and each Z is independently -H, alkyl, alkenyl, alkynyl, heteroalkyl, heteroalkenyl, heteroalkynyl, aryl, heterocycle, protecting group or prodrug moiety, while two Z bonded to a nitrogen atom can be taken together with the nitrogen atom to which they are bondend to form a heterocycle. Alkyl(ene), alkenyl(ene), and alkynyl(ene) groups may also be similarly substituted.

Any substituent designation that is found in more than one site in a compound of this invention shall be independently selected.

Substituents optionally are designated with or without bonds. Regardless of bond indications, if a substituent is polyvalent (based on its position in the structure referred to), then any and all possible orientations of the substituent are intended.

References herein to compounds of the invention are also intended to encompass solvates of such compounds. As used herein and unless otherwise stated, the term "solvate" includes any combination which may be formed by a derivative of this invention with a suitable inorganic solvent (e.g. hydrates) or organic solvent, such as but not limited to alcohols, ketones, esters, ethers, nitriles and the like.

The compounds of the invention optionally are bound covalently to an insoluble matrix and used for affinity chromatography separations, depending on the nature of the groups of the compounds, for example compounds with pendant aryl are useful in hydrophobic affinity separations.

The compounds of the invention are employed for use in the treatment or prophylaxis of viral infections, more particularly retroviral infections, in particular HIV infections. When using one or more compounds of the invention and of the formulae as defined herein:

- the compound(s) may be administered to the animal or mammal (including a human) to be treated by any means well known in the art, i.e. orally, intranasally, subcutaneously, intramuscularly, intradermally, intravenously, intra-arterially, parenterally or by catheterization.
- the therapeutically effective amount of the preparation of the compound(s), especially for the treatment of viral infections in humans and other mammals, preferably is a retroviral replication inhibiting amount of the formulae as defined herein and corresponds to an amount which ensures a plasma level of between 1 μg/ml and 100 mg/ml, optionally of 10 mg/ml.

Also described is a method for preventing or treating a viral infections in a subject or patient by administering to the patient in need thereof a therapeutically effective amount of the compounds of the present invention. The therapeutically effective amount of the compound(s), especially for the treatment of viral infections in humans and other mammals, preferably is a retroviral replication inhibiting amount. The suitable dosage is usually in the range of 0.001 mg to 60 mg, optionally 0.01 mg to 10 mg, optionally 0.1 mg to 1 mg per day per kg bodyweight for humans. Depending upon the pathologic condition to be treated and the patient's condition, the said effective amount may be divided into several sub-units per day or may be administered at more than one day intervals.

As is conventional in the art, the evaluation of a synergistic effect in a drug combination may be made by analyzing the quantification of the interactions between individual drugs, using the median effect principle described by Chou et al. in Adv. Enzyme Reg. (1984) 22:27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1a. 2a), or in combination with the second or respectively first drug (1c, 2c), which is needed to produce a given effect. The said first and second drug have synergistic or additive or antagonistic effects depending upon $CI < 1$, $CI = 1$, or $CI > 1$, respectively.

Synergistic activity of the pharmaceutical compositions or combined preparations of this invention against viral infection may also be readily determined by means of one or more tests such as, but not limited to, the isobologram method, as previously described by Elion et al. in J. Biol. Chem. (1954) 208:477-488 and by Baba et al. in Antimicrob. Agents Chemother. (1984) 25:515-517, using $EC_{50}$ for calculating the fractional inhibitory concentration (hereinafter referred as FIC). When the minimum FIC index corresponding to the FIC of combined compounds (e.g., $FIC_x$ + FICy) is equal to 1.0, the combination is said to be additive; when it is between 1.0 and 0.5, the combination is defined as subsynergistic, and when it is lower than 0.5, the combination is by defined as synergistic. When the minimum FIC index is between 1.0 and 2.0, the combination is defined as subantagonistic and, when it is higher than 2.0, the combination is defined as antagonistic. This principle may be applied to a combination of different antiviral drugs of the invention or to a combination of the antiviral drugs of the invention with other drugs that exhibit anti-HIV activity. The invention thus relates to a pharmaceutical composition or combined preparation having synergistic effects against a viral infection and containing: Either:

A)

(a) a combination of two or more of the compounds of the present invention, and

(b) optionally one or more pharmaceutical excipients or pharmaceutically acceptable carriers,

for simultaneous, separate or sequential use in the treatment or prevention of a retroviridae infection or

B)

(c) one or more antiviral agents, and

(d) at least one of the compounds of the present invention, and

(e) optionally one or more pharmaceutical excipients or pharmaceutically acceptable carriers,

for simultaneous, separate or sequential use in the treatment or prevention of a retroviridae infection.

The agents that may be used in combination with the compounds of the present invention include, but are not limited to, those useful as HIV protease inhibitors, HIV reverse transcriptase inhibitors, non-nucleoside HIV reverse transcriptase inhibitors, HIV integrase inhibitors, CCR5 inhibitors, HIV fusion inhibitors or other inhibitors of HIV entry, maturation inhibitors, agents that act to perturb HIV capsid multimerisation or viral core stability, compounds targeting host proteins required for viral replication or immune evasion (such as but not limited to PSIP1), compounds useful as immunomodulators, compounds that inhibit the HIV virus by an unknown mechanism, compounds useful for the treatment of herpes viruses, compounds useful as anti-infectives, and others as described below.

Compounds useful as HIV protease inhibitors that may be used in combination with the compound of the present invention include, but are not limited to, 141 W94 (amprenavir), CGP-73547, CGP-61755, DMP-450 (mozenavir), nelfinavir, ritonavir, saquinavir (invirase), lopinavir, TMC-126, atazanavir, palinavir, GS-3333, KN I-413, KNI-272, LG-71350, CGP-61755, PD 173606, PD 177298, PD 178390, PD 178392, U-140690, ABT-378, DMP-450, AG-1776, MK-944, VX-478, indinavir, tipranavir, TMC-114 (darunavir), DPC-681, DPC-684, fosamprenavir calcium (Lexiva), benzenesulfonamide derivatives disclosed in WO 03053435, R-944, Ro-03-34649, VX-385 (brecanavir), GS-224338, OPT-TL3, PL-100, SM-309515, AG-148, DG-35-VIII, DMP-850, GW-5950X, KNI-1039, L-756423, LB-71262, LP-130, RS-344, SE-063, UIC-94-003, Vb-19038, A-77003, BMS-182193, BMS-186318, SM-309515, JE-2147, GS-9005, telinavir (SC-52151), BILA-2185 BS, DG-17, PPL-100, A-80987, GS-8374, DMP-323, U-103017, CGP-57813, and CGP-53437.

Compounds useful as inhibitors of the HIV reverse transcriptase enzyme that may be used in combination with the compound of the present invention include, but are not limited to, abacavir, emtricitabine (FTC), GS-840 (adefovir), lamivudine, adefovir dipivoxil, beta-fluoro-ddA, zalcitabine, didanosine, stavudine, zidovudine, tenofovir, tenofovir disoproxil fumarate, amdoxovir, SPD-754 (apricitabine), SPD-756, racivir, reverset (DPC-817), MIV-210 (FLG), beta-L-Fd4C (ACH-126443, elvucitabine), MIV-310 (alovudine, FLT), dOTC, DAPD, entecavir, GS-7340, stampidine, D-d4FC (dexelvucitabine), phospahzide, fozivudine tidoxil, and fosalvudine tidoxil.

Compounds useful as non-nucleoside inhibitors of the HIV reverse transcriptase enzyme that may be used in combination with the compound of the present invention include, but are not limited to, efavirenz, HBY-097, nevirapine, dapivirine (TMC-120), TMC-125, etravirine, delavirdine, DPC-083, DPC-961, TMC-120, capravirine, GW-678248, GW-695634, calanolide, rilpivirine (TMC-278), loviride, emivirine (MKC-442), DPC-963, MIV-150, BILR 355 BS, VRX-840773, lersivirine (UK-453061), RDEA806, and tricyclic pyrimidinone derivatives as disclosed in WO 03062238.

Compounds useful as CCR5 inhibitors that may be used in combination with the compound of the present invention include, but are not limited to, TAK-779, SC-351125, SCH-D, UK-427857 (maraviroc), PRO-140, and GW-873140 (aplaviroc, Ono-4128, AK-602), SCH-417690 (viciviroc, SCH-D), INCB-9471, INCB-15050, TBR-220 (TAK-220), CCR5 mAb004. Other compounds useful as CCR5 inhibitors that may be used in combination with the compound of the present invention include, but are not limited to, (N-{(1S)-3-[3-isopropyl-5-methyl-4H-1,2,4-triazole-4-yl]-exo-8-azabicyclo[3.2.1]oct-8-yl}-1-phenylpropyl)-4,4-difluorocyclohexanecarboxamide), methyl 1-*endo*-{8-[(3S)-3-(acetylamino)-3-(3-fluorophenyl)propyl]-8-azabicyclo[3.2.1]oct-3-yl}-2-methyl-4,5,6,7-tetrahydro-1*H*-imidazo[4,5-c]pyridine-5-carboxylate, and *N*-{(1*S*)-3-[3-*endo*-(5-Isobutyryl-2-methyl-4,5,6,7-tetrahydro-1*H*-imidazo[4,5-c]pyridin-1-yl)-8-azabicyclo[3.2.1]oct-8-yl]-1-(3-fluorophenyl)propyl}acetamide).

Compounds useful as inhibitors of HIV integrase enzyme that may be used in combination with the compound of the present invention include, but are not limited to, raltegravir, elvitegravir (GS-9137, JTK-303), GSK-364735, MK-2048, BMS-707035, S-1360 (GW-810781), L-870810, L-870812, AR-177, BA-011, 1,5-naphthyridine-3-carboxamide derivatives disclosed in WO 03062204, compounds disclosed in WO 03047564, compounds disclosed in WO 03049690, 5-hydroxypyrimidine-4-carboxamide derivatives disclosed in WO 03035076, and L-000810810.

Fusion inhibitors for the treatment of HIV that may be used in combination with the compound of the present invention include, but are not limited to enfuvirtide (T-20), T-1249, AMD-3100, sifuvirtide, FB-006M, TRI-1144, PRO-2000 and fused tricyclic compounds disclosed in JP 2003171381.

Maturation inhibitors for the treatment of HIV that may be used in combination with the compound of the present invention include, but are not limited to bevirimat and vivecon.

HIV fixed drug combinations for the treatment of HIV that may be used in combination with the compound of the present invention include, but are not limited to, combivir, atripla, trizivir, truvada, kaletra and epzicom.

CXCR4 inhibitors for the treatment of HIV that may be used in combination with the compound of the present invention include, but are not limited to, AMD-070.

Entry inhibitors for the treatment of HIV that may be used in combination with the compound of the present invention include, but are not limited to, SP-01A.

Gp 120 inhibitors for the treatment of HIV that may be used in combination with the compound of the present invention include, but are not limited to, BMS-488043 and BMS-378806.

G6PD and NADH-oxidase inhibitors for the treatment of HIV that may be used in combination with the compound of the present invention include, but are not limited to, immunitin.

Other compounds that are useful inhibitors of HIV that may be used in combination with the compound of the present invention include, but are not limited to, Soluble CD4, PRO-542, ibalizumab (TNX-355), and compounds disclosed in JP 2003119137.

Compounds useful in the treatment or management of infection from viruses other than HIV that may be used in combination with the compound of the present invention include, but are not limited to, acyclovir, fomivirsen, penciclovir, HPMPC, oxetanocin G, AL-721, cidofovir, cytomegalovirus immune globin, cytovene, fomivganciclovir, famciclovir, foscarnet sodium, Isis 2922, KNI-272, valacyclovir, virazole ribavirin, valganciclovir, ME-609, PCL-016, DES6, ODN-93, ODN-112, VGV-1, ampligen, HRG-214, cytolin, VGX-410, KD-247, AMZ-0026, CYT-99007A-221, DEBIO-025, BAY 50-4798, MDX-010 (ipilimumab), PBS-119, ALG-889, PA-1050040 (PA-040) and filibuvir (PF-00868554).

Compounds that act as immunomodulators and may be used in combination with the compound of the present invention include, but are not limited to, AD-439, AD-519, Alpha Interferon, AS-101, bropirimine, acemannan, CL246,738, EL10, FP-21399, gamma interferon, granulocyte macrophage colony stimulating factor, IL-2, immune globulin intravenous, IMREG-1, IMREG-2, imuthiol diethyl dithio carbamate, alpha-2 interferon, methionine-enkephalin, MTP-PE, granulocyte colony stimulating sactor, remune, rCD4, recombinant soluble human CD4, interferon alfa-2, SK&F106528, soluble T4 yhymopentin, tumor necrosis factor (TNF), tucaresol, recombinant human interferon beta, and interferon alfa n-3.

Anti-infectives that may be used in combination with the compound of the present invention include, but are not limited to, atovaquone, azithromycin, clarithromycin, trimethoprim, trovafloxacin, pyrimethamine, daunorubicin, clindamycin with primaquine, pastill, omidyl, eflornithine pentamidine, rifabutin, spiramycin, intraconazole-R51211, trimetrexate, daunorubicin, chloroquine, recombinant human erythropoietin, recombinant human growth hormone, megestrol acetate, testerone, and total enteral nutrition.

Antifungals that may be used in combination with the compound of the present invention include, but are not limited to, anidulafungin, C31G, caspofungin, DB-289, fluconzaole, itraconazole, ketoconazole, micafungin, posaconazole, and voriconazole.

Other compounds that may be used in combination with the compound of the present invention include, but are not limited to, acemannan, ansamycin, LM 427, AR177, BMS-232623, BMS-234475, CI-1012, curdlan sulfate, dextran sulfate, STOCRINE EL10, hypericin, lobucavir, novapren, peptide T octabpeptide sequence, trisodium phosphonoformate, probucol, and RBC-CD4.

In addition, the compound of the present invention may be used in combination with anti-proliferative agents for the treatment of conditions such as Kaposi's sarcoma. Such agents include, but are not limited to, inhibitors of metallo-matrix proteases, A-007, bevacizumab, BMS-275291, halofuginone, interleukin-12, rituximab, paditaxel, porfimer sodium, rebimastat, and COL-3.

According to a particular embodiment of the invention, the compounds of the invention may be employed in combination with other therapeutic agents for the treatment or prophylaxis of retroviral infections, more preferably HIV. The invention therefore relates to the use of a composition comprising:

(a) one or more compounds of the formulae herein, and

(b) one or more retroviral enzyme inhibitors as biologically active agents in respective proportions such as to provide a synergistic effect against a viral infection, particularly a retroviral infection in a mammal, for instance in the form of a combined preparation for simultaneous, separate or sequential use in viral infection therapy, such as of HIV.

More generally, the invention relates to the compounds of formulae (A), (B), (C), (D), (E), (F) and embodiments thereof being useful as agents having biological activity (particularly antiviral activity) or as diagnostic agents. Any of the uses mentioned with respect to the present invention may be restricted to a non-medical use, a non-therapeutic use, a non-diagnostic use, or exclusively an in vitro use, or a use related to cells remote from an animal.

Those of skill in the art will also recognize that the compounds of the invention may exist in many different protonation states, depending on, among other things, the pH of their environment. While the structural formulae provided herein depict the compounds in only one of several possible protonation states, it will be understood that these structures

are illustrative only, and that the invention is not limited to any particular protonation state - any and all protonated forms of the compounds are intended to fall within the scope of the invention.

The term "pharmaceutically acceptable salts" as used herein means the therapeutically active non-toxic salt forms which the compounds of formulae herein are able to form. Therefore, the compounds of this invention optionally comprise salts of the compounds herein, especially pharmaceutically acceptable non-toxic salts containing, for example, $Na^+$, $Li^+$, $K^+$, $Ca^{2+}$ and $Mg^{2+}$. Such salts may include those derived by combination of appropriate cations such as alkali and alkaline earth metal ions or ammonium and quaternary amino ions with an acid anion moiety, typically a carboxylic acid. The compounds of the invention may bear multiple positive or negative charges. The net charge of the compounds of the invention may be either positive or negative. Any associated counter ions are typically dictated by the synthesis and/or isolation methods by which the compounds are obtained. Typical counter ions include, but are not limited to ammonium, sodium, potassium, lithium, halides, acetate, trifluoroacetate, etc., and mixtures thereof. It will be understood that the identity of any associated counter ion is not a critical feature of the invention, and that the invention encompasses the compounds in association with any type of counter ion. Moreover, as the compounds can exist in a variety of different forms, the invention is intended to encompass not only forms of the compounds that are in association with counter ions (e.g., dry salts), but also forms that are not in association with counter ions (e.g., aqueous or organic solutions). Metal salts typically are prepared by reacting the metal hydroxide with a compound of this invention. Examples of metal salts which are prepared in this way are salts containing $Li^+$, $Na^+$, and $K^+$. A less soluble metal salt can be precipitated from the solution of a more soluble salt by addition of the suitable metal compound. In addition, salts may be formed from acid addition of certain organic and inorganic acids to basic centers, typically amines, or to acidic groups. Examples of such appropriate acids include, for instance, inorganic acids such as hydrohalogen acids, e.g. hydrochloric or hydrobromic acid, sulfuric acid, nitric acid, phosphoric acid and the like; or organic acids such as, for example, acetic, propanoic, hydroxyacetic, 2-hydroxypropanoic, 2-oxopropanoic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclohexanesulfamic, salicylic (i.e. 2-hydroxybenzoic), p-aminosalicylic and the like. Furthermore, this term also includes the solvates which the compounds of formulae herein as well as their salts are able to form, such as for example hydrates, alcoholates and the like. Finally, it is to be understood that the compositions herein comprise compounds of the invention in their unionized, as well as zwitterionic form, and combinations with stoichiometric amounts of water as in hydrates.

Also included within the scope of this invention are the salts of the parental compounds with one or more amino acids, especially the naturally-occurring amino acids found as protein components. The amino acid typically is one bearing a side chain with a basic or acidic group, e.g., lysine, arginine or glutamic acid, or a neutral group such as glycine, serine, threonine, alanine, isoleucine, or leucine.

The compounds of the invention also include physiologically acceptable salts thereof. Examples of physiologically acceptable salts of the compounds of the invention include salts derived from an appropriate base, such as an alkali metal (for example, sodium), an alkaline earth (for example, magnesium), ammonium and $NX_4^+$ (wherein X is $C_1$-$C_4$ alkyl). Physiologically acceptable salts of an hydrogen atom or an amino group include salts of organic carboxylic acids such as acetic, benzoic, lactic, fumaric, tartaric, maleic, malonic, malic, isethionic, lactobionic and succinic acids; organic sulfonic acids, such as methanesulfonic, ethanesulfonic, benzenesulfonic and p-toluenesulfonic acids; and inorganic acids, such as hydrochloric, sulfuric, phosphoric and sulfamic acids. Physiologically acceptable salts of a compound containing a hydroxy group include the anion of said compound in combination with a suitable cation such as $Na^+$ and $NX_4^+$ (wherein X typically is independently selected from H or a $C_1$-$C_4$ alkyl group). However, salts of acids or bases which are not physiologically acceptable may also find use, for example, in the preparation or purification of a physiologically acceptable compound. All salts, whether or not derived from a physiologically acceptable acid or base, are within the scope of the present invention.

As used herein and unless otherwise stated, the term "enantiomer" means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

The term "isomers" as used herein means all possible isomeric forms, including tautomeric and stereochemical forms, which the compounds of formulae herein may possess, but not including position isomers. Typically, the structures shown herein exemplify only one tautomeric or resonance form of the compounds, but the corresponding alternative configurations are contemplated as well. Unless otherwise stated, the chemical designation of compounds denotes the mixture of all possible stereochemically isomeric forms, said mixtures containing all diastereomers and enantiomers (since the compounds of formulae herein may have at least one chiral center) of the basic molecular structure, as well as the stereochemically pure or enriched compounds. More particularly, stereogenic centers may have either the R- or S-configuration, and multiple bonds may have either cis- or trans-configuration.

The compounds of the present invention may have a chiral centre adjacent to the carboxyl group. Thus, compounds which do not also exhibit atropisomerism (described in more detail below), may exist as two stereoisomers (i.e.

enantiomers). For example:

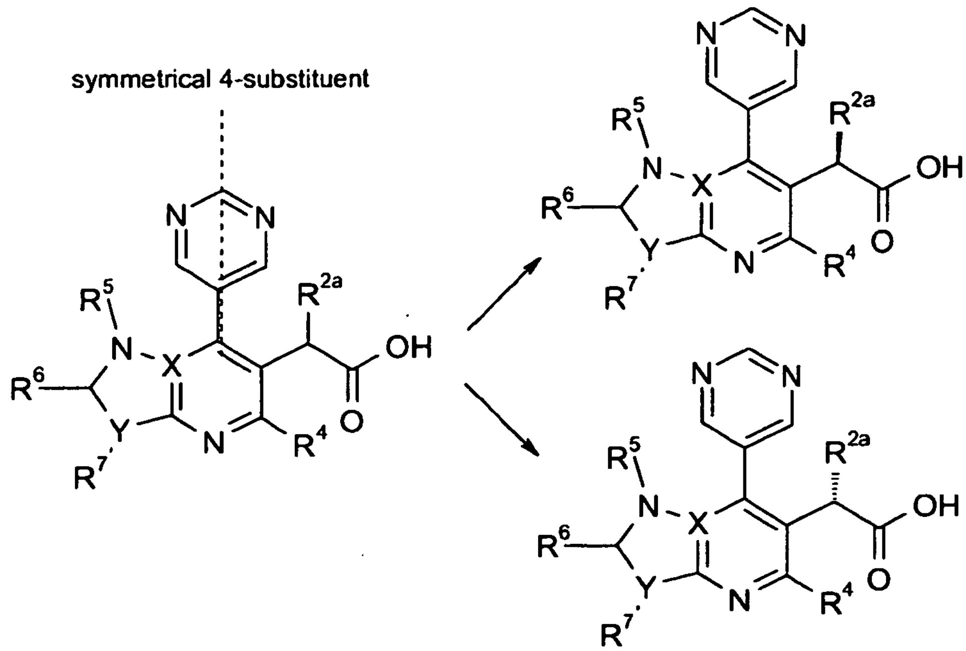


When the 4-substituent is not symmetrical about the plane of the bond at the 4-position, atropisomerism may also arise. This is because the aromatic ring of the 4-substituent and the pyridine/pyrimidine portion of the fused bicyclic ring lie more or less orthogonal to one another and rotation about the bond at the 4-position of the 2,3,4-substituted bicyclic compounds of the present invention may be restricted. Such compounds may therefore exist as four stereoisomers (i.e. diastereoisomers). For example:

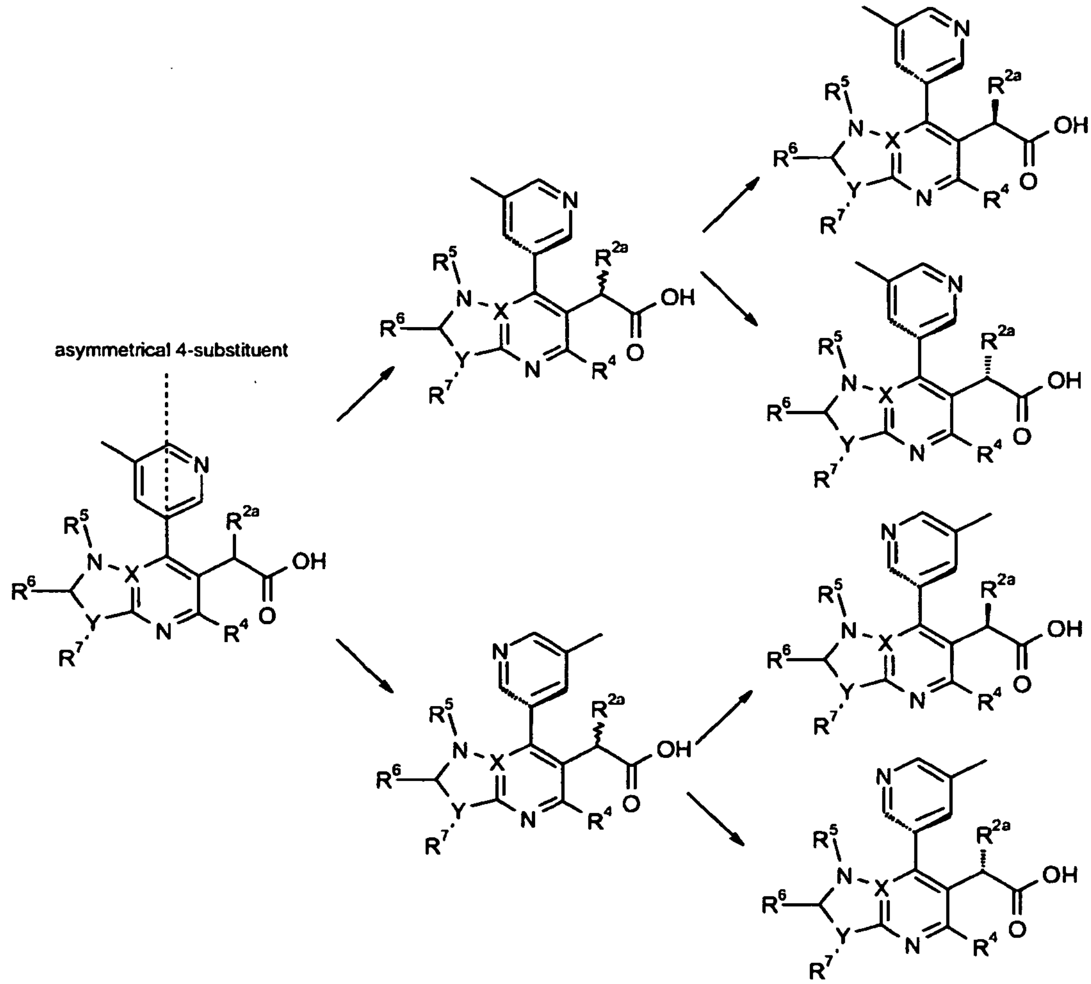


Pure isomeric forms of the said compounds are defined as isomers substantially free of other enantiomeric or diastereomeric forms of the same basic molecular structure. In particular, the term "stereoisomerically pure" or "chirally pure" relates to compounds having a stereoisomeric excess of at least about 80% (i.e. at least 90% of one isomer and at most 10% of the other possible isomers), preferably at least 90%, more preferably at least 94% and most preferably at least 97%. The terms "enantiomerically pure" and "diastereomerically pure" should be understood in a similar way, having regard to the enantiomeric excess, respectively the diastereomeric excess, of the mixture in question.

Separation of stereoisomers is accomplished by standard methods known to those in the art. One enantiomer of a compound of the invention can be separated substantially free of its opposing enantiomer by a method such as formation of diastereomers using optically active resolving agents ("Stereochemistry of Carbon Compounds," (1962) by E. L. Eliel, McGraw Hill; Lochmuller, C. H., (1975) J. Chromatogr., 113:(3) 283-302). Separation of isomers in a mixture can be accomplished by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure enantiomers, or (3) enantiomers can be separated directly under chiral conditions. Under method (1), diastereomeric salts can be formed by reaction of enantiomerically pure chiral bases such as brucine, quinine, ephedrine, strychnine, a-methyl-b-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts. Alternatively, by method (2), the substrate to be resolved may be reacted with one enantiomer of a chiral compound to form a diastereomeric pair (Eliel, E. and Wilen, S. (1994) Stereochemistry of Organic Compounds, John Wiley & Sons, Inc., p. 322). Diastereomeric compounds can be formed by reacting asymmetric compounds with enantiomerically pure chiral derivatizing reagents, such as menthyl derivatives, followed by separation of the diastereomers and hydrolysis to yield the free, enantiomerically enriched xanthene. A method of determining optical purity involves making chiral esters, such as a menthyl ester or Mosher ester, a-methoxy-a-(trifluoromethyl)phenyl acetate (Jacob III. (1982) J. Org. Chem.

47:4165), of the racemic mixture, and analyzing the NMR spectrum for the presence of the two atropisomeric diastereomers. Stable diastereomers can be separated and isolated by normal- and reverse-phase chromatography following methods for separation of atropisomeric naphthyl-isoquinolines (Hoye, T., WO 96/15111). Under method (3), a racemic mixture of two asymmetric enantiomers is separated by chromatography using a chiral stationary phase. Suitable chiral stationary phases are, for example, polysaccharides, in particular cellulose or amylose derivatives. Commercially available polysaccharide based chiral stationary phases are ChiralCel™ CA, OA, OB5, OC5, OD, OF, OG, OJ and OK, and Chiralpak™ AD, AS, OP(+) and OT(+). Appropriate eluents or mobile phases for use in combination with said polysaccharide chiral stationary phases are hexane and the like, modified with an alcohol such as ethanol, isopropanol and the like. ("Chiral Liquid Chromatography" (1989) W. J. Lough, Ed. Chapman and Hall, New York; Okamoto, (1990) "Optical resolution of dihydropyridine enantiomers by High-performance liquid chromatography using phenylcarbamates of polysaccharides as a chiral stationary phase", J. of Chromatogr. 513:375-378).

The terms cis and trans are used herein in accordance with Chemical Abstracts nomenclature and include reference to the position of the substituents on a ring moiety. The absolute stereochemical configuration of the compounds of formula (1) may easily be determined by those skilled in the art while using well-known methods such as, for example, X-ray diffraction.

The compounds of the invention may be formulated with conventional carriers and excipients, which will be selected in accordance with standard practice. Tablets will contain excipients, glidants, fillers, binders and the like. Aqueous formulations are prepared in sterile form, and when intended for delivery by other than oral administration generally will be isotonic. Formulations optionally contain excipients such as those set forth in the "Handbook of Pharmaceutical Excipients" (1986) and include ascorbic acid and other antioxidants, chelating agents such as EDTA, carbohydrates such as dextrin, hydroxyalkylcellulose, hydroxyalkylmethylcellulose, stearic acid and the like.

Subsequently, the term "pharmaceutically acceptable carrier" as used herein means any material or substance with which the active ingredient is formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, suspensions, ointments, creams, tablets, pellets or powders.

Suitable pharmaceutical carriers for use in the said pharmaceutical compositions and their formulation are well known to those skilled in the art, and there is no particular restriction to their selection within the present invention. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-steps procedure, with the selected carrier material and, where appropriate, the other additives such as surface-active agents may also be prepared by micronisation, for instance in view to obtain them in the form of microspheres usually having a diameter of about 1 to 10 gm, namely for the manufacture of microcapsules for controlled or sustained release of the active ingredients.

Suitable surface-active agents, also known as emulgent or emulsifier, to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable from coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcoholamine salts of dodecylbenzene sulphonic acid or dibutyl-naphthalenesulphonic acid or a naphthalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanylphosphatidylcholine, dipalmitoylphoshatidyl -choline and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenol -polyethoxyethanol, castor oil polyglycolic ethers, polypropylene/polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, particularly halides, having 4 hydrocarbon radicals optionally substituted with halo, phenyl, substituted phenyl or hydroxy; for instance quaternary ammonium salts containing as N-substituent at least one C8C22 alkyl radical (e.g. cetyl, lauryl, palmityl, myristyl, oleyl and the like) and, as further substituents, unsubstituted or halogenated lower alkyl, benzyl and/or hydroxy-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbucw', 2 d ed. (Hanser Verlag, Vienna, 1981) and "Encyclopaedia of Surfactants, (Chemical Publishing Co., New York, 1981).

Compounds of the invention and their physiologically acceptable salts (hereafter collectively referred to as the active ingredients) may be administered by any route appropriate to the condition to be treated, suitable routes including oral, rectal, nasal, topical (including ocular, buccal and sublingual), vaginal and parenteral (including subcutaneous, intramuscular, intravenous, intradermal, intrathecal and epidural). The preferred route of administration may vary with for example the condition of the recipient.

While it is possible for the active ingredients to be administered alone, it is preferable to present them as pharmaceutical formulations. The formulations, both for veterinary and for human use, of the present invention comprise at least one active ingredient, as above described, together with one or more pharmaceutically acceptable carriers therefore and optionally other. therapeutic ingredients. The carrier(s) optimally are "acceptable" in the sense of being compatible with the other ingredients of the formulation and not deleterious to the recipient thereof. The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein. For infections of the eye or other external tissues e.g. mouth and skin, the formulations are optionally applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base. If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG400) and mixtures thereof. The topical formulations may desirably include a compound which enhances absorption or penetration of the active ingredient through the skin or other affected areas. Examples of such dermal penetration enhancers include dimethylsulfoxide and related analogs.

The oily phase of the emulsions of this invention may be constituted from known ingredients in a known manner.

While the phase may comprise merely an emulsifier (otherwise known as an emulgent), it desirably comprises a mixture of at least one emulsifier with a fat or an oil or with both a fat and an oil. Optionally, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

The choice of suitable oils or fats for the formulation is based on achieving the desired cosmetic properties, since the solubility of the active compound in most oils likely to be used in pharmaceutical emulsion formulations is very low. Thus the cream should optionally be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is optionally present in such formulations in a concentration of 0.5 to 20%, advantageously 0.5 to 10% particularly about 1.5% w/w. Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate. Formulations suitable for nasal administration wherein the carrier is a solid include a coarse powder having a particle size for example in the range 20 to 500 microns (including particle sizes in a range between 20 and 500 microns in increments of 5 microns such as 30 microns, 35 microns, etc), which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient. Formulations suitable for aerosol administration may be prepared according to conventional methods and may be delivered with other therapeutic agents.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavoring agents.

Compounds of the invention can be used to provide controlled release pharmaceutical formulations containing as active ingredient one or more compounds of the invention ("controlled release formulations") in which the release of the active ingredient can be controlled and regulated to allow less frequency dosing or to improve the pharmacokinetic or toxicity profile of a given invention compound. Controlled release formulations adapted for oral administration in which discrete units comprising one or more compounds of the invention can be prepared according to conventional methods.

Additional ingredients may be included in order to control the duration of action of the active ingredient in the composition. Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition may require protective coatings. Pharmaceutical forms suitable for injectionable use include sterile aqueous solutions or dispersions and sterile powders

for the extemporaneous preparation thereof. Typical carriers for this purpose therefore include biocompatible aqueous buffers, ethanol, glycerol, propylene glycol, polyethylene glycol and the like and mixtures thereof.

In view of the fact that, when several active ingredients are used in combination, they do not necessarily bring out their joint therapeutic effect directly at the same time in the mammal to be treated, the corresponding composition may also be in the form of a medical kit or package containing the two ingredients in separate but adjacent repositories or compartments. In the latter context, each active ingredient may therefore be formulated in a way suitable for an administration route different from that of the other ingredient, e.g. one of them may be in the form of an oral or parenteral formulation whereas the other is in the form of an ampoule for intravenous injection or an aerosol.

Another embodiment of this invention relates to various precursor or "prodrug" forms of the compounds of the present invention. It may be desirable to formulate the compounds of the present invention in the form of a chemical species which itself is not significantly biologically-active, but which when delivered to the animal will undergo a chemical reaction catalyzed by the normal function of the body of the animal, *inter alia,* enzymes present in the stomach or in blood serum, said chemical reaction having the effect of releasing a compound as defined herein. The term "pro-drug" thus relates to these species which are converted *in vivo* into the active pharmaceutical ingredient.

The prodrugs of the present invention can have any form suitable to the formulator, for example, esters are non-limiting common pro-drug forms. In the present case, however, the pro-drug may necessarily exist in a form wherein a covalent bond is cleaved by the action of an enzyme present at the target *locus*. For example, a C-C covalent bond may be selectively cleaved by one or more enzymes at said target *locus* and, therefore, a pro-drug in a form other than an easily hydrolysable precursor, *inter alia* an ester, an amide, and the like, may be used. The counterpart of the active pharmaceutical ingredient in the pro-drug can have different structures such as an amino acid or peptide structure, alkyl chains, sugar moieties and others as known in the art.

For the purpose of the present invention the term "therapeutically suitable prodrug" is defined herein as "a compound modified in such a way as to be transformed *in vivo* to the therapeutically active form, whether by way of a single or by multiple biological transformations, when in contact with the tissues of the animal, mammal or human to which the pro-drug has been administered, and without undue toxicity, irritation, or allergic response, and achieving the intended therapeutic outcome ".

More specifically the term "prodrug", as used herein, relates to an inactive or significantly less active derivative of a compound such as represented by the structural formula (I), which undergoes spontaneous or enzymatic transformation within the body in order to release the pharmacologically active form of the compound. For a comprehensive review, reference is made to Rautio J. et al. ("Prodrugs: design and clinical applications" Nature Reviews Drug Discovery, 2008, doi: 10.1038/nrd2468).

The compounds of the invention can be prepared while using a series of chemical reactions well known to those skilled in the art, altogether making up the process for preparing said compounds and exemplified further. The processes described further are only meant as examples and by no means are meant to limit the scope of the present invention.

The compounds of the present invention can be prepared according to the following general procedures depicted hereunder:

## Scheme 1:

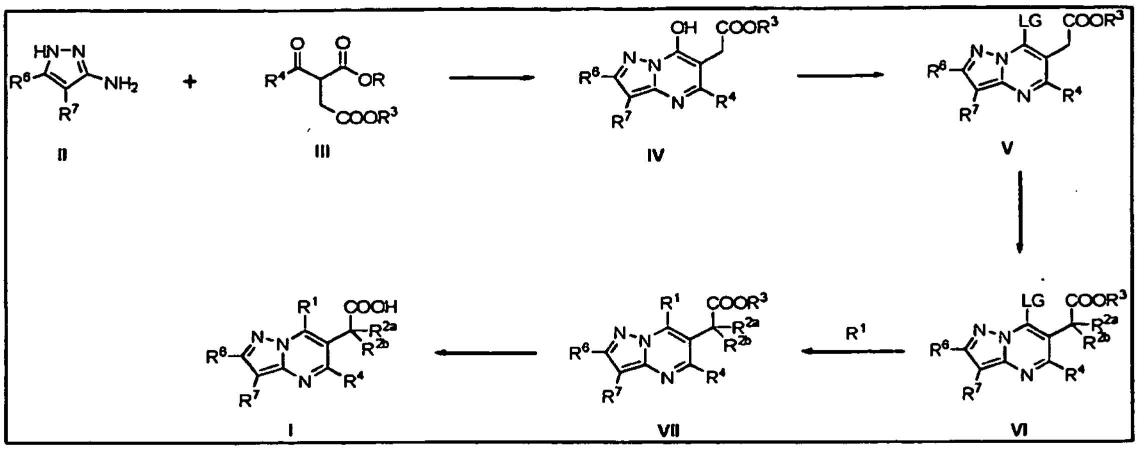

**Scheme 1: all $R^1$, $R^{2a}$, $R^{2b}$, $R^3$, $R^4$, $R^6$, $R^7$ and LG are as described for the compounds of the present invention and its embodiments and formulae.**

Condensation of a 3-aminopyrazole of general formula II (commercially available or synthesized by procedures known to the skilled in the art) with an intermediate of formula III, wherein R is an ester protecting group (e.g., methyl, ethyl and the like) in the presence of an apolar aprotic solvent (e.g., benzene, toluene, xylene and the like) at a temperature raising from 80 to 140°C, provides the desired intermediates of formula IV. The intermediates IV are then converted in intermediates of formula V by procedures known to the skilled in the art or as set forth in the examples below, and wherein LG is a leaving group only selected from halogen. Alkylation of intermediates of formula V, by procedures known to the skilled in the art or as set forth in the examples below, provides compounds of formula VI. Coupling of intermediates VI with a suitable $R^1$ precursor by procedures known to the skilled in the art or as set forth in examples below, provides compounds of formula VII which can be converted in the desired compounds of formula I using standard hydrolysis conditions.

Alternatively, compounds of general formula I can also be prepared as outlined in Scheme 2 below.

Scheme 2:

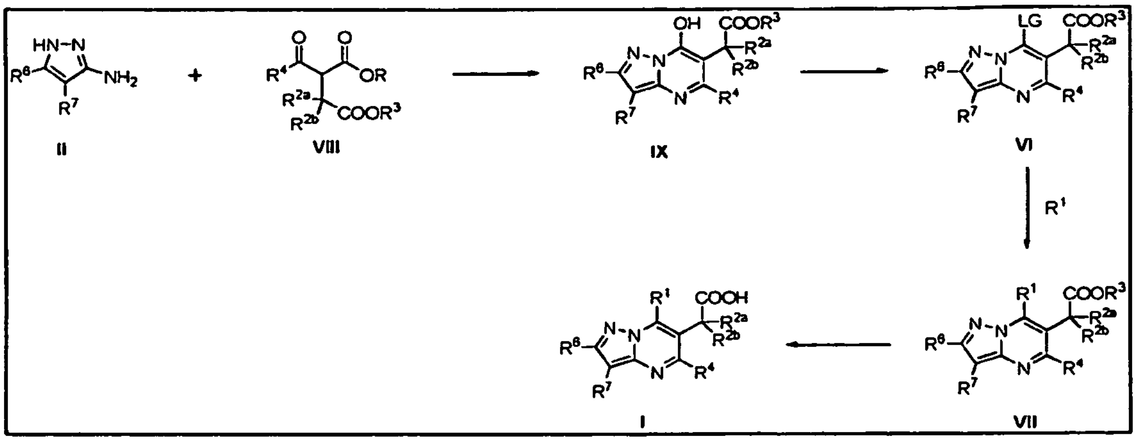

**Scheme 2: all $R^1$, $R^{2a}$, $R^{2b}$, $R^4$, $R^6$, $R^7$ and LG are as described for the compounds of the present invention and its embodiments and formulae.**

Condensation of a 3-aminopyrazole of general formula II (commercially available or synthesized by procedures known to the skilled in the art) with an intermediate of formula VIII (commercially available or synthesized by procedures known to the skilled in the art), wherein R is an ester protecting group (e.g., methyl, ethyl and the like) in the presence of an apolar aprotic solvent (e.g., benzene, toluene, xylene and the like) at a temperature raising from 80 to 140°C, provides the desired intermediates of formula IV. The intermediates IX are then converted in intermediates of formula VI by procedures known to the skilled in the art or as set forth in the examples below, and wherein LG is a leaving group only selected from halogen. Coupling of intermediates VI with a suitable $R^1$ precursor by procedures known to the skilled in the art or as set forth in examples below, provides compounds of formula VII which can be converted in the desired compounds of formula I using standard hydrolysis conditions.

In another embodiment, compounds of general formula Ia can be prepared as outlined in Scheme 3 below.

Scheme 3:

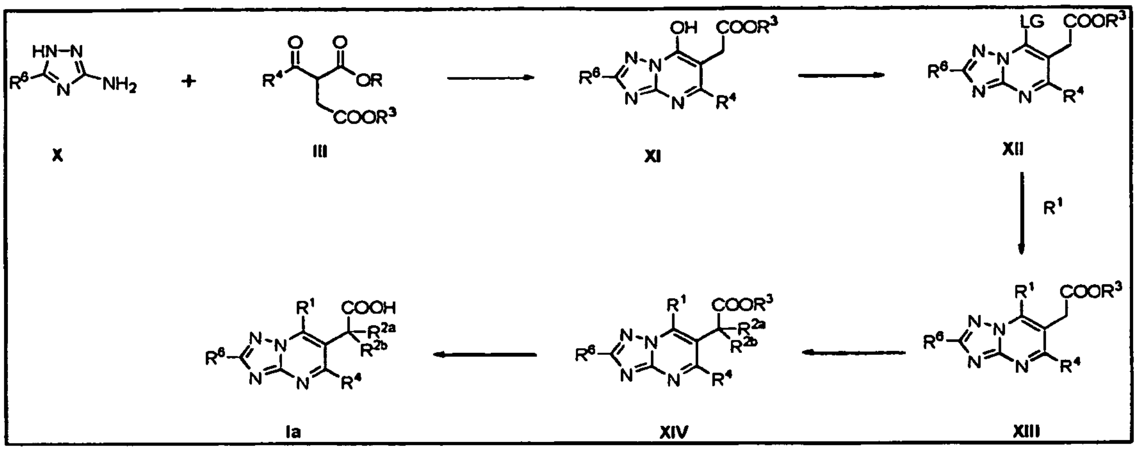

Scheme 3: all $R^1$, $R^{2a}$, $R^{2b}$, $R^4$, $R^6$ and LG are as described for the compounds of the present invention and its embodiments and formulae.

Condensation of a 3-amino-1,2,4-triazole of general formula X (commercially available or synthesized by procedures known to the skilled in the art) with an intermediate of formula III, wherein R is an ester protecting group (e.g., methyl, ethyl and the like) in the presence of an apolar aprotic solvent (e.g., benzene, toluene, xylene and the like) at a temperature raising from 80 to 140°C, provides the desired intermediates of formula XI. The intermediates XI are then converted in intermediates of formula XII by procedures known to the skilled in the art or as set forth in the examples below, and wherein LG is a leaving group only selected from halogen. Coupling of intermediates XII with a suitable $R^1$ precursor by procedures known to the skilled in the art (amination, Suzuki coupling, Negishi coupling, Stille coupling and the like) or as set forth in examples below, provides compounds of formula XIII. Alkylation of intermediates of formula XIII, by procedures known to the skilled in the art or as set forth in the examples below, provides compounds of formula XIV which can be converted in the desired compounds of formula Ia using standard hydrolysis conditions.

In another embodiment, compounds of general formula Ia can be prepared as outlined in Scheme 4 below.

Scheme 4:

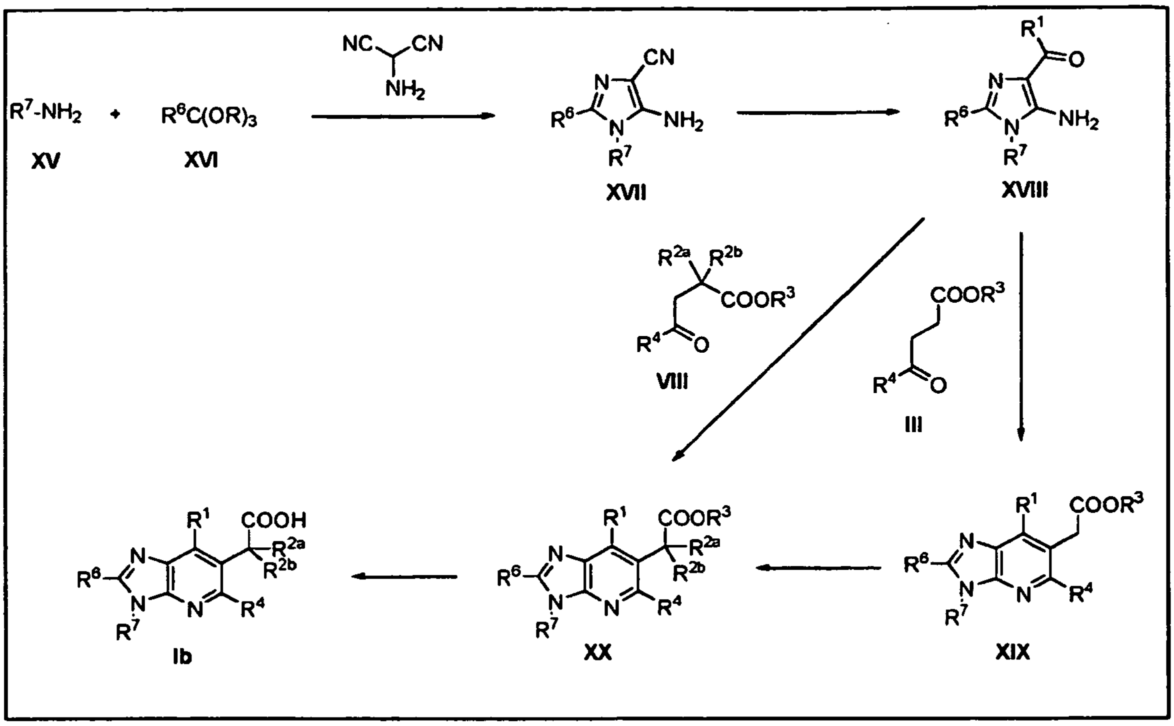

Scheme 4: all $R^1$, $R^{2a}$, $R^{2b}$, $R^4$, $R^6$, $R^7$ and LG are as described for the compounds of the present invention and its embodiments and formulae.

The condensation of a primary amine of formula XV with an orthoester of formula XVI, wherein R is an alkyl group (methyl or ethyl) with aminomalonirile, in the presence of a strong base (e.g., triethylamine, diisopropylethylamine and the like) in a polar solvent (e.g., acetonitrile, THF, and the like) at a temperature raising from 0°C to 80°C provides the desired intermediates of formula XVII. More detailed information can be found in the following reference (US 2006/0094706 A1). The intermediates of formula XVII are then reacted with a Grignard reagent to provide intermediates of formula XVIII which can the be condensed with intermediates of formula III in the presence of trimethylsilyl chloride to provide intermediates of formula XIX. Alkylation of intermediates of formula XIX, by procedures known to the skilled in the art or as set forth in the examples below, provides compounds of formula XX which can be converted in the desired compounds of formula Ib using standard hydrolysis conditions. Alternatively, intermediates of formula XX can also be obtained from the condensation of intermediates of formula XVIII with intermediates of formula VIII wherein R is an ester protecting group (e.g., methyl, ethyl and the like).

In another embodiment, compounds of general formula Ia can be prepared as outlined in Scheme 5 below.

Scheme 5:

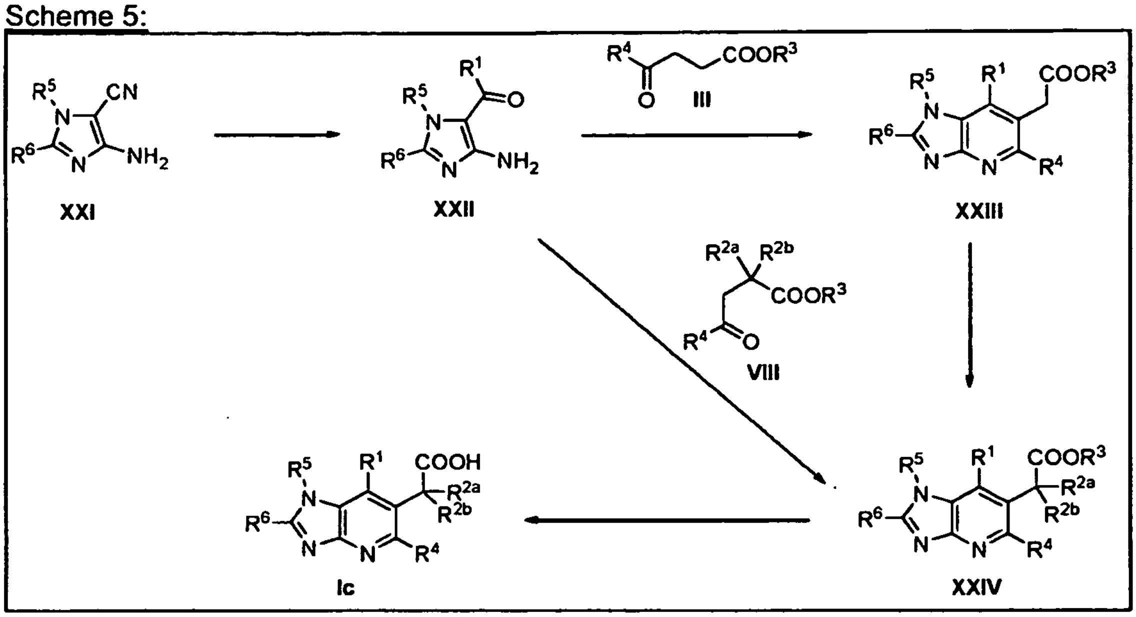

Scheme 5: all $R^1$, $R^{2a}$, $R^{2b}$, $R^4$, $R^5$, $R^6$ and LG are as described for the compounds of the present invention and its embodiments and formulae.

The condensation of intermediates of formula XXI (commercially available or synthesized by procedures known to the skilled in the art or as set forth in the examples below) with a Grignard reagent provides intermediates of formula XXII which can the be condensed with intermediates of formula III in the presence of trimethylsilyl chloride to provide intermediates of formula XXIII. Alkylation of intermediates of formula XXIII, by procedures known to the skilled in the art or as set forth in the examples below, provides compounds of formula XXIV which can be converted in the desired compounds of formula Ic using standard hydrolysis conditions. Alternatively, intermediates of formula XXIV can also be obtained from the condensation of intermediates of formula XXII with intermediates of formula VIII wherein R is an ester protecting group (e.g., methyl, ethyl and the like).

## Examples

The following examples are provided for the purpose of illustrating the present invention and by no means should be interpreted to limit the scope of the present invention.

Part A represents the preparation of the compounds (intermediates and final compounds) whereas Part B describes the antiviral activity of the compounds of the invention.

Table 1: Structures of example compounds of the invention and their respective codes.

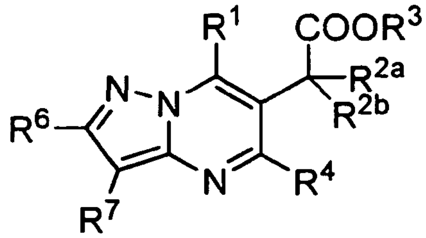

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-01 | 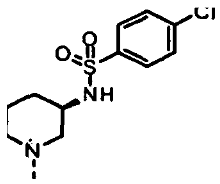 | n-propyl | H | Et | Me | H | H |
| CPD-02 | 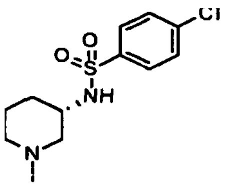 | n-propyl | H | Et | Me | H | H |
| CPD-03 | 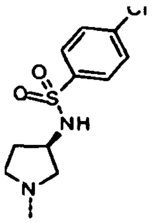 | n-propyl | H | Et | Me | H | H |
| CPD-04 | 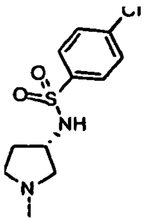 | n-propyl | H | Et | Me | H | H |
| CPD-05 | phenyl | n-propyl | H | Me | Me | H | H |
| CPD-06 | p-tolyl | n-propyl | H | Me | Me | H | H |
| CPD-07 | phenyl | n-propyl | H | Me | Me | tBu | H |
| CPD-08 | p-tolyl | n-propyl | H | Me | Me | tBu | H |
| CPD-09 | p-tolyl | n-propyl | H | Me | Me | phenyl | H |
| CPD-10 | 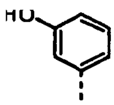 | n-propyl | H | Me | Me | tBu | H |
| CPD-11 | 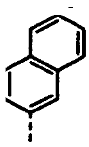 | n-propyl | H | Me | Me | tBu | H |
| CPD-12 | 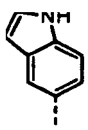 | n-propyl | H | Me | Me | tBu | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-13 | 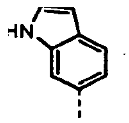 | n-propyl | H | Me | Me | tBu | H |
| CPD-14 | 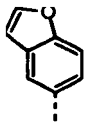 | n-propyl | H | Me | Me | tBu | H |
| CPD-15 | 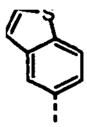 | n-propyl | H | Me | Me | tBu | H |
| CPD-16 | 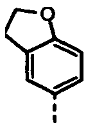 | n-propyl | H | Me | Me | tBu | H |
| CPD-17 | 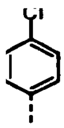 | n-propyl | H | Me | Me | tBu | H |
| CPD-18 | 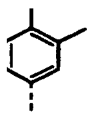 | n-propyl | H | Me | Me | tBu | H |
| CPD-19 | 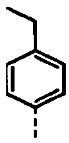 | n-propyl | H | Me | Me | tBu | H |
| CPD-20 | 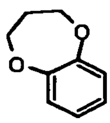 | n-propyl | H | Me | Me | tBu | H |
| CPD-21 | 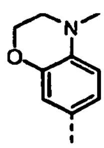 | n-propyl | H | Me | Me | tBu | H |
| CPD-22 | p-tolyl | n-propyl | H | Et | Me | H | Br |
| CPD-23 | p-tolyl | n-propyl | H | Et | Me | H | phenyl |
| CPD-24 | p-tolyl | n-propyl | H | Et | Me | H | p-tolyl |
| CPD-25 | p-tolyl | n-propyl | H | Me | Me | tBu | Br |
| CPD-26 | p-tolyl | n-propyl | H | Me | Me | tBu | p-tolyl |
| CPD-27 | p-tolyl | n-propyl | H | Me | Me | n-propyl | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-28 | p-tolyl | n-propyl | H | Me | Me | 2-furanyl | H |
| CPD-29 | 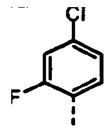 | n-propyl | H | Me | Me | tBu | H |
| CPD-30 | 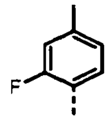 | n-propyl | H | Me | Me | tBu | H |
| CPD-31 | p-tolyl | 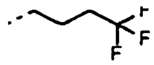 | H | Me | Me | tBu | H |
| CPD-32 | p-tolyl | benzyl | H | Me | Me | tBu | H |
| CPD-33 | 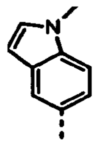 | n-propyl | H | Me | Me | tBu | H |
| CPD-34 | 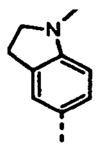 | n-propyl | H | Me | Me | tBu | H |
| CPD-35 | 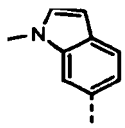 | n-propyl | H | Me | Me | tBu | H |
| CPD-36 | 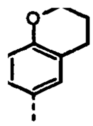 | n-propyl | H | Me | Me | tBu | H |
| CPD-37 | p-tolyl | 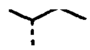 | H | Me | Me | tBu | H |
| CPD-38 | p-tolyl | | H | Me | Me | tBu | Cl |
| CPD-39 | p-tolyl | 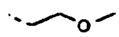 | H | Me | Me | tBu | H |
| CPD-40 | 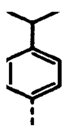 | n-propyl | H | Me | Me | tBu | H |
| CPD-41 | 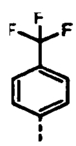 | n-propyl | H | Me | Me | tBu | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-42 | 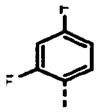 | n-propyl | H | Me | Me | tBu | H |
| CPD-43 | 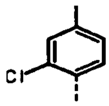 | n-propyl | H | Me | Me | tBu | H |
| CPD-44 | 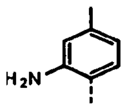 | n-propyl | H | Me | Me | tBu | H |
| CPD-45 | 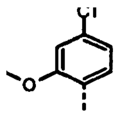 | n-propyl | H | Me | Me | tBu | H |
| CPD-46 | 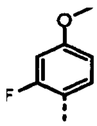 | n-propyl | H | Me | Me | tBu | H |
| CPD-47 | 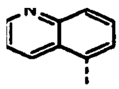 | n-propyl | H | Me | Me | tBu | H |
| CPD-48 | 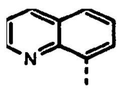 | n-propyl | H | Me | Me | tBu | H |
| CPD-49 | 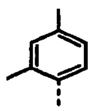 | n-propyl | H | Me | Me | tBu | H |
| CPD-50 | p-tolyl | n-propyl | H | Me | Me | tBu | Me |
| CPD-51 | p-tolyl | n-propyl | H | Me | Me | tBu | phenyl |
| CPD-52 | 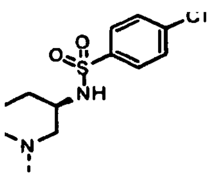 | n-propyl | H | H | Me | H | H |
| CPD-53 | 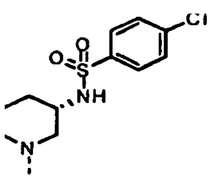 | n-propyl | H | H | Me | H | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-54 | 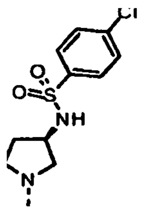 | n-propyl | H | H | Me | H | H |
| CPD-55 | 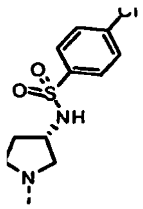 | n-propyl | H | H | Me | H | H |
| CPD-56 | phenyl | n-propyl | H | H | Me | H | H |
| CPD-57 | p-tolyl | n-propyl | H | H | Me | H | H |
| CPD-58 | phenyl | n-propyl | H | H | Me | tBu | H |
| CPD-59 | p-tolyl | n-propyl | H | H | Me | tBu | H |
| CPD-60 | p-tolyl | n-propyl | H | H | Me | tBu | Br |
| CPD-61 | p-tolyl | n-propyl | H | H | Me | phenyl | H |
| CPD-62 | 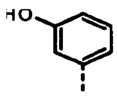 | n-propyl | H | H | Me | tBu | H |
| CPD-63 | 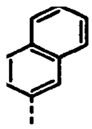 | n-propyl | H | H | Me | tBu | H |
| CPD-64 | 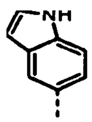 | n-propyl | H | H | Me | tBu | H |
| CPD-65 | 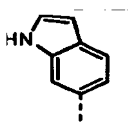 | n-propyl | H | H | Me | tBu | H |
| CPD-66 | 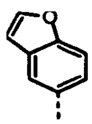 | n-propyl | H | H | Me | tBu | H |
| CPD-67 | 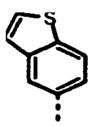 | n-propyl | H | H | Me | tBu | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-68 | 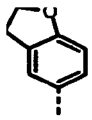 | n-propyl | H | H | Me | tBu | H |
| CPD-69 | 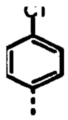 | n-propyl | H | H | Me | tBu | H |
| CPD-70 | 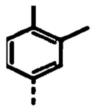 | n-propyl | H | H | Me | tBu | H |
| CPD-71 | 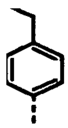 | n-propyl | H | H | Me | tBu | H |
| CPD-72 | 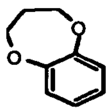 | n-propyl | H | H | Me | tBu | H |
| CPD-73 | 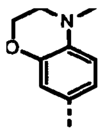 | n-propyl | H | H | Me | tBu | H |
| CPD-74 | p-tolyl | n-propyl | H | H | Me | H | phenyl |
| CPD-75 | p-tolyl | n-propyl | H | H | Me | H | p-tolyl |
| CPD-76 | p-tolyl | n-propyl | H | H | Me | tBu | p-tolyl |
| CPD-77 | p-tolyl | n-propyl | H | H | Me | n-propyl | H |
| CPD-78 | p-tolyl | n-propyl | H | H | Me | 2-furanyl | H |
| CPD-79 | 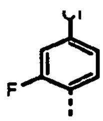 | n-propyl | H | H | Me | tBu | H |
| CPD-80 | 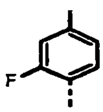 | n-propyl | H | H | Me | tBu | H |
| CPD-81 | p-tolyl | 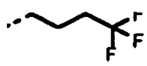 | H | H | Me | tBu | H |
| CPD-82 | P-tolyl | benzyl | H | H | Me | tBu | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-83 | 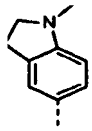 | n-propyl | H | H | Me | tBu | H |
| CPD-84 | 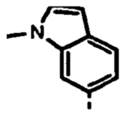 | n-propyl | H | H | Me | tBu | H |
| CPD-85 | 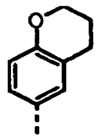 | n-propyl | H | H | Me | tBu | H |
| CPD-86 | p-tolyl | 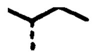 | H | H | Me | tBu | H |
| CPD-87 | p-tolyl | n-propyl | H | H | Me | tBu | Cl |
| CPD-88 | p-tolyl | 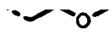 | H | H | Me | tBu | H |
| CPD-89 | 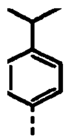 | n-propyl | H | H | Me | tBu | H |
| CPD-90 | 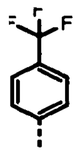 | n-propyl | H | H | Me | tBu | H |
| CPD-91 | 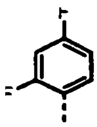 | n-propyl | H | H | Me | tBu | H |
| CPD-92 | 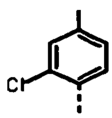 | n-propyl | H | H | Me | tBu | H |
| CPD-93 | 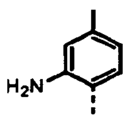 | n-propyl | H | H | Me | tBu | H |
| CPD-94 | 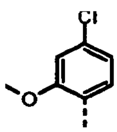 | n-propyl | H | H | Me | tBu | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-95 | 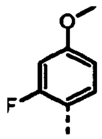 | n-propyl | H | H | Me | tBu | H |
| CPD-96 | 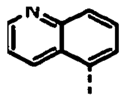 | n-propyl | H | H | Me | tBu | H |
| CPD-97 | 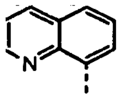 | n-propyl | H | H | Me | tBu | H |
| CPD-98 | 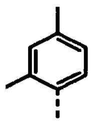 | n-propyl | H | H | Me | tBu | H |
| CPD-99 | p-tolyl | n-propyl | H | H | Me | tBu | Me |
| CPD-100 | p-tolyl | n-propyl | H | H | Me | tBu | phenyl |
| CPD-110 | 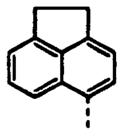 | n-propyl | H | Me | Me | tBu | H |
| CPD-111 | 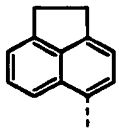 | n-propyl | H | H | Me | tBu | H |
| CPD-112 | 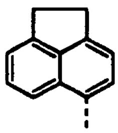 | n-propyl | H | H | Me | tBu | H |
| CPD-113 | 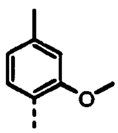 | n-propyl | H | Me | Me | tBu | H |
| CPD-114 | 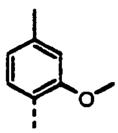 | n-propyl | H | Me | Me | tBu | H |
| CPD-115 | 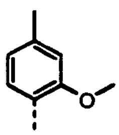 | n-propyl | H | H | Me | tBu | H |

(continued)

| Cpd code | $R^1$ | $R^{2a}$ | $R^{2b}$ | $R^3$ | $R^4$ | $R^6$ | $R^7$ |
|---|---|---|---|---|---|---|---|
| CPD-116 | 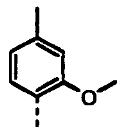 | n-propyl | H | H | Me | tBu | H |
| CPD-117 | 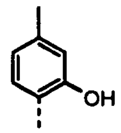 | n-propyl | H | H | Me | tBu | H |

Table 2: Structures of example compounds of the invention and their respective codes.

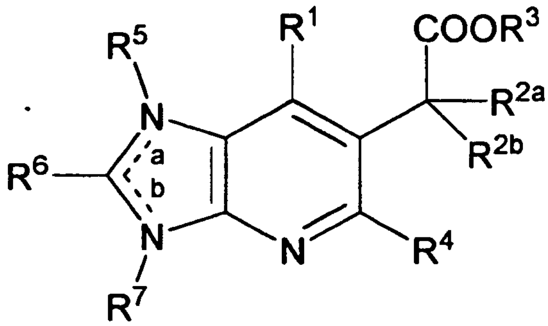

| Cpd code | Example | a | b | $R^1$ | $R^{2a}$ | $R^{2b}$ | $R^3$ | $R^4$ | $R^5$ | $R^6$ | $R^7$ |
|---|---|---|---|---|---|---|---|---|---|---|---|
| CPD-101 | 52 | bond | - | p-tolyl | H | H | Me | Me | - | Me | Me |
| CPD-102 | 53 | bond | - | p-tolyl | n-propyl | H | Me | Me | - | Me | Me |
| CPD-103 | 55 | bond | - | p-tolyl | n-propyl | H | Et | Me | - | n-propyl | Me |
| CPD-104 | 56 | bond | - | P- tolyl | n-propyl | H | Et | Me | - | i-propyl | He |
| CPD-105 | 107 | bond | - | p-tolyl | n-propyl | H | H | Me | - | Me | Me |
| CPD-106 | 109 | bond | - | p-tolyl | n-propyl | H | H | Me | - | n-propyl | Me |
| CPD-107 | 110 | bond | - | p-tolyl | n-propyl | H | H | Me | - | i-propyl | Me |
| CPD-108 | 54 | - | bond | p-tolyl | n-propyl | H | Et | Me | Me | Me | - |
| CPD-109 | 108 | - | bond | p-tolyl | n-propyl | H | H | Me | Me | Me | - |

Table 3: Structures of example compounds of the invention and their respective codes.

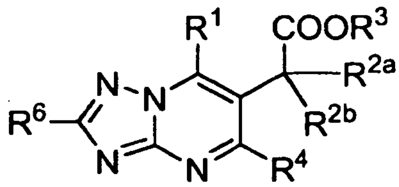

| Cpd code | $R^1$ | $R^{2a}$ | $R^{2b}$ | $R^3$ | $R^4$ | $R^6$ |
|---|---|---|---|---|---|---|
| CPD-118 | p-tolyl | H | H | Me | Me | iPr |
| CPD-119 | p-tolyl | H | H | H | Me | iPr |
| CPD-120 | p-tolyl | n-propyl | H | Me | Me | iPr |

(continued)

| Cpd code | $R^1$ | $R^{2a}$ | $R^{2b}$ | $R^3$ | $R^4$ | $R^6$ |
|---|---|---|---|---|---|---|
| CPD-121 | p-tolyl | n-propyl | H | H | Me | iPr |
| CPD-122 | p-totyl | n-propyl | H | Et | Me | benzyl |
| CPD-123 | p-tolyl | n-propyl | H | H | Me | benzyl |

**EXAMPLE DESCRIBING THE MATERIALS USED, GENERAL PREPARATION METHODS AND SYNTHESIS OF INTERMEDIATES**

All the preparative HPLC purifications mentioned in this experimental part have been carried out with the following system: a Waters 2489 UV/Visible Detector, a Waters 2545 Binary Gradient Module, a Waters Fraction Collector III and a Waters Dual Flex Injector.

The separations were performed with a SunFire Prep C18 ODB column (5 μm; 19 x 100 mm) equipped with a SunFire C18 guard column (5 μm; 19 x 10 mm). Elutions were carried out with the methods described in the following tables, and detection wavelengths were fixed at 210 and 254 nm.

HPLC method 1

| Time (min) | Flow Rate (mL/min) | Solvent A (%) | Solvent B (%) |
|---|---|---|---|
| 0 | 20 | 80 | 20 |
| 2.00 | 20 | 80 | 20 |
| 8.00 | 20 | 10 | 90 |
| 10.80 | 20 | 10 | 90 |
| 11.00 | 20 | 80 | 20 |
| 16.00 | 20 | 80 | 20 |
| Solvent A: Formic Acid LC-MS grade 0.1% in milliQ water<br>Solvent B: Acetonitrile HPLC grade. | | | |

HPLC method 2

| Time (min) | Flow Rate (mL/min) | Solvent A (%) | Solvent B (%) |
|---|---|---|---|
| 0 | 20 | 50 | 50 |
| 2.00 | 20 | 50 | 50 |
| 9.00 | 20 | 10 | 90 |
| 11.00 | 20 | 10 | 90 |
| 11.20 | 20 | 50 | 50 |
| 16.00 | 20 | 50 | 50 |
| Solvent A: Formic Add LC-MS grade 0.1% in milliQ water<br>Solvent B: Acetonitrile HPLC grade. | | | |

General procedure A :

A mixture of a 3-aminopyrazole (1 equivalent) and a dialkyl acetylsuccinate (1.1 equivalent) in toluene (1

mL/mmol of default reagent) was heated to reflux under with Dean Stark system until the theoric volume of water distilled in the trap. The precipitate was filtered-off, washed with toluene and diethylether to afford the expected alkyl 2-(7-hydroxy-5-methylpyrazolo[1,5-a]pyrimidin-6-yl)acetate, which was used for the next step without any further purification.

General procedure B :

The alkyl 2-(7-hydroxy-5-methylpyrazolo[1,5-a]pyrimidin-6-yl)acetate was suspended in phosphorus oxychloride (1 mL/mmol) and dimethylaniline (0.2 to 0.25 mL/mmol) was added under nitrogen atmosphere. The well-stirred reaction mixture was heated (temperature from 30 to 60°C) until disappearance of starting material. Phosphorus oxychloride in excess was removed under reduced pressure and the remaining oil was placed in an ice-bath. A cold saturated sodium hydrogenocarbonate solution was carefully added until neutralization. The aqueous layer was extracted with ethyl acetate, the organics were combined, dried over sodium sulfate, concentrated under reduced pressure. The residue was purified by flash chromatography on silica gel to afford the expected alkyl 2-(7-chloropyrazolo[1,5-a]pyrimidin-6-yl)acetate.

General procedure C :

To a solution of alkyl 2-(7-chloropyrazolo[1,5-a]pyrimidin-6-yl)acetate (1 equivalent) in dry DMF at -10°C was slowly added a 1N solution of LHMDS in tetrahydrofurane (1.1 to 2 equivalents). Then, the halide derivative (1.5 to 2 equivalents) was added and the reaction mixture was stirred at room temperature until disappearance of default compound. The reaction mixture was quenched by addition of a saturated solution of ammonium chloride and the mixture was extracted with ethyl acetate. The organic layer was washed with water, brine, dried over sodium sulfate and concentrated under reduced pressure. The residue was purified by flash chromatography on silica gel to afford the expected product.

General procedure D :

To a sonicated solution of alkyl 2-(7-chloropyrazolo[1,5-a]pyrimidin-6-yl)-2-alkyl-acetate (1 equivalent) and arylboronic acid (1.5 to 3 equivalents) in a mixture of water/DME (1/3) were added palladiumtetrakistriphenylphosphine (0.1 to 0.2 equivalent) and diisopropylethylamine (2 to 4 equivalents). The solution was stirred for 20 min at 140°C under microwave irradiation. Ethyl acetate was added to the reaction mixture and the solution was washed with a 1 N hydrochloric acid solution, a 1N sodium hydrogenocarbonate and brine. The organic phase was dried over magnesium sulfate, filtered and concentrated under reduced pressure. The residue was purified by flash chromatography on silica gel to afford the expected product.

General procedure E :

To a sonicated solution of alkyl 2-(7-chloropyrazolo[1,5-a]pyrimidin-6-yl)-2-alkyl-acetate (1 equivalent) and arylboronic acid (1.5 to 3 equivalents) in a mixture of water/DME (1/3) were added palladiumtetrakistriphenylphosphine (0.1 to 0.2 equivalent) and diisopropylethylamine (3 to 4 equivalents). The reaction mixture was heated at a temperature between 80 and 140°C under inert atmosphere until disappearance of default compound. Ethyl acetate was added to the reaction mixture and the solution was washed with a 1N hydrochloric acid solution, a 1 N sodium hydrogenocarbonate and brine. The organic phase was dried over magnesium sulfate, filtered and concentrated under reduced pressure. The residue was purified by flash chromatography on silica gel to afford the expected product.

INTERMEDIATE 1 : PREPARATION OF Methyl 2-(7-hydroxy-5-methylpyrazolo[1,5-a]pyrimidin-6-yl)acetate

This intermediate was prepared according to the procedure A from dimethyl acetylsuccinate (4.1 g; 22 mmol) and 3-aminopyrazole (1.66 g; 20 mmol) in toluene (20 mL) for 18 h. The 4.2 g of the title compound (95%) was obtained as a white solid. ESI/APCI(+): 222 (M+H).

INTERMEDIATE 2 : PREPARATION OF Ethyl 2-(7-hydroxy-5-methylpyrazolo[1,5-a]pyrimidin-6-yl)acetate

This intermediate was prepared according to the procedure A from diethyl acetylsuccinate (22 mL; 110 mmol) and 3-aminopyrazole (8.3 g; 100 mmol) in toluene (100 mL) for 18 h. The 20.2 g of the title compound (86%) was obtained as a white solid. ESI/APCI(+): 236 (M+H).

INTERMEDIATE 3 : PREPARATION OF Methyl 2-(2-*tert*-butyl-7-hydroxy-5-methylpyrazolo[1,5-a]pyrimidin-6-yl)acetate

This intermediate was prepared according to the procedure A from dimethyl acetylsuccinate (3 g; 15.9 mmol) and 3-Amino-5-*tert*-butyl-1*H*-pyrazole (2.01 g; 14.4 mmol) in toluene (25 mL) for 18 h. The 3.32 g of the title compound (83%) was obtained as a white solid. ESI/APCI(+): 278 (M+H). $^1$H-NMR (DMSO-$d_6$) $\delta$ 12.08 (1H, bs); 5.96 (1 H, s); 3.60 (3H, s); 3.52 (2H, s); 2.28 (3H, s); 1.30 (9H, s).

INTERMEDIATE 4 : PREPARATION OF Methyl 2-(7-hydroxy-5-methyl-2-phenylpyrazolo[1,5-a]pyrimidin-6-yl)acetate

This intermediate was prepared according to the procedure A from dimethyl acetylsuccinate (3.92 g; 20.8 mmol) and 3-Amino-5-phenyl-1*H*-pyrazole (3 g; 18.9 mmol) in toluene (40 mL) for 18 h. The 5.33 g of the title compound (95%) was obtained as a white solid. ESI/APCI(+): 298 (M+H). ESI/APCI(-): 296 (M-H). $^1$H-NMR (DMSO-$d_6$) (ppm) $\delta$ 12.38 (1 H, bs); 7.98 (2H, d, J=6.82 Hz); 7.41-7.50 (3H, M); 6.58 (1H, s); 3.62 (3H, s); 3.57 (2H, s), 2.32 (3H, s).

INTERMEDIATE 5 : PREPARATION OF Methyl 2-(7-hydroxy-5-methyl-2-propylpyrazolo[1,5-a]pyrimidin-6-yl)acetate

This intermediate was prepared according to the procedure A from dimethyl acetylsuccinate (1.65 g; 8.8 mmol) and 3-Amino-5- *n*-propyl l-1*H*-pyrazole (1 g; 8 mmol) in toluene (8 mL) for 20 h. The 1.79 g of the title compound (85%) was obtained as a white solid. ESI/APCI(+): 264 (M+H).

INTERMEDIATE 6 : PREPARATION OF Methyl 2-(2-(furan-2-yl)-7-hydroxy-5-methylpyrazolo[1,5-a]pyrimidin-6-yl)acetate

This intermediate was prepared according to the procedure A from dimethyl acetylsuccinate (1.4 g; 7.4 mmol) and 5-(furan-2-yl)-1H-pyrazol-3-amine (1 g; 6.7 mmol) in toluene (7 mL) for 20 h. The 1.76 g of the title compound (92%) was obtained as a white solid. ESI/APCI(+): 287 (M+H).

INTERMEDIATE 7 : PREPARATION OF Methyl 2-(7-hydroxy-2-isopropyl-5-methyl-[1,2,4]triazolo[1,5-a]pyrimidin-6-yl)acetate

A mixture of 3-amino-5-isopropyl-1*H*-1,2,4-triazole (1.00 g; 7.93 mmol) and dimethylacetylsuccinate (1.85 g; 9.83 mmol) in toluene (35 mL) was heated to reflux under a Dean Stark system for 24 h. After cooling, the volatiles were removed under reduced pressure and the remaining oily residue was coevaporated several times with methanol and then crystallized in methanol. The white solid was filtered and washed with methanol to furnish 0.396 g. The filtrate was evaporated under reduced pressure, acetic acid (3 mL) was added and the solution was allowed to cristallize a second time to furnish 0.187 g of white solid. Purification of the concentrated filtrate by flash-chromatography on silica using a gradient of ethyl acetate (10 - 100%) in heptane furnished another 0.72 g of a white solid. Global yield of this step is 62%. $^1$H-NMR (400 MHz, DMSO-$d_6$) (ppm) $\delta$: 13.11 (1 H, bs); 3.61 (3H, s); 3.55 (2H,s); 3.05 (1 H, h, $J$ = 6.9 Hz); 2.31 (3 H, s); 1.28 (6 H, d, $J$ = 6.9 Hz).

INTERMEDIATE 8 : PREPARATION OF Methyl 2-(7-chloro-5-methylpyrazolo[1,5-a]pyrimidin-6-yl)acetate

This intermediate was prepared according to the procedure B from methyl 2-(7-hydroxy-5-methylpyrazolo[1,5-a]pyrimidin-6-yl)acetate (4.4 g; 20 mmol), phosphorus oxychloride (20 mL) and dimethylaniline (4 mL) for 3 days. Purification by flash chromatography on silica gel using a gradient of ethyl acetate (5 - 40%) in dichloromethane furnished 2.4 g (50%) of the title compound as a white solid.
ESI/APCI(+): 240 (M+H).

INTERMEDIATE 9 : PREPARATION OF Ethyl 2-(7-chloro-5-methylpyrazolo[1,5-a]pyrimidin-6-yl)acetate

This intermediate was prepared according to the procedure B from ethyl 2-(7-hydroxy-5-methylpyrazolo[1,5-a]pyrimidin-6-yl)acetate (11.75 g ; 50 mmol), phosphorus oxychloride (50 mL) and dimethylaniline (10 mL) for 3 days. Purification by flash chromatography on silica gel using a gradient of ethyl acetate (5 - 40%) in dichloromethane furnished 8.2 g (64%) of the title compound as a white solid. ESI/APCI(+): 240 (M+H).

INTERMEDIATE 10 : PREPARATION OF Methyl 2-(2-*tert*-butyl-7-chloro-5-methylpyrazolo[1,5-a]pyrimidin-6-yl)acetate

This intermediate was prepared according to the procedure B from methyl 2-(2-*tert*-butyl-7-hydroxy-5-methylpyrazolo[1,5-a]pyrimidin-6-yl)acetate (2.00 g ; 7.21 mmol), phosphorus oxychloride (8 mL) and dimethylaniline (1.6 mL) for 3 days. Purification by flash chromatography on silica gel using a gradient of ethyl acetate (0 - 15%) in dichloromethane furnished 1.45 g (68%) of the title compound as a yellow solid. ESI/APCI(+): 296-298 (M+H).

INTERMEDIATE 11 : PREPARATION OF Methyl 2-(7-chloro-5-methyl-2-phenylpyrazolo[1,5-a]pyrimidin-6-yl)acetate

This intermediate was prepared according to the procedure B from methyl 2-(7-hydroxy-5-methyl-2-phenylpyrazolo[1,5-a]pyrimidin-6-yl)acetate (3.00 g ; 10.09 mmol), phosphorus oxychloride (12 mL) and dimethylaniline (3 mL) for 3 days. Purification by flash chromatography on silica gel using a gradient of ethyl acetate (0 - 25%) in dichloromethane furnished 2.07 g (65%) of the title compound as a white solid. ESI/APCI(+): 317-319 (M+H).

INTERMEDIATE 12 : PREPARATION OF Methyl 2-(7-chloro-5-methyl-2-propylpyrazolo[1,5-a]pyrimidin-6-yl)acetate

This intermediate was prepared according to the procedure B from methyl 2-(7-hydroxy-5-methyl-2-propylpyrazolo[1,5-a]pyrimidin-6-yl)acetate (1.79 g; 6.8 mmol), phosphorus oxychloride (6.8 mL) and dimethylaniline (1.4 mL) for 3 days. Purification by flash chromatography on silica gel using a gradient of ethyl acetate (1 - 20%) in dichloromethane furnished 0.573 g (30%) of the title compound as a solid. ESI/APCI(+): 282 (M+H).

INTERMEDIATE 13 : PREPARATION OF Methyl 2-(7-chloro-2-(furan-2-yl)-5-methylpyrazolo[1,5-a]pyrimidin-6-yl)acetate

This intermediate was prepared according to the procedure B from methyl 2-(2-(furan-2-yl)-7-hydroxy-5-methylpyrazolo[1,5-a]pyrimidin-6-yl)acetate (2.75 g ; 9.61 mmol), phosphorus oxychloride (10 mL) and dimethylaniline (2 mL) for 3 days. Purification by flash chromatography on silica gel using a gradient of ethyl acetate (1 - 20%) in dichloromethane furnished 2.12 g (73%) of the title compound as a light yellow solid. ESI/APCI(+): 305 (M+H).

INTERMEDIATE 14 : PREPARATION OF Methyl 2-(7-chloro-2-isopropyl-5-methyl-[1,2,4]triazolo[1,5-a]pyrimidin-6-yl)acetate

A solution of methyl 2-(7-hydroxy-2-isopropyl-5-methyl-[1,2,4]-triazolo[1,5-a]pyrimidin-6-yl)acetate (0.58 g; 2.19 mmol) in phosphorus oxychloride (5 mL) was heated 100°C for 24 h. Phosphorus oxychloride in excess was removed under reduced pressure and the remaining oil was placed in an ice-bath. A cold saturated sodium hydrogenocarbonate solution was carefully added until neutralization. The aqueous layer was extracted with ethyl acetate, the organics were combined, dried over sodium sulfate, concentrated under reduced pressure. Purification by flash chromatography on silica gel using a gradient of ethyl acetate 10 - 60 %) in heptane furnished 0.403 g (65%) of the title compound as a yellow solid. ESI/APCI (+): 283 - 285 (M+H)

INTERMEDIATE 15 : PREPARATION OF Methyl 2-(7-chloro-5-methylpyrazolo[1,5-a]pyrimidin-6-yl)pentanoate

This intermediate was prepared according to the procedure C from methyl 2-(7-hydroxy-5-methylpyrazolo[1,5-a]pyrimidin-6-yl)acetate (1.8 g ; 7.8 mmol), LHMDS (8.3 mL; 8.3 mmol), 1-iodopropane (1.1 mL; 11.25 mmol) in DMF (22 mL) for 3 h. Purification by flash chromatography on silica gel using a gradient of ethyl acetate (5 - 20%) in dichloromethane furnished 1.8 g (85%) of the title compound as a brown oil. ESI/APCI(+): 282-284 (M+H).

INTERMEDIATE 16 : PREPARATION OF Ethyl 2-(7-chloro-5-methylpyrazolo[1,5-a]pyrimidin-6-yl)pentanoate

This intermediate was prepared according to the procedure C from ethyl 2-(7-hydroxy-5-methylpyrazolo[1,5-a]pyrimidin-6-yl)acetate (2.53 g; 10 mmol), LHMDS (11 mL; 11 mmol), 1-iodopropane (1.45 mL; 15 mmol) in DMF (30 mL) for 3 h. Purification by flash chromatography on silica gel using a gradient of ethyl acetate (5 - 20%) in dichloromethane furnished 2.4 g (81%) of the title compound as a brown oil. ESI/APCI(+): 296-298 (M+H).

INTERMEDIATE 17 : PREPARATION OF Methyl 2-(2-*tert*-butyl-7-chloro-5-methylpyrazolo[1,5-a]pyrimidin-6-yl)pentanoate

This intermediate was prepared according to the procedure C from methyl 2-(2-*tert*-butyl-7-chloro-5-methylpyrazolo[1,5-a]pyrimidin-6-yl)acetate (1 g ; 3.38 mmol), LHMDS (4 mL; 4 mmol), 1-iodopropane (0.50 mL; 5.12 mmol) in DMF (10 mL) for 3.5 h. Purification by flash chromatography on silica gel using a gradient of acetone (0 - 10%) in heptane furnished 0.90 g (79%) of the title compound as a yellow oil. ESI/APCI(+): 338-340 (M+H).

INTERMEDIATE 18 : PREPARATION OF Methyl 2-(7-chloro-5-methyl-2-phenylpyrazolo[1,5-a]pyrimidin-6-yl)pentanoate

This intermediate was prepared according to the procedure C from methyl 2-(7-chloro-5-methyl-2-phenylpyrazolo[1,5-a]pyrimidin-6-yl)acetate (1.50 g; 4.75 mmol), LHMDS (5.25 mL; 5.25 mmol), 1-iodopropane (0.70 mL; 7.17 mmol) in DMF (15 mL) for 3.5 h. Purification by flash chromatography on silica gel using a gradient of acetone (0 - 20%) in heptane furnished 0.90 g (41%) of the title compound as an orange oil. ESI/APCI(+): 358-360 (M+H).

INTERMEDIATE 19 : PREPARATION OF Methyl 2-(7-chloro-5-methyl-2-propylpyrazolo[1,5-a]pyrimidin-6-yl)pentanoate

This intermediate was prepared according to the procedure C from methyl 2-(7-chloro-5-methyl-2-propylpyrazolo[1,5-a]pyrimidin-6-yl)acetate (0.281 g ; 1 mmol), LHMDS (1.1 mL; 1.1 mmol), 1-iodopropane (0.146 mL; 1.5 mmol) in DMF (4 mL) for 20 h. Purification by flash chromatography on silica gel using a gradient of ethyl acetate (2 - 20%) in dichloromethane furnished 0.229 g (70%) of the title compound as an orange oil. ESI/APCI(+): 324-326 (M+H).

INTERMEDIATE 20 : PREPARATION OF Methyl 2-(7-chloro-5-methyl-2-(furan-2-yl)pyrazolo[1,5-a]pyrimidin-6-yl)pentanoate

This intermediate was prepared according to the procedure C from methyl 2-(7-chloro-5-methyl-2-(furan-2-yl)pyrazolo[1,5-a]pyrimidin-6-yl)acetate (0.305 g; 1 mmol), LHMDS (1.1 mL; 1.1 mmol), 1-iodopropane (0.146 mL; 1.5 mmol) in DMF (4 mL) for 20 h. Purification by flash chromatography on silica gel using a gradient of ethyl acetate (2 - 20%) in dichloromethane furnished 0.229 g (52%) of the title compound as an oil. ESI/APCI(+): 348-350 (M+H).

INTERMEDIATE 21 : PREPARATION OF Methyl 2-(2-*tert*-butyl-7-chloro-5-methylpyrazolo[1,5-a]pyrimidin-6-yl)-6,6,6-trifluorohexanoate

This intermediate was prepared according to the procedure C from methyl 2-(2-*tert*-butyl-7-chloro-5-methylpyrimido[1,2-b]indazol-3-yl)acetate (0.303 g; 1.02 mmol), LHMDS (1.1 mL; 1.1 mmol), 1,1,1-trifluoro-4-iodobutane (0.210 mL; 1.62 mmol) in DMF (3 mL) for 20 h. Purification by flash chromatography on silica gel using a gradient of ethyl acetate (2 - 25%) in dichloromethane furnished 0.175 g (42%) of the title compound as an oil. ESI/APCI(+): 406-408 (M+H).

INTERMEDIATE 22 : PREPARATION OF Methyl 2-(2-*tert*-butyl-7-chloro-5-methylpyrazolo[1,5-a]pyrimidin-6-yl)-3-phenylpropanoate

This intermediate was prepared according to the procedure C from methyl 2-(2-*tert*-butyl-7-chloro-5-methylpyrimido[1,2-b]indazol-3-yl)acetate (0.504 g; 1.70 mmol), LHMDS (2 mL; 2 mmol), benzylbromide (0.300 mL; 2.53 mmol) in DMF (5 mL) for 20 h. Potassium iodide (0.420 g; 2.53 mmol) was added in the mixture after adding benzylbromide. Purification by flash chromatography on silica gel using a gradient of ethyl acetate (2 - 25%) in dichloromethane furnished 0.551 g (84%) of the title compound as an orange solid. ESI/APCI(+): 368-370 (M+H). ESI/APCI(-): 366-368 (M-H).

INTERMEDIATE 23 : PREPARATION OF Methyl 2-(2-*tert*-butyl-7-chloro-5-methylpyrazolo[1,5-a]pyrimidin-6-yl)-3-methylpentanoate

This intermediate was prepared according to the procedure C from methyl 2-(2-*tert*-butyl-7-chloro-5-methylpyrimido[1,2-b]indazol-3-yl)acetate (0.401 g; 1.36 mmol), LHMDS (1.5 mL; 1.5 mmol), 2-iodobutane (0.250 mL; 2.17 mmol) in DMF (4 mL) for 20 h. Purification by flash chromatography on silica gel using a gradient of ethyl acetate (2 - 15%) in dichloromethane furnished 0.057 g (12%) of the title compound as an orange solid. ESI/APCI(+): 338-340 (M+H).

INTERMEDIATE 24 : PREPARATION OF Methyl 2-(2-*tert*-butyl-7-chloro-5-methylpyrazolo[1,5-a]pyrimidin-6-yl)-4-methoxybutanoate

This intermediate was prepared according to the procedure C from methyl 2-(2-*tert*-butyl-7-chloro-5-methylpyrimido[1,2-b]indazol-3-yl)acetate (0.500 g; 1.70 mmol), LHMDS (2 mL; 2 mmol), bromoethyl methylether (0.300 mL; 2.53 mmol) in DMF (5 mL) for 20 h. Purification by flash chromatography on silica gel using a gradient of ethyl acetate (2 - 35%) in dichloromethane furnished 0.220 g (36%) of the title compound as an orange solid. ESI/APCI(+): 354-356 (M+H).

INTERMEDIATE 25 : PREPARATION OF Ethyl 2-(3-bromo-7-chloro-5-methylpyrazolo[1,5-a]pyrimidin-6-yl)pentanoate

To a cooled solution of ethyl 2-(7-chloro-5-methylpyrazolo[1,5-a]pyrimidin-6-yl)pentanoate (0.100 g; 0.338 mmol) in dichloromethane (1.3 mL) was added N-bromosuccinimide (0.085 g; 0.478 mmol) and the reaction mixture was stirred at room temperature for 1 h. The solution was diluted with ethyl acetate (10 mL) and the resulting solution was washed with a saturated sodium hydrogenosulfate solution (10 mL), a 1M sodium hydrogenocarbonate solution (10 mL) and brine (10 mL), dried over $MgSO_4$, filtered and concentrated under reduced pressure. The 0.127 g of the crude remaining residue (98%) was used in the next step without any further purification. ESI/APCI(+): 374-376-378 (M+H).

INTERMEDIATE 26 : PREPARATION OF Methyl 2-(3-bromo-2-*tert*-butyl-7-chloro-5-methylpyrazolo[1,5-a]pyrimidin-6-yl)pentanoate

To a cooled solution of methyl 2-(2-*tert*-butyl-7-chloro-5-methylpyrazolo[1,5-a]pyrimidin-6-yl)pentanoate (0.162 g; 0.480 mmol) in dichloromethane (2 mL) was added N-bromosuccinimide (0.122 g; 0.685 mmol) and the reaction mixture was stirred at room temperature for 1 h. The solution was diluted with ethyl acetate (10 mL) and the resulting solution was washed with a saturated sodium hydrogenosulfate solution (2 x 10 mL), a 1M sodium hydrogenocarbonate solution (10 mL) and brine (10 mL), dried over $MgSO_4$, filtered and concentrated under reduced pressure. The 0.182 g of the crude remaining orange oil (91%) was used in the next step without any further purification. ESI/APCI(+): 416-418-420 (M+H).

INTERMEDIATE 27 : PREPARATION OF Ethyl 3-(2-methyl-1,3-dioxolan-2-yl)propanoate

In a flask equipped by a Dean-Stark trap, a mixture of ethyl levulinate (28.83 g; 200 mmol), ethylene glycol (37.24 g; 600 mmol) and a catalytic amount of pyridinium para-toluenesulfonic acid in toluene (200 mL) was heated to reflux . The trap was purged 3 times until the expected volume of water distilled. After cooling, the mixture was washed with a saturated sodium hydrogenocarbonate solution. The basic layer was extracted with diethylether and the organics were combined, then washed with brine and water. The organic layer was dried over sodium sulfate and concentrated under reduced pressure to afford a colorless oil. ESI/APCI(+): 189 (M+H).

INTERMEDIATE 28 : PREPARATION OF Ethyl 2-((2-methyl-1,3-dioxolan-2-yl)methyl)pentanoate

To a cooled (-78°C) solution of lithium diisopropylamide 2N in tetrahydrofurane (30 mL, 60 mmol) in THF (8 mL) was added hexamethylphosphoramide (12 mL) and the solution was stirred for 30 min. A solution of ethyl 3-(2-methyl-1,3-dioxolan-2-yl)propanoate (9.4 g; 50 mmol), in tetrahydrofurane (9 mL), was added over 30 min and stirring was continued for 1 h. Propyl iodide (6.84 mL; 70 mmol) was slowly added and the solution was allowed to warm to room temperature for 4 h. the reaction was quenched by adding a saturated ammonium chloride solution and water. The two phases were separated and the aqueous layer was extracted with ethyl acetate (50 mL) and the combined organic layers were sodium sulfate, filtered and concentrated under reduced pressure. Purification of the remaining orange oil by flash-chromatography on silica gel using a gradient of ethyl acetate (0 - 40%) in heptane furnished 9.8 g (85%) of an oil. ESI/APCI(+): 231 (M+H).

INTERMEDIATE 29 : PREPARATION OF Ethyl 4-oxo-2-propylpentanoate

To a solution of ethyl 2-((2-methyl-1,3-dioxolan-2-yl)methyl)pentanoate (9.8 g; 42.55 mmol) in hexane (106 mL) at -78°C under nitrogen atmosphere was added borontribromide (1 M in dichloromethane) (55 mL; 55 mmol) and the reaction mixture was stirred at -20°C for 2 h. Water (50 mL) and ethyl acetate (50 mL) were added to the reaction mixture and both phases were separated. The aqueous layer was extracted with ethyl acetate and organics were combined, dried over sodium sulphate, filtered and concentrated under reduced pressure. Purification by flash chromatography on silica gel using a gradient of ethyl acetate (1 - 40%) in heptane furnished 6.41 g (81%) of the title compound as a light yellow oil. ESI/APCI(+): 187 (M+H).

INTERMEDIATE 30 : PREPARATION OF 5-Amino-1,2-dimethyl-1*H*-imidazole-4-carbonitrile

To a suspension of aminomalonitrile *p*-toluenesulfonate salt (0.5 g; 1.974 mmol) in acetonitrile (9 mL) was added a 0.5M ammonia solution in tetrahydrofurane (4 mL; 2 mmol). The reaction mixture was stirred at room temperature for 2.5 h. The solids were filtered and washed with tetrahydrofurane. The filtrate was concentrated to a volume of 10 mL. Triethylorthoacetate (0.361 mL; 1.969 mmol) was added and the reaction mixture was refluxed for 1h. After cooling

to 0°C, triethylamine (0.330 mL; 2.368 mmol) and a 2M methylamine solution in tetrahydrofurane (1.2 mL; 2.400 mmol) were added. The reaction mixture was allowed to warm to room temperature and was stirred for 18 h at room temperature. The solvents were evaporated and the residue was purified by flash chromatography on silica gel using a gradient of methanol (5 - 10%) in dichloromethane to give 0.090 g (33%) of title compound as a yellow solid. ESI/APCI (+): 137 (M+H). ESI/APCI (-): 135 (M-H).

INTERMEDIATE 31 : PREPARATION OF (5-Amino-1,2-dimethyl-1*H*-imidazol-4-yl)(*p*-tolyl)methanone

To a solution of 5-amino-1,2-dimethy)-1*H*-imidazole-4-carbonitrile (0.090 g; 0.661 mmol) in tetrahydrofurane (27 mL) was added a 1 M *p*-methylphenylmagnesium bromide solution in tetrahydrofurane (3.3 mL; 3.300 mmol). After 2 h stirring at room temperature, a 3 M hydrochloric acid solution (27 mL) was added and the reaction mixture was stirred at room temperature for 18 h and 1 h more at 80 °C. The reaction mixture was then cooled to 0°C, basified with a 6 M sodium hydroxide solution (pH = 9) and extracted twice with ethyl acetate. The organic phases were combined, washed with water and brine, dried over sodium sulfate, filtered and concentrated under reduced pressure. The remaining residue was purified by flash chromatography on silica gel using a gradient of methanol (2 - 30%) in dichloromethane to give 0.077 mg (51%) of title compound as a yellow solid. ESI/APCI (+): 230 (M+H); 252 (M+Na); 481 (2M+Na).

INTERMEDIATE 32 : PREPARATION OF Ethyl N-cyanoacetimidate

To a solution of 1,1,1-triethoxyethane (3.66 mL; 20 mmol) in acetonitrile (40 mL) was add cyanamide (0.882 g; 21 mmol) and the reaction mixture was stirred at room temperature for 21 h. No reaction so the mixture was heated to reflux for 18 h. The volatiles were removed under reduced pressure and the 2.24 g (100%) of the crude white solid were dried and used without further purification. ESI/APCI(+): 113 (M+H). NMR ($^1H$) : DMSO-d6 4.24 (q, 2H, $CH_2$), 2.37 (s, 3H, $CH_3$), 1.27 (t, 3H, $CH_3$).

INTERMEDIATE 33 : PREPARATION OF N'-cyano-N-(cyanomethyl)-N-methylacetimidamide

To a solution of ethyl N-cyanoacetimidate (1.12 g; 10 mmol) in ethanol (20 mL) was add 2-(methylamino)acetonitrile (0.736 g; 10.5 mmol) and the reaction mixture was stirred at room temperature for 24 h. The volatiles were removed under reduced pressure and the 1.36 g (100%) of the crude white solid were dried and used without further purification. ESI/APCI(+): 137 (M+H).

INTERMEDIATE 34 : PREPARATION OF 4-amino-1,2-dimethyl-1H-imidazole-5-carbonitrile

To a solution of N'-cyano-N-(cyanomethyl)-N-methylacetimidamide (1.36 g; 10 mmol) in dry ethanol (50 mL) under nitrogen atmosphere was added sodium ethanolate (4.85 mL; 13 mmol) and the reaction mixture was stirred at room temperature for 18 h. After cooling, the volatiles were removed under reduced pressure and the remaining crude was purified by flash-chromatography on silica gel using a gradient of methanol (0 - 20%) in dichloromethane to afford 1.23 g (61 %) of the title compound as a light beige solid. ESI/APCI(+): 137 (M+H).

INTERMEDIATE 35 : PREPARATION OF (4-amino-1,2-dimethyl-1H-imidazol-5-yl)(p-tolyl)methanone

To a solution of 4-amino-1,2-dimethyl-1H-imidazole-5-carbonitrile (0.272 g; 2 mmol) in dry tetrahydrofurane (60 mL) under nitrogen atmosphere was slowly added a 1M p-tolylmagnesium bromide solution in tetrahydrofurane (10 mL; 10 mmol). The resulting solution was stirred at room temperature for 21 h. A 3N hydrochloric acid solution (40 mL) was added to hydrolyse the intermediate imine and the reaction mixture was heated to reflux for 2 h. The volatiles were removed under reduced pressure and the crude residue was dissolved in ethyl acetate. The well-stirred mixture was basified by adding a 3N sodium hydroxide solution. Purification by flash-chromatography on silica gel using a gradient of methanol (1 - 20%) in dichloromethane furnished 0.172 g (37 %) of the title compound as a deep yellow oil. ESI/APCI(+): 230 (M+H)

INTERMEDIATE 36 : PREPARATION OF 5-amino-1-methyl-2-propyl-1H-imidazole-4-carbonitrile

To a suspension of aminomalonitrile *p*-toluenesulfonate (1.26 g; 5 mmol) in acetonitrile was added a 0.5M solution of ammonia in tetrahydrofurane (12 mL; 6 mmol) and the reaction mixture was stirred at room temperature for 2.5 h. The suspension was filtered, washed with acetonitrile and the filtrate was concentrated until approximatively 20 mL. Trimethylorthobutyrate was added and the reaction mixture was heated to reflux for 5 h.

After cooling at 0°C, triethylamine (1.69 mL; 6 mmol) and methyl amine (2M in tetrahydrofurane) (3 mL; 6 mmol)

were added. The solution was stirred at room temperature for 21 h. The volatiles were removed under reduced pressure and the crude residue was purified by flash-chromatography on silica gel using a gradient of methanol (1 - 20%) in dichloromethane to afford 0.351 g (42%) of the title compound as a brownish solid. ESI/APCI(+): 164 (M+H)

INTERMEDIATE 37 : PREPARATION OF (5-amino-1-methyl-2-propyl-1H-imidazol-4-yl)(*p*-tolyl)methanone

To a solution of 5-amino-1-methyl-2-*n*-propyl-1H-imidazole-4-carbonitrile (0.328 g; 2 mmol) in dry tetrahydrofurane (20 mL) under nitrogen atmosphere was slowly added a 1M p-tolylmagnesium bromide solution in tetrahydrofurane (10 mL; 10 mmol). The resulting solution was stirred at room temperature for 2 h. A 3N hydrochloric acid solution (20 mL) was added to hydrolyse the intermediate imine and the reaction mixture was heated to reflux for 1 h and at room temperature for 18 h. After cooling at 0°C, the reaction mixture was basified with a 6N sodium hydroxide solution. The product was extracted with ethyl acetate (80 mL) and the organics were dried over sodium sulfate, filtered and concentrated under reduced pressure. Purification by flash-chromatography on silica gel using a gradient of methanol (1 - 20%) in dichloromethane furnished 0.180 g (35 %) of the title compound as a deep yellow oil. ESI/APCI(+): 258 (M+H)

INTERMEDIATE 38 : PREPARATION OF 5-amino-1-methyl-2-*iso*-propyl-1H-imidazole-4-carbonitrile

To a suspension of aminomalonitrile *p*-toluenesulfonate (2.53 g; 10 mmol) in acetonitrile (40 mL) was added a 0.5M solution of ammonia in tetrahydrofurane (24 mL; 12 mmol) and the reaction mixture was stirred at room temperature for 2.5 h. The suspension was filtered, washed with acetonitrile and tetrahydrofurane and the filtrate was concentrated until approximatively 40 mL. Trimethylorthobutyrate was added and the reaction mixture was heated to reflux for 21 h. After cooling at 0°C, triethylamine (3.4 mL; 12 mmol) and methyl amine (2M in tetrahydrofurane) (6 mL; 12 mmol) were added. The solution was stirred at room temperature for 21 h. The volatiles were removed under reduced pressure and the crude residue was purified by flash-chromatography on silica gel using a gradient of methanol (1 - 20%) in dichloromethane to afford 0.901 g (55 %) of the title compound as a brown solid. ESI/APCI(+): 164 (M+H)

INTERMEDIATE 39 : PREPARATION OF (5-amino-1-methyl-2-*iso*-propyl-1*H*-imidazol-4-yl)(p-tolyl)methanone

To a solution of 5-amino-1-methyl-2-isopropyl-1H-imidazole-4-carbonitrile (0.328 g; 2 mmol) in dry tetrahydrofurane (20 mL) under nitrogen atmosphere was slowly added a 1M p-tolylmagnesium bromide solution in tetrahydrofurane (10 mL; 10 mmol). The resulting solution was stirred at room temperature for 2 h. A 3N hydrochloric acid solution (20 mL) was added to hydrolyse the intermediate imine and the reaction mixture was heated to reflux for 2 h and at room temperature for 18 h. After cooling at 0°C, the reaction mixture was basified with a 6N sodium hydroxide solution. The product was extracted with ethyl acetate (80 mL) and the organics were dried over sodium sulfate, filtered and concentrated under reduced pressure. Purification by flash-chromatography on silica gel using a gradient of methanol (1 - 20%) in dichloromethane furnished 0.304 g (59 %) of the title compound as a deep yellow oil. ESI/APCI(+): 258 (M+H)

INTERMEDIATE 40 : PREPARATION OF Methyl 2-(7-hydroxy-2-isopropyl-5-methyl-[1,2,4]-triazolo[1,5-*a*]pyrimidin-6-yl)acetate

A mixture of 3-amino-5-isopropyl-1*H*-1,2,4-triazole (1 g; 7.93 mmol) and dimethyl acetylsuccinate (1.85 g; 9.83 mmol) in toluene (35 mL) was heated for 24 hours under reflux in a flask equipped with a Dean-Stark apparatus. The solvent was evaporated under reduced pressure and the remaining residue was coevaporated in methanol before crystallizing in methanol to furnish 0.396 g of the title compound as a white solid. The filtrate was evaporated, acetic acid (3 mL) was added and the solution was allowed to crystallize a second time to furnish 0.187 g of the title compound. The filtrate was concentrated under reduced pressure and the residue was purified by flash chromatography on silica gel using a gradient of ethyl acetate (20 - 100%) in heptane to furnish 0.72 g of the title compound as a white solid (62% overall yield). $^1$H-NMR (400 MHz, DMSO-$d_6$) (ppm) $\delta$: 13.11 (bs, 1H, OH); 3.61 (s, 3H, $OCH_3$); 3.55 (s, 2H, $CH_2$); 3.05 (m, 1H, $CH(CH_3)_2$); 2.31 (s, 3H, $CH_3$); 1.28 (d, $J$ = 6.9 Hz, 6H, $CH(CH_3)_2$).

INTERMEDIATE 41 : PREPARATION OF Methyl 2-(7-chloro-2-isopropyl-5-methyl-[1,2,4]-triazolo[1,5-a]pyrimidin-6-yl)acetate

A stirred solution of methyl 2-(7-hydroxy-2-isopropyl-5-methyl-[1,2,4]-triazolo[1,5-a]pyrimidin-6-yl)acetate (0.50 g; 1.89 mmol) in phosphorus oxychloride (5 mL; 53.6 mmol) was heated at 105°C for 18 hours. Dimethylaniline (1 mL; 7.89 mmol) was then added and the solution was stirred for 3 hours at 105°C. After cooling, the volatiles were removed under reduced pressure and the residue was dissolved in dichloromethane (15 mL). The organic solution was successively washed with a 1 N solution of sodium hydroxide (2 x 10 mL), a 1 N solution of hydrochloric acid (10 mL) and brine (2 x

15 mL). The organic layer was dried over magnesium sulphate, filtered and concentrated under reduced pressure. Purification by flash chromatography on silica gel using a gradient of ethyl acetate (10 - 60%) in heptane furnished 0.325 g (61%) of the title compound as a beige solid. ESI/APCI (+): 283 - 285 (M+H).

INTERMEDIATE 42 : PREPARATION OF 2-methoxy-4-methylaniline

To a solution of 2-methoxy-4-methyl-1-nitrobenzene (5 g; 29.9 mmol) in methanol (200 mL) was added tin(II)chloride dihydrate (33.7 g; 150 mmol) and the mixture was heated under reflux for 3 hours. The solvent was removed under reduced pressure, the residue was dissolved in ethyl acetate and a saturated solution of sodium hydrogenocarbonate was added until a basic pH was reached. The suspension was filtered over a plug of celite, the organic phase was separated, washed with a saturated solution of sodium hydrogenocarbonated, dried over magnesium sulphate and concentrated under reduced pressure. The residue was used as such in the next reaction.

INTERMEDIATE 43 : PREPARATION OF 1-bromo-2-methoxy-4-methylbenzene

To a solution of copper(II)bromide (6.35 g; 28.4 mmol) in acetonitrile (25 mL) was added tert-butyl nitrite (2.85 ml; 24.06 mmol) and the mixture was heated at 65°C under nitrogen atmosphere. A solution of 2-methoxy-4-methylaniline (3 g, 21,87 mmol) in 25 ml acetonitrile was added carefully and the mixture was stirred for 20 min at 65 °C. The solvent was removed under reduced pressure, the residue was dissolved in ethyl acetate and washed with a 5% solution of ammonia, water, a solution of EDTA, water and brine. The organic layer was dried over sodium sulphate, filtered and concentrated under reduced pressure. Purification by flash chromatography on silica gel using a gradient of ethyl acetate (2 - 50%) in heptane furnished 2.32 g (53%) of the title compound. $^1$H-NMR (300 MHz, $CDCl_3$) (ppm) $\delta$: 6.63-6.60 (m, 3H, 3 x H*arom.*); 3.82 (s, 3H, $OCH_3$); 2.26 (s, 3H, $CH_3$).

INTERMEDIATE 44 : PREPARATION OF 2-methoxy-4-methylphenylboronic acid

To a cooled (-78°C) solution of 1-bromo-2-methoxy-4-methylbenzene (1.67 g; 8.31 mmol) in dry THF (40 mL) under argon atmosphere, was added dropwise a *tert*-butyllithium solution 1.5M in pentane (12.18 ml; 18,27 mmol). After 10 min, trimethyl borate (1.415 mL; 12.46 mmol) was added dropwise as a neat liquid and the reaction was stirred at -78°C for 1 hour. The reaction mixture was allowed to warm up to room temperature and stirring was carried on for an additional hour. The mixture was quenched with a saturated solution of ammonium chloride and the organic volatiles were removed under reduced pressure. The residue was acidified with a 2N solution of hydrochloric acid and the mixture was extracted with dichloromethane. The extract was washed with brine, dried over magnesium sulphate, filtered and concentrated under reduced pressure. The crude product was precipitated out off a DCM/heptane solution, washed with heptane and dried under high vacuum to give 0.2 g (14.5%) of the title compound as a off-white solid. $^1$H-NMR (300 MHz, $CDCl_3$) (ppm) $\delta$: 7.57 (d, $J$ = 8.1 Hz, 1 H, $H^6$); 6.86 (s, 1 H, $H^3$); 6.79 (d, $J$ = 8.1 Hz, 1 H, $H^5$); 3.99 (s, 3H, $OCH_3$); 2.40 (s, 3H, $CH_3$).

INTERMEDIATE 45 : PREPARATION OF Methyl 2-(7-hydroxy-2-isopropyl-5-methyl-[1,2,4]-triazolo[1,5-*a*]pyrimidin-6-yl)pentanoate

A mixture of diethyl 2-acetyl-3-propylsuccinate (1.50 g; 5.81 mmol) and 3-amino-5-isopropyl-1,2,4-triazole (0.50 g; 3.96 mmol) in toluene (20 mL) was heated under reflux for 4 days in a flask equipped with a Dean-Stark apparatus. After cooling, the solvent was evaporated and diethyl ether (5 mL) was added and the solution was cooled at 4°C for a few hours. The formed white precipitate was filtered and then dissolved in THF (3.75 mL). A 5% solution of sodium hydroxide (1.25 mL; 1.56 mmol) was added and the reaction mixture was stirred at room temperature for 24 hours. Solid sodium hydroxide (0.100 g; 2.50 mmol) was added and the reaction mixture was heated to 75°C for 18 hours. The solvent was evaporated and a 1 N solution of hydrochloric solution was added to the residue. The aqueous solution was extracted with ethyl acetate and the combined organic phases were washed with brine, dried over magnesium sulphate, filtered and concentrated under reduced pressure. The crude material was dissolved in methanol (2 mL) and thionyl chloride (0.050 mL; 0.685 mmol) was added. The solution was stirred at room temperature for 40 hours. The volatiles were removed under reduced pressure, the residue was dissolved in ethyl acetate, washed with a 1 N solution of hydrochloric acid and brine. The organic layer was dried over magnesium sulphate, filtered and concentrated under reduced pressure to yield 0.133 g (11%) of the title compound as a pale oil. ESI/APCI (+): 307 (M+H). ESI/APCI (-): 305 (M-H)

INTERMEDIATE 46 : PREPARATION OF Methyl 2-(7-chloro-2-isopropyl-5-methyl-[1,2,4]-triazolo[1,5-*a*]pyrimidin-6-yl)pentanoate

To a solution of methyl 2-(7-hydroxy-2-isopropyl-5-methyl-[1,2,4]triazolo[1,5-*a*]pyrimidin-6-yl)pentanoate (0.133 g; 0.434 mmol) in phosphorus oxychloride (2 mL) was added dimethylaniline (0.050 mL; 0.394 mmol) and the solution was stirred at 45°C for 3 days and at 110°C for an additional 24 hours. After cooling, the solvent was evaporated and a 1 N solution of sodium hydroxide was added to the residue. The product was extracted with ethyl acetate and the combined organics were washed with a 1N solution of hydrochloric acid, brine, dried over magnesium sulphate, filtered and concentrated under reduced pressure. The title compound (0.122 g, 87%) was isolated as a brownish oil. ESI/APCI (+): 325-327 (M+H).

INTERMEDIATE 47 : PREPARATION OF Ethyl 2-(2-benzyl-7-hydroxy-5-methyl-[1,2,4]-triazolo[1,5-*a*]pyrimidin-6-yl)pentanoate

3-amino-5-benzyl-1*H*-1,2,4-triazole (0.700 g; 4.02 mmol) and diethyl 2-acetyl-3-propylsuccinate (1.50 g; 5.81 mmol) were dissolved in toluene (20 mL) and the solution was heated for 4 days under reflux in a flask equipped with a Dean-Stark apparatus. The solvent was evaporated under reduced pressure. Purification by flash chromatography on silica gel using a gradient of methanol (0 - 3%) in dichloromethane furnished 0.571 g (39%) of the title compound as a white foam. ESI/APCI (+): 369 (M+H)

INTERMEDIATE 48 : PREPARATION OF Ethyl 2-(2-benzyl-7-chloro-5-methyl-[1,2,4]-triazolo[1,5-*a*]pyrimidin-6-yl)pentanoate

To a solution of ethyl 2-(-2benzyl-7-hydroxy-5-methyl-[1,2,4]triazolo[1,5-a]pyrimidin-6-yl)pentanoate (0.571 g; 1.55 mmol) in phosphorus oxychloride (7 mL) was added dimethylaniline (0.200 mL; 1.58 mmol) and the solution was stirred at 110°C for 5 hours. After cooling, the excess of phosphorus oxychloride was removed and ethyl acetate was added to the residue. The solution was washed with a 1N solution of sodium hydroxide. The aqueous layer was extracted with ethyl acetate and the combined organic layers were washed with a 1 N solution of hydrochloric acid, brine, dried over magnesium sulphate, filtered and concentrated under reduced pressure. Purification by flash chromatography on silica gel using a gradient of ethyl acetate (0 - 50%) in heptane furnished 0.390 g (65%) of the title compound as a colorless oil. ESI/APCI (+): 387 - 389 (M+H)

**EXAMPLE 1: PREPARATION OF Ethyl 2-(7-((R)-3-(4-chlorophenylsulfonamido)piperidin-1-yl)-5-methylpyrazolo[1,5-a]pyrimidin-6-yl)pentanoate**

To a suspension of ethyl 2-(7-chloro-5-methylpyrazolo[1,5-a]pyrimidin-6-yl)pentanoate (0.295 g; 1 mmol) in dry toluene (1 mL) were added (R)-3-Bocaminopiperidine (0.4 g ; 2 mmol), diisopropylethylamine (0.331 mL ; 2 mmol) and the reaction mixture was heated at 90°C for 18 h in a seal-tube. After cooling, the volatiles were removed under reduced pressure and the remaining residue was purified by flash chromatography on silica gel using a gradient of ethyl acetate (2 - 50%) in dichloromethane to furnish 0.413 g (93%) of ethyl 2-(7-((R)-3-(tert-butoxycarbonylamino)piperidin-1-yl)-5-methylpyrazolo[1,5-a]pyrimidin-6-yl)pentanoate as a yellow oil. ESI/APCI(+): 460 (M+H).

The last ethyl 2-(7-((R)-3-(tert-butoxycarbonylamino)piperidin-1-yl)-5-methylpyrazolo[1,5-a]pyrimidin-6-yl)pentanoate (0.378 g, 0.82 mmol) was dissolved in dichloromethane (4 mL) and trifluoroacetic acid (1 mL) was added dropewise. After 30 min stirring, toluene (5 ml) was added and the volatiles were removed under reduced pressure. The remaining residue was dissolved in a mixture of dichloromethane-triethylamine (1:1, 6 mL) and para-chlorobenzensulfonyl chloride (0.211 g; 1 mmol) was added. The reaction mixture was stirred at room temperature for 1 h. The mixture was quenched by adding a saturated ammonium chloride solution, the aqueous layer was extracted with dichloromethane. Organic layer was concentrated and the residue was purified by flash chromatography on silica gel using a gradient of ethyl acetate (2 - 50%) in heptane to furnish 0.380 g (87%) of title compound as an oil. ESI/APCI(+): 534-536 (M+H).

**EXAMPLE 2 : PREPARATION OF Ethyl 2-(7-((S)-3-(4-chlorophenylsulfonamido)piperidin-1-yl)-5-methylpyrazolo[1,5-a]pyrimidin-6-yl)pentanoate**

To a suspension of ethyl 2-(7-chloro-5-methylpyrazolo[1,5-a]pyrimidin-6-yl)pentanoate (0.295 g ; 1 mmol) in dry toluene (1 mL) were added (S)-3-Bocaminopiperidine (0.4 g ; 2 mmol), diisopropylethylamine (0.331 mL ; 2 mmol) and the reaction mixture was heated at 90°C for 3 h in a seal-tube. After cooling, the volatiles were removed under reduced pressure and the remaining residue was purified by flash chromatography on silica gel using a gradient of ethyl acetate (2 - 50%) in dichloromethane to furnish 0.401 g (87%) of ethyl 2-(7-((S)-3-(tert-butoxycarbonylamino)piperidin-1-yl)-5-methylpyrazolo[1,5-a]pyrimidin-6-yl)pentanoate as a yellow oil. ESI/APCI(+): 460 (M+H).

The last ethyl 2-(7-((S)-3-(tert-butoxycarbonylamino)piperidin-1-yl)-5-methylpyrazolo[1,5-a]pyrimidin-6-yl)pentanoate (0.368 g, 0.80 mmol) was dissolved in dichloromethane (4 mL) and trifluoroacetic acid (1 mL) was added dropewise. After 30 min stirring, toluene (5 ml) was added and the volatiles were removed under reduced pressure. The remaining residue was dissolved in a mixture of dichloromethane-triethylamine (1:1, 6 mL) and para-chlorobenzensulfonyl chloride (0.211 g; 1 mmol) was added. The reaction mixture was stirred at room temperature for 1 h. The mixture was quenched by adding a saturated ammonium chloride solution, the aqueous layer was extracted with dichloromethane. Organic layer was concentrated and the residue was purified by flash chromatography on silica gel using a gradient of ethyl acetate (2 - 50%) in heptane to furnish 0.280 g (65%) of title compound as an oil. ESI/APCI(+): 534-536 (M+H).

**EXAMPLE 3 : PREPARATION OF Ethyl 2-(7-((R)-3-(4-chlorophenylsulfonamido)pyrrolidin-1-yl)-5-methylpyrazolo[1,5-a]pyrimidin-6-yl)pentanoate**

To a suspension of ethyl 2-(7-chloro-5-methylpyrazolo[1,5-a]pyrimidin-6-yl)pentanoate (0.281 g ; 1 mmol) in dry toluene (1 mL) were added (R)-3-Bocaminopyrrolidine (0.370 g ; 2 mmol), diisopropylethylamine (0.414 mL ; 2.5 mmol) and the reaction mixture was heated at 90°C for 3 h in a seal-tube. After cooling, the volatiles were removed under reduced pressure and the remaining residue was purified by flash chromatography on silica gel using a gradient of ethyl acetate (2 - 50%) in dichloromethane to furnish 0.394 g (92%) of ethyl 2-(7-((R)-3-(tert-butoxycarbonylamino)pyrrolidin-1-yl)-5-methylpyrazolo[1,5-a]pyrimidin-6-yl)pentanoate as a yellow oil. ESI/APCI(+): 446 (M+H).

The last ethyl 2-(7-((R)-3-(tert-butoxycarbonylamino)pyrrolidin-1-yl)-5-methylpyrazolo[1,5-a]pyrimidin-6-yl)pentanoate (0.394 g, 0.92 mmol) was dissolved in dichloromethane (4 mL) and trifluoroacetic acid (1 mL) was added dropewise. After 30 min stirring, toluene (5 ml) was added and the volatiles were removed under reduced pressure. The remaining residue was dissolved in a mixture of dichloromethane-triethylamine (1:1, 6 mL) and para-chlorobenzensulfonyl chloride (0.211 g; 1 mmol) was added. The reaction mixture was stirred at room temperature for 1 h. The mixture was quenched by adding a saturated ammonium chloride solution, the aqueous layer was extracted with dichloromethane. Organic layer was concentrated and the residue was purified by flash chromatography on silica gel using a gradient of ethyl acetate (2 - 50%) in heptane to furnish 0.316 g (67%) of title compound as an oil. ESI/APCI(+): 520-522 (M+H).

**EXAMPLE 4 : PREPARATION OF Ethyl 2-(7-((S)-3-(4-chlorophenylsulfonamido)pyrrolidin-1-yl)-5-methylpyrazolo[1,5-a]pyrimidin-6-yl)pentanoate**

To a suspension of ethyl 2-(7-chloro-5-methylpyrazolo[1,5-a]pyrimidin-6-yl)pentanoate (0.281 g ; 1 mmol) in dry toluene (1 mL) were added (S)-3-Bocaminopyrrolidine (0.370 g ; 2 mmol), diisopropylethylamine (0.414 mL ; 2.5 mmol) and the reaction mixture was heated at 90°C for 3 h in a seal-tube. After cooling, the volatiles were removed under reduced pressure and the remaining residue was purified by flash chromatography on silica gel using a gradient of ethyl acetate (2 - 50%) in dichloromethane to furnish 0.402 g (93%) of ethyl 2-(7-((S)-3-(tert-butoxycarbonylamino)pyrrolidin-1-yl)-5-methylpyrazolo[1,5-a]pyrimidin-6-yl)pentanoate as a yellow oil. ESI/APCI(+): 446 (M+H).

The last ethyl 2-(7-((S)-3-(tert-butoxycarbonylamino)pyrrolidin-1-yl)-5-methylpyrazolo[1,5-a]pyrimidin-6-yl)pentanoate (0.394 g, 0.92 mmol) was dissolved in dichloromethane (4 mL) and trifluoroacetic acid (1 mL) was added dropewise. After 30 min stirring, toluene (5 ml) was added and the volatiles were removed under reduced pressure. The remaining residue was dissolved in a mixture of dichloromethane-triethylamine (1:1, 6 mL) and para-chlorobenzensulfonyl chloride (0.211 g; 1 mmol) was added. The reaction mixture was stirred at room temperature for 1 h. The mixture was quenched by adding a saturated ammonium chloride solution, the aqueous layer was extracted with dichloromethane. Organic layer was concentrated and the residue was purified by flash chromatography on silica gel using a gradient of ethyl acetate (2 - 50%) in heptane to furnish 0.300 g (64%) of title compound as an oil. ESI/APCI(+): 520-522 (M+H).

**EXAMPLE 5 : PREPARATION OF methyl 2-(5-methyl-7-phenylpyrazolo[1,5-a]pyrimidin-6-yl)pentanoate**

This intermediate was prepared according to the procedure D from methyl 2-(7-chloro-5-methyl-2-phenylpyrazolo[1,5-a]pyrimidin-6-yl)pentanoate (0.281 g ; 1 mmol), phenylboronic acid (0.366 mg ; 3 mmol), tetrakistriphenylphosphine palladium (0.231 mg ; 0.2 mmol) and diisopropylethylamine (0.663 mL ; 4 mmol) in DME-water (3 :1 ; 4 mL) at 130°C for 20 min. Purification by flash chromatography on silica gel using a gradient of ethyl acetate (2 - 50%) in dichloromethane furnished 0.266 g (82%) of the title compound as an oil contaminated with an impurity. ESI/APCI(+): 324 (M+H).

**EXAMPLE 6 : PREPARATION OF methyl 2-(5-methyl-7-*p*-tolylpyrazolo[1,5-a]pyrimidin-6-yl)pentanoate**

This intermediate was prepared according to the procedure D from methyl 2-(7-chloro-5-methyl-2-phenylpyrazolo[1,5-a]pyrimidin-6-yl)pentanoate (0.281 g ; 1 mmol), *p*-tolylboronic acid (0,268 g; 2 mmol), tetrakistriphenylphosphine palladium (0.231 mg ; 0.2 mmol) and diisopropylethylamine (0.414 mL ; 2.5 mmol) in DME-water (3 :1 ; 4 mL) at 140°C for 40 min. Purification by flash chromatography on silica gel using a gradient of ethyl acetate (2 - 50%) in dichloromethane furnished 0.337 g (82%) of the title compound as an oil contaminated with an impurity. ESI/APCI(+): 338 (M+H).

**EXAMPLE 7 : PREPARATION OF Methyl 2-(2-*tert*-butyl-5-methyl-7-phenylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.202 g; 0.598 mmol), phenylboronic acid (0.146 g; 1.20 mmol), tetrakistriphenylphosphine palladium (0.092 g ; 0.079 mmol) and diisopropylethylamine (0.350 mL ; 2 mmol) in DME-water (3:1 ; 2.5 mL) at 80°C for 18 h. Purification by flash chromatography on silica gel using a gradient of ethyl acetate (2 - 50%) in dichloromethane furnished 0.141 g (62%) of the title compound as an oil contaminated with an impurity. ESI/APCI(+): 380 (M+H).

**EXAMPLE 8 : PREPARATION OF Methyl 2-(2-*tert*-butyl-5-methyl-7-*p*-tolylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.152 g; 0.450 mmol), p-tolylboronic acid (0.120 g; 0.833 mmol), tetrakistriphenylphosphine palladium (0.080 g ; 0.069 mmol) and diisopropylethylamine (0.250 mL; 1.44 mmol) in DME-water (3 :1 ; 1.7 mL) at 140°C for 40 h. Purification by flash chromatography on silica gel using a gradient of ethyl acetate (0 - 40%) in dichloromethane furnished 0.126 g (71%) of the title compound as an oil contaminated with an impurity. ESI/APCI(+): 394 (M+H).

**EXAMPLE 9 : PREPARATION OF Methyl 2-(5-methyl-2-phenyl-7-*p*-tolylpyrazolo[1,5-a]pyrimidin-6-yl)pentanoate**

This intermediate was prepared according to the procedure E from methyl 2-(7-chloro-5-methyl-2-phenylpyrazolo[1,5-a]pyrimidin-6-yl)pentanoate (0.100 g; 0.279 mmol), *p*-tolylboronic acid (0.075 g; 0.552 mmol), tetrakistriphenylphosphine palladium (0.069 g ; 0.059 mmol) and diisopropylethylamine (0.200 mL ; 1.15 mmol) in DME-water (3:1 ; 1 mL) at 140°C for 40 h. Purification by flash chromatography on silica gel using a gradient of ethyl acetate (0 - 40%) in dichloromethane furnished 0.109 g (94%) of the title compound as an oil contaminated with an impurity. ESI/APCI(+): 414 (M+H).

**EXAMPLE 10 : PREPARATION OF Methyl 2-(2-*tert*-butyl-7-(3-hydroxyphenyl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.171 g; 0.506 mmol), *p*-tolylboronic acid (0.075 g; 0.552 mmol), tetrakistriphenylphosphine palladium (0.067 g ; 0.058 mmol) and diisopropylethylamine (0.300 mL ; 1.72 mmol) in DME-water (3 :1 ; 2 mL) at 80°C for 4 days. Purification by flash chromatography on silica gel using a gradient of ethyl acetate (0 - 40%) in dichloromethane furnished 0.172 g (86%) of the title compound as an oil contaminated with an impurity. ESI/APCI(+): 396 (M+H).

**EXAMPLE 11 : PREPARATION OF Methyl 2-(2-*tert*-butyl-7-(2-naphthyl)-5-methylpyrazolo[1,5-a]pyrimidin-6-yl)pentanoate**

This intermediate was prepared according to the procedure E from methyl 2-(2-tert-butyl-7-chloro-5-methylpyrazolo[1,5-a]pyrimidin-6-yl)pentanoate (0.111 g; 0.328 mmol), 2-naphthylboronic acid (0.106 g; 0.616 mmol), tetrakistriphenylphosphine palladium (0.042 g ; 0.039 mmol) and diisopropylethylamine (0.220 mL ; 1.26 mmol) in DME-water (3 :1 ; 2.5 mL) at 90°C for 48 h. Purification by flash chromatography on silica gel using a gradient of ethyl acetate (0 - 20%) in dichloromethane furnished 0.143 g (100%) of the title compound as an oil contaminated with an impurity. ESI/APCI(+): 430 (M+H).

**EXAMPLE 12 : PREPARATION OF Methyl 2-(2-*tert*-butyl-7-(1*H*-indol-5-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.106 g; 0.313 mmol), 1*H*-indol-5-ylboronic acid (0.106 g; 0.659 mmol), tetrakistriphenylphosphine palladium (0.039 g ; 0.033 mmol) and diisopropylethylamine (0.220 mL ; 1.26 mmol) in DME-water (3 :1 ; 2.5 mL) at 90°C for 48 h. Purification by flash chromatography on silica gel using a gradient of ethyl acetate (0 - 35%) in dichloromethane furnished 0.129 g (98%) of the title compound as a yellow foam contaminated with an impurity. ESI/APCI(+): 419 (M+H).

**EXAMPLE 13 : PREPARATION OF Methyl 2-(2-*tert*-butyl-7-(1*H*-indol-6-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.100 g; 0.295 mmol), 1*H*-indol-6-ylboronic acid (0.102 g; 0.634 mmol), tetrakistriphenylphosphine palladium (0.041 g ; 0.035 mmol) and diisopropylethylamine (0.220 mL; 1.26 mmol) in DME-water (3 :1 ; 2.5 mL) at 90°C for 48 h. Purification by flash chromatography on silica gel using a gradient of ethyl acetate (0 - 35%) in dichloromethane furnished 0.104 g (84%) of the title compound as a yellow foam contaminated with an impurity. ESI/APCI(+): 419 (M+H).

**EXAMPLE 14 : PREPARATION OF Methyl 2-(2-*tert*-butyl-7-(1-benzofuran-5-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.100 g; 0.296 mmol), 5-(4,4,5,5-tetramethyl-1,3,2-dioxaborolan-2-yl)-1-benzofuran (0.152 g; 0.623 mmol), tetrakistriphenylphosphine palladium (0.039 g ; 0.034 mmol) and diisopropylethylamine (0.220 mL ; 1.26 mmol) in DME-water (3 :1 ; 2 mL) at 90°C for 48 h. Purification by flash chromatography on silica gel using a gradient of ethyl acetate (0 - 15%) in dichloromethane furnished 0.114 g (92%) of the title compound as a yellow oil contaminated with an impurity. ESI/APCI(+): 420 (M+H).

**EXAMPLE 15 : PREPARATION OF Methyl 2-(2-*tert*-butyl-7-(1-benzothiophen-5-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.83 g; 0.244 mmol), 2-(1-benzothiophen-5-yl)-4,4,5,5-tetra-methyl-1,3,2-dioxaborolane (0.120 g; 0.461 mmol), tetrakistriphenylphosphine palladium (0.039 g ; 0.034 mmol) and diisopropylethylamine (0.220 mL ; 1.26 mmol) in DME-water (3 :1 ; 2 mL) at 90°C for 48 h. Purification by flash chromatography on silica gel using a gradient of ethyl acetate (0 - 15%) in dichloromethane furnished 0.102 g (96%) of the title compound as a yellow oil contaminated with an impurity. ESI/APCI(+): 436 (M+H).

**EXAMPLE 16 : PREPARATION OF Methyl 2-(2-*tert*-butyl-7-(2,3-dihydrobenzofuran-5-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.097 g; 0.287 mmol), 2,3-dihydroxbenzofuran-5-ylboronic acid (0.096 g; 0.585 mmol), tetrakistriphenylphosphine palladium (0.042 g ; 0.036 mmol) and diisopropylethylamine (0.220 mL; 1.26 mmol) in DME-water (3 :1 ; 2.5 mL) at 90°C for 48 h. Purification by flash chromatography on silica gel using a gradient of ethyl acetate (0 - 35%) in dichloromethane furnished 0.096 g (79%) of the title compound as a yellow oil contaminated with an impurity. ESI/APCI(+): 422 (M+H).

**EXAMPLE 17 : PREPARATION OF Methyl 2-(2-*tert*-butyl-7-(4-chlorophenyl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyimidin-6-yl)pentanoate (0.104 g; 0.302 mmol), 4-chlorophenylboronic acid (0.105 g; 0.671 mmol), tetrakistriphenylphosphine palladium (0.066 g ; 0.057 mmol) and diisopropylethylamine (0.220 mL; 1.26 mmol) in DME-water (3 :1 ; 2.5 mL) at 90°C for 18 h. The crude material was used in the next step without any further purification. ESI/APCI(+): 414-416 (M+H).

**EXAMPLE 18 : PREPARATION OF Methyl 2-(2-*tert*-butyl-7-(3,4-dimethylphenyl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.104 g; 0.308 mmol), 3,4-dimethylphenylboronic acid (0.087 g; 0.580 mmol), tetrakistriphenylphosphine palladium (0.057 g ; 0.049 mmol) and diisopropylethylamine (0.220 mL ; 1.26 mmol) in DME-water (3 :1 ; 2.5 mL) at 90°C for 18 h. The crude material was used in the next step without any further purification. ESI/APCI(+): 408 (M+H).

**EXAMPLE 19 : PREPARATION OF Methyl 2-(2-*tert*-butyl-7-(4-ethylphenyl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.108 g; 0.320 mmol), 4-ethylphenylboronic acid (0.090 g; 0.600 mmol), tetrakistriphenylphosphine palladium (0.066 g ; 0.057 mmol) and diisopropylethylamine (0.220 mL ; 1.26 mmol) in DME-water (3 :1 ; 2.5 mL) at 90°C for 18 h. The crude material was used in the next step without any further purification. ESI/APCI(+): 408 (M+H).

**EXAMPLE 20 : PREPARATION OF Methyl 2-(2-*tert*-butyl-7-(3,4-dihydro-2*H*-benzo[*b*][1,4]dioxepin-7-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.098 g; 0.290 mmol), 3,4-dihydro-2*H*-benzo[*b*][1,4]dioxepin-7-yl-boronic acid (0.121 g; 0.624 mmol), tetrakistriphenylphosphine palladium (0.034 g ; 0.029 mmol) and diisopropylethylamine (0.220 mL ; 1.26 mmol) in DME-water (3 :1 ; 2.5 mL) at 90°C for 48 h. Purification by flash chromatography on silica gel using a gradient of ethyl acetate (0 - 15%) in dichloromethane furnished 0.114 g (87%) of the title compound as a yellow oil contaminated with an impurity. ESI/APCI(+): 452 (M+H).

**EXAMPLE 21 : PREPARATION OF Methyl 2-(2-*tert*-butyl-7-(2-(7-(4-methyl-3,4-dihydro-2H-benzo[*b*][1,4]oxazin-7-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.068 g; 0.201 mmol), 4-methyl-7-(4,4,5,5-tetramethyl-1,3,2-dioxaborolan-2-yl)-3,4dihydro-2*H*-benzo[*b*][1,4]oxazine (0.112 g; 0.407 mmol), tetrakistriphenylphosphine palladium (0.034 g ; 0.030 mmol) and diisopropyl ethylamine (0.220 mL ; 1.26 mmol) in DME-water (3 :1 ; 2 mL) at 90°C for 48 h. Purification by flash chromatography on silica gel using a gradient of ethyl acetate (0 - 35%) in dichloromethane furnished 0.071 g (78%) of the title compound as a yellow oil contaminated with an impurity. ESI/APCI(+): 451 (M+H).

**EXAMPLE 22 : PREPARATION OF Ethyl 2-(3-bromo-5-methyl-7-*p*-tolylpyrazolo[1,5-a]pyrimidin-6-yl)pentanoate**

To a solution of ethyl 2-(3-bromo-7-chloro-5-methylpyrazolo[1,5-a]pyimidin-6-yl)pentanoate (0.509 g; 1.36 mmol) in dry tetrahydrofurane (15 mL) was added *p*-tolylmagnesium bromide (3.2 mL ; 2.36 mmol). The reaction mixture was stirred under nitrogen atmosphere at room temperature for 3 h. The organic solution was poured with a saturated ammonium chloride solution (50 mL) and water (5 mL) was added to the mixture and the product was extracted with ethyl acetate (45 mL). The organic phase was washed with brine (40 mL), dried over magnesium sulfate, filtered and concentrated under reduced pressure. Purification by flash chromatography on silica gel using a gradient of ethyl acetate (0 - 15%) in heptane furnished 0.381 g (65%) of the title compound as a pale yellow oil contaminated with an impurity. ESI/APCI (+): 430 - 432 (M+H)

**EXAMPLE 23 : PREPARATION OF Ethyl 2-(5-methyl-3-phenyl-7-*p*-tolylpyrazolo[1,5-a]pyrimidin-6-yl)pentanoate**

This intermediate was prepared according to the procedure E from ethyl 2(3-bromo-5-methyl-7-*p*-tolylpyrazolo[1,5-a]pyrimidin-6-yl)pentanoate (0.070 g; 0.163 mmol), phenylboronic acid (0.045 g; 0.372 mmol), tetrakistriphenylphosphine palladium (0.023 g ; 0.020 mmol) and diisopropylethylamine (0.100 mL ; 0.574 mmol) in DME-water (3 :1 ; 1.5 mL) at 90°C for 18 h. Purification by flash chromatography on silica gel using a gradient of ethyl acetate (0 - 10%) in heptane furnished 0.050 g (72%) of the title compound as a yellow oil contaminated with an impurity. ESI/APCI(+): 428 (M+H).

**EXAMPLE 24 : PREPARATION OF Ethyl 2-(5-methyl-3,7-di-*p*-tolylpyrazolo[1,5-a]pyrimidin-6-yl)pentanoate**

This intermediate was prepared according to the procedure E from ethyl 2-(3-bromo-7-chloro-5methylpyrazolo[1,5-a]pyrimidin-6-yl)pentanoat (0.123 g; 0.328 mmol), *p*-tolylboronic acid (0.100 g; 0.736 mmol), tetrakistriphenylphosphine palladium (0.029 g ; 0.025 mmol) and diisopropylethylamine (0.240 mL; 1.38 mmol) in DME-water (3 :1 ; 1.5 mL) at 80°C for 24 h. Purification by flash chromatography on silica gel using a gradient of ethyl acetate (0 - 20%) in heptane furnished 0.086 g (61%) of the title compound as a yellow oil contaminated with an impurity. ESI/APCI(+): 442 (M+H).

**EXAMPLE 25 : PREPARATION OF Methyl 2-(3-bromo-2-*tert*-butyl-5-methyl-7-*p*-tolylpyrazolo[1,5-a]pyrimidin-6-yl)pentanoate**

To a cooled solution of methyl 2-(2-*tert*-butyl-7-chloro-5-methylpyrazolo[1,5-*a*]pyrimidin-6-yl)pentanoate (0.312 g; 0.793 mmol) in dichloromethane (5 mL) was added N-bromosuccinimide (0.214 g; 1.2 mmol) and the reaction mixture was stirred at room temperature for 1.5 h. The solution was diluted with ethyl acetate (20 mL) and the resulting solution was washed with a saturated sodium hydrogenosulfate solution (2 x 20 mL), a 1M sodium hydrogenocarbonate solution (20 mL) and brine (20 mL), dried over $MgSO_4$, filtered and concentrated under reduced pressure. The 0.369 g of the crude remaining brown sticky solid (99%) was used in the next step without any further purification. ESI/APCI(+): 472-474 (M+H). $^1$H-NMR (400 MHz, DMSO-$d_6$) (ppm) δ: 7.36 (4 H, m); 3.72 (1 H, t, *J* = 6.6 Hz); 3.64 (3 H, s); 2.48 (3 H, s); 2.42 (3 H, s); 2.01 (1 H, m); 1.60 (1 H, m); 1.33 (9 H, s); 1.01 (2 H, m), 0.63 (3 H, t, *J* = 7.2 Hz).

**EXAMPLE 26 : PREPARATION OF Methyl 2-(2-*tert*-butyl-5-methyl-3,7-di-*p*-tolylpyrazolo[1,5-a]pyrimidin-6-yl)pentanoate**

This intermediate was prepared according to the procedure E from methyl 2-(3-bromo-2-*tert*-butyl-5-methylpyrazolo[1,5-*a*]pyrimidin-6-yl)pentanoate (0.047 g; 0.112 mmol), *p*-tolylboronic acid (0.065 g; 0.479 mmol), tetrakistriphenylphosphine palladium (0.017 g ; 0.016 mmol) and diisopropylethylamine (0.120 mL; 0.689 mmol) in DME-water (3:1 ; 1 mL) at 120°C for 24 h. Purification by flash chromatography on silica gel using a gradient of ethyl acetate (0 - 12%) in heptane furnished 0.014 g (25%) of the title compound as a yellow oil contaminated with an impurity. ESI/APCI(+): 484 (M+H).

**EXAMPLE 27 : PREPARATION OF Methyl 2-(5-methyl-2-propyl-7-*p*-tolylpyrazolo[1,5-a]pyrimidin-6-yl)pentanoate**

This intermediate was prepared according to the procedure D from methyl 2-(7-chloro-5-methyl-2-propylpyrazolo[1,5-a]pyrimidin-6-yl)pentanoate (0.229 g; 0.7 mmol), 4-methylphenylboronic acid (0.192 mg ; 1.41 mmol), tetrakistriphenylphosphine palladium (0.122 mg ; 0.106 mmol) and diisopropylethylamine (0.351 mL; 2.12 mmol) in DME-water (3 :1 ; 2.8 mL) at 140°C for 20 min. Purification by flash chromatography on silica gel using a gradient of ethyl acetate (1 - 30%) in dichloromethane furnished 0.105 g (39%) of the title compound as an oil contaminated with an impurity. ESI/APCI(+): 380 (M+H).

**EXAMPLE 28 : PREPARATION OF Methyl 2-(2-(furan-2-yl)-5-methyl-7-*p*-tolylpyrazolo[1,5-a]pyrimidin-6-yl)pentanoate**

This intermediate was prepared according to the procedure D from methyl 2-(7-chloro-2-(furan-2-yl)-5-methylpyrazolo[1,5-a]pyrimidin-6-yl)pentanoate (0.182 g; 0.52 mmol), 4-methylphenylboronic acid (0.142 mg ; 1.04 mmol), tetrakistriphenylphosphine palladium (0.090 mg ; 0.078 mmol) and diisopropylethylamine (0.260 mL; 1.57 mmol) in DME-water (3 :1 ; 2.1 mL) at 140°C for 20 min. Purification by flash chromatography on silica gel using a gradient of ethyl acetate (1 - 40%) in dichloromethane furnished 0.123 g (58%) of the title compound as an oil. ESI/APCI(+): 404 (M+H).

**EXAMPLE 29 : PREPARATION OF Methyl 2-(2-*tert*-butyl-7-(4-chloro-2-fluorophenyl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-*a*]pyrimidin-6-yl)pentanoate (0.097 g; 0.287 mmol), 4-chloro-2-fluorophenylboronic acid (0.096 g; 0.551 mmol), tetrakistriphenylphosphine palladium (0.033 g ; 0.029 mmol) and diisopropylethylamine (0.200 mL; 1.15 mmol) in DME-water (3 :1 ; 2.5 mL) at 90°C for 3 days. Purification by flash chromatography on silica gel using a gradient of ethyl acetate (0 - 25%) in heptane furnished 0.101 g (81%) of the title compound as a yellow oil contaminated with an

impurity. ESI/APCI(+): 432-434 (M+H).

**EXAMPLE 30 : PREPARATION OF Methyl 2-(2-*tert*-butyl-7-(2-fluoro-4-methylphenyl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-*a*]pyrimidin-6-yl)pentanoate (0.100 g; 0.296 mmol), 2-fluoro-4-methylphenylboronic acid (0.086 g; 0.559 mmol), tetrakistriphenylphosphine palladium (0.031 g ; 0.027 mmol) and diisopropylethylamine (0.200 mL; 1.15 mmol) in DME-water (3 :1 ; 2.5 mL) at 90°C for 3 days. Purification by flash chromatography on silica gel using a gradient of ethyl acetate (0 - 25%) in heptane furnished 0.122 g (87%) of the title compound as a yellow oil contaminated with an impurity. ESI/APCI(+): 412 (M+H).

**EXAMPLE 31 : PREPARATION OF Methyl 2-(2-*tert*-butyl-7-*p*-tolyl-5-methylpyrazolo[1,5-a]pyrimidin-6-yl)-6,6,6-trifluorohexanoate**

This intermediate was prepared according to the procedure D from methyl 2-(2-*tert*-butyl-7-chloro-5-methylpyrazolo[1,5-a]pyrimidin-6-yl)-6,6,6-trifluorohexanoate (0.175 g; 0.431 mmol), p-tolylboronic acid (0.120 mg; 0.883 mmol), tetrakistriphenylphosphine palladium (0.050 g ; 0.043 mmol) and diisopropylethylamine (0.300 mL; 1.72 mmol) in DME-water (3 :1 ; 4 mL) at 140°C for 20 min. Purification by flash chromatography on silica gel using a gradient of ethyl acetate (0 - 25%) in heptane furnished 0.135 g (68%) of the title compound as a yellow oil contaminated with impurities. ESI/APCI(+): 462 (M+H).

**EXAMPLE 32 : PREPARATION OF Methyl 2-(2-*tert*-butyl-7-*p*-tolyl-5-methylpyrazolo[1,5-a]pyrimidin-6-yl)-3-phenylpropanoate**

This intermediate was prepared according to the procedure D from methyl 2-(2-*tert*-butyl-7-chloro-5-methylpyrazolo[1,5-*a*]pyrimidin-6-yl)-3-phenylpropanoate (0.150 g; 0.287 mmol), p-tolylboronic acid (0.100 mg; 0.500 mmol), tetrakistriphenylphosphine palladium (0.045 g ; 0.039 mmol) and diisopropylethylamine (0.270 mL; 1.55 mmol) in DME-water (3 :1 ; 3.5 mL) at 120°C for 20 min. Purification by flash chromatography on silica gel using a gradient of ethyl acetate (0 - 25%) in heptane furnished 0.118 g (69%) of the title compound as a yellow oil contaminated with impurities. ESI/APCI(+): 442 (M+H).

**EXAMPLE 33 : PREPARATION OF Methyl 2-(2-tert-butyl)-5-methyl-7-1-(1-metyl-1H-indol-5-yl) pyrazolo[1,5-a]pyrimidin-6-yl)pentanoate**

This intermediate was prepared according to the procedure E from methyl 2-(2-*tert*-butyl-7-chloro-5-methylpyrazolo[1,5-*a*]pyrimidin-6-yl)pentanoate (0.099 g; 0.292 mmol), 1-methyl-5-(4,4,5,5-tetramethyl-1,3,2-dioxaborolan-2-yl)-1H-indole (0.143 g; 0.556 mmol), tetrakistriphenylphosphine palladium (0.038 g; 0.033 mmol) and diisopropylethylamine (0.220 mL ; 1.26 mmol) in DME-water (3 :1 ; 2.5 mL) at 90°C for 21 h. Purification by flash chromatography on silica gel using a gradient of ethyl acetate (0 - 20%) in heptane furnished 0.097 g (77%) of the title compound as a yellow foam. ESI/APCI(+): 433 (M+H).

**EXAMPLE 34 : PREPARATION OF Methyl 2-(2-*tert*-butyl-5-methyl-7-(1-methylindolin-5-yl)pyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.079 g; 0.234 mmol), 1-methyl-5-(4,4,5,5-tetramethyl-1,3,2-dioxaborolan-2-yl)indoline-(0.099 mg; 0.382 mmol), tetrakistriphenylphosphine palladium (0.025 g ; 0.022 mmol) and diisopropylethylamine (0.160 mL ; 0.919 mmol) in DME-water (3 :1 ; 2 mL) at 90°C for 3 days. Purification by flash chromatography on silica gel using a gradient of ethyl acetate (0 - 35%) in heptane furnished 0.170 g (73%) of the title compound as a yellow oil contaminated with an impurity. ESI/APCI(+): 435 (M+H).

**EXAMPLE 35 : PREPARATION OF Methyl 2-(2-*tert*-butyl-5-methyl-7-(1-methyl-1H-indol-6-yl)pyrazolo[1,5-a]pyrimidin-6-yl)pentanoate**

This intermediate was prepared according to the procedure E from methyl 2-(2-*tert*-butyl-7-chloro-5-methylpyrazolo[1,5-a]pyrimidin-6-yl)pentanoate (0.077 g; 0.227 mmol), 1-methyl-6-(4,4,5,5-tetramethyl-1,3,2-dioxaborolan-2-yl)-1-H-indole (0.102 mg; 0.397 mmol), tetrakistriphenylphosphine palladium (0.028 g ; 0.024 mmol) and diisopropylethylamine (0.220 mL ; 1.26 mmol) in DME-water (3 :1 ; 2 mL) at 90°C for 2 days. Purification by flash chromatography on silica gel using a gradient of ethyl acetate (0 - 20%) in heptane furnished 0.079 g (80%) of the title compound as a yellow oil contaminated with an impurity. ESI/APCI(+): 433 (M+H).

**EXAMPLE 36 : PREPARATION OF Methyl 2-(2-*tert*-butyl-7-(chroman-6-yl)-5-methylpyrazolo[1,5-a]pyrimidin-6-yl)pentanoate**

This intermediate was prepared according to the procedure E from methyl 2-(2-*tert*-butyl-7-chloro-5-methylpyrazolo[1,5-*a*]pyrimidin-6-yl)pentanoate (0.075 g; 0.222 mmol), 2-(chroman-6-yl)-4,4,5,5-tetramethyl-1,3,2-dioxaborolane (0.120 mg; 0.461 mmol), tetrakistriphenylphosphine palladium (0.035 g ; 0.030 mmol) and diisopropylethylamine (0.220 mL ; 1.26 mmol) in DME-water (3 :1 ; 2 mL) at 90°C for 2 days. Purification by flash chromatography on silica gel using a gradient of ethyl acetate (0 - 15%) in heptane furnished 0.095 g (98%) of the title compound as a yellow oil contaminated with an impurity. ESI/APCI(+): 436 (M+H).

**EXAMPLE 37 : PREPARATION OF Methyl 2-(2-*tert*-butyl-7-*p*-tolyl-5-methylpyrazolo[1,5-a]pyrimidin-6-yl)-3-methylpentanoate**

This intermediate was prepared according to the procedure D from methyl 2-(2-*tert*-butyl-7-chloro-5-methylpyrazolo[1,5-*a*]pyrimidin-6-yl)-6,6,6-trifluorohexanoate (0.057 g; 0.162 mmol), *p*-tolylboronic acid (0.045 mg; 0.883 mmol), tetrakistriphenylphosphine palladium (0.020 g ; 0.017 mmol) and diisopropylethylamine (0.120 mL; 0.070 mmol) in DME-water (3:1 ; 1 mL) at 140°C for 20 min. Purification by flash chromatography on silica gel using a gradient of ethyl acetate (0 - 25%) in heptane furnished 0.051 g (77%) of the title compound as a yellow oil contaminated with impurities. ESI/APCI(+): 408 (M+H).

**EXAMPLE 38 : PREPARATION OF Methyl 2-(2-*tert*-butyl-3-chloro-7-*p*-tolyl-5-methylpyrazolo[1,5-a]pyrimidin-6-yl)pentanoate**

To a solution of methyl 2(2-*tert*-butyl-5-methyl-7-*p*-tolylpyrazolo[1,5-*a*]pyrimidin-6-yl)pentanoate (0.097 g; 0.246 mmol) in dichloromethane (1.5 mL) was added N-Chlorosuccinimide (0.050 g; 0.374 mmol) and the solution was stirred at room temperature for 4 h. Ethyl acetate (10 mL) was added to the reaction mixture and the solution was washed with a 5% sodium hydrogenosulfate solution (10 mL), a 1N sodium hydrogenocarbonate solution (10 mL) and brine (10 mL). The organic phase was dried over magnesium sulfate, filtered and concentrated under reduced pressure. Purification by flash-chromatography on silica gel using a gradient of ethyl acetate (0 - 25%) in heptane furnished 0.070 g (66%) of the title compound as a yellowish oil. ESI/APCI (+): 428 - 430 (M+H).

**EXAMPLE 39 : PREPARATION OF Methyl 2-(2-*tert*-butyl-7-*p*-tolyl-5-methylpyrazolo[1,5-a]pyrimidin-6-yl)-4-methoxybutanoate**

This intermediate was prepared according to the procedure D from methyl 2-(2-*tert*-butyl-7-chloro-5-methylpyrazolo[1,5-*a*]pyrimidin-6-yl)-4-methoxybutanoate (0.220 g; 0.622 mmol), *p*-tolylboronic acid (0.186 mg; 1.67 mmol), tetrakistriphenylphosphine palladium (0.061 g ; 0.053 mmol) and diisopropylethylamine (0.450 mL; 2.58 mmol) in DME-water (3 :1 ; 4 mL) at 140°C for 30 min. Purification by flash chromatography on silica gel using a gradient of ethyl acetate (0 - 25%) in heptane furnished 0.211 g (83%) of the title compound as a yellow oil contaminated with impurities. ESI/APCI(+): 408 (M+H).

**EXAMPLE 40 : PREPARATION OF Methyl 2-(2-*tert*-butyl-5-methyl-7-(4-*iso*-propylphenyl)pyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.205 g; 0.607 mmol), 4-isopopylphenylboronic acid (0.186 g; 1.13 mmol), tetrakistriphenylphosphine palladium (0.063 g; 0.055 mmol) and diisopropylethylamine (0.410 mL; 2.35 mmol) in DME-water (3:1 ; 4 mL) at 140°C for 30 min. Purification by flash chromatography on silica gel using a gradient of ethyl acetate (0 - 25%) in heptane furnished 0.180 g (70%) of the title compound as a yellow solid. ESI/APCI (+): 422 (M+H).

**EXAMPLE 41 : PREPARATION OF Methyl 2-(2-*tert*-butyl-5-methyl-7-(4-trifluoromethylphenyl)pyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyimidin-6-yl)-pentanoate (0.209 g; 0.619 mmol), 4-(trifluoromethyl)phenylboronic acid (0.170 g; 0.964 mmol), tetrakistriphenylphosphine palladium (0.079 g; 0.069 mmol) and diisopropylethylamine (0.410 mL; 2.35 mmol) in DME-water (3:1 ; 4 mL) at 140°C for 40 min. Purification by flash chromatography on silica gel using a gradient of ethyl acetate (0 - 25%) in heptane furnished 0.220 g (79%) of the title compound as a yellow solid. ESI/APCI (+): 448 (M+H).

**EXAMPLE 42 : PREPARATION OF Methyl 2-(2-tert-butyl-7-(2,4-difluorophenyl)-5-methylpyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.152 g; 0.450 mmol), 2,4-difluorophenylboronic acid (114 mg; 0.722 mmol), tetrakistriphenylphosphine palladium (0.052 g; 0.045 mmol) and diisopropylethylamine (0.320 mL ; 1.84 mmol) in DME-water (3 :1 ; 3.5 mL) at 140°C for 40 min. Purification by flash chromatography on silica gel using a gradient of ethyl acetate (0 - 20%) in heptane furnished 0.188 g (100 %) of the title compound as a yellow oil contaminated with an impurity. ESI/APCI (+): 416 (M+H).

**EXAMPLE 43 : PREPARATION OF Methyl 2-(2-*tert*-butyl-7-(2-chloro-4-methylphenyl)-5-methylpyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.152 g; 0.450 mmol), 2-chloro-4-methylphenylboronic acid (110 mg; 0.646 mmol), tetrakistriphenylphosphine palladium (0.052 g; 0.045 mmol) and diisopropylethylamine (0.320 mL ; 1.84 mmol) in DME-water (3 :1 ; 3.5 mL) at 140°C for 40 min. Purification by flash chromatography on silica gel using a gradient of ethyl acetate (0 - 20%) in heptane furnished 0.188 g (93 %) of the title compound as a yellow oil contaminated with an impurity. ESI/APCI (+): 428-430 (M+H).

**EXAMPLE 44 : PREPARATION OF Methyl 2-(7-(2-amino-4-methylphenyl)-2-*tert*-butyl-5-methylpyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.100 g; 0.296 mmol), 5-methyl-(2-4,4,5,5-tetramethyl-1,3,2-dioxoborolan-2-yl)aniline (100 mg; 0.429 mmol), tetrakistriphenylphosphine palladium (0.040 g; 0.035 mmol) and diisopropylethylamine (0.210 mL; 1.21 mmol) in DME-water (3 :1 ; 2 mL) at 140°C for 40 min. Purification by flash chromatography on silica gel using a gradient of ethyl acetate (0 - 30%) in heptane furnished 0.041 g (34 %) of the title compound as a yellow oil contaminated with an impurity. ESI/APCI (+): 409 (M+H).

**EXAMPLE 45 : PREPARATION OF Methyl 2-(2-*tert*-butyl-7-(2-methoxy-4-chlorophenyl)-5-methylpyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.100 g; 0.296 mmol), 4-chloro-2-methoxyphenylboronic acid (0.100 g; 0.429 mmol), tetrakistriphenylphosphine palladium (0.040 g; 0.035 mmol) and diisopropylethylamine (0.210 mL ; 1.21 mmol) in DME-water (3 :1 ; 2 mL) at 140°C for 40 min. Purification by flash chromatography on silica gel using a gradient of ethyl acetate (0 - 20%) in heptane furnished 0.075 g (40 %) of the title compound as a yellow oil contaminated with an impurity. ESI/APCI (+): 444-446 (M+H).

**EXAMPLE 46 : PREPARATION OF Methyl 2-(2-*tert*-butyl-7-(2-fluoro-4-methoxyphenyl)-5-methylpyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.142 g; 0.420 mmol), 2-fluoro-4-methoxyphenylboronic acid (0.103 g; 0.606 mmol), tetrakistriphenylphosphine palladium (0.040 g; 0.035 mmol) and diisopropylethylamine (0.320 mL ; 1.84 mmol) in DME-water (3 :1 ; 3 mL) at 140°C for 40 min. Purification by flash chromatography on silica gel using a gradient of ethyl acetate (0 - 25%) in heptane furnished 0.174 g (97 %) of the title compound as a yellow oil contaminated with an impurity. ESI/APCI (+): 428 (M+H).

**EXAMPLE 47 : PREPARATION OF Methyl 2-(2-*tert*-butyl-5-methyl-7-(5-quinoline)pyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.149 g; 0.449 mmol), quinoline-5-boronic acid (0.115 g; 0.655 mmol), tetrakistriphenylphosphine palladium (0.040 g; 0.035 mmol) and diisopropylethylamine (0.320 mL ; 1.84 mmol) in DME-water (3 :1 ; 3 mL) at 140°C for 40 min. Purification by flash chromatography on silica gel using a gradient of ethyl acetate (0 - 50%) in heptane furnished 0.144 g (76 %) of the title compound as a yellow oil contaminated with an impurity. ESI/APCI (+): 431 (M+H).

**EXAMPLE 48 : PREPARATION OF Methyl 2-(2-*tert*-butyl-5-methyl-7-(8-quinoline)pyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.139 g; 0.411 mmol), quinoline-8-boronic acid (0.110 g; 0.636 mmol), tetrakistriphenylphosphine palladium (0.043 g; 0.038 mmol) and diisopropylethylamine (0.320 mL; 1.84 mmol) in DME-water (3 :1 ; 3 mL) at 140°C for 40 min. Purification by flash chromatography on silica gel using a gradient of ethyl acetate (0 - 40%) in heptane furnished 0.065 g (32 %) of the title compound as a yellow oil contaminated with an impurity. ESI/APCI (+): 431 (M+H).

**EXAMPLE 49 : PREPARATION OF Methyl 2-(2-*tert*-butyl-7-(2,4-dimethylphenyl)-5-methylpyrazolo[1,5-a]pyrimidin-6-yl)pentanoate**

This intermediate was prepared according to the procedure D from methyl 2-(2-*tert*-butyl-7-chloro-5-methylpyrazolo[1,5-*a*]pyrimidin-6-yl)-pentanoate (0.144 g; 0.426 mmol), 2,4-dimethylbenzeneboronic acid (0.105 g; 0.700 mmol), tetrakistriphenylphosphine palladium (0.042 g; 0.037 mmol) and diisopropylethylamine (0.320 mL; 1.84 mmol) in DME-water (3 :1 ; 3 mL) at 140°C for 40 min. Purification by flash chromatography on silica gel using a gradient of ethyl acetate (0 - 30%) in heptane furnished 0.138 g (79 %) of the title compound as a yellow oil contaminated with an impurity. ESI/APCI (+): 408 (M+H).

**EXAMPLE 50 : PREPARATION OF Methyl 2-(2-*tert*-butyl-3,5-dimethyl-7-*p*-tolylpyrazolo[1,5-a]pyrimidin-6-yl)pentanoate**

In a seal-tube were placed methyl 2-(3-bromo-2-*tert*-butyl-5-methyl-7-*p*-tolylpyrazolo[1,5-a]pyrimidin-6-yl)pentanoate (0.050 g; 0,106 mmol), potassium carbonate (0.0449 g; 0,318 mmol), trimethylboroxin (0.080 g; 0,635 mmol) and (1,1'-Bis(diphenylphosphino)ferrocene)-dichloropalladium(II) complex with dichloromethane (0.008 g; 0.011 mmol) with dimethylformamid (0.550 mL). The reaction mixture was purged with nitrogen, sealed and stirred under microwave irradiation at 150 °C for 45 min. The reaction mixture was partitiioned between dichloromethane and a saturated sodium chloride solution, filtered over magnesium sulfate and concentrated under reduced pressure. Purification by flash chromatography on silica gel using a gradient of ethyl acetate (5 - 30%) in heptane furnished 0.025 g (58 %) of the title compound as a yellow oil. ESI/APCI (+): 408 (M+H).

**EXAMPLE 51 : PREPARATION OF Methyl 2-(2-*tert*-butyl-5-methyl-3-phenyl-7-*p*-tolylpyrazolo[1,5-a]pyrimidin-6-yl)pentanoate**

This intermediate was prepared according to the procedure E from methyl 2-(3-bromo-2-*tert*-butyl-5-methyl-7-*p*-tolylpyrazolo[1,5-a]pyrimidin-6-yl)pentanoate (0.057 g; 0.121 mmol), benzeneboronic acid (0.036 g; 0.300 mmol), tetrakistriphenylphosphine palladium (0.014 g; 0.013 mmol) and diisopropylethylamine (0.075 mL; 0.431 mmol) in DME-water (3 :1 ; 1 mL) at 140°C for 40 min. Purification by flash chromatography on silica gel using a gradient of ethyl acetate (0 - 15%) in heptane furnished 0.041 g (73 %) of the title compound as a yellow oil contaminated with an impurity. ESI/APCI (+): 470 (M+H).

**Example 52 : PREPARATION OF Methyl 2-(2,3,5-trimethyl-7-*p*-tolyl-3*H*-imidazo[4,5-*b*]pyridin-6-yl)acetate**

To a solution of (5-amino-1,2-dimethyl-1*H*-imidazol-4-yl)(*p*-tolyl)methanone (0.077 g; 0.336 mmol) and methyl levunilate (0.080 mL; 0.646 mmol) in DMF (2 mL) placed in a safety pressure tube was slowly added chlorotrimethylsilane (0.340 mL; 2.679 mmol). The tube was sealed and heated to 110 °C for 24 h. Extra volumes of methyl levulinate (0.040 mL; 0.323 mmol) and chlorotrimethylsilane (0.170 mL; 1.339 mmol) were added the stirring at 110 °C was maintained

for 23 h. After cooling to room temperature, the reaction mixture was diluted with ethyl acetate and washed with a saturated sodium hydrogenocarbonate solution, water and brine. The organic phase was dried over sodium sulfate, filtered and concentrated under reduced pressure. The residue was purified by flash chromatography on silica gel using a gradient of methanol (0 - 10%) in dichloromethane to give 0.047 g (43%) of title compound as a brown solid. ESI/APCI (+): 324 (M+H).

**Example 53 : PREPARATION OF Methyl 2-(2,3,5-trimethyl-7-*p*-tolyl-3H-imidazo[4,5-*b*]pyridin-6-yl)pentanoate**

To a solution of methyl 2-(2,3,5-trimethyl-7-*p*-tolyl-3H-imidazo[4,5-*b*]pyridin-6-yl)acetate (0.051 g; 0.158 mmol) in dry DMF (2.6 mL) at -10°C was slowly added a 1N solution of LHMDS in THF (0.187 mL; 0.187 mmol). After 35 minutes at -15 °C, 1-iodopropane (0.027 mL; 0.277 mmol) was added and the reaction mixture was stirred at room temperature for 6.5 h. The reaction mixture was quenched by addition of a saturated solution of ammonium chloride and the mixture was extracted with ethyl acetate. The organic layer was washed with water, brine, dried over sodium sulfate, filtered and concentrated under reduced pressure. The residue was purified by flash chromatography on silica gel using gradient of methanol (2 - 10%) in dichloromethane to give 0.037 g (64%) of title compound as a yellow oil. ESI/APCI (+): 366 (M+H); 388 (M+Na).

**Example 54 : PREPARATION OF Ethyl 2-(1,2,5-trimethyl-7-*p*-tolyl-1H-imidazo[4,5-b]pyridin-6-yl)pentanoate**

To a solution of (4-amino-1,2-dimethyl-1H-imidazol-5-yl)(*p*-tolyl)methanone (0.172 g; 0.750 mmol) and ethyl 4-oxo-2-propylpentanoate (0.279 g; 1.5 mmol) in dry DMF (3 mL) under nitrogen atmosphere was added chlorotrimethylsilane (1.15 mL; 9 mmol). The mixture was stirred in a seal-tube and heated to 100°C for 72 h. After cooling, the mixture was poured with water and the non-homogeneous mixture vigorously stirred for 10 min. The aqueous layer was extracted twice with ethyl acetate, the organics were combined, dried over sodium sulfate and concentrated under reduced pressure. Purification by flash-chromatography on silica gel using a gradient of methanol (0 - 20%) in dichloromethane as eluent furnished 0.270 g (94 %) of the expected compound as a brown oil. ESI/APCI(+): 380 (M+H).

**Example 55 : PREPARATION OF Ethyl 2-(3,5-dimethyl-2-propyl-7-*p*-tolyl-3H-imidazo[4,5-b]pyridin-6-yl)pentanoate**

To a solution of (5-amino-2-propyl-1-methyl-1H-imidazol-4-yl)(*p*-tolyl)methanone (0.180 g; 0.700 mmol) and ethyl 4-oxo-2-propylpentanoate (0.260 g; 1.4 mmol) in dry DMF (2.8 mL) under nitrogen atmosphere was added chlorotrimethylsilane (1.07 mL; 8.4 mmol). The mixture was stirred in a seal-tube and heated to 100°C for 72 h. After cooling, the mixture was poured with water and the non-homogeneous mixture vigorously stirred for 10 min. The aqueous layer was extracted twice with ethyl acetate, the organics were combined, dried over sodium sulfate and concentrated under reduced pressure. Purification by flash-chromatography on silica gel using a gradient of methanol (0 - 20%) in dichloromethane as eluent furnished 0.190 g (66 %) of the expected cyclic compound as a brown oil. ESI/APCI(+): 408 (M+H).

**Example 56 : PREPARATION OF Ethyl 2-(3,5-dimethyl-2-isopropyl-7-*p*-tolyl-3H-imidazo[4,5-b]pyridin-6-yl)pentanoate**

To a solution of (5-amino-2-isopropyl-1-methyl-1H-imidazol-4-yl)(*p*-tolyl)methanone (0.296 g; 1.15 mmol) and ethyl 4-oxo-2-propylpentanoate (0.429 g; 2.3 mmol) in dry DMF (4.5 mL) under nitrogen atmosphere was added trimethylsilyl chloride (1.76 mL; 13.80 mmol). The mixture was stirred in a seal-tube and heated to 100°C for 72 h.. After cooling, the mixture was poured with water and the non-homogeneous mixture vigorously stirred for 10 min. The aqueous layer was extracted twice with ethyl acetate, the organics were combined, dried over sodium sulfate and concentrated under reduced pressure. Purification by flash-chromatography on silica gel using a gradient of methanol (0 - 20%) in dichloromethane as eluent furnished 0.367 g (78%) of the expected compound as a brown oil. ESI/APCI(+): 408 (M+H).

**EXAMPLE 57 : PREPARATION OF 2-(7-((R)-3-(4-chlorophenylsulfonamido)piperidin-1-yl)-5-methylpyrazolo[1,5-a]pyrimidin-6-yl)pentanoic acid**

To a solution of Ethyl 2-(7-((R)-3-(4-chlorophenylsulfonamido)piperidin-1-yl)-5-methylpyrazolo[1,5-a]pyrimidin-6-yl)pentanoate (0.380 g; 0.7 mmol) in ethanol (7 mL) was added a 2N sodium hydroxide solution (7 mL) and the reaction mixture was stirred at room temperature for 18 h. An extra volume of a 6N sodium hydroxide solution (1 ml) was added and the mixture was stirred 3 hours more. The volatiles were removed under reduced pressure, the pH was adjusted to 2 by adding of a cold 2N hydrochloric acid solution and the precipitate was filtered. Purification by flash chromatography on silica gel using a gradient of methanol (0.5 - 8%) in dichloromethane furnished 0.067 g (18%) of the title compound

as a white solid. ESI/APCI(+): 506-508 (M+H).

**EXAMPLE 58 : PREPARATION OF 2-(7-((S)-3-(4-chlorophenylsulfonamido)piperidin-1-yl)-5-methylpyrazolo[1,5-a]pyrimidin-6-yl)pentanoic acid**

To a solution of Ethyl 2-(7-((S)-3-(4-chlorophenylsulfonamido)piperidin-1-yl)-5-methylpyrazolo[1,5-a]pyrimidin-6-yl)pentanoate (0.280 g; 0.52 mmol) in ethanol (5.2 mL) was added a 2N sodium hydroxide solution (5.2 mL) and the reaction mixture was stirred at room temperature for 18 h. An extra volume of a 6N sodium hydroxide solution (0.8 ml) was added and the mixture was stirred 3 hours more. The volatiles were removed under reduced pressure, the pH was adjusted to 2 by adding of a cold 2N hydrochloric acid solution and the precipitate was filtered. Purification by flash chromatography on silica gel using a gradient of methanol (0.5 - 8%) in dichloromethane furnished 0.011 g (4%) of the title compound as a white solid. ESI/APCI(+): 506-508 (M+H).

**EXAMPLE 59 : PREPARATION OF 2-(7-((R)-3-(4-chlorophenylsulfonamido)pyrrolidin-1-yl)-5-methylpyrazolo[1,5-a]pyrimidin-6-yl)pentanoic acid**

To a solution of Ethyl 2-(7-((R)-3-(4-chlorophenylsulfonamido)pyrrolidin-1-yl)-5-methylpyrazolo[1,5-a]pyrimidin-6-yl)pentanoate (0.316 g; 0.61 mmol) in ethanol (6.1 mL) was added a 2N sodium hydroxide solution (6.1 mL) and the reaction mixture was stirred at room temperature for 18 h. An extra volume of a 6N sodium hydroxide solution (0.9 ml) was added and the mixture was stirred 3 hours more. The volatiles were removed under reduced pressure, the pH was adjusted to 2 by adding of a cold 2N hydrochloric acid solution and the precipitate was filtered. Purification by flash chromatography on silica gel using a gradient of methanol (0.5 - 8%) in dichloromethane furnished 0.019 g (7%) of the title compound as a white solid. ESI/APCI(+): 492-494 (M+H).

**EXAMPLE 60 : PREPARATION OF 2-(7-((S)-3-(4-chlorophenylsulfonamido)pyrrolidin-1-yl)-5-methylpyrazolo[1,5-a]pyrimidin-6-yl)pentanoic acid**

To a solution of Ethyl 2-(7-((S)-3-(4-chlorophenylsulfonamido)pyrrolidin-1-yl)-5-methylpyrazolo[1,5-a]pyrimidin-6-yl)pentanoate (0.300 g; 0.57mmol) in ethanol (5.7 mL) was added a 2N sodium hydroxide solution (5.7 mL) and the reaction mixture was stirred at room temperature for 18 h. An extra volume of a 6N sodium hydroxide solution (0.8 ml) was added and the mixture was stirred 3 hours more. The volatiles were removed under reduced pressure, the pH was adjusted to 2 by adding of a cold 2N hydrochloric acid solution and the precipitate was filtered. Purification by flash chromatography on silica gel using a gradient of methanol (0.5 - 8%) in dichloromethane furnished 0.040 g (14%) of the title compound as a white solid. ESI/APCI(+): 492-494 (M+H).

**EXAMPLE 61 : PREPARATION OF 2-(5-methyl-7-phenylpyrazolo[1,5-a]pyrimidin-6-yl)pentanoic acid**

To a solution of methyl 2-(5-methyl-7-phenylpyrazolo[1,5-a]pyrimidin-6-yl)pentanoate (0.266 g; 0.8 mmol) in a mixture DMSO/water (8 mL/0.8 mL) was added a 10N sodium hydroxide solution (0.8 mL) and the mixture was stirred at 65°C for 1 hour. Water (12 mL) was added and the pH was adjusted to 1 with a 6N hydrochloric acid solution and the aqueous phase was extracted with ethyl acetate The organic layer was concentrated under reduced pressure until dryness and water was added to the crude material. The white precipitate was filtered, washed with cold water and dried under reduced pressure to furnish the title compound 0.0 42 g (17 %) as a white solid. ESI/APCI(+): 310 (M+H).

**EXAMPLE 62 : PREPARATION OF 2-(5-methyl-7-*p*-tolylpyrazolo[1,5-a]pyrimidin-6-yl)pentanoic acid**

To a solution of methyl 2-(5-methyl-7-*p*-tolylpyrazolo[1,5-a]pyrimidin-6-yl)pentanoate (0.33 g; 0.98 mmol) in methanol (10 mL) was added a 10N sodium hydroxide solution (1 ml) and the mixture was heated to 80°C for 18 h. An extra volume of base was added (0.5 mL) and the reaction mixture was stirred 24 h more. After cooling, the volatiles were removed under reduced pressure, the pH was adjusted to 1 by adding of a cold 2N hydrochloric acid solution and the acid layer was extracted with ethyl acetate. The organics were collected, washed with brine, dried over magnesium sulfate and concentrated under reduced pressure. The crude solid was crystallized in a mixture ethyl acetate-heptane to give 0.106 g (33%) of title compound. ESI/APCI(+): 324 (M+H).

**EXAMPLE 63 : PREPARATION OF 2-(2-*tert*-butyl-5-methyl-7-phenylpyrazolo[1,5-a]pyrimidin-6-yl)pentanoic acid**

To a solution of methyl 2-(2-*tert*-butyl-5-methyl-7-phenylpyrazolo[1,5-a]pyrimidin-6-yl)pentanoate (0.141 g, 0.372 mmol) in a mixture methanol/water (8 mL/0.4 mL) was added a 10N sodium hydroxide solution (0.400 ml; 4.00

mmol) and the solution was stirred at 65 °C for 18 h. The volatiles were evaporated, a 5% citric acid solution (15 mL) was added to the residue and the product was extracted with ethyl acetate (15 mL). The organic layer was washed with brine (15 mL), dried over magnesium sulfate and concentrated under reduced pressure. Purification by flash-chromatography on silica gel using a gradient of methanol (0 - 7%) in dichloromethane furnished 0.036 g (27%) of the title compound as a yellow oil, which slowly solidified. ESI/APCI (+): 366 (M+H). ESI/APCI (-): 364 (M-H)

**EXAMPLE 64 : PREPARATION OF 2-(2-*tert*-butyl-5-methyl-7-p-tolylpyrazolol[1,5-a]pyrimidin-6-yl)pentanoic acid**

To a solution of methyl 2-(2-*tert*-butyl-5-methyl-7-*p*-tolylpyrazolo[1,5-a]pyrimidin-6-yl)pentanoate (0.126 g, 0.372 mmol) in a mixture methanol/water (6 mL/0.3 mL) was added a 10N sodium hydroxide solution (0.32 ml; 3.2 mmol) and the solution was stirred at 65 °C for 18 h. The volatiles were evaporated, a 5% citric acid solution (15 mL) was added to the residue and the product was extracted with ethyl acetate (15 mL). The organic layer was washed with brine (15 mL), dried over magnesium sulfate and concentrated under reduced pressure. Purification by flash-chromatography on silica gel using a gradient of methanol (0 - 7%) in dichloromethane furnished 0.067 g (55%) of the title compound as a yellow oil, which slowly solidified. ESI/APCI (+): 380 (M+H). ESI/APCI (-): 378 (M-H)

**EXAMPLE 65 : PREPARATION OF 2-(3-bromo-2-*tert*-butyl-5-methyl-7-p-tolylpyrazolo[1,5-a]pyrimidin-6-yl)pentanoic acid**

To a solution of methyl 2-(3-bromo-2-*tert*-butyl-5-methyl-7-*p*-tolylpyrazolo[1,5-a]pyrimidin-6-yl)pentanoate (0.052 g, 0.110 mmol) in methanol (5 mL) was added a 1N lithium hydroxide solution (0.15 ml; 0.15 mmol) and the solution was stirred at room temperature for 18 h. An extra volume of base (0.250 mL; 0.250 mmol) was added and stirring was continued for 24 h. An extra volume of base (0.400 mL; 0.400 mmol) was added and stirring was continued for 48 h. The volatiles were evaporated, a 1 N hydrochloric acid solution (10 mL) was added to the residue and the product was extracted with ethyl acetate (10 mL). The organic layer was dried over magnesium sulfate and concentrated under reduced pressure. Purification by flash-chromatography on silica gel using a gradient of methanol (0 - 20%) in dichloromethane furnished 0.041 g (81%) of the title compound as a yellow oil, which slowly solidified. ESI/APCI (+): 458-460 (M+H)

**EXAMPLE 66 : PREPARATION OF 2-(5-methyl-2-phenyl-7-p-tolylpyrazolo[1,5-a]pyrimidin-6-yl)pentanoic acid**

To a solution of methyl 2-(5-methyl-2-phenyl-7-p-tolylpyrazolo[1,5-a]pyrimidin-6-yl)pentanoate (0.109 g, 0.264 mmol) in a mixture methanol/water (5 mL/0.25 mL) was added a 10N sodium hydroxide solution (0.25 ml; 2.50 mmol) and the solution was stirred at 65 °C for 18 h. The volatiles were evaporated, a 5% citric acid solution (15 mL) was added to the residue and the product was extracted with ethyl acetate (15 mL). The organic layer was washed with brine (15 mL), dried over $MgSO_4$ and concentrated under reduced pressure. Purification by flash-chromatography on silica gel using a gradient of methanol (0 - 7%) in dichloromethane furnished 0.041 g (39%) of the title compound as a white solid. ESI/APCI (+): 400 (M+H). ESI/APCI (-): 398 (M-H)

**EXAMPLE 67 : PREPARATION OF 2-(2-*tert*-butyl-7-(3-hydroxyphenyl)-5-methylpyrazolo[1,5-a]pyrimidin-6-yl)pentanoic acid**

To a solution of methyl 2-(2-*tert*-butyl-7-(3-hydroxyphenyl)-5-methylpyrazolo[1,5-a]pyrimidin-6-yl)pentanoate (0.176 g, 0.445 mmol) in a mixture methanol/water (9 mL/0.5 mL) was added a 10N sodium hydroxide solution (0.450 ml; 4.50 mmol) and the solution was stirred at 65 °C for 18 h. The volatiles were evaporated, a 5% citric acid solution (15 mL) was added to the residue and the product was extracted with ethyl acetate (3 x 15 mL). The organic layer was washed with brine (15 mL), dried over $MgSO_4$ and concentrated under reduced pressure. Purification by flash-chromatography on silica gel using a gradient of methanol (0 - 10%) in dichloromethane furnished 0.065 g (40%) of the title compound as a yellow oil, which slowly solidified. ESI/APCI (+): 382 (M+H). ESI/APCI (-): 380 (M-H)

**EXAMPLE 68 : PREPARATION OF 2-(2-*tert*-butyl-5-methyl-7-(2-naphthyl)pyrazolo[1,5-a]pyrimidin-6-yl)pentanoic acid**

To a solution of methyl 2-(2-*tert*-butyl-5-methyl-7-(2-naphthyl)pyrazolo[1,5-a]pyrimidin-6-yl)pentanoate (0.143 g, 0.333 mmol) in methanol (7 mL) was added a 5N sodium hydroxide solution (0.700 ml; 3.50 mmol) and the solution was stirred at 50 °C for 18 h. The volatiles were evaporated, a 5% citric acid solution (15 mL) was added to the residue and the product was extracted with ethyl acetate (3 x 15 mL). The organic layer was washed with brine (15 mL), dried over $MgSO_4$ and concentrated under reduced pressure. Purification by flash-chromatography on silica gel using a

gradient of methanol (0 - 20%) in dichloromethane furnished 0.114 g (82%) of the title compound as a pale yellow foam. ESI/APCI (+): 416 (M+H). ESI/APCI (-): 414 (M-H); 370 (M-COOH)

**EXAMPLE 69 : PREPARATION OF 2-(2-*tert*-butyl-7-(1*H*-indol-5-yl)-5-methylpyrazolo[1,5-a]pyrimidin-6-yl)pentanoic acid**

To a solution of methyl 2-(2-*tert*-butyl-7-(1*H*-indol-5-yl)-5-methylpyrazolo[1,5-a]pyrimidin-6-yl)pentanoate (0.129 g, 0.308 mmol) in methanol (7 mL) was added a 5N sodium hydroxide solution (0.700 ml; 3.50 mmol) and the solution was stirred at 50 °C for 18 h. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 20%) in dichloromethane furnished 0.101 g (75%) of the title compound as a brown foam. ESI/APCI (+): 405 (M+H). ESI/APCI (-): 403 (M-H); 359 (M-COOH)

**EXAMPLE 70 : PREPARATION OF 2-(2-*tert*-butyl-7-(1*H*-indol-6-yl)-5-methylpyrazolo[1,5-a]pyrimidin-6-yl)pentanoic acid**

To a solution of methyl 2-(2-*tert*-butyl-7-(1*H*-indol-6-yl)-5-methylpyrazolo[1,5-a]pyrimidin-6-yl)pentanoate (0.104 g, 0.248 mmol) in methanol (5 mL) was added a 5N sodium hydroxide solution (0.500 ml; 2.50 mmol) and the solution was stirred at 50 °C for 18 h. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 20%) in dichloromethane furnished 0.058 g (58%) of the title compound as a white solid. ESI/APCI (+): 405 (M+H). ESI/APCI (-): 403 (M-H); 359 (M-COOH)

**EXAMPLE 71 : PREPARATION OF 2-(7-(benzofuran-5-yl)-2-*tert*-butyl-5-methylpyrazolo[1,5-a]pyrimidin-6-yl)pentanoic acid**

To a solution of methyl 2-(7-(benzofuran-5-yl)-2-*tert*-butyl-5-methylpyrazolo[1,5-a]pyrimidin-6-yl)pentanoate (0.114 g, 0.272 mmol) in a mixture methanol/water (5.5 mL/0.55 mL) was added a 5N sodium hydroxide solution (0.550 ml; 2.50 mmol) and the solution was stirred at 50 °C for 18 h. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 20%) in dichloromethane furnished 0.084 g (76%) of the title compound as a white solid. ESI/APCI (+): 406 (M+H). ESI/APCI (-): 404 (M-H); 360 (M-COOH)

**EXAMPLE 72 : PREPARATION OF 2-(7-(benzo[*b*]thiophen-5-yl)-2-*tert*-butyl-5-methylpyrazolo[1,5-a]pyrimidin-6-yl)pentanoic acid**

To a solution of methyl 2-(7-(benzo[*b*]thiophen-5-yl)-2-*tert*-butyl-5-methylpyrazolo[1,5-a]pyrimidin-6-yl)pentanoate (0.102 g, 0.234 mmol) in a mixture methanol/water (5 mL/0.5 mL) was added a 5N sodium hydroxide solution (0.5 ml; 2.50 mmol) and the solution was stirred at 50 °C for 18 h. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 20%) in dichloromethane furnished 0.078 g (79%) of the title compound as a yellow oil, which slowly solidified. ESI/APCI (+): 422 (M+H). ESI/APCI (-): 420 (M-H); 346 (M-COOH)

**EXAMPLE 73 : PREPARATION OF 2-(2-*tert*-butyl-7-(2,3-dihydrobenzofuran-5-yl)-5-methylpyrazolo[1,5-a]pyrimidin-6-yl)pentanoic acid**

To a solution of methyl 2-(2-tert-butyl-7-(2,3-dihydrobenzofuran-5-yl)-5-methylpyrazolo[1,5-a]pyrimidin-6-yl)pentanoate (0.096 g, 0.228 mmol) in a mixture methanol/water (5 mL/0.5 mL) was added a 5N sodium hydroxide solution (0.5 ml; 2.50 mmol) and the solution was stirred at 50 °C for 18 h. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 20%) in dichloromethane furnished 0.076 g (82%) of the title compound as a white solid. ESI/APCI (+): 408 (M+H). ESI/APCI (-): 406 (M-H); 362 (M-COOH)

**EXAMPLE 74 : PREPARATION OF 2-(2-*tert*-butyl-7-(4-chlorophenyl)-5-methylpyrazolo[1,5-a]pyrimidin-6-yl)pentanoic acid**

To a solution of methyl 2-(2-tert-butyl-7-(4-chlorophenyl)-5-methylpyrazolo[1,5-a]pyrimidin-6-yl)pentanoate (0.125 g, 0.302 mmol) in methanol (10 mL) was added a 1 N lithium hydroxide solution (0.6 ml; 0.6 mmol) and the solution was stirred at room temperature for 24 h. An extra volume of base (0.900 mL; 0.900 mmol) was added and stirring was continued for 24 h. An extra volume of 5N sodium hydroxide solution (0.300 mL; 1.5 mmol) was added and the reaction mixture was heated at 70°C for 24 h. The volatiles were evaporated, a 1N hydrochloric acid solution (18 mL) was added to the residue and the product was extracted with ethyl acetate (25 mL). The organic layer was washed with brine (10 mL), dried over $MgSO_4$ and concentrated under reduced pressure. Purification by flash-chromatography on silica gel using a gradient of methanol (0 - 20%) in dichloromethane and by preparative HPLC according to described method 1furnished 0.062 g (51%) of the title compound as a white solid. ESI/APCI (+): 400-402 (M+H). ESI/APCI (-): 398-400 (M-H); 354-356 (M-COOH)

**EXAMPLE 75 : PREPARATION OF 2-(2-*tert*-butyl-7-(3,4-dimethylphenyl)-5-methylpyrazolo[1,5-a]pyrimidin-6-yl)pentanoic acid**

To a solution of methyl 2-(2-tert-butyl-7-(3,4-dimethylphenyl)-5-methylpyrazolo[1,5-a]pyrimidin-6-yl)pentanoate (0.125 g, 0.302 mmol) in methanol (10 mL) was added a 1 N lithium hydroxide solution (0.6 ml; 0.6 mmol) and the solution was stirred at room temperature for 24 h. An extra volume of base (0.900 mL; 0.900 mmol) was added and stirring was continued for 24 h. An extra volume of 5N sodium hydroxide solution (0.300 mL; 1.5 mmol) was added and the reaction mixture was heated at 70°C for 24 h. The volatiles were evaporated, a 1 N hydrochloric acid solution (10 mL) was added to the residue and the product was extracted with ethyl acetate (2 x 10 mL). The organic layer was washed with brine (10 mL), dried over $MgSO_4$ and concentrated under reduced pressure. Purification by flash-chromatography on silica gel using a gradient of methanol (0 - 20%) in dichloromethane and by preparative HPLC according to described method 2 furnished 0.057 g (48 %) of the title compound as a white solid. ESI/APCI (+): 394 (M+H). ESI/APCI (-): 392 (M-H); 348 (M-COOH)

**EXAMPLE 76 : PREPARATION OF 2-(2-*tert*-butyl-7-(4-ethylphenyl)-5-methylpyrazolo[1,5-a]pyrimidin-6-yl)pentanoic acid**

To a solution of methyl 2-(2-tert-butyl-7-(4-ethylphenyl)-5-methylpyrazolo[1,5-a]pyimidin-6-yl)pentanoate (0.130 g, 0.319 mmol) in methanol (10 mL) was added a 1 N lithium hydroxide solution (0.65 ml; 0.65 mmol) and the solution was stirred at room temperature for 24 h. An extra volume of base (1 mL; 1 mmol) was added and stirring was continued for 24 h. An extra volume of 5N sodium hydroxide solution (0.300 mL; 1.5 mmol) was added and the reaction mixture was heated at 70°C for 24 h. The volatiles were evaporated, a 1N hydrochloric acid solution (10 mL) was added to the residue and the product was extracted with ethyl acetate (2 x 10 mL). The organic layer was washed with brine (10 mL), dried over $MgSO_4$ and concentrated under reduced pressure. Purification by flash-chromatography on silica gel using a gradient of methanol (0 - 20%) in dichloromethane and by preparative HPLC according to described method 2 furnished 0.057 g (45 %) of the title compound as a white solid. ESI/APCI (+): 394 (M+H). ESI/APCI (-): 392 (M-H); 348 (M-COOH)

**EXAMPLE 77 : PREPARATION OF 2-(2-*tert*-butyl-7-(3,4-dihydro-2*H*-benzo[*b*][1,4]dioxepin-7-yl)-5-methylpyrazolo[1,5-a]pyrimidin-6-yl)pentanoic acid**

To a solution of methyl 2-(2-*tert*-butyl-7-(3,4-dihydro-2*H*-benzo[*b*][1,4]dioxepin-7-yl)-5-methylpyrazolo[1,5-a]pyrimidin-6-yl)pentanoate (0.114 g, 0.252 mmol) in a mixture methanol/water (5.5 mL/0.55 mL) was added a 5N sodium hydroxide solution (0.55 ml; 2.75 mmol) and the solution was stirred at 50 °C for 18 h. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 20%) in dichloromethane furnished 0.056 g (50%) of the title compound as a white solid. ESI/APCI (+): 438 (M+H). ESI/APCI (-): 436 (M-H)

**EXAMPLE 78 : PREPARATION OF 2-(2-*tert*-butyl-7-(4-methyl-3,4-dihydro-2*H*-benzo[b][1,4]oxazin-7-yl)-5-methylpyrazolo[1,5-a]pyrimidin-6-yl)pentanoic acid**

To a solution of methyl 2-(2-tert-butyl-7-(3,4-dihydro-2*H*-benzo[b][1,4]dioxepin-7-yl)-5-methylpyrazolo[1,5-a]pyrimidin-6-yl)pentanoate (0.097 g, 0.224 mmol) in a mixture methanol/water (3.5 mL/0.35 mL) was added a 5N sodium

hydroxide solution (0.35 ml; 1.75 mmol) and the solution was stirred at 50 °C for 18 h. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 20%) in dichloromethane and by preparative HPLC according to described method 2 furnished 21 mg (21 %) of a bright yellow solid. ESI/APCI (+): 437 (M+H). ESI/APCI (-): 435 (M-H); 391 (M-COOH)

**EXAMPLE 79 : PREPARATION OF 2-(5-methyl-3-phenyl-7-p-tolylpyrazolo[1,5-a]pyrimidin-6-yl)pentanoic acid**

To a solution of ethyl 2-(5-methyl-3-phenyl-7-*p*-tolylpyrazolo[1,5-*a*]pyrimidin-6-yl)pentanoate (0.050 g, 0.117 mmol) in ethanol (2.5 mL) was added a 5N sodium hydroxide solution (0.25 ml; 1.25 mmol) and the solution was stirred at 70 °C for 3 days. The volatiles were evaporated, a 5% citric acid solution (10 mL) was added to the residue and the product was extracted with ethyl acetate (10 mL). The organic layer was washed with brine (10 mL), dried over $MgSO_4$ and concentrated under reduced pressure. Purification by flash-chromatography on silica gel using a gradient of methanol (0 - 7%) in dichloromethane and by preparative HPLC according described method 2 furnished 6.5 mg (14%) of a white solid. ESI/APCI (+): 400 (M+H). ESI/APCI (-): 398 (M-H)

**EXAMPLE 80 : PREPARATION OF 2-3,7-di-*p*-tolyl-5-methylpyrazolo[1,5-a]pyrimidin-6-yl)pentanoic acid**

To a solution of ethyl 2-(3,7-di-*p*-tolyl-5-methylpyrazolo[1,5-a]pyrimidin-6-yl)pentanoate (0.086 mg; 0.195 mmol) in a mixture methanol/water (4 mL/0.2 mL) was added a 5 N sodium hydroxide solution (0.4 mL; 2.0 mmol) and the resulting mixture was stirred at room temperature. After 24 h stirring, the solution was heated at 70°C for 3 days. The volatiles were removed under reduced pressure and ethyl acetate (10 mL) was added to the remaining residue. The solution was washed with a 1N hydrochloric acid solution (2 x 10 mL) and brine (10 mL), dried over $MgSO_4$, filtered and concentrated under reduced pressure. Purification by flash-chromatography on silica using a gradient of methanol (0 - 4%) in dichloromethane furnished 0.048 g (60 %) of the title compound as a bright yellow oil, which solidified slowly. ESI/APCI (+): 414 (M+H). ESI/APCI (-): 412 (M-H)

**EXAMPLE 81 : PREPARATION OF 2-(2-*tert*-butyl-3,7-di-*p*-tolyl-5-methylpyrazolo[1,5-a]pyrimidin-6-yl)pentanoic acid**

To a solution of methyl 2-(2-*tert*-butyl-3,7-di-*p*-tolyl-5-methylpyrazolo[1,5-a]pyrimidin-6-yl)pentanoate (0.014 g, 0.029 mmol) in methanol (1 mL) was added a 5N sodium hydroxide solution (0.1 ml; 0.5 mmol) and the solution was stirred at 60 °C for 3 days. The volatiles were evaporated, a 1 N hydrochloric acid solution (10 mL) was added to the residue and the product was extracted with ethyl acetate (10 mL). The organic layer was washed with brine (10 mL), dried over $MgSO_4$ and concentrated under reduced pressure to furnish 14 mg (96%) of a pale yellow solid. ESI/APCI (+): 470 (M+H). ESI/APCI (-): 368 (M-H)

**EXAMPLE 82 : PREPARATION OF 2-(5-methyl-2-propyl-7-*p*-tolylpyrazolo[1,5-a]pyrimidin-6-yl)pentanoic acid**

To a solution of methyl 2-(5-methyl-2-propyl-7-*p*-tolylpyrazolo[1,5-a]pyrimidin-6-yl)pentanoate (0.105 g; 0.277 mmol) in a mixture methanol-ethanol (2:1) (9 mL) was added a 5% sodium hydroxide solution (6.6 mL; 8.3 mmol) and the reaction mixture was heated to 60°C for 18 h. The organic volatiles were removed under reduced pressure and the remaining basic solution was acidified till pH 2 with a 6N hydrochloric acid solution and the aqueous layer was extracted with ethyl acetate. The organics were combined, dried over magnesium sulfate and concentrated under reduced pressure. Purification by flash chromatography on silica gel using a gradient of methanol (1 - 20%) in dichloromethane and by preparative HPLC according to described method 2 furnished 0.021 g (21 %) of the title compound.
ESI/APCI(+): 366 (M+H). ESI/APCI(-): 364 (M-H).

**EXAMPLE 83 : PREPARATION OF 2-(2-(furan-2-yl)-5-methyl-7-*p*-tolylpyrazolo[1,5-a]pyrimidin-6-yl)pentanoic acid**

To a solution of methyl 2-(2-(furan-2-yl)-5-methyl-7-*p*-tolylpyrazolo[1,5-a]pyrimidin-6-yl)pentanoate (0.123 g; 0.3 mmol) in a mixture methanol-ethanol (2:1) (9 mL) was added a 5% sodium hydroxide solution (7.3 mL; 9.1 mmol) and the reaction mixture was heated to 60°C for 18 h. The organic volatiles were removed under reduced pressure and the remaining basic solution was acidified till pH 2 with a 6N hydrochloric acid solution and the aqueous layer was extracted with ethyl acetate. The organics were combined, dried over magnesium sulfate and concentrated under reduced pressure. Purification by flash chromatography on silica gel using a gradient of methanol (1 - 20%) in dichloromethane

and by preparative HPLC according to described method 2 furnished 0.014 g (12%) of the title compound. ESI/APCI(+): 390 (M+H). ESI/APCI(-): 389 (M-H).

**EXAMPLE 84 : PREPARATION OF 2-(2-*tert*-butyl-7-(4-chloro-2-fluorophenyl)-5-methylpyrazolo[1,5-a]pyrimidin-6-yl)pentanoic acid**

To a solution of methyl 2-(2-*tert*-butyl-7-(4-chloro-2-fluorophenyl)-5-methylpyrazolo[1,5-a]pyrimidin-6-yl)pentanoate (0.101 g; 0.234 mmol) in methanol (10 mL) was added a 5N sodium hydroxide solution (0.5 mL; 2.5 mmol) and the reaction mixture was heated to 70°C for 3 days. Ethyl acetate (10 mL) was added to the reaction mixture and the solution was washed with a 1 N hydrochloric acid solution (10 mL) and brine (10 mL). The organic phase was dried over magnesium sulfate, filtered and concentrated under reduced. Crystallisation from ethyl acetate/heptane (twice) furnished 0.034 g (35%) of the title compound as a white solid. ESI/APCI(+): 418 (M+H). ESI/APCI(-): 416 (M-H). $^1$H-NMR (400 MHz, DMSO-$d_6$) (ppm) $\delta$: 12.9 (1 H, bs); 7.76 (1 H, d, $J$ = 9.66 Hz); 7.51 (2 H, m); 6.54 (1 H, s); 3.54 (1 H, t, $J$ = 5.63 Hz) 2.51 (3 H, s); 2.00 (1 H, m); 1.65 (1 H, m); 1.33 (9 H, s); 0.97 (2 H, m), 0.66 (3 H, t, $J$ = 7.12 Hz).

**EXAMPLE 85 : PREPARATION OF 2-(2-*tert*-butyl-7-(2-fluoro-4-methylphenyl)-5-methylpyrazolo[1,5-a]pyrimidin-6-yl)pentanoic acid**

To a solution of methyl 2-(2-*tert*-butyl-7-(2-fluoro-4-methylphenyl)-5-methylpyrazolo[1,5-a]pyrimidin-6-yl)pentanoate (0.106 g; 0.258 mmol) in methanol (10 mL) was added a 5N sodium hydroxide solution (0.5 mL; 2.5 mmol) and the reaction mixture was heated to 70°C for 3 days. The organic volatiles were removed under reduced pressure and the remaining basic solution was acidified till pH 2 with a 1 N hydrochloric acid solution and the aqueous layer was extracted with ethyl acetate. The organics were combined, dried over magnesium sulfate and concentrated under reduced pressure. Crystallisation from ethyl acetate/heptane (twice) furnished 0.061 g (60%) of the title compound as a white solid. ESI/APCI (+): 398 (M+H). ESI/APCI (-): 396 (M-H). $^1$H-NMR (400 MHz, DMSO-$d_6$) (ppm) $\delta$: 12.9 (1 H, bs); 7.30 (3 H, m); 6.51 (1 H, s); 3.54 (1H, t, $J$ = 5.7 Hz); 2.48 (3 H, s); 2.44 (3 H, s); 1.99 (1 H, m); 1.64 (1 H, m); 1.33 (9 H, s); 0.95 (2 H, m), 0.63 (3 H, t, $J$ = 7.3 Hz).

**EXAMPLE 86 : PREPARATION OF 2-(2-*tert*-butyl-7-*p*-tolyl-5-methylpyrazolo[1,5-a]pyrimidin-6-yl)-6,6,6-trifluorohexanoic acid**

To a solution of methyl 2-(2-*tert*-butyl-5-methyl-7-*p*-tolylpyrazolo[1,5-a]pyrimidin-6-yl)-6,6,6-trifluorohexanoate (0.135 g; 0.293 mmol) in methanol (12 mL) was added a 5N sodium hydroxide solution (0.6 mL; 3 mmol) and the reaction mixture was heated to 70°C for 3 days. The organic volatiles were removed under reduced pressure and the remaining basic solution was acidified till pH 2 with a 1 N hydrochloric acid solution and the aqueous layer was extracted with ethyl acetate. The organics were combined, dried over magnesium sulfate and concentrated under reduced pressure. Purification by by preparative HPLC according to described method 2 gave 0.063 g (46%) of a white solid. ESI/APCI (+): 448 (M+H). ESI/APCI (-): 446 (M-H). $^1$H-NMR (400 MHz, DMSO-$d_6$) (ppm) $\delta$: 12.9 (1 H, bs); 7.37 (4 H, m); 6.49 (1 H, s); 3.57 (1 H, t, $J$ = 7.0 Hz); 2.47 (3 H, s); 2.45 (3 H, s); 2.03 (3 H, M); 1.68 (1 H, m); 1.23 (9 H, s); 1.18 (2 H, m).

**EXAMPLE 87 : PREPARATION OF 2-(2-*tert*-butyl-7-*p*-tolyl-5-methylpyrazolo[1,5-a]pyrimidin-6-yl)-3-phenylpropanoic acid**

To a solution of methyl 2-(2-*tert*-butyl-5-methyl-7-*p*-tolylpyrazolo[1,5-a]pyrimidin-6-yl)-3-phenylpropanoate (0.118 g; 0.293 mmol) in methanol (6 mL) was added a 5N sodium hydroxide solution (0.6 mL; 3 mmol) and the reaction mixture was heated to 95°C for 16 h. The organic volatiles were removed under reduced pressure and the remaining basic solution was acidified till pH 2 with a 1N hydrochloric acid solution and the aqueous layer was extracted with ethyl acetate. The organics were combined, dried over magnesium sulfate and concentrated under reduced pressure. Crystallisation from ethyl acetate/heptane furnished 0.069 g (60%) of the title compound as a white solid. ESI/APCI (+): 428 (M+H). ESI/APCI (-): 426 (M-H).

**EXAMPLE 88 : PREPARATION OF 2-(2-*tert*-butyl-7-(1*H*-indol-6-yl)-5-methylpyrazolo[1,5-a]pyrimidin-6-yl)pentanoic acid**

To a solution of methyl 2-(2-*tert*-butyl-7-(1*H*-indol-6-yl)-5-methylpyrazolo[1,5-a]pyrimidin-6-yl)-3-phenylpropanoate (0.097 g; 0.224 mmol) in methanol (5 mL) was added a 5N sodium hydroxide solution (0.5 mL; 2.5 mmol) and the reaction mixture was heated to 50°C for 7 days. The organic volatiles were removed under reduced pressure and the remaining basic solution was acidified till pH 2 with a 1N hydrochloric acid solution and the aqueous layer was

extracted with ethyl acetate. The organics were combined, washed with a 1 N sodium hydrogenocarbonate, brine, dried over magnesium sulfate and concentrated under reduced pressure. Purification by preparative HPLC according to described method 2 furnished 0.010 g (11%) of the title compound as a white solid. ESI/APCI (+): 418 (M+H).

**EXAMPLE 89 : PREPARATION OF 2-(2-*tert*-butyl-5-methyl-7-(1-methylindolin-5-yl)pyrazolo[1,5-a]pyrimidin-6-yl)pentanoic acid**

To a solution of methyl 2-(2-*tert*-butyl-5-methyl-7-(1-methylindolin-5-yl)pyrazolo[1,5-a]pyrimidin-6-yl)pentanoate (0.074 g; 0.170 mmol) in methanol (8 mL) was added a 5N sodium hydroxide solution (0.35 mL; 1.75 mmol) and the reaction mixture was heated to 70°C for 3 days. The organic volatiles were removed under reduced pressure and the remaining basic solution was acidified till pH 2 with a 1N hydrochloric acid solution and the aqueous layer was extracted with ethyl acetate. The organics were combined, dried over magnesium sulfate and concentrated under reduced pressure. Purification by preparative HPLC according to described method 2 furnished 0.012 g (17%) of the title compound as a yellow solid. ESI/APCI (+): 421 (M+H). ESI/APCI (-): 419 (M-H).

**EXAMPLE 90 : PREPARATION OF 2-(2-*tert*-butyl-5-methyl-7-(1-methyl-1H-indol-6-yl)pyrazolo[1,5-a]pyrimidin-6-yl)pentanoic acid**

To a solution of methyl 2-(2-*tert*-butyl-5-methyl-7-(1-methyl-1H-indol-6-yl)pyrazolo[1,5-a]pyrimidin-6-yl)pentanoate (0.079 g; 0.183 mmol) in methanol (4 mL) was added a 5N sodium hydroxide solution (0.4 mL; 2 mmol) and the reaction mixture was heated to 50°C for 7 days. The organic volatiles were removed under reduced pressure and the remaining basic solution was acidified till pH 2 with a 1 N hydrochloric acid solution and the aqueous layer was extracted with ethyl acetate. The organics were combined, dried over magnesium sulfate and concentrated under reduced pressure. Purification by preparative HPLC according to described method 2 furnished 0.010 g (13%) of the title compound as a yellow solid. ESI/APCI (+): 419 (M+H). ESI/APCI (-): 417 (M-H).

**EXAMPLE 91 : PREPARATION OF 2-(2-*tert*-butyl-7-(chroman-6-yl)-5-methylpyrazolo[1,5-a]pyrimidin-6-yl)pentanoic acid**

To a solution of methyl 2-(2-*tert*-butyl-7-(chroman-6-yl)-5-methylpyrazolo[1,5-a]pyrimidin-6-yl)pentanoate (0.095 g; 0.218 mmol) in methanol (5 mL) was added a 5N sodium hydroxide solution (0.5 mL; 2.5 mmol) and the reaction mixture was heated to 50°C for 7 days. The organic volatiles were removed under reduced pressure and the remaining basic solution was acidified till pH 2 with a 1 N hydrochloric acid solution and the aqueous layer was extracted with ethyl acetate. The organics were combined, dried over magnesium sulfate and concentrated under reduced pressure. Purification by flash-chromatography on silica using a gradient of methanol (0 - 20%) in dichloromethane gave the expected compound, contaminated with an impurity. Purification by preparative HPLC according to described method 2 furnished 0.054 g (58%) of the title compound as a yellow solid. ESI/APCI (+): 422 (M+H). ESI/APCI (-): 420 (M-H).

**EXAMPLE 92 : PREPARATION OF 2-(2-*tert*-butyl-7-p-tolyl-5-methylpyrazolo[1,5-a]pyrimidin-6-yl)-3-methylpentanoic acid**

To a solution of methyl 2-(2-*tert*-butyl-7-*p*-tolyl-5-methylpyrazolo[1,5-a]pyrimidin-6-yl)-3-methylpentanoate (0.055 g; 0.135 mmol) in ethanol (6 mL), water (1 mL) was added a 5N sodium hydroxide solution (0.6 mL; 3.0 mmol) and the solution was heated at 140°C for 2 h under microwave irradiation. The organic volatiles were removed under reduced pressure and the remaining basic solution was acidified till pH 2 with a 1N hydrochloric acid solution and the aqueous layer was extracted with ethyl acetate. The organics were combined, washed with a 1 N sodium hydrogenocarbonate solution, brine, dried over magnesium sulfate and concentrated under reduced pressure. Purification by flash-chromatography on silica using a gradient of methanol (0 - 20%) in dichloromethane gave the expected compound, contaminated with an impurity. Crystallisation in heptane furnished 0.028 g (52%) of the title compound as a white solid. ESI/APCI (+): 394 (M+H). ESI/APCI (-): 392 (M-H).

**EXAMPLE 93 : PREPARATION OF 2-(2-*tert*-butyl-3-chloro-7-*p*-tolyl-5-methylpyrazolo[1,5-a]pyrimidin-6-yl)pentanoic acid**

To a solution of methyl 2-(2-*tert*-butyl-3-chloro-5-methyl-7-p-tolylpyrazolo[1,5-a]pyrimidin-6-yl)pentanoate (0.070 g; 0.164 mmol) in methanol (6 mL) was added a 5N sodium hydroxide solution (0.3 mL; 1.5 mmol) and the solution was heated at 75°C for 18 h. The organic volatiles were removed under reduced pressure and the remaining basic solution was acidified till pH 2 with a 1N hydrochloric acid solution and the aqueous layer was extracted with ethyl acetate.

The organics were combined, washed with a 1N sodium hydrogenocarbonate solution, brine, dried over magnesium sulfate and concentrated under reduced pressure. The remaining bright yellow oil crystallized slowly to give 0.066 g (98%) of the title compound. ESI/APCI (+): 414-416 (M+H).

**EXAMPLE 94 : PREPARATION OF 2-(2-*tert*-butyl-7-*p*-tolyl-5-methylpyrazolo[1,5-a]pyrimidin-6-yl)-4-methoxybutanoic acid**

To a solution of methyl 2-(2-*tert*-butyl-7-*p*-tolyl-5-methylpyrazolo[1,5-a]pyrimidin-6-yl)-3-methyl-4-methoxybutanoate (0.211 g; 0.515 mmol) in methanol (11 mL) was added a 5N sodium hydroxide solution (1.1 mL; 5.5 mmol) and the solution was heated at 70°C for 24 h. The organic volatiles were removed under reduced pressure and the remaining basic solution was acidified till pH 2 with a 1N hydrochloric acid solution and the aqueous layer was extracted with ethyl acetate. The organics were combined, washed with a 1 N sodium hydrogenocarbonate solution, brine, dried over magnesium sulfate and concentrated under reduced pressure. Purification by preparative HPLC according to described method 2 furnished 0.111 g (55%) of the title compound as a yellow solid. ESI/APCI (+): 396 (M+H). ESI/APCI (-): 394 (M-H).

**EXAMPLE 95 : PREPARATION OF 2-(2-*tert*-butyl-5-methyl-7-(4-*iso*propylphenyl)pyrazolo[1,5-a]pyrimidin-6-yl)pentanoic acid**

To a solution of methyl 2-(2-*tert*-butyl-7-(4-*iso*-propylphenyl)-5-methylpyrazolo[1,5-*a*]pyrimidin-6-yl)-pentanoate (0.180 g; 0.427 mmol) in methanol (5 mL) was added a 5N sodium hydroxide solution (0.43 mL; 2.15 mmol) and the solution was heated at 75°C for 24 h. The organic volatiles were removed under reduced pressure and the remaining basic solution was acidified till pH 2 with a 1N hydrochloric acid solution and the aqueous layer was extracted with ethyl acetate. The organics were combined, washed with a 1N sodium hydrogenocarbonate solution, brine, dried over magnesium sulfate and concentrated under -reduced pressure. Purification by flash-chromatography using a gradient of ethyl acetate (0 - 25 %) in heptane furnished 0.069 g (40%) of the title compound as a white solid. ESI/APCI (+): 408 (M+H). ESI/APCI (-): 406 (M-H).

**EXAMPLE 96 : PREPARATION OF 2-(2-tert-butyl-5-methyl-7-(4-trifluoromethylphenyl)pyrazolo[1,5-a]pyrimidin-6-yl)pentanoic acid**

To a solution of methyl 2-(2-*tert*-butyl-5-methyl-7-(4-trifluorophenyl)pyrazolo[1,5-*a*]pyrimidin-6-yl)-pentanoate (0.220 g; 0.427 mmol) in methanol (5 mL) was added a 5N sodium hydroxide solution (0.5 mL; 2.5 mmol) and the solution was heated at 75°C for 24 h. The organic volatiles were removed under reduced pressure and the remaining basic solution was acidified till pH 2 with a 1 N hydrochloric acid solution and the aqueous layer was extracted with ethyl acetate. The organics were combined, washed with a 1N sodium hydrogenocarbonate solution, brine, dried over magnesium sulfate and concentrated under reduced pressure. Purification by flash-chromatography using a gradient of ethyl acetate (0 - 25 %) in heptane furnished 0.151 g (71 %) of the title compound as a pale yellow solid. ESI/APCI (+): 434 (M+H). ESI/APCI (-): 432 (M-H).

**EXAMPLE 97 : PREPARATION OF 2-(2-*tert*-butyl-7-(2,4-difluorophenyl)-5-methylpyrazolo[1,5-a]pyrimidin-6-yl)pentanoic acid**

To a solution of methyl 2-(2-*tert*-butyl-7-(2,4-difluorophenyl)-5-methylpyrazolo[1,5-*a*]pyrimidin-6-yl)pentanoate (0.188 g; 0.433 mmol) in methanol (5 mL) was added a 5N sodium hydroxide solution (0.5 mL; 2.5 mmol) and the solution was heated at 75°C for 24 h. The organic volatiles were removed under reduced pressure and the remaining basic solution was acidified till pH 2 with a 1N hydrochloric acid solution and the aqueous layer was extracted with ethyl acetate. The organics were combined, washed with a 1N sodium hydrogenocarbonate solution, brine, dried over magnesium sulfate and concentrated under reduced pressure to furnish 0.161 g (89%) of the title compound as a pale yellow solid. ESI/APCI (+): 402 (M+H). ESI/APCI (-): 400 (M-H).

**EXAMPLE 98 : PREPARATION OF 2-(2-*tert*-butyl-7-(2-chloro-4-methylphenyl)-5-methylpyrazolo[1,5-a]pyrimidin-6-yl)pentanoic acid**

To a solution of methyl 2-(2-tert-butyl-7-(2-chloro-4-methylphenyl)-5-methylpyrazolo[1,5-*a*]pyimidin-6-yl)-pentanoate (0.180 g; 0.450 mmol) in methanol (5 mL) was added a 5N sodium hydroxide solution (0.5 mL; 2.5 mmol) and the solution was heated at 75°C for 24 h. The organic volatiles were removed under reduced pressure and the remaining basic solution was acidified till pH 2 with a 1 N hydrochloric acid solution and the aqueous layer was extracted with ethyl

acetate. The organics were combined, washed with a 1N sodium hydrogenocarbonate solution, brine, dried over magnesium sulfate and concentrated under reduced pressure to furnish 0.163 g (93%) of the title compound as a pale yellow solid. ESI/APCI (+): 413-415 (M+H).

**EXAMPLE 99 : PREPARATION OF 2-(7-(2-amino-4-methylphenyl)-2-*tert*-butyl-5-methylpyrazolo[1,5-a]pyrimidin-6-yl)pentanoic acid and its corresponding lactam**

To a solution of methyl 2-(7-(2-amino-4-methylphenyl)-2-*tert*-butyl-5-methylpyrazolo[1,5-*a*]pyrimidin-6-yl)pentanoate (0.041 g; 0.010 mmol) in methanol (2 mL) was added a 5N sodium hydroxide solution (0.2 mL; 1 mmol) and the solution was heated at 75°C for 20 h. The organic volatiles were removed under reduced pressure and the remaining basic solution was acidified till pH 2 with a 1N hydrochloric acid solution and the aqueous layer was extracted with ethyl acetate. The organics were combined, washed with a 1N sodium hydrogenocarbonate solution, brine, dried over magnesium sulfate and concentrated under reduced pressure. Purification by preparative HPLC according to described method 2 furnished 0.003 g (7 %) of the 2-(7-(2-amino-4-methylphenyl)-2-tert-butyl-5-methylpyrazolo[1,5-a]pyrimidin-6-yl)pentanoic acid as a white solid. ESI/APCI (+): 395 (M+H).

**EXAMPLE 100 : PREPARATION OF 2-(2-*tert*-butyl-7-(2-methoxy-4-chlorophenyl)-5-methylpyrazolo[1,5-a]pyrimidin-6-yl)pentanoic acid**

To a solution of methyl 2-(2-*tert*-butyl-7-(2-methoxy-4-chlorophenyl)-5-methylpyrazolo[1,5-a]pyrimidin-6-yl)pentanoate (0.075 g; 0.17 mmol) in methanol (3.5 mL) was added a 5N sodium hydroxide solution (0.350 mL; 1.75 mmol) and the solution was heated at 75°C for 24 h. The organic volatiles were removed under reduced pressure and the remaining basic solution was acidified till pH 2 with a 1N hydrochloric acid solution and the aqueous layer was extracted with ethyl acetate. The organics were combined, washed with a 1N sodium hydrogenocarbonate solution, brine, dried over magnesium sulfate and concentrated under reduced pressure. Purification by flash-chromatography using a mixture acetic acid -dichloromethane (2-98) as eluent gave a yellow oil. Purification by preparative HPLC according to described method 2 furnished 0.034 g (47%) of the title compound as a yellow solid. ESI/APCI (+): 430-432 (M+H).

**EXAMPLE 101 : PREPARATION OF 2-(2-*tert*-butyl-7-(2-fluoro-4-methoxyphenyl)-5-methylpyrazolo[1,5-a]pyrimidin-6-yl)pentanoic acid**

To a solution of methyl 2-(2-*tert*-butyl-7-(2-fluoro-4-methoxyphenyl)-5-methylpyrazolo[1,5-a]pyrimidin-6-yl)pentanoate (0.174 g; 0.407 mmol) in methanol (4 mL) was added a 5N sodium hydroxide solution (0.410 mL; 2.05 mmol) and the solution was heated at 75°C for 18 h. The organic volatiles were removed under reduced pressure and the remaining basic solution was acidified till pH 2 with a 1N hydrochloric acid solution and the aqueous layer was extracted with ethyl acetate. The organics were combined, washed with a 1N sodium hydrogenocarbonate solution, brine, dried over magnesium sulfate and concentrated under reduced pressure to furnish 0.148 g (88%) of the title compound as a beige powder. ESI/APCI (+): 414 (M+H).

**EXAMPLE 102 : PREPARATION OF 2-(2-tert-butyl-5-methyl-7-(5-quinoline)pyrazolo[1,5-a]pyrimidin-6-yl)pentanoic acid**

To a solution of methyl 2-(2-tert-butyl-5-methyl-7-(5-quinoline)pyrazolo[1,5-a]pyrimidin-6-yl)pentanoate (0.141 g; 0.327 mmol) in methanol (3.5 mL) was added a 5N sodium hydroxide solution (0.350 mL; 1.75 mmol) and the solution was heated at 75°C for 48 h. The organic volatiles were removed under reduced pressure and the remaining basic solution was acidified till pH 2 with a 1N hydrochloric acid solution and the aqueous layer was extracted with ethyl acetate. The organics were combined, washed with a 1N sodium hydrogenocarbonate solution, brine, dried over magnesium sulfate and concentrated under reduced pressure to furnish 0.118 g (87%) of the title compound as a beige solid. ESI/APCI (+): 417 (M+H).

**EXAMPLE 103 : PREPARATION OF 2-(2-tert-butyl-5-methyl-7-(8-quinoline)pyrazolo[1,5-a]pyrimidin-6-yl)pentanoic acid**

To a solution of methyl 2-(2-tert-butyl-5-methyl-7-(8-quinoline)pyrazolo[1,5-a]pyrimidin-6-yl)pentanoate (0.059 g; 0.137 mmol) in methanol (2 mL) was added a 5N sodium hydroxide solution (0.150 mL; 0.75 mmol) and the solution was heated at 75°C for 48 h. The organic volatiles were removed under reduced pressure and the remaining basic solution was acidified till pH 2 with a 1N hydrochloric acid solution and the aqueous layer was extracted with ethyl acetate. The organics were combined, washed with a 1N sodium hydrogenocarbonate solution, brine, dried over magnesium

sulfate and concentrated under reduced pressure to furnish 0.047 g (82%) of the title compound as a beige solid. ESI/APCI (+): 417 (M+H).

**EXAMPLE 104 : PREPARATION OF 2-(2-tert-butyl-7-(2,4-dimethylphenyl)-5-methylpyrazolo[1,5-a]pyrimidin-6-yl)pentanoic acid**

To a solution of methyl 2-(2-*tert*-butyl-7-(2,4-dimethylphenyl)-5-methylpyrazolo[1,5-a]pyrimidin-6-yl)pentanoate (0.138 g; 0.339 mmol) in methanol (3.5 mL) was added a 5N sodium hydroxide solution (0.350 mL; 1.75 mmol) and the solution was heated at 75°C for 48 h. The organic volatiles were removed under reduced pressure and the remaining basic solution was acidified till pH 2 with a 1 N hydrochloric acid solution and the aqueous layer was extracted with ethyl acetate. The organics were combined, washed with a 1N sodium hydrogenocarbonate solution, brine, dried over magnesium sulfate and concentrated under reduced pressure. Purification by flash-chromatography using a gradient of methanol (0 -7%) in dichloromethane as eluent furnished 0.064 g (48%) of the title compound as a white solid. ESI/APCI (+): 394 (M+H). ESI/APCI (-): 392 (M-H).

**EXAMPLE 105 : PREPARATION OF 2-(2-*tert*-butyl-3,5-dimethyl-7-*p*-tolylpyrazolo[1 ,5-a]pyrimidin-6-yl)pentanoic acid**

To a solution of methyl 2-(2-*tert*-butyl-3,5-dimethyl-7-*p*-tolylpyrazolo[1,5-a]pyrimidin-6-yl)pentanoate (0.025 g; 0.061 mmol) in methanol (1 mL) was added a 10 N sodium hydroxide solution (0.100 mL; 1 mmol) and the mixture was heated to 60 °C in a sealed tube for 20 h. After cooling, the volatiles were removed under reduced pressure and the residue was dissolved in water, the mixture was then acidified by adding a 2 N hydrochloric acid solution until pH 2. The precipitate was filtered, washed with water and dried under reduced pressure. To furnish 0.014g (56 %) of the title compound as a light yellow solid. ESI/APCI(+): 394 (M+H).

**EXAMPLE 106 : PREPARATION OF 2-(2-*tert*-butyl-5-methyl-3-phenyl-7-*p*-tolylpyrazolo[1,5-a]pyrimidin-6-yl)pentanoic acid**

To a solution of methyl 2-(3-bromo-2-*tert*-butyl-5-methyl-7-*p*-tolylpyrazolo[1,5-a]pyrimidin-6-yl)pentanoate (0.041 g; 0.087 mmol) in methanol (4 mL) was added a 5 N sodium hydroxide solution (0.200 mL; 1 mmol) and the mixture was heated to 70 °C for 72 h. After cooling, the volatiles were removed under reduced pressure and the residue was dissolved in water. Tthe mixture was then acidified by adding a 1 N hydrochloric acid solution until pH 2 and the aqueous layer was extracted with ethyl acetate. The organics were combined, washed with a 1N sodium hydrogenocarbonate solution, brine, dried over magnesium sulfate and concentrated under reduced pressure. Purification by preparative according to describted method 2 furnished 0.023g (58 %) of the title compound as a light yellow solid. ESI/APCI (+): 456 (M+H). ESI/APCI (-): 454 (M-H).

**Example 107 : PREPARATION OF 2-(2,3,5-Trimethyl-7-*p*-tolyl-3*H*-imidazo[4,5-*b*]pyridin-6-yl)pentanoic acid**

To a solution of methyl 2-(2,3,5-trimethyl-7-*p*-tolyl-3*H*-imidazo[4,5-*b*]pyridin-6-yl)pentanoate (0.037 g; 0.101 mmol) in a mixture methanol-ethanol (2:1, 3 mL) was added a 5% sodium hydroxide solution (2.7 mL; 3.375 mmol). The reaction mixture was heated to reflux for 4 h. The solvents were evaporated and the residue was taken up with water, acidified with a 1N hydrochloric acid solution and extracted with ethyl acetate. The organic phase was washed with water and brine, dried over sodium sulfate, filtered and concentrated under reduced pressure. The residue was purified by flash chromatography on silica gel using a gradient of methanol (3 - 20%) in dichloromethane to give 0.019 g (53%) of title compound as a beige solid. ESI/APCI (+): 352 (M+H); 374 (M+Na). ESI/APCI (-): 350 (M+H). 1 H NMR (dmso-$d_6$) δ 0.58 (3H, t); 0.87 (2H, m); 1.52 (1H, m); 1.96 (1H, m); 2.39 (3H, s); 2.46 (3H, s); 2.50 (3H, s); 3.70 (3H, s); 3.85 (1 H, m); 7.26 (4H, m).

**Example 108 : PREPARATION OF 2-(1,2,5-trimethyl-7-*p*-tolyl-1H-imidazo[4,5-b]pyridin-6-yl)pentanoic acid**

To a suspension of ethyl 2-(1,2,5-trimethyl-7-*p*-tolyl-1H-imidazo[4,5-b]pyridin-6-yl)pentanoate (0.270 g; 0.711 mmol) in a mixture methanol-ethanol (2:1) (24 mL) was added a 5% sodium hydroxide solution (21.34 mmol; 17 mL) and the reaction mixture was heated to 60°C for 18 h. The organic volatiles were removed under reduced pressure and the remaining basic solution was acidified till pH 2 with a hydrochloric solution (1 N) and extracted with ethyl acetate twice. The organics were combined, dried over sodium sulphate and concentrated under reduced pressure. Purification by preparative HPLC according to described method 1 furnished 0.021 g (8%) of the title compound as a beige solid. ESI/APCI(+): 352 (M+H).

**Example 109 : PREPARATION OF 2-(2-propyl-3,5-dimethyl-7-*p*-tolyl-3H-imidazo[4,5-b]pyridin-6-yl)pentancic acid**

To a suspension of ethyl 2-(2-propyl-3,5-dimethyl-7-*p*-tolyl-3H-imidazo[4,5-b]pyridin-6-yl)pentanoate (0.190 g; 0.466 mmol) in a mixture methanol-ethanol (2:1) (15 mL) was added a 5% sodium hydroxide solution (14 mmol; 11.2 mL) and the reaction mixture was heated to 60°C for 18 h. The organic volatiles were removed under reduced pressure and the remaining basic solution was acidified till pH 2 with a hydrochloric solution (1 N) and extracted with ethyl acetate twice. The organics were combined, dried over sodium sulphate and concentrated under reduced pressure. Purification by preparative HPLC according to described method 1 furnished 0.014 g (8%) of the title compound as a beige solid. ESI/APCI(+): 380 (M+H).

**Example 110 : PREPARATION OF 2-(2-isopropyl-3,5-dimethyl-7-*p*-tolyl-3H-imidazo[4,5-b]pyridin-6-yl)pentanoate**

To a suspension of ethyl 2-(2-isopropyl-3,5-dimethyl-7-*p*-tolyl-3H-imidazo[4,5-b]pyridin-6-yl)pentanoate (0.367 g; 0.900 mmol) in a mixture methanol-ethanol (2:1) (30 mL) was added a 5% sodium hydroxide solution (27.02 mmol; 21.6 mL) and the reaction mixture was heated to 60°C for 18 h. The organic volatiles were removed under reduced pressure and the remaining basic solution was acidified till pH 2 with a hydrochloric solution (1 N) and extracted with ethyl acetate twice. The organics were combined, dried over sodium sulphate and concentrated under reduced pressure. Purification by preparative HPLC according to described method 1 0.030 g (9%) of the title compound as a beige solid. ESI/APCI(+): 380 (M+H).

**EXAMPLE 111 : PREPARATION OF Methyl 2-(2-isopropyl-5-methyl-7-*p*-tolyl-[1,2,4]-triazolo[1,5-*a*]pyrimidin-6-yl)acetate**

To a sonicated solution of methyl 2-(7-chloro-2-isopropyl-5-methyl-[1,2,4]triazolo[1,5-*a*]pyrimidin-6-yl)-acetate (0.150 g; 0.531 mmol) and 4-tolylboronic acid (0.114 g; 0.839 mmol) in a mixture of water/DME (1/3) (4 mL) were added palladiumtetrakistriphenylphosphine (0.056 g; 0.049 mmol) and diisopropylethylamine (0.500 mL; 1.84 mmol). The solution was stirred for 40 min at 140°C under microwave irradiation. Ethyl acetate (20 mL) was added to the reaction mixture and the solution was washed with a 1N solution of hydrochloric acid, a 1N solution of sodium hydrogenocarbonate and brine. The organic layer was dried over magnesium sulphate, filtered and concentrated under reduced pressure. Purification by flash chromatography on silica gel using a gradient of ethyl acetate (20 - 70%) in heptane furnished 0.089 g (50%) of the title compound as a white solid. ESI/APCI (+): 339 (M+H).

**EXAMPLE 112 : PREPARATION OF 2-(2-isopropyl-5-methyl-7-*p*-tolyl-[1,2,4]-triazolo[1,5-*a*]pyrimidin-6-yl)acetic acid**

To a solution of methyl 2-(2-isopropyl-5-methyl-7-*p*-tolyl-[1,2,4]triazolo[1,5-a]pyrimidin-6-yl)acetate (0.089 g; 0.263 mmol) in methanol (5 mL) was added a 5N solution of sodium hydroxide (0.550 mL; 2.75 mmol) and the reaction mixture was heated at 75°C for 6 hours. After cooling, a 1N solution of hydrochloric acid was added to the reaction mixture and the organics were removed under reduced pressure. The aqueous layer was extracted with ethyl acetate, washed with brine, dried over magnesium sulphate, filtered and concentrated under reduced pressure. Crystallization from a mixture ethyl acetate-heptane furnished 0.044 g (52%) of the title compound as a white solid. ESI/APCI (+): 325 (M+H). ESI/APCI (-): 323 (M-H).

**EXAMPLE 113 : PREPARATION OF Methyl 2-[2-*tert*-butyl-7-(1,2-dihydroacenaphthylen-5-yl)-5-methylpyrazolo[1,5-a]pyrimidin-6-yl]acetate**

To a solution of methyl 2-(2-*tert*-butyl-7-chloro-5-methylpyrazolo[1,5-*a*]pyrimidin-6-yl)-pentanoate (0.080 g; 0.237 mmol) and 2-(1,2-dihydroacenaphthylen-5-yl)-4,4,5,5-tetramethyl-1,3,2-dioxaborolane (0.095 g; 0.339 mmol) in a mixture of water/DME (1/3) (1.5 mL) were added palladiumtetrakistriphenylphosphine (0.015 g; 0.013 mmol) and diisopropylethylamine (0.170 mL; 0.976 mmol). The solution was stirred for 40 min at 140°C under microwave irradiation. Ethyl acetate was added to the reaction mixture and the solution was successively washed with a 1N solution of hydrochloric acid, a 1N solution of sodium hydrogenocarbonate and brine. The organic layer was dried over magnesium sulphate, filtered and concentrated under reduced pressure. Purification by flash chromatography on silica gel using a gradient of ethyl acetate (0 - 20%) in heptane furnished 0.092 g (85%) of the title compound as a bright yellow solid. ESI/APCI (+): 408 (M+H).

**EXAMPLE 114 : PREPARATION OF 2-[2-*tert*-butyl-7-(1,2-dihydroacenaphthylen-5-yl)-5-methylpyrazolo[1,5-a]pyrimidin-6-yl]acetic acid**

To a solution of methyl 2-(2-*tert*-butyl-7-(1,2-dihydroacenaphthylen-5-yl)-5-methylpyrazolo[1,5-*a*]pyrimidin-6-yl)-pentanoate (0.092 mg; 0.202 mmol) in methanol (3 mL) was added a 5N solution of sodium hydroxide (0.200 mL; 1.00 mmol) and the solution was heated at 75°C for 5 days. The volatiles were evaporated and a 1N solution of hydrochloric acid was added to the residue. The precipitate was filtered and washed with water. Purification by preparative HPLC according to described method 2 furnished 2 peaks with the same mass. The first peak was eluted at Rt=8.20 min (0.0079 g, 9%) and the second peak was eluted at Rt=8.58 min (0.0187 g, 21 %). ESI/APCI (+): 442 (M+H).

**EXAMPLE 115 : PREPARATION OF Methyl 2-[2-*tert*-butyl-7-(2-methoxy-4-methylphenyl)-5-methylpyrazolo[1,5-a]pyrimidin-6-yl]acetate**

To a solution of methyl 2-(2-*tert*-butyl-7-chloro-5-methylpyrazolo[1,5-*a*]pyrimidin-6-yl)-pentanoate (0.120 g; 0.355 mmol) and 2-methoxy-4-methylphenylboronic acid (0.085 g; 0.512 mmol) in a mixture of water/DME (1/3) (3 mL) were added palladiumtetrakistriphenylphosphine (0.043 g; 0.038 mmol) and diisopropylethylamine (0.3 mL; 1.72 mmol). The solution was stirred for 1 hour at 140°C under microwave irradiation. Ethyl acetate was added to the reaction mixture and the solution was successively washed with a 1N solution hydrochloric acid, a 1N sodium hydrogenocarbonate and brine. The organic layer was dried over magnesium sulphate, filtered and concentrated under reduced pressure. Purification by flash chromatography on silica gel using a gradient of ethyl acetate (0 - 20%) in heptane furnished 0.168 g of the title compound as a bright yellow oil, contaminated with methyl 2-(2-tert-butyl-5-methylpyrazolo[1,5-a]pyrimidin-6-yl)pentanoate. ESI/APCI (+): 423 (M+H).

**EXAMPLE 116 : PREPARATION OF 2-[2-*tert*-butyl-7-(2-methoxy-4-methylphenyl)-5-methylpyrazolo[1,5-a]pyrimidin-6-yl]acetic acid**

To a solution of methyl 2-(2-*tert*-butyl-7-(2-methoxy-4-methylphenyl)-5-methylpyrazolo[1,5-*a*]pyrimidin-6-yl)pentanoate (0.128 g; 0.3 mmol) in methanol (3 mL) was added a 5N solution of sodium hydroxide (0.300 mL; 1.50 mmol) and the reaction mixture was heated at 75°C for 24 hours. The mixture was acidified with a 1N solution of hydrochloric acid and the product was extracted with ethyl acetate. The organic layer was washed with a saturated solution of sodium hydrogenocarbonate and brine, dried over magnesium sulphate, filtered and concentrated under reduced pressure. Purification by preparative HPLC according to described method 2 furnished 2 peaks with the same mass. The first peak was eluted at Rt=6.37 min (0.0207 g, 17%) and the second peak was eluted at Rt=6.80 min (0.0074 g, 6%). ESI/APCI (+): 410 (M+H).

**EXAMPLE 117 : PREPARATION OF 2-[2-*tert*-butyl-7-(2-hydroxy-4-methylphenyl)-5-methylpyrazolo[1,5-a]pyrimidin-6-yl]acetic acid**

To a solution of 2-(2-*tert*-butyl-7-(2-methoxy-4-methylphenyl)-5-methylpyrazolo[1,5-a]pyrimidin-6-yl)pentanoic acid (0.080 g; 0.195 mmol) in pyridine (0.75 mL) was added lithium iodide (0.135 g; 1.01 mmol) and the solution was heated at 170°C for 90 min. under microwave irradiation. Ethyl acetate was added to the reaction mixture and it was washed with a 1 N solution of hydrochloric acid. The aqueous layer was extracted with ethyl acetate, the combined organic layers were washed with brine, dried over magnesium sulphate, filtered and concentrated under reduced pressure. Purification by preparative HPLC according to described method 2 furnished 0.018 g (23%) of the title compound as a white solid. ESI/APCI (+): 396 (M+H).

**EXAMPLE 118 : PREPARATION OF Methyl 2-(2-isopropyl-5-methyl-7-*p*-tolyl-[1,2,4]-triazolo[1,5-*a*]pyrimidin-6-yl)pentanoate**

To a solution of methyl 2-(7-chloro-2-isopropyl-5-methyl-[1,2,4]triazolo[1,5-a]pyrimidin-6-yl)pentanoate (0.122 g; 0.376 mmol) in water/DME (1/3, 3 mL) were added 4-tolylboronic acid (0.100 g; 0.736 mmol), palladiumtetrakistriphenylphosphine (0.045 g; 0.039 mmol) and diisopropylethylamine (0.265 mL; 1.52 mmol). The solution was heated for 40 min at 140°C under microwave irradiation. A 1N solution of hydrochloric acid was added to the reaction mixture and the product was extracted with ethyl acetate. The organic layer was washed with a 1N solution of sodium hydrogenocabonate, brine, dried over magnesium sulphate, filtered and concentrated under reduced pressure. Purification by flash chromatography on silica gel using a gradient of ethyl acetate (0 - 40%) in heptane furnished 0.103 g (72%) of the title compound as a pale yellow oil. ESI/APCI (+): 381 (M+H).

**EXAMPLE 119 : PREPARATION OF 2-(2-isopropyl-5-methyl-7-*p*-tolyl-[1,2,4]-triazolo[1,5-a]pyrimidin-6-yl)pentanoic acid**

To a solution of methyl 2-(2-isopropyl-5-methyl-7-*p*-tolyl-[1,2,4]triazolo[1,5-*a*]pyrimidin-6-yl)pentanoate (0.103 g; 0.271 mmol) in methanol (2.5 mL) was added a 5N solution of sodium hydroxide (0.300 mL; 1.50 mmol) and the solution was heated under reflux for 3 hours. The solvent was evaporated and a 1N solution of hydrochloric acid was added to the residue. The mixture was sonicated for 5 min and the precipitate was filtered, washed with water and dried under high vacuum to furnish 0.082 g (83%) of the title compound as a white solid. ESI/APCI (+): 367 (M+H). ESI/APCI (-): 365 (M-H).

**EXAMPLE 120 : PREPARATION OF Ethyl 2-(2-benzyl-5-methyl-7-*p*-tolyl-[1,2,4]-triazolo[1,5-*a*]pyrimidin-6-yl)pentanoate**

To a solution of ethyl 2-(2-benzyl-7-chloro-5-methyl-[1,2,4]triazolo[1,5-a]pyrimidin-6-yl)-pentanoate (0.134 g; 0.346 mmol) and 4-tolylboronic acid (0.091 g; 0.669 mmol) in a mixture of water/DME (1/3) (3 mL) were added palladiumtetrakistriphenylphosphine (0.038 g; 0.033 mmol) and diisopropylethylamine (0.250 mL; 1.44 mmol). The solution was heated for 40 min at 140°C under microwave irradiation. A 1N solution of hydrochloric acid was added to the reaction mixture and the product was extracted with ethyl acetate. The organic layer was washed with a 1 N solution of sodium hydrogenocarbonate, brine, dried over magnesium sulphate, filtered and concentrated under reduced pressure. Purification by flash chromatography on silica gel using a gradient of ethyl acetate (0 - 50%) in heptane furnished 0.152 g (99%) of the title compound as a sticky white solid. ESI/APCI (+): 443 (M+H).

**EXAMPLE 121 : PREPARATION OF Ethyl 2-(2-benzyl-5-methyl-7-*p*-tolyl-[1,2,4]-triazolo[1,5-*a*]pyrimidin-6-yl)pentanoate**

To a solution of ethyl 2-(2-benzyl-5-methyl-7-*p*-tolyl-[1,2,4]triazolo[1,5-a]pyrimidin-6-yl)-pentanoate (0.152 mg; 0.344 mmol) in methanol (7 mL) was added a 5N solution of sodium hydroxide (0.350 mL; 1.75 mmol) and the solution was heated at reflux for 24 hours. The volatiles were evaporated and a 1N solution of hydrochloric acid was added to the residue. The product was extracted with ethyl acetate and the combined organic fractions were washed with a 1N solution of hydrochloric acid, brine, dried over magnesium sulphate, filtered and concentrated under reduced pressure. Purification by preparative HPLC according to described method 1 furnished 0.077 g (54%) of the title compound as a white solid. ESI/APCI (+): 415 (M+H). ESI/APCI (-): 413 (M-H).

PART B: ANTIVIRAL ACTIVITY OF THE COMPOUNDS

**EXAMPLE 122 : EVALUATION OF THE ANTI-HIV ACTIVITY OF THE COMPOUNDS OF THE INVENTION**

A rapid and automated assay procedure was used for the in vitro evaluation of anti-HIV agents. An HTLV-1 transformed T4-cell line MT-4, which was previously shown to be highly susceptible to and permissive for HIV infection, served as the target cell line. Inhibition of the HIV-induced cytopathogenic effect was used as the end point. The viabitlity of both HIV-and mock-infected cells was assessed spectrophotometrically via in situ reduction of 3-(4,5-dimethylthiazol-2-yl)-2,5-diphenyltetrazolium bromide (MTT). The 50 % cytotoxic concentration ($CC_{50}$ in μg/ml) was defined as the concentration of compound that reduced the absorbance of the mock-infected control sample by 50%. The percent protection achieved by the compound in HIV-infected cells was calculated by the following formula:

$$\frac{(OD_T)_{HIV} - (OD_C)_{HIV}}{(OD_C)_{MOCK} - (OD_C)_{HIV}} \quad \text{expressed in \%}$$

whereby $(OD_T)_{HIV}$ is the optical density measured with a given concentration of the test compound in HIV-infected cells; $(OD_C)_{HIV}$ is the optical density measured for the control untreated HIV-infected cells; $(OD_C)_{MOCK}$ is the optical density measured for the control untreated mock-infected cells; all optical density values were determined at 540 nm. The dose achieving 50% protection according to the above formula was defined as the 50% inhibitory concentration ($EC_{50}$ in μg/ml or μM). The ratio of $CC_{50}$ to $EC_{50}$ was defined as the selectivity index (SI). Examples of $EC_{50}$, $CC_{50}$ and SI values for inhibition of proliferation of HIV by particular compounds of the invention are listed in table 3 herein below.

Examples of inhibition of cell proliferation by particular compounds of the invention can be found by looking at

the respective $CC_{50}$ values in the MT-4 cell line.

Cells: MT-4 cells (Miyoshi *et al.*, 1982) were grown and maintained in RPMI 1640medium supplemented with 10% heat-inactivated fetal calf serum, 2 mM I-glutamine, 0.1% sodium bicarbonate, and 20æg of gentamicin per ml.

Viruses: The HIV-1(NL4.3) strain (Adachi *et al.*, 1986) is a molecular clone obtained from the National Institutes of Health (Bethesda, MD). The HIV-2(ROD) (Barr,-Sinoussi et al., 1983) stock was obtained from culture supernatant of HIV-2 infected cell lines.

References:

Adachi, A., Gendelman, H., Koenig, S., Folks, T., Willey, R., Rabson, A. and Martin, M (1986) Production of acquired immunodeficiency syndrome-associated retrovirus in human and nonhuman cells transfected with an infectious molecular clone, J. Virol., 59, 284-291.

Barr-Sinoussi, F., Chermann, J.C., Rey, F., Nugeyre, M.T., Chamaret, S., Gruest, J., Dauguet, C., Axler-Blin, C., V,zinet-Brun, F., Rouzioux, C., Rozenbaum, W., Montagnier, L. (1983) Isolation of a T-lymphotropic retrovirus from patient at risk for AIDS, Science (Wash DC) 220, 868-871.Miyoshi, I., Taguchi, H., Kobonishi, I., Yoshimoto, S., Ohtsuki, Y., Shiraishi, Y. andAkagi,T. (1982) Type C virus-producing cell lines derived from adult T cell leukemia, Gann mongr, 28, 219-228.

**EXAMPLE 123: ALPHASCREEN ASSAY TO MEASURE THE LEDGF-INTEGRASE INTERACTION INHIBITORY ACTIVITY OF COMPOUNDS OF THE INVENTION**

The AlphaScreen assay was performed according to the manufacturer's protocol (Perkin Elmer, Benelux). Reactions were performed in 25 μl final volume in 384-well Optiwell™ microtiter plates (Perkin Elmer). The reaction buffer contained 25 mM Tris-HCl (pH 7.4), 150 mM NaCl, 1 mM $MgCl_2$, 0.01% (v/v) Tween-20 and 0.1% (w/v) bovine serum albumin. $His_6$-tagged integrase (300nM final concentration) was incubated with the compounds for 30 min at 4°C. The compounds were added at varying concentrations spanning a wide range from 0.1 up to 100 μM. Afterwards 100 nM flag-LEDGF/p75 was added and incubation was prolonged for an additional hour at 4°C. Subsequently 5 μl of Ni-chelate - coated acceptor beads and 5 μl anti-flag donor beads were added to a final concentration of 20 μg/ml of both beads. Proteins and beads were incubated for 1 h at 30°C in order to allow association to occur. Exposure of the reaction to direct light was omitted as much as possible and the emission of light from the acceptor beads was measured in the EnVision plate reader (Perkin Elmer, Benelux) and analyzed using the EnVision manager software. IN/DNA binding was analyzed in a similar setting using $His_6$-tagged integrase (1 μM final concentration) and an oligodeoxynucleotide mimicking the IN ELISA oligonucleotide substrate (30 nM final concentration). Counterscreens with JPO2 or PogZ, respectively, were essentially performed as described previously. Expression and purification of recombinant proteins: $His_6$-tagged HIV-1 integrase, 3xflag-tagged LEDGF/p75, MBP-JPO2 and MBP-PogZ were purified for AlphaScreen applications as described previously.

References:

Bartholomeeusen, K., et al. Differential interaction of HIV-1 integrase and JPO2 with the C terminus of LEDGF/p75. J. Mol. Biol. 372, 407-421 (2007).

Bartholomeeusen, K., et al. Lens Epithelium Derived Growth Factor/p75 interacts with the transposase derived DDE domain of pogZ. J. Biol. Chem. (2009).

Busschots, K., et al. The interaction of LEDGF/p75 with integrase is lentivirus-specific and promotes DNA binding. J. Biol. Chem. 280, 17841-17847 (2005).

Compounds of the invention showed an anti-HIV activity and examples thereof are listed in Table 4.

Table 4

| Cpd code | Alpha screen % (100μM) | EC50 (100μM) | EC50 (μm) | CC50 (μM) | SI |
|---|---|---|---|---|---|
| CPD-57 | 84 | 27,19+/-0,24 | 191,6+/-31,9 | >250 | >1 |
| CPD-58 | 77 | 35.04 | 27,28 | 121,33+/- 17,04 | 5 |

(continued)

| Cpd code | Alpha screen % (100μM) | EC50 (100μM) | EC50 (μm) | CC50 (μM) | SI |
|---|---|---|---|---|---|
| CPD-59 | 92 | 47,72+/- 12,84 | 14,33+/-0,43 | >250 | >18 |
| CPD-60 | 92 | 6.12 | 2,39+/-0,58 | 56,33+/- 17,95 | 23 |
| CPD-61 | 94 | 20.65 | 30,25+/-7,1 | 104+/-6,55 | 4 |
| CPD-65 | 30 | | 5,69+/-0,49 | 46,5+/-12,5 | 8 |
| CPD-66 | 92 | 11.93 | 10,5+/-0,07 | 99+/-8 | 9 |
| CPD-67 | 93 | 6.99 | 2,94+/-0,77 | 70+/-12 | 24 |
| CPD-68 | 93 | 3.89 | 6,42+/-0,7 | 101+/-4 | 16 |
| CPD-69 | 93 | 2.91 | 1,61+/-0,12 | 40,33+/- 16,92 | 25 |
| CPD-70 | 93 | 1.01 | 72,13+/- 35,71 | >125 | >1 |
| CPD-71 | 74 | | 2,8+/-1,03 | 73+/-5 | 26 |
| CPD-72 | 93 | 1.55 | 22,63+/- 10,14 | >125 | <6 |
| CPD-77 | 83 | 29,2+/-3,8 | 28,1 | 203+/-13 | 7 |
| CPD-78 | 83 | 17.68 | 21,16+/-1,8 | 127+/-5 | 6 |
| CPD-79 | 75 | 41,8+/-14,2 | 1,89 | 76+/-25 | 40 |
| CPD-80 | 75 | 97,3+/-15,82 | 4,08+/-0,65 | 191,5+/-59,5 | 47 |
| CPD-81 | 65 | 62.67 | 16,41 | 56 | 3 |
| CPD-83 | 50 | | 25,2+/-8 | 91 +/-8 | 9 |
| CPD-84 | 34 | | 45,06 | 72,66+/-6,65 | >1 |
| CPD-85 | 76 | 5.1 | 2,54+/-1,0 | 125,33+/- 27,22 | 49 |
| CPD-86 | | | | 77 | |
| CPD-87 | 67 | 6.3 | 2,33+/-1,04 | 38,33+/- 15,63 | 16 |
| CPD-88 | 44 | | 38,03+/-3,33 | >250 | >7 |
| CPD-89 | 89 | | 23,98 | 139 | 6 |
| CPD-90 | 70 | | 6,15 | 22 | 4 |
| CPD-91 | 90 | 9.4 | 15,69+/-6,35 | 85,5+/-7,5 | 5 |
| CPD-92 | 95 | 5.4 | 9,13+/-4,1 | 60,5+/-6,5 | 7 |
| CPD-93 | 90 | | 7.4+/-0.32 | 119+/-6 | 16 |
| CPD-95 | 83 | | 7,48 | 115 | 15 |
| CPD-98 | 89 | | 5,91 | 69 | 12 |
| CPD-99 | 81 | | 1,01 | 34 | 34 |
| CPD-100 | 92 | 13.71 | 6,24 | 23 | 4 |
| PD-105 | | | 23,23 | 235 | 10 |
| CPD-111 | 89 | 52.25 | | 50 | |
| CPD-112 | 93 | >100 | | 59 | |
| CPD-117 | 95 | 2.15 | 2.06 | 116.5 | 57 |
| CPD-121 | | 76.1 | 123.51 | >250 | >2 |
| CPD-123 | | 15.2 | 26.08 | 126 | 5 |

The invention has been described in detail sufficient to allow one of ordinary skill in the art to make and use the subject matter of the following Embodiments. Many modifications are possible in the embodiments without departing from the teachings thereof. All such modifications are intended to be encompassed within the claims of the invention.

## Claims

1. A compound according to the formula (A):

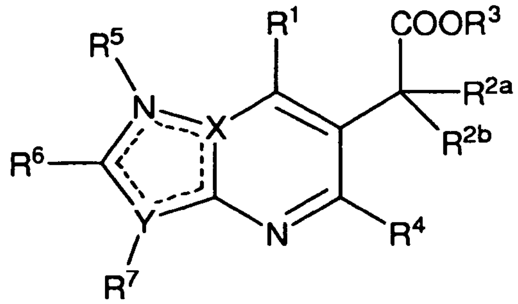

(A)

wherein,

- each dotted line represents an optional double bond whereby two dotted lines of the 5 dotted lines constitute a double bond and these 2 double bonds are non-adjacent;
- each of X and Y are independently selected from C or N, whereby at least one of X and Y is N;
- $R^1$ is independently selected from cycloalkyl; cycloalkenyl; cycloalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl or heterocycle-heteroalkynyl;
and wherein said cycloalkyl, cycloalkenyl, cycloalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more $R^{10}$;
- each of $R^{2a}$ and $R^{2b}$ is independently selected from hydrogen; cyano; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl, heterocycle-heteroalkenyl, or heterocycle-heteroalkynyl; or $R^{2a}$ and $R^{2b}$ can be taken together to form vinyl or vinylalkyl;
and wherein said alkyl, alkenyl, alkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, - C(O)OH or $NH_2$;
- $R^3$ is independently selected from hydrogen; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- $R^4$ is independently selected from hydrogen; alkyl; alkenyl or alkynyl; wherein said alkyl, alkenyl or alkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
- $R^5$ is not present or is selected from hydrogen; alkyl; alkenyl; alkynyl; heteroalkyl; heteroalkenyl; and heteroalkynyl;

wherein said alkyl, alkenyl, alkynyl, heteroalkyl, heteroalkenyl, and heteroalkynyl can be unsubstituted or substituted with one or more one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;

- $R^6$ is selected from hydrogen; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl;

wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;

- $R^7$ is selected from being not present; hydrogen; halogen; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl;

wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O halogen, -SH, =S, trifluoromethyl, $-OCF_3$, cyano, nitro, -C(O)OH or $NH_2$;

- each $R^{10}$ is independently selected from the group consisting of halogen; -OR"; =O -SR"; =S; $-S(O)R^{12}$; $-S(O)_2R^{12}$; $-S(O)_2NR^{13}R^{14}$; trifluoromethyl; nitro; $-NR^{13}R^{14}$; $-NR^{11}S(O)_2R^{12}$; cyano; $-C(O)OR^{11}$; $-C(O)NR^{13}R^{14}$; $-C(O)R^{12}$; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl, or heterocycle-heteroalkynyl;

and wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;

- each R" is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl; heterocycle-heteroalkenyl; and heterocycle-heteroalkynyl;

wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocycle-alkynyl, heterocycle-heteroalkyl, heterocycle-heteroalkenyl, and heterocycle-heteroalkynyl can be unsubstituted or substituted with one or more independently selected alkyl, alkenyl, alkynyl, heteroalkyl, heteroalkenyl, heteroalkynyl, hydroxyl, =O halogen, -SH, =S, trifluoromethyl, $-OCF_3$, cyano, nitro, -C(O)OH or $NH_2$;

- each $R^{12}$ is independently selected from hydrogen; hydroxyl; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl; heterocycle-heteroalkenyl; and heterocycle-heteroalkynyl;

wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocycle-alkynyl, heterocycle-heteroalkyl, heterocycle-heteroalkenyl, and heterocycle-heteroalkynyl can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;

- each $R^{13}$ and $R^{14}$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl; heterocycle-heteroalkenyl; and heterocycle-heteroalkynyl;

and wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocycle-alkynyl, heterocycle-heteroalkyl, heterocycle-heteroalkenyl, and heterocycle-heteroalkynyl

can be unsubstituted or substituted with one or more independently selected alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH or $NH_2$;
and wherein $R^{13}$ and $R^{14}$ can be taken together with the N to which they are attached in order to form a (5-, 6-, or 7-membered) heterocycle which can be unsubstituted or substituted with one or more independently selected alkyl, alkenyl, alkynyl, heteroalkyl, heteroalkenyl, heteroalkynyl, hydroxyl, =O halogen, -SH, =S, trifluoromethyl, $-OCF_3$, cyano, nitro, -C(O)OH or $NH_2$;

or a pharmaceutically acceptable salt thereof.

**2.** The compound according to claim 1, wherein $R^3$ is H.

**3.** The compound according to claim 1 or 2, wherein $R^1$ is selected from aryl or heteroaryl, wherein said aryl or heteroaryl can be unsubstituted or substituted with one or more $R^{10}$.

**4.** The compound according to claims 1 to 3, wherein one of $R^{2a}$ and $R^{2b}$ is hydrogen, and the other of $R^{2a}$ and $R^{2b}$ is selected from hydrogen; cyano; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocycle-alkynyl; heterocycle-heteroalkyl, heterocycle-heteroalkenyl, or heterocycle-heteroalkynyl; or $R^{2a}$ and $R^{2b}$ can be taken together to form vinyl or vinylalkyl;
and wherein said alkyl, alkenyl, alkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted;

**5.** The compound according to claims 1 to 4, wherein the compound has a structure according to formula (C-I) or (C-II),

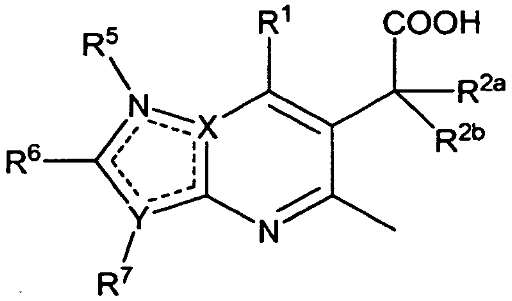
(C-I)

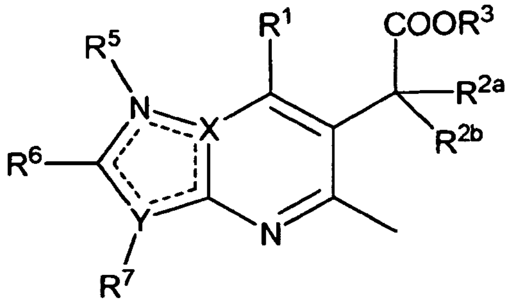
(C-II)

wherein each of X, Y, the dotted lines, $R^1$, $R^{2a}$, $R^{2b}$, $R^5$, $R^6$, and $R^7$ are as in claims 1 to 4.

**6.** The compound according to claims 1 to 5, wherein the compound has a structure according to formula (E), which consist of formulas (E-I), (E-II), (E-III), or (E-IV),

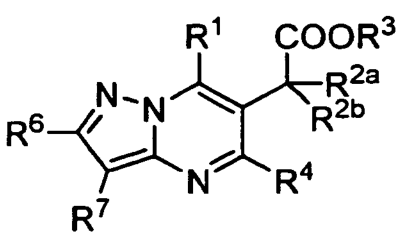
(E-I)

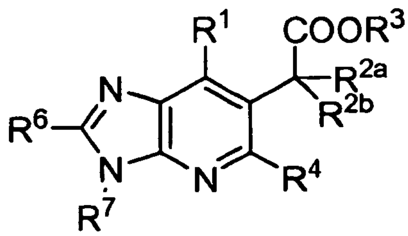
(E-II)

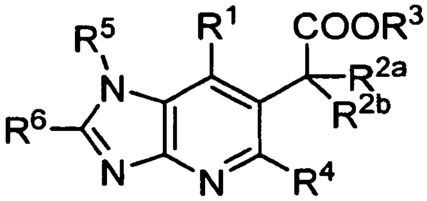

(E-III)

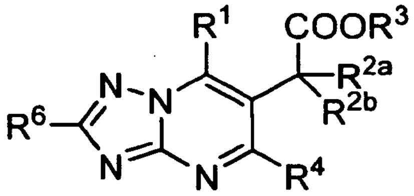

(E-IV)

wherein each of $R^1$, $R^{2a}$, $R^{2b}$, $R^3$, $R^4$, $R^5$, $R^6$, and $R^7$are as in claims 1 to 4.

7. The compound according to claim 1, which is:

ethyl 2-(7-((R)-3-(4-chlorophenylsulfonamido)piperidin-1-yl)-5-methylpyrazolo[1,5-a]pyrimidin-6-yl)pentanoate;
ethyl 2-(7-((S)-3-(4-chlorophenylsulfonamido)piperidin-1-yl)-5-methylpyrazolo[1,5-a]pyrimidin-6-yl)pentanoate;
ethyl 2-(7-((R)-3-(4-chlorophenylsulfonamido)pyrrolidin-1-yl)-5-methylpyrazolo[1,5-a]pyrimidin-6-yl)pentanoate;
ethyl 2-(7-((S)-3-(4-chlorophenylsulfonamido)pyrrolidin-1-yl)-5-methylpyrazolo[1,5-a]pyrimidin-6-yl)pentanoate;
methyl 2-(5-methyl-7-phenylpyrazolo[1,5-a]pyrimidin-6-yl)pentanoate;
methyl 2-(5-methyl-7-p-tolylpyrazolo[1,5-a]pyrimidin-6-yl)pentanoate;
methyl 2-(2-tert-butyl-5-methyl-7-phenylpyrazolo[1,5-a]pyrimidin-6-yl)pentanoate;
methyl 2-(2-tert-butyl-5-methyl-7-p-tolylpyrazolo[1,5-a]pyrimidin-6-yl)pentanoate;
methyl 2-(5-methyl-2-phenyl-7-p-tolylpyrazolo[1,5-a]pyrimidin-6-yl)pentanoate;
methyl 2-(2-tert-butyl-7-(3-hydroxyphenyl)-5-methylpyrazolo[1,5-a]pyrimidin-6-yl)pentanoate;
methyl 2-(2-tert-butyl-7-(2-naphthyl)-5-methylpyrazolo[1,5-a]pyrimidin-6-yl)pentanoate;
methyl 2-(2-tert-butyl-7-(1H-indol-5-yl)-5-methylpyrazolo[1,5-a]pyrimidin-6-yl)pentanoate;
methyl 2-(2-tert-butyl-7-(1H-indol-6-yl)-5-methylpyrazolo[1,5-a]pyrimidin-6-yl)pentanoate;
methyl 2-(2-tert-butyl-7-(1-benzofuran-5-yl)-5-methylpyrazolo[1,5-a]pyrimidin-6-yl)pentanoate;
methyl 2-(2-tert-butyl-7-(1-benzothiophen-5-yl)-5-methylpyrazolo[1,5-a]pyrimidin-6-yl)pentanoate;
methyl 2-(2-tert-butyl-7-(2,3-dihydrobenzofuran-5-yl)-5-methylpyrazolo[1,5-a]pyrimidin-6-yl)pentanoate;
methyl 2-(2-tert-butyl-7-(4-chlorophenyl)-5-methylpyrazolo[1,5-a]pyrimidin-6-yl)pentanoate;
methyl 2-(2-tert-butyl-7-(3,4-dimethylphenyl)-5-methylpyrazolo[1,5-a]pyrimidin-6-yl)pentanoate;
methyl 2-(2-tert-butyl-7-(4-ethylphenyl)-5-methylpyrazolo[1,5-a]pyrimidin-6-yl)pentanoate;
methyl 2-(2-tert-butyl-7-(3,4-dihydro-2H-benzo[b][1,4]dioxepin-7-yl)-5-methylpyrazolo[1,5-a]pyrimidin-6-yl)pentanoate;
methyl 2-(2-tert-butyl-7-(2-(7-(4-methyl-3,4-dihydro-2H-benzo[b][1,4]oxazin-7-yl)-5-methylpyrazolo[1,5-a]pyrimidin-6-yl)pentanoate;
ethyl 2-(3-bromo-5-methyl-7-p-tolylpyrazolo[1,5-a]pyrimidin-6-yl)pentanoate;
ethyl 2-(5-methyl-3-phenyl-7-p-tolylpyrazolo[1,5-a]pyrimidin-6-yl)pentanoate;
ethyl 2-(5-methyl-3,7-di-p-tolylpyrazolo[1,5-a]pyrimidin-6-yl)pentanoate;
methyl 2-(3-bromo-2-tert-butyl-5-methyl-7-p-tolylpyrazolo[1,5-a]pyrimidin-6-yl)pentanoate;
methyl 2-(2-tert-butyl-5-methyl-3,7-di-p-tolylpyrazolo[1,5-a]pyrimidin-6-yl)pentanoate;
methyl 2-(5-methyl-2-propyl-7-p-tolylpyrazolo[1, 5-a]pyrim idin-6-yl)pentanoate;
methyl 2-(2-(furan-2-yl)-5-methyl-7-p-tolylpyrazolo[1,5-a]pyrimidin-6-yl)pentanoate;
methyl 2-(2-tert-butyl-7-(4-chloro-2-fluorophenyl)-5-methylpyrazolo[1,5-a]pyrimidin-6-yl)pentanoate;
methyl 2-(2-tert-butyl-7-(2-fluoro-4-methylphenyl)-5-rnethylpyrazolo[1,5-a]pyrimidin-6-yl)pentanoate;
methyl 2-(2-tert-butyl-7-p-tolyl-5-methylpyrazolo[1,5-a]pyrimidin-6-yl)-6,6,6-trifluorohexanoate;
methyl 2-(2-tert-butyl-7-p-tolyl-5-methylpyrazolo[1,5-a]pyrimidin-6-yl)-3-phenylpropanoate;
methyl 2-(2-tert-butyl-7-(1-methylindol-5-yl)-5-methylpyrazolo[1,5-a]pyrimidin-6-yl)-3-phenylpropanoate;
methyl 2-(2-tert-butyl-5-methyl-7-(1-methylindolin-5-yl)pyrazolo[1,5-a]pyrimidin-6-yl)pentanoate;
methyl 2-(2-tert-butyl-5-methyl-7-(1-methyl-1H-indol-6-yl)pyrazolo[1,5,a]pyimidin-6-yl)pentanoate;
methyl 2-(2-tert-butyl-7-(chroman-6-yl)-5-methylpyrazolo[1,5-a]pyrimidin-6-yl)pentanoate;
methyl 2-(2-tert-butyl-7-p-tolyl-5-rnethylpyrazolo[1,5-a]pyrimidin-6-yl)-3-methylpentanoate;
methyl 2-(2-tert-butyl-3-chloro-7-p-tolyl-5-methylpyrazolo[1,5-a]pyrimidin-6-yl)pentanoate;

methyl 2-(2-tert-butyl-7-p-tolyl-5-methylpyrazolo[1,5-a]pyrimidin-6-yl)-4-methoxybutanoate;
methyl 2-(2-tert-butyl-5-methyl-7-(4-iso-propylphenyl)pyrazolo[1,5-a]pyrimidin-6-yl)pentanoate;
methyl 2-(2-tert-butyl-5-methyl-7-(4-trifluoromethylphenyl)pyrazolo[1,5-a]pyrimidin-6-yl)pentanoate;
methyl 2-(2-tert-butyl-7-(2,4-difluorophenyl)-5-methylpyrazolo[1,5-a]pyrimidin-6-yl)pentanoate;
methyl 2-(2-tert-butyl-7-(2-chloro-4-methylphenyl)-5-methylpyrazolo[1,5-a]pyrimidin-6-yl)pentanoate;
methyl 2-(7-(2-amino-4-methylphenyl)-2-tert-butyl-5-methylpyrazolo[1,5-a]pyrimidin-6-yl)pentanoate;
methyl 2-(2-tert-butyl-7-(2-methoxy-4-chlorophenyl)-5-methylpyrazolo[1,5-a]pyrimidin-6-yl)pentanoate;
methyl 2-(2-tert-butyl-7-(2-fluoro-4-methoxyphenyl)-5-methylpyrazolo[1,5-a]pyrimidin-6-yl)pentanoate;
methyl 2-(2-tert-butyl-5-methyl-7-(5-quinoline)pyrazolo[1,5-a]pyrimidin-6-yl)pentanoate;
methyl 2-(2-tert-butyl-5-methyl-7-(8-quinoline)pyrazolo[1,5-a]pyrimidin-6-yl)pentanoate;
methyl 2-(2-tert-butyl-7-(2,4-dimethylphenyl)-5-methylpyrazolo[1,5-a]pyrimidin-6-yl)pentanoate;
methyl 2-(2-tert-butyl-3,5-dimethyl-7-p-tolylpyrazolo[1,5-a]pyrimidin-6-yl)pentanoate;
methyl 2-(2-tert-butyl-5-methyl-3-phenyl-7-p-tolylpyrazolo[1,5-a]pyrimidin-6-yl)pentanoate;
methyl 2-(2,3,5-trimethyl-7-p-tolyl-3H-imidazo[4,5-b]pyridin-6-yl)acetate;
methyl 2-(2,3,5-trimethyl-7-p-tolyl-3H-imidazo[4,5-b]pyridin-6-yl)pentanoate;
ethyl 2-(1,2,5-trimethyl-7-p-tolyl-1H-imidazo[4,5-b]pyridin-6-yl)pentanoate;
ethyl 2-(3,5-dimethyl-2-propyl-7-p-tolyl-3H-imidazo[4,5-b]pyridin-6-yl)pentanoate;
ethyl 2-(3,5-dimethyl-2-isopropyl-7-p-tolyl-3H-imidazo[4,5-b]pyridin-6-yl)pentanoate;
2-((7-((R)-3-(4-chlorophenylsulfonamido)piperidin-1-yl)-5-methylpyrazolo[1,5-a]pyrimidin-6-yl)pentanoic acid;
2-((7-((S)-3-(4-chlorophenylsulfonamido)piperidin-1-yl)-5-methylpyrazolo[1,5-a]pyrimidin-6-yl)pentanoic acid;
2-((7-((R)-3-(4-chlorophenylsulfonamido)pyrrolidin-1-yl)-5-methylpyrazolo[1,5-a]pyrimidin-6-yl)pentanoic acid;
2-((7-((S)-3-(4-chlorophenylsulfonamido)pyrrolidin-1-yl)-5-methylpyrazolo[1,5-a]pyrimidin-6-yl)pentanoic acid;
2-((5-methyl-7-phenylpyrazolo[1,5-a]pyrimidin-6-yl)pentanoic acid;
2-((5-methyl-7-p-tolylpyrazolo(1,5-a]pyrimidin-6-yl)pentanoic acid;
2-((2-tert-butyl-5-methyl-7-phenylpyrazolo[1,5-a]pyrimidin-6-yl)pentanoic acid;
2-((2-tert-butyl-5-methyl-7-p-tolylpyrazolo[1,5-a]pyrimidin-6-yl)pentanoic acid;
2-((3-bromo-2-tert-butyl-5-methyl-7-p-tolylpyrazolo[1,5-a]pyrimidin-6-yl)pentanoic acid;
2-((5-methyl-2-phenyl-7-p-tolylpyrazolo[1,5-a]pyrimidin-6-yl)pentanoic acid;
2-((2-tert-butyl-7-(3-hydroxyphenyl)-5-methylpyrazolo[1,5-a]pyrimidin-6-yl)pentanoic acid;
2-((2-tert-butyl-5-methyl-7-(2-naphthyl)pyrazolo[1,5-a]pyrimidin-6-yl)pentanoic acid;
2-((2-tert-butyl-7-(1H-indol-5-yl)-5-methylpyrazolo[1,5-a]pyrimidin-6-yl)pentanoic acid;
2-((2-tert-butyl-7-(1H-indol-6-yl)-5-methylpyrazolo[1,5-a]pyrimidin-6-yl)pentanoic acid;
2-((7-(benzofuran-5-yl)-2-tert-butyl-5-methylpyrazolo[1,5-a]pyrimidin-6-yl)pentanoic acid;
2-((7-(benzo[b]thiophen-5-yl)-2-tert-butyl-5-methylpyrazolo[1,5-a]pyrimidin-6-yl)pentanoic acid;
2-((2-tert-butyl-7-(2,3-dihydrobenzofuran-5-yl)-5-methylpyrazolo[1,5-a]pyrimidin-6-yl)pentanoic acid;
2-((2-tert-butyl-7-(4-chlorophenyl)-5-methylpyrazolo[1,5-a]pyrimidin-6-yl)pentanoic acid;
2-((2-tert-butyl-7-(3,4-dimethylphenyl)-5-methylpyrazolo[1,5-a]pyrimidin-6-yl)pentanoic acid;
2-((2-tert-butyl-7-(4-ethylphenyl)-5-methylpyrazolo[1,5-a]pyrimidin-6-yl)pentanoic acid;
2-((2-tert-butyl-7-(3,4-dihydro-2H-benzo[b][1,4]dioxepin-7-yl)-5-methylpyrazolo[1,5-a]pyrimidin-6-yl)pentanoic acid;
2-((2-tert-butyl-7-(4-methyl-3,4-dihydro-2H-benzo[b][1,4]oxazin-7-yl)-5-methylpyrazolo[1,5. a]pyrimidin-6-yl)pentanoic acid;
2-((5-methyl-3-phenyl-7-p-tolylpyrazolo[1,5-a]pyrimidin-6-yl)pentanoic acid;
2-((3,7-di-p-tolyl-5-methylpyrazolo[1,5-a]pyrimidin-6-yl)pentanoic acid;
2-((2-tert-butyl-3,7-di-p-tolyl-5-methylpyrazolo[1,5-a]pyrimidin-6-yl)pentanoic acid;
2-((5-methyl-2-propyl-7-p-tolylpyrazolo[1,5-a]pyrimidin-6-yl)pentanoic acid;
2-((2-(furan-2-yl)-5-methyl-7-p-tolylpyrazolo[1,5-a]pyrimidin-6-yl)pentanoic acid;
2-((2-tert-butyl-7-(4-chloro-2-fluorophenyl)-5-methylpyrazolo[1,5-a]pyrimidin-6-yl)pentanoic acid;
2-((2-tert-butyl-7=(2=fluoro-4-methylphenyl)-5-methylpyrazolo[1,5-a]pyrimidin-6-yl)pentanoic acid;
2-((2-tert-butyl-7-p-tolyl-5-methylpyrazolo[1,5-a]pyrimidin-6-yl)-6,6;6-trifluorohexanoic acid;
2-((2-tert-butyl-7-p-tolyl-5-methylpyrazolo[1,5-a]pyrimidin-6-yl)-3-phenylpropanoic acid;
2-((2-tert-butyl-5-methyl-7-(1-methylindolin-5-yl)pyrazolo[1,5-a]pyrimidin-6-yl)pentanoic acid;
2-((2-tert-butyl-5-methyl-7-(1-methyl-1H-indol-6-yl)pyrazolo[1,5-a]pyrimidin-6-yl)pentanoic acid,
2-((2-tert-butyl-7-(chrorman-6-yl)-5-methylpyrazolo[1,5-a]pyrimidin-6-yl)pentanoic acid;
2-((2-tert-butyl-7-p-tolyl-5-methylpyrazolo[1,5-a]pyrimidin-6-yl)-3-methylpentanoic acid;
2-((2-tert-butyl-3-chloro-7-p-tolyl-5-methylpyrazol[1,5-a]pyrimidin-6-yl)pentanoic acid;
2-((2-tert-butyl-7-p-tolyl-5-methylpyrazolo[1,5-a]pyrimidin-6-yl)-4-methoxybutanoic acid;
2-((2-tert-butyl-5-methyl-7-(4-isopropylphenyl)pyrazolo[1,5-a]pyrimidin-6-yl)pentanoic acid;

2-((2-tert-butyl-5-methyl-7-(4-trifluoromethylphenyl)pyrazolo[1,5-a]pyrimidin-6-yl)pentanoic acid;
2-((2-tert-butyl-7-(2,4-difluorophenyl)-5-methylpyrazolo[1,5-a]pyrimidin-6-yl)pentanoic acid; 2-(2-tert-butyl-7-(2-chloro-4-methylphenyl)-5-methylpyrazolo[1,5-a]pyrimidin-6-yl)pentanoic acid;
2-((7-(2-amino-4-methylphenyl)-2-tert-butyl-5-methylpyrazolo[1,5-a]pyrimidin-6-yl)pentanoic acid;
2-((2-tert-butyl-7-(2-methoxy-4-chlorophenyl)-5-methylpyrazolo[1,5-a]pyrimidin-6-yl)pentanoic acid;
2-((2-tert-butyl-7-(2-fluoro-4-methoxyphenyl)-5-methylpyrazolo[1,5-a]pyrimidin-6-yl)pentanoic acid;
2-((2-tert-butyl-5-methyl-7-(5-quinoline)pyrazolo[1,5-a]pyrimidin-6-yl)pentanoic acid;
2-((2-tert-butyl-5-methyl-7-(8-quinoline)pyrazolo[1,5-a]pyrimidin-6-yl)pentanoic acid;
2-((2-tert-butyl-7-(2,4-dimethylphenyl)-5-methylpyrazolo[1,5-a]pyrimidin-6-yl)pentanoic acid;
2-((2-tert-butyl-3,5-dimethyl-7-p-tolylpyrazolo[1,5-a]pyrimidin-6-yl)pentanoic acid;
2-((2-tert-butyl-5-methyl-3-phenyl-7-p-tolylpyrazolo-[1,5-a]pyrimidin-6-yl)pentanoic acid;
2-((2,3,5-trimethyl-7-p-tolyl-3H-imidazo[4,5-b]pyridin-6-yl)pentanoic acid;
2-((1,2,5-trimemyl-7-p-tolyl-1H-imidazo[4,5.b]pyridin-6-yl)pentanoic acid;
2-((2-propyl-3,5-dimethyl-7-p-tolyl-3H-imidazo[4,5-b]pyidin-6-yl)pentanoic acid;
2-((2-isopropyl-3,5-dimethyl-7-p-tolyl-3H-imidazo[4,5-b]pyridin-6-yl)pentanoate; methyl 2-(2-isopropyl-5-methyl-7-*p*-tolyl-[1,2,4]-triazolo(1,5-*a*]pyrimidin-6-yl)acetate;
2-((2-isopropyl-5-methyl-7-*p*-tolyl-[1,2,4]-triazolo[1,5-*a*]pyrimidin-6-yl)acetic acid;
methyl 2-[2-*tert*-butyl-7-(1,2-dihydroacenaphthylen-5-yl)-5-methylpyrazolo[1,5-a]pyrimidin-6-yl]acetate;
2-[2-*tert*-butyl-7-(1,2-dihydroacenaphthylen-5-yl)-5-methylpyrazolo[1,5-a]pyrimidin-6-yl]acetic acid;
methyl 2-[2-*tert*-butyl-7-(2-methoxy-4-methyl)-5-methylpyrazolo[1,5-a]pyrimidin yl]acetate;
2-[2-*tert*-butyl-7-(2-methoxy-methylpheny)-5-methylpyrazolo[1,5-a]pyrimidin-6-yl]acetic acid;
2-[2-*tert*-butyl-7-(2-hydroxy-4-methylphenyl)-5-methylpyrazolo[1,5-a]pyrimidin-6-yl-acetic acid;
methyl 2-(2-isopropy-5-methyl-7-*p*-tolyl-[1,2,4]-triazolo-[1,5-*a*]pyrimidin-6-yl)pentanoate
2-((2-isopropyl-5-methyl-7-*p*-tolyl-[1,2,4]-triazolo[1,5-*a*]pyrimidin-6-yl)pentanoic acid;
2-((2-benzyl-5-methyl-7-*p*-tolyl-[1,2,4]-triazolo[1,5-*a*]pyrimidin-6-yl)pentanoic acid; or
ethyl 2-(2-benzyl-5-methyl-7-*p*-tolyl-[1,2,4]-triazolo[1,5-*a*]pyrimidin-6-yl)pentanoate;

or a pharmaceutically acceptable salt thereof.

**8.** The compounds according to claims 1 to 7, for use as a medicine.

**9.** The compounds according to claim 8, for use as a medicine for the prevention or treatment of a viral infection in an animal.

**10.** The compounds according to claim 9, wherein said viral infection is an infection with HIV.

**11.** The compounds according to claim 9 or 10, wherein said animal is a human.

**12.** The use of compounds according to claims 1 to 7 for the manufacture of a medicament for the prevention or treatment of a viral infection in an animal.

**13.** The use according to claim 12, wherein the viral infection is an infection with HIV.

**14.** A pharmaceutical composition comprising the compounds according to claims 1 to 7 as an active ingredient in admixture with at least a pharmaceutically acceptable carriers.

**15.** The pharmaceutical composition according to Claim 14, further comprising at least one other compound with antiviral activity.

**Patentansprüche**

**1.** Verbindung der Formel (A):

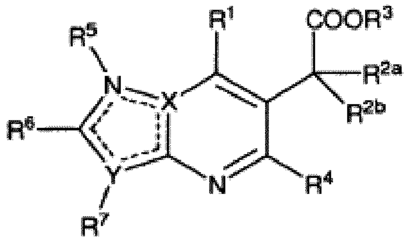

(A)

worin

- jede gepunktete Linie für eine optionale Doppelbindung steht, wobei zwei gepunktete Linien der 5 gepunkteten Linien eine Doppelbindung bilden und diese 2 Doppelbindungen nicht benachbart sind;
- jedes X und Y unabhängig ausgewählt ist aus C oder N, wobei mindestens eines von X und Y N ist;
- $R^1$ unabhängig ausgewählt ist aus Cycloalkyl; Cycloalkenyl; Cycloalkinyl; Aryl; Heterocyclus; Arylalkyl; Arylalkenyl; Arylalkinyl; Arylheteroalkyl; Arylheteroalkenyl; Arylheteroalkinyl; Heterocyclus-Alkyl; Heterocyclus-Alkenyl; Heterocyclus-Alkinyl; Heterocyclus-Heteroalkyl; Heterocyclus-Heteroalkenyl oder Heterocyclus-Heteroalkinyl;
und worin das Cycloalkyl, Cycloalkenyl, Cycloalkinyl, Aryl, Heterocyclus, Arylalkyl, Arylalkenyl, Arylalkinyl, Arylheteroalkyl, Arylheteroalkenyl, Arylheteroalkinyl, Heterocyclus-Alkyl, Heterocyclus-Alkenyl, Heterocyclus-Alkinyl, Heterocyclus-Heteroalkyl, Heterocyclus-Heteroalkenyl oder Heterocyclus-Heteroalkinyl mit einem oder mehreren $R^{10}$ substituiert oder unsubstituiert sein kann;
- jedes $R^{2a}$ und $R^{2b}$ unabhängig ausgewählt ist aus Wasserstoff; Cyano; Alkyl; Alkenyl; Alkinyl; Heteroalkyl; Heteroalkenyl; Heteroalkinyl; Aryl; Arylalkyl; Arylalkenyl; Arylalkinyl; Arylheteroalkyl; Arylheteroalkenyl; Arylheteroalkinyl; Heterocyclus; Heterocyclus-Alkyl; Heterocyclus-Alkenyl; Heterocyclus-Alkinyl; Heterocyclus-Heteroalkyl, Heterocyclus-Heteroalkenyl oder Heterocyclus-Heteroalkinyl; oder $R^{2a}$ und $R^{2b}$ zusammengenommen werden können, um Vinyl oder Vinylalkyl zu bilden;
und worin das Alkyl, Alkenyl, Alkinyl, Aryl, Heterocyclus, Arylalkyl, Arylalkenyl, Arylalkinyl, Arylheteroalkyl, Arylheteroalkenyl, Arylheteroalkinyl, Heterocyclus-Alkyl, Heterocyclus-Alkenyl, Heterocyclus-Alkinyl, Heterocyclus-Heteroalkyl, Heterocyclus-Heteroalkenyl oder Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, - C(O)OH oder $NH_2$, substituiert oder unsubstituiert sein kann;
- $R^3$ unabhängig ausgewählt ist aus Wasserstoff; Alkyl; Alkenyl; Alkinyl; Heteroalkyl; Heteroalkenyl; Heteroalkinyl; Aryl; Heterocyclus; Arylalkyl; Arylalkenyl; Arylalkinyl; Arylheteroalkyl; Arylheteroalkenyl; Arylheteroalkinyl; Heterocyclus-Alkyl; Heterocyclus-Alkenyl; Heterocyclus-Alkinyl; Heterocyclus-Heteroalkyl, Heterocyclus-Heteroalkenyl oder Heterocyclus-Heteroalkinyl;
worin das Alkyl, Alkenyl, Alkinyl, Heteroalkyl, Heteroalkenyl, Heteroalkinyl, Aryl, Heterocyclus, Arylalkyl, Arylalkenyl, Arylalkinyl, Arylheteroalkyl, Arylheteroalkenyl, Arylheteroalkinyl, Heterocyclus-Alkyl, Heterocyclus-Alkenyl, Heterocyclus-Alkinyl, Heterocyclus-Heteroalkyl, Heterocyclus-Heteroalkenyl oder Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $NH_2$, substituiert oder unsubstituiert sein kann;
- $R^4$ unabhängig ausgewählt ist aus Wasserstoff; Alkyl; Alkenyl oder Alkinyl; worin das Alkyl, Alkenyl oder Alkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $NH_2$, substituiert oder unsubstituiert sein kann;
- $R^5$ nicht vorhanden ist oder ausgewählt ist aus Wasserstoff; Alkyl; Alkenyl; Alkinyl; Heteroalkyl; Heteroalkenyl und Heteroalkinyl;
worin das Alkyl, Alkenyl, Alkinyl Heteroalkyl, Heteroalkenyl und Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $NH_2$, substituiert oder unsubstituiert sein kann;
- $R^6$ ausgewählt ist aus Wasserstoff; Alkyl; Alkenyl; Alkinyl; Heteroalkyl; Heteroalkenyl; Heteroalkinyl; Aryl; Heterocyclus; Arylalkyl; Arylalkenyl; Arylalkinyl; Arylheteroalkyl; Arylheteroalkenyl; Arylheteroalkinyl; Heterocyclus-Alkyl; Heterocyclus-Alkenyl; Heterocyclus-Alkinyl; Heterocyclus-Heteroalkyl, Heterocyclus-Heteroalkenyl oder Heterocyclus-Heteroalkinyl;

worin das Alkyl, Alkenyl, Alkinyl, Heteroalkyl, Heteroalkenyl, Heteroalkinyl, Aryl, Heterocyclus, Arylalkyl, Arylalkenyl, Arylalkinyl, Arylheteroalkyl, Arylheteroalkenyl, Arylheteroalkinyl, Heterocyclus-Alkyl, Heterocyclus-Alkenyl, Heterocyclus-Alkinyl, Heterocyclus-Heteroalkyl, Heterocyclus-Heteroalkenyl oder Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $NH_2$, substituiert oder unsubstituiert sein kann;

- $R^7$ entweder nicht vorhanden ist oder ausgewählt ist aus Wasserstoff; Halogen; Alkyl; Alkenyl; Alkinyl; Heteroalkyl; Heteroalkenyl; Heteroalkinyl; Aryl; Heterocyclus; Arylalkyl; Arylalkenyl; Arylalkinyl; Arylheteroalkyl; Arylheteroalkenyl; Arylheteroalkinyl; Heterocyclus-Alkyl; Heterocyclus-Alkenyl; Heterocyclus-Alkinyl; Heterocyclus-Heteroalkyl, Heterocyclus-Heteroalkenyl oder Heterocyclus-Heteroalkinyl;

worin das Alkyl, Alkenyl, Alkinyl, Heteroalkyl, Heteroalkenyl, Heteroalkinyl, Aryl, Heterocyclus, Arylalkyl, Arylalkenyl, Arylalkinyl, Arylheteroalkyl, Arylheteroalkenyl, Arylheteroalkinyl, Heterocyclus-Alkyl, Heterocyclus-Alkenyl, Heterocyclus-Alkinyl, Heterocyclus-Heteroalkyl, Heterocyclus-Heteroalkenyl oder Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $NH_2$, substituiert oder unsubstituiert sein kann;

- jedes $R^{10}$ unabhängig ausgewählt ist aus der Gruppe, bestehend aus Halogen; $-OR^{11}$; =O; $-SR^{11}$; =S; $-S(O)R^{12}$; $-S(O)_2R^{12}$; - $S(O)_2NR^{13}R^{14}$; Trifluormethyl; Nitro; $-NR^{13}R^{14}$. $-NR^{11}S(O)_2R^{12}$; Cyano; $-C(O)OR^{11}$; $-C(O)NR^{13}R^{14}$; $-C(O)R^{12}$; Alkyl; Alkenyl; Alkinyl; Heteroalkyl; Heteroalkenyl; Heteroalkinyl; Aryl; Heterocyclus; Arylalkyl; Arylalkenyl; Arylalkinyl; Arylheteroalkyl; Arylheteroalkenyl; Arylheteroalkinyl; Heterocyclus-Alkyl; Heterocyclus-Alkenyl; Heterocyclus-Alkinyl; Heterocyclus-Heteroalkyl, Heterocyclus-Heteroalkenyl oder Heterocyclus-Heteroalkinyl;

und worin das Alkyl, Alkenyl, Alkinyl, Heteroalkyl, Heteroalkenyl, Heteroalkinyl, Aryl, Heterocyclus, Arylalkyl, Arylalkenyl, Arylalkinyl, Arylheteroalkyl, Arylheteroalkenyl, Arylheteroalkinyl, Heterocyclus-Alkyl, Heterocyclus-Alkenyl, Heterocyclus-Alkinyl, Heterocyclus-Heteroalkyl, Heterocyclus-Heteroalkenyl oder Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $NH_2$, substituiert oder unsubstituiert sein kann;

- jedes $R^{11}$ unabhängig ausgewählt ist aus Wasserstoff; Alkyl; Alkenyl; Alkinyl; Heteroalkyl; Heteroalkenyl; Heteroalkinyl; Aryl; Arylalkyl; Arylalkenyl; Arylalkinyl; Arylheteroalkyl; Arylheteroalkenyl; Arylheteroalkinyl; Heterocyclus; Heterocyclus-Alkyl; Heterocyclus-Alkenyl; Heterocyclus-Alkinyl; Heterocyclus-Heteroalkyl, Heterocyclus-Heteroalkenyl und Heterocyclus-Heteroalkinyl;

worin das Alkyl, Alkenyl, Alkinyl, Heteroalkyl, Heteroalkenyl, Heteroalkinyl, Aryl, Arylalkyl, Arylalkenyl, Arylalkinyl, Arylheteroalkyl, Arylheteroalkenyl, Arylheteroalkinyl, Heterocyclus, Heterocyclus-Alkyl, Heterocyclus-Alkenyl, Heterocyclus-Alkinyl, Heterocyclus-Heteroalkyl, Heterocyclus-Heteroalkenyl und Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, - SH, =S, Trifluormethyl, $-OCF_3$, Cyano, Nitro, -C(O)OH oder $NH_2$, substituiert oder unsubstituiert sein kann;

- jedes $R^{12}$ unabhängig ausgewählt ist aus Wasserstoff; Hydroxyl; Alkyl; Alkenyl; Alkinyl; Heteroalkyl; Heteroalkenyl; Heteroalkinyl; Aryl; Arylalkyl; Arylalkenyl; Arylalkinyl; Arylheteroalkyl; Arylheteroalkenyl; Arylheteroalkinyl; Heterocyclus; Heterocyclus-Alkyl; Heterocyclus-Alkenyl; Heterocyclus-Alkinyl; Heterocyclus-Heteroalkyl, Heterocyclus-Heteroalkenyl und Heterocyclus-Heteroalkinyl;

worin das Alkyl, Alkenyl, Alkinyl, Heteroalkyl, Heteroalkenyl, Heteroalkinyl, Aryl, Arylalkyl, Arylalkenyl, Arylalkinyl, Arylheteroalkyl, Arylheteroalkenyl, Arylheteroalkinyl, Heterocyclus, Heterocyclus-Alkyl, Heterocyclus-Alkenyl, Heterocyclus-Alkinyl, Heterocyclus-Heteroalkyl, Heterocyclus-Heteroalkenyl und Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, - SH, =S, Trifluormethyl, $-OCF_3$, Cyano, Nitro, -C(O)OH oder $NH_2$, substituiert oder unsubstituiert sein kann;

- jedes $R^{13}$ und $R^{14}$ unabhängig ausgewählt ist aus Wasserstoff; Alkyl; Alkenyl; Alkinyl; Heteroalkyl; Heteroalkenyl; Heteroalkinyl; Aryl; Arylalkyl; Arylalkenyl; Arylalkinyl; Arylheteroalkyl; Arylheteroalkenyl; Arylheteroalkinyl; Heterocyclus; Heterocyclus-Alkyl; Heterocyclus-Alkenyl; Heterocyclus-Alkinyl; Heterocyclus-Heteroalkyl, Heterocyclus-Heteroalkenyl und Heterocyclus-Heteroalkinyl;

und worin das Alkyl, Alkenyl, Alkinyl, Heteroalkyl, Heteroalkenyl, Heteroalkinyl, Aryl, Arylalkyl, Arylalkenyl, Arylalkinyl, Arylheteroalkyl, Arylheteroalkenyl, Arylheteroalkinyl, Heterocyclus, Heterocyclus-Alkyl, Heterocyclus-Alkenyl, Heterocyclus-Alkinyl, Heterocyclus-Heteroalkyl, Heterocyclus-Heteroalkenyl und Heterocyclus-Heteroalkinyl mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, - SH, =S, Trifluormethyl, $-OCF_3$, Cyano, Nitro, -C(O)OH oder $NH_2$, substituiert oder unsubstituiert sein kann;

und worin $R^{13}$ und $R^{14}$ mit dem N, an das sie gebunden sind, zusammengenommen werden können, um einen (5-, 6- oder 7-gliedrigen) Heterocyclus zu bilden, der mit einem oder mehreren, die unabhängig ausgewählt sind aus Alkyl, Alkenyl, Alkinyl, Heteroalkyl, Heteroalkenyl, Heteroalkinyl, Hydroxyl, =O, Halogen, - SH, =S, Trifluormethyl, -$OCF_3$, Cyano, Nitro, -C(O)OH oder $NH_2$;
oder einem pharmazeutisch verträglichen Salz davon, substituiert oder unsubstituiert sein kann.

**2.** Verbindung nach Anspruch 1, wobei $R^3$ H ist.

**3.** Verbindung nach Anspruch 1 oder Anspruch 2, wobei $R^1$ ausgewählt ist aus Aryl oder Heteroaryl, wobei das Aryl oder Heteroaryl mit einem oder mehreren $R^{10}$ substituiert oder unsubstituiert sein kann.

**4.** Verbindung nach Anspruch 1 bis 3, wobei eines von $R^{2a}$ und $R^{2b}$ Wasserstoff ist und das andere von $R^{2a}$ und $R^{2b}$ ausgewählt ist aus Wasserstoff; Cyano; Alkyl; Alkenyl; Alkinyl; Heteroalkyl; Heteroalkenyl; Heteroalkinyl; Aryl; Arylalkyl; Arylalkenyl; Arylalkinyl; Arylheteroalkyl; Arylheteroalkenyl; Arylheteroalkinyl; Heterocyclus; Heterocyclus-Alkyl; Heterocyclus-Alkenyl; Heterocyclus-Alkinyl; Heterocyclus-Heteroalkyl, Heterocyclus-Heteroalkenyl oder Heterocyclus-Heteroalkinyl; oder $R^{2a}$ und $R^{2b}$ zusammengenommen werden können, um Vinyl oder Vinylalkyl zu bilden; und wobei das Alkyl, Alkenyl, Alkinyl, Aryl, Heterocyclus, Arylalkyl, Arylalkenyl, Arylalkinyl, Arylheteroalkyl, Arylheteroalkenyl, Arylheteroalkinyl, Heterocyclus-Alkyl, Heterocyclus-Alkenyl, Heterocyclus-Alkinyl, Heterocyclus-Heteroalkyl, Heterocyclus-Heteroalkenyl oder Heterocyclus-Heteroalkinyl substituiert oder unsubstituiert sein kann;

**5.** Verbindung nach Anspruch 1 bis 4, wobei die Verbindung eine Struktur der Formel (C-I) oder (C-II) hat,

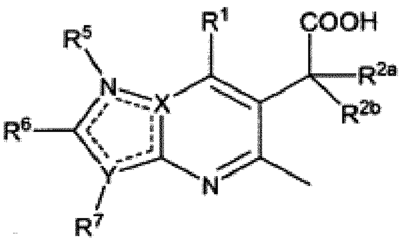
(C-I)
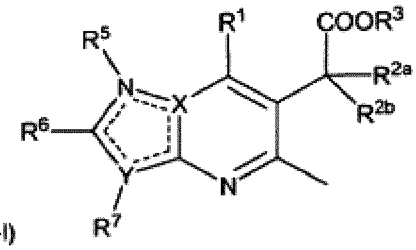
(C-II)

worin jedes X, Y, die gepunkteten Linien, $R^1$, $R^{2a}$, $R^{2b}$, $R^5$, $R^6$ und $R^7$ wie in Anspruch 1 bis 4 sind.

**6.** Verbindung nach Anspruch 1 bis 5, wobei die Verbindung eine Struktur der Formel (E) aufweist, die aus den Formeln (E-I), (E-II), (E-III) oder (E-IV) besteht,

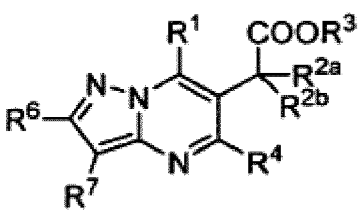
(E-I)
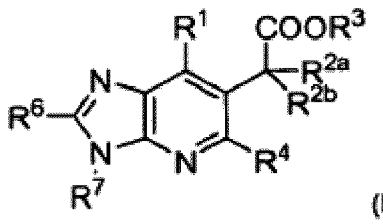
(E-II)
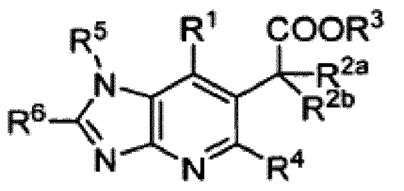
(E-III)
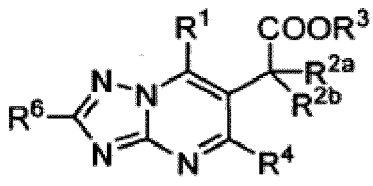
(E-IV)

worin jedes $R^1$, $R^{2a}$, $R^{2b}$, $R^3$, $R^4$, $R^5$, $R^6$ und $R^7$ wie in Anspruch 1 bis 4 ist.

**7.** Verbindung nach Anspruch 1, die Folgendes ist:

Ethyl-2-(7-((R)-3-(4-chlorophenylsulfonamido)piperidin-1-yl)-5-methylpyrazolo[1,5-a]pyrimidin-6-yl)pentanoat;
Ethyl-2-(7-((S)-3-(4-chlorophenylsulfonamido)piperidin-1-yl)-5-methylpyrazolo[1,5-a]pyrimidin-6-yl)pentanoat;
Ethyl-2-(7-((R)-3-(4-chlorophenylsulfonamido)pyrrolidin-1-yl)-5-methylpyrazolo[1,5-a]pyrimidin-6-yl)pentanoat;
Ethyl-2-(7-((S)-3-(4-chlorophenylsulfonamido)pyrrolidin-1-yl)-5-methylpyrazolo[1,5-a]pyrimidin-6-yl)pentanoat;
Methyl-2-(5-methyl-7-phenylpyrazolo[1,5-a]pyrimidin-6-yl)pentanoat;
Methyl-2-(5-methyl-7-p-tolylpyrazolo[1,5-a]pyrimidin-6-yl)pentanoat;
Methyl-2-(2-tert-butyl-5-methyl-7-phenylpyrazolo[1,5-a]pyrimidin-6-yl)pentanoat;
Methyl-2-(2-tert-butyl-5-methyl-7-p-tolylpyrazolo[1,5-a]pyrimidin-6-yl)pentanoat;
Methyl-2-(5-methyl-2-phenyl-7-p-tolylpyrazolo[1,5-a]pyrimidin-6-yl)pentanoat;
Methyl-2-(2-tert-butyl-7-(3-hydroxyphenyl)-5-methylpyrazolo[1,5-a]pyrimidin-6-yl)pentanoae;
Methyl-2-(2-tert-butyl-7-(2-naphthyl)-5-methylpyrazolo[1,5-a]pyrimidin-6-yl)pentanoat;
Methyl-2-(2-tert-butyl-7-(1 H-indol-5-yl)-5-methylpyrazolo[1,5-a]pyrimidin-6-yl)pentanoat;
Methyl-2-(2-tert-butyl-7-(1 H-indol-6-yl)-5-methylpyrazolo[1,5-a]pyrimidin-6-yl)pentanoat;
Methyl-2-(2-tert-butyl-7-(1-benzofuran-5-yl)-5-methylpyrazolo[1,5-a]pyrimidin-6-yl)pentanoat;
Methyl-2-(2-tert-butyl-7-(1-benzothiophen-5-yl)-5-methylpyrazolo[1,5-a]pyrimidin-6-yl)pentanoat;
Methyl-2-(2-tert-butyl-7-(2,3-dihydrobenzofuran-5-yl)-5-methylpyrazolo[1,5-a]pyrimidin-6-yl)pentanoat;
Methyl-2-(2-tert-butyl-7-(4-chlorophenyl)-5-methylpyrazolo[1,5-a]pyrimidin-6-yl)pentanoat;
Methyl-2-(2-tert-butyl-7-(3,4-dimethylphenyl)-5-methylpyrazolo[1,5-a]pyrimidin-6-yl)pentanoat;
Methyl-2-(2-tert-butyl-7-(4-ethylphenyl)-5-methylpyrazolo[1,5-a]pyrimidin-6-yl)pentanoat;
Methyl-2-(2-tert-butyl-7-(3,4-dihydro-2H-benzo[b][1,4]dioxepin-7-yl)-5-methylpyrazolo[1,5-a]pyrimidin-6-yl)pentanoat;
Methyl-2-(2-tert-butyl-7-(2-(7-(4-methyl-3,4-dihydro-2H-benzo[b][1,4]oxazin-7-yl)-5-methylpyrazolo[1,5-a]pyrimidin-6-yl)pentanoat;
Ethyl-2-(3-bromo-5-methyl-7-p-tolylpyrazolo[1,5-a]pyrimidin-6-yl)pentanoat;
Ethyl-2-(5-methyl-3-phenyl-7-p-tolylpyrazolo[1,5-a]pyrimidin-6-yl)pentanoat;
Ethyl-2-(5-methyl-3,7-di-p-tolylpyrazolo[1,5-a]pyrimidin-6-yl)pentanoat;
Methyl-2-(3-bromo-2-tert-butyl-5-methyl-7-p-tolylpyrazolo[1,5-a]pyrimidin-6-yl)pentanoat;
Methyl-2-(2-tert-butyl-5-methyl-3,7-di-p-tolylpyrazolo[1,5-a]pyrimidin-6-yl)pentanoat;
Methyl-2-(5-methyl-2-propyl-7-p-tolylpyrazolo[1,5-a]pyrimidin-6-yl)pentanoat;
Methyl-2-(2-(furan-2-yl)-5-methyl-7-p-tolylpyrazolo[1,5-a]pyrimidin-6-yl)pentanoat;
Methyl-2-(2-tert-butyl-7-(4-chloro-2-fluorophenyl)-5-methylpyrazolo[1,5-a]pyrimidin-6-yl)pentanoat;
Methyl-2-(2-tert-butyl-7-(2-fluoro-4-methylphenyl)-5-methylpyrazolo[1,5-a]pyrimidin-6-yl)pentanoat;
Methyl-2-(2-tert-butyl-7-p-tolyl-5-methylpyrazolo[1,5-a]pyrimidin-6-yl)-6,6,6-trifluorohexanoat;
Methyl-2-(2-tert-butyl-7-p-tolyl-5-methylpyrazolo[1,5-a]pyrimidin-6-yl)-3-phenylpropanoat;
Methyl-2-(2-tert-butyl-7-(1-methylindol-5-yl)-5-methylpyrazolo[1,5-a]pyrimidin-6-yl)-3-phenylpropanoat;
Methyl-2-(2-tert-butyl-5-methyl-7-(1-methylindolin-5-yl)pyrazolo[1,5-a]pyrimidin-6-yl)pentanoat;
Methyl-2-(2-tert-butyl-5-methyl-7-(1-methyl-1H-indol-6-yl)pyrazolo[1,5-a]pyrimidin-6-yl)pentanoat;
Methyl-2-(2-tert-butyl-7-(chroman-6-yl)-5-methylpyrazolo[1,5-a]pyrimidin-6-yl)pentanoat;
Methyl-2-(2-tert-butyl-7-p-tolyl-5-methylpyrazolo[1,5-a]pyrimidin-6-yl)-3-methylpentanoat;
Methyl-2-(2-tert-butyl-3-chloro-7-p-tolyl-5-methylpyrazolo[1,5-a]pyrimidin-6-yl)pentanoat;
Methyl-2-(2-tert-butyl-7-p-tolyl-5-methylpyrazolo[1,5-a]pyrimidin-6-yl)-4-methoxybutanoat;
Methyl-2-(2-tert-butyl-5-methyl-7-(4-iso-propylphenyl)pyrazolo[1,5-a]pyrimidin-6-yl)pentanoat;
Methyl-2-(2-tert-butyl-5-methyl-7-(4-trifluoromethylphenyl)pyrazolo[1,5-a]pyrimidin-6-yl)pentanoat;
Methyl-2-(2-tert-butyl-7-(2,4-difluorophenyl)-5-methylpyrazolo[1,5-a]pyrimidin-6-yl)pentanoat;
Methyl-2-(2-tert-butyl-7-(2-chloro-4-methylphenyl)-5-methylpyrazolo[1,5-a]pyrimidin-6-yl)pentanoat;
Methyl-2-(7-(2-amino-4-methylphenyl)-2-tert-butyl-5-methylpyrazolo[1,5-a]pyrimidin-6-yl)pentanoat;
Methyl-2-(2-tert-butyl-7-(2-methoxy-4-chlorophenyl)-5-methylpyrazolo[1,5-a]pyrimidin-6-yl)pentanoat;
Methyl-2-(2-tert-butyl-7-(2-fluoro-4-methoxyphenyl)-5-methylpyrazolo[1,5-a]pyrimidin-6-yl)pentanoat;
Methyl-2-(2-tert-butyl-5-methyl-7-(5-chinolin)pyrazolo[1,5-a]pyrimidin-6-yl)pentanoat;
Methyl-2-(2-tert-butyl-5-methyl-7-(8-chinolin)pyrazolo[1,5-a]pyrimidin-6-yl)pentanoat;
Methyl-2-(2-tert-butyl-7-(2,4-dimethylphenyl)-5-methylpyrazolo[1,5-a]pyrimidin-6-yl)pentanoat;
Methyl-2-(2-tert-butyl-3,5-dimethyl-7-p-tolylpyrazolo[1,5-a]pyrimidin-6-yl)pentanoat;
Methyl-2-(2-tert-butyl-5-methyl-3-phenyl-7-p-tolylpyrazolo[1,5-a]pyri midin-6-yl)pentanoat;
Methyl-2-(2,3,5-trimethyl-7-p-tolyl-3H-imidazo[4,5-b]pyridin-6-yl)acetat;
Methyl-2-(2,3,5-trimethyl-7-p-tolyl-3H-imidazo[4,5-b]pyridin-6-yl)pentanoat;
Ethyl-2-(1,2,5-trimethyl-7-p-tolyl-1H-imidazo[4,5-b]pyridin-6-yl)pentanoat;

Ethyl-2-(3,5-dimethyl-2-propyl-7-p-tolyl-3H-imidazo[4,5-b]pyridin-6-yl)pentanoat;
Ethyl-2-(3,5-dimethyl-2-isopropyl-7-p-tolyl-3H-imidazo[4,5-b]pyridin-6-yl)pentanoat;
2-((7-((R)-3-(4-Chlorophenylsulfonamido)piperidin-1-yl)-5-methylpyrazolo[1,5-a]pyrimidin-6-yl)pentansäure;
2-((7-((S)-3-(4-Chlorophenylsulfonamido)piperidin-1-yl)-5-methylpyrazolo[1,5-a]pyrimidin-6-yl)pentansäure;
2-((7-((R)-3-(4-Chlorophenylsulfonamido)pyrrolidin-1-yl)-5-methylpyrazolo[1,5-a]pyrimidin-6-yl)pentansäure;
2-((7-((S)-3-(4-Chlorophenylsulfonamido)pyrrolidin-1-yl)-5-methylpyrazolo[1,5-a]pyrimidin-6-yl)pentansäure;
2-((5-Methyl-7-phenylpyrazolo[1,5-a]pyrimidin-6-yl)pentansäure;
2-((5-Methyl-7-p-tolylpyrazolo[1,5-a]pyrimidin-6-yl)pentansäure;
2-((2-Tert-butyl-5-methyl-7-phenylpyrazolo[1,5-a]pyrimidin-6-yl)pentansäure;
2-((2-Tert-butyl-5-methyl-7-p-tolylpyrazolo[1,5-a]pyrimidin-6-yl)pentansäure;
2-((3-Bromo-2-tert-butyl-5-methyl-7-p-tolylpyrazolo[1,5-a]pyrimidin-6-yl)pentansäure;
2-((5-Methyl-2-phenyl-7-p-tolylpyrazolo[1,5-a]pyrimidin-6-yl)pentansäure;
2-((2-tert-Butyl-7-(3-hydroxyphenyl)-5-methylpyrazolo[1,5-a]pyrimidin-6-yl)pentansäure;
2-((2-tert-Butyl-5-methyl-7-(2-naphthyl)pyrazolo[1,5-a]pyrimidin-6-yl)pentansäure;
2-((2-tert-Butyl-7-(1 H-indol-5-yl)-5-methylpyrazolo[1,5-a]pyrimidin-6-yl)pentansäure;
2-((2-tert-Butyl-7-(1 H-indol-6-yl)-5-methylpyrazolo[1,5-a]pyrimidin-6-yl)pentansäure;
2-((7-(Benzofuran-5-yl)-2-tert-butyl-5-methylpyrazolo[1,5-a]pyrimidin-6-yl)pentansäure;
2-((7-(Benzo[b]thiophen-5-yl)-2-tert-butyl-5-methylpyrazolo[1,5-a]pyrimidin-6-yl)pentansäure;
2-((2-tert-Butyl-7-(2,3-dihydrobenzofuran-5-yl)-5-methylpyrazolo[1,5-a]pyrimidin-6-yl)pentansäure;
2-((2-tert-Butyl-7-(4-chlorophenyl)-5-methylpyrazolo[1,5-a]pyrimidin-6-yl)pentansäure;
2-((2-tert-Butyl-7-(3,4-dimethylphenyl)-5-methylpyrazolo[1,5-a]pyrimidin-6-yl)pentansäure;
2-((2-tert-Butyl-7-(4-ethylphenyl)-5-methylpyrazolo[1,5-a]pyrimidin-6-yl)pentansäure;
2-((2-tert-Butyl-7-(3,4-dihydro-2H-benzo[b][1,4]dioxepin-7-yl)-5-methylpyrazolo[1,5-a]pyrimidin-6-yl)pentansäure;
2-((2-tert-Butyl-7-(4-methyl-3,4-dihydro-2H-benzo[b][1,4]oxazin-7-yl)-5-methylpyrazolo[1,5-a]pyrimidin-6-yl)pentansäure;
2-((5-Methyl-3-phenyl-7-p-tolylpyrazolo[1,5-a]pyrimidin-6-yl)pentansäure;
2-((3,7-Di-p-tolyl-5-methylpyrazolo[1,5-a]pyrimidin-6-yl)pentansäure;
2-((2-tert-Butyl-3,7-di-p-tolyl-5-methylpyrazolo[1,5-a]pyrimidin-6-yl)pentansäure;
2-((5-Methyl-2-propyl-7-p-tolylpyrazolo[1,5-a]pyrimidin-6-yl)pentansäure;
2-((2-(Furan-2-yl)-5-methyl-7-p-tolylpyrazolo[1,5-a]pyrimidin-6-yl)pentansäure;
2-((2-tert-Butyl-7-(4-chloro-2-fluorophenyl)-5-methylpyrazolo[1,5-a]pyrimidin-6-yl)pentansäure;
2-((2-tert-Butyl-7-(2-fluoro-4-methylphenyl)-5-methylpyrazolo(1,5-a]pyrimidin-6-yl)pentansäure;
2-((2-tert-Butyl-7-p-tolyl-5-methylpyrazolo(1,5-a]pyrimidin-6-yl)-6,6,6-triluorhexansäure;
2-((2-tert-Butyl-7-p-tolyl-5-methylpyrazolo[1,5-a]pyrimidin-6-yl)-3-phenylpropansäure;
2-((2-tert-Butyl-5-methyl-7-(1-methylindolin-5-yl)pyrazolo[-1,5-a]pyrimidin-6-yl)pentansäure;
2-((2-tert-Butyl-5-methyl-7-(1-methyl-1H-indol-6-yl)pyrazolo[1,5-a]pyrimidin-6-yl)pentansäure;
2-((2-tert-Butyl-7-(chroman-6-yl)-5-methylpyrazolo[1,5-a]pyrimidin-6-yl)pentansäure;
2-((2-tert-Butyl-7-p-tolyl-5-methylpyrazolo[1,5-a]pyrimidin-6-yl)-3-methylpentansäure;
2-((2-tert-Butyl-3-chloro-7-p-tolyl-5-methylpyrazolo[1,5-a]pyrimidin-6-yl)pentansäure;
2-((2-tert-Butyl-7-p-tolyl-5-methylpyrazolo[1,5-a]pyrimidin-6-yl)-4-methoxybutansäure;
2-((2-tert-Butyl-5-methyl-7-(4-iso-propylphenyl)pyrazolo[1,5-a]pyrimidin-6-yl)pentansäure;
2-((2-tert-Butyl-5-methyl-7-(4-trifluormethylphenyl)pyrazolo[1,5-a]pyrimidin-6-yl)pentansäure;
2-((2-tert-Butyl-7-(2,4-difluorophenyl)-5-methylpyrazolo[1,5-a]pyrimidin-6-yl)pentansäure;
2-((2-tert-Butyl-7-(2-chloro-4-methylphenyl)-5-methylpyrazolo[1,5-a]pyrimidin-6-yl)pentansäure;
2-((7-(2-Amino-4-methylphenyl)-2-tert-butyl-5-methylpyrazolo(1,5-a]pyrimidin-6-yl)pentansäure;
2-((2-tert-Butyl-7-(2-methoxy-4-chlorophenyl)-5-methylpyrazolo[1,5-a]pyrimidin-6-yl)pentansäure;
2-((2-tert-Butyl-7-(2-fluoro-4-methoxyphenyl)-5-methylpyrazolo[1,5-a]pyrimidin-6-yl)pentansäure;
2-((2-tert-Butyl-5-methyl-7-(5-chinolin)pyrazolo[1,5-a]pyrimidin-6-yl)pentansäure;
2-((2-tert-Butyl-5-methyl-7-(8-chinolin)pyrazolo[1,5-a]pyrimidin-6-yl)pentansäure;
2-((2-tert-Butyl-7-(2,4-dimethylphenyl)-5-methylpyrazolo{1,5-a]pyrimidin-6-yl)pentansäure;
2-((2-tert-Butyl-3,5-dimethyl-7-p-tolylpyrazolo[1,5-a]pyrimidin-6-yl)pentansäure;
2-((2-tert-Butyl-5-methyl-3-phenyl-7-p-tolylpyrazolo(1,5-a]pyrimidin-6-yl)pentansäure;
2-((2,3,5-Trimethyl-7-p-tolyl-3H-imidazo[4,5-b]pyridin-6-yl)pentansäure;
2-((1,2,5-trimethyl-7-p-tolyl-1H-imidazo[4,5-b]pyridin-6-yl)pentansäure;
2-((2-Propyl-3,5-dimethyl-7-p-tolyl-3H-imidazo[4,5-b]pyridin-6-yl)pentansäure;
2-((2-Isopropyl-3,5-dimethyl-7-p-tolyl-3H-imidazo[4,5-b]pyridin-6-yl)pentanoat;
Methyl-2-(2-isopropyl-5-methyl-7-p-tolyl-(1,2,4]-triazolo[1,5-a]pyrimidin-6-yl)acetat;

2-((2-Isopropyl-5-methyl-7-p-tolyl-[1,2,4]-triazolo[1,5-a]pyrimidin-6-yl)essigsäure;
Methyl-2-[2-tert-butyl-7-(1,2-dihydroacenaphthylen-5-yl)-5-methylpyrazolo[1,5-a]pyrimidin-6-yl]acetat;
2-[2-tert-Butyl-7-(1,2-dihydroacenaphthylen-5-yl)-5-methylpyrazolo[1,5-a]pyrimidin-6-yl]essigsäure;
Methyl-2-[2-tert-butyl-7-(2-methoxy-4-methylphenyl)-5-methylpyrazolo[1,5-a]pyrimidin-6-yl]acetat;
2-[2-tert-Butyl-7-(2-methoxy-4-methylphenyl)-5-methylpyrazolo[1,5-a]pyrimidin-6-yl]essigsäure;
2-[2-tert-Butyl-7-(2-hydroxy-4-methylphenyl)-5-methylpyrazolo[1,5-a]pyrimidin-6-yl]essigsäure;
Methyl-2-(2-isopropyl-5-methyl-7-p-tolyl-[1,2,4]-triazolo[1,5-a]pyrimidin-6-yl)pentanoat;
2-((2-Isopropyl-5-methyl-7-p-tolyl-[1,2,4]-triazolo[1,5-a]pyrimidin-6-yl)pentansäure;
(2-((2-Benzyl-5-methyl-7-p-tolyl-[1,2,4]-triazolo[1,5-a]pyrimidin-6-yl)pentansäure; oder
Ethyl-2-(2-benzyl-5-methyl-7-p-tolyl-[1,2,4]-triazolo[1,5-a]pyrimidin-6-yl)pentanoat;

oder ein pharmazeutisch unbedenkliches Salz davon.

**8.** Verbindungen nach Anspruch 1 bis 7 zur Verwendung als Medikament.

**9.** Verbindungen nach Anspruch 8 zur Verwendung als eine Arznei für die Verhütung oder Behandlung einer Virusinfektion in einem Tier.

**10.** Verbindungen nach Anspruch 9, wobei die Virusinfektion eine Infektion mit HIV ist.

**11.** Verbindungen nach Anspruch 9 oder 10, wobei das Tier ein Mensch ist.

**12.** Verwendung der Verbindungen nach Anspruch 1 bis 7 zur Herstellung eines Medikaments zur Verhütung oder Behandlung einer Virusinfektion in einem Tier.

**13.** Verwendung nach Anspruch 12, wobei die Virusinfektion eine Infektion mit HIV ist.

**14.** Pharmazeutische Zusammensetzung, umfassend die Verbindungen nach Anspruch 1 bis 7 als Wirkstoff in Beimischung mit mindestens einem pharmazeutisch unbedenklichen Träger.

**15.** Pharmazeutische Zusammensetzung nach Anspruch 14, ferner umfassend mindestens eine weitere Verbindung mit antiviraler Wirkung.

## Revendications

**1.** Composé de formule (A) :

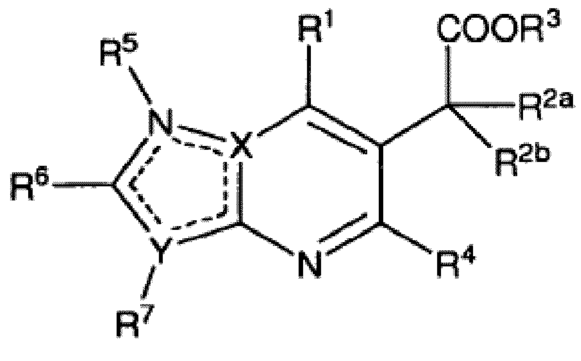

(A)

dans laquelle,

- chaque ligne en pointillés représente une double liaison facultative, moyennant quoi deux lignes en pointillés parmi les 5 lignes en pointillés constituent une double liaison et ces 2 doubles liaisons ne sont pas adjacentes ;
- X et Y sont chacun choisis indépendamment parmi C ou N, moyennant quoi au moins un parmi X et Y est N ;
- $R^1$ est choisi indépendamment parmi un cycloalkyle ; un cycloalcényle ; un cycloalcynyle ; un aryle ; un hétérocycle ; arylalkyle ; arylalcényle ; un arylalcynyle ; un arylhétéroalkyle ; un arylhétéroalcényle ; un

arylhétéroalcynyle ; un hétérocycle-alkyle ; un hétérocycle-alcényle ; un hétérocycle-alcynyle ; un hétérocycle-hétéroalkyle ; un hétérocycle-hétéroalcényle ou un hétérocycle-hétéroalcynyle ;

et dans lequel ledit cycloalkyle, cycloalcényle, cycloalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs $R^{10}$ ;

- $R^{2a}$ et $R^{2b}$ sont chacun choisis indépendamment parmi un hydrogène ; un cyano ; un alkyle ; un alcényle ; un alcynyle ; un hétéroalkyle ; un hétéroalcényle ; un hétéroalcynyle ; un aryle ; un arylalkyle ; arylalcényle ; un arylalcynyle ; un arylhétéroalkyle ; un arylhétéroalcényle ; un arylhétéroalcynyle ; un hétérocycle ; un hétérocycle-alkyle ; un hétérocycle-alcényle ; un hétérocycle-alcynyle ; un hétérocycle-hétéroalkyle, un hétérocycle-hétéroalcényle ou un hétérocycle-hétéroalcynyle ; ou $R^{2a}$ et $R^{2b}$ peuvent être pris ensemble pour former un vinyle ou un vinylalkyle ;

et dans lequel ledit alkyle, alcényle, alcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, $-OCF_3$, un cyano, un nitro, -C(O)OH ou $NH_2$ ;

- $R^3$ est choisi indépendamment parmi un hydrogène ; un alkyle ; un alcényle ; un alcynyle ; un hétéroalkyle ; un hétéroalcényle ; un hétéroalcynyle ; un aryle ; un hétérocycle ; un arylalkyle ; un arylalcényle ; un arylalcynyle ; un arylhétéroalkyle ;

un arylhétéroalcényle ; un arylhétéroalcynyle ; un hétérocycle-alkyle ; un hétérocycle-alcényle ; un hétérocycle-alcynyle ; un hétérocycle-hétéroalkyle, un hétérocycle-hétéroalcényle ou un hétérocycle-hétéroalcynyle ;

dans lequel ledit alkyle, alcényle, alcynyle, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, $-OCF_3$, un cyano, un nitro, -C(O)OH ou $NH_2$ ;

- $R^4$ est choisi indépendamment parmi un hydrogène ; un alkyle ; un alcényle ou un alcynyle ; dans lequel ledit alkyle, alcényle ou alcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, $-OCF_3$, un cyano, un nitro, -C(O)OH ou $NH_2$ ;

- $R^5$ n'est pas présent ou est choisi parmi un hydrogène ; un alkyle ; un alcényle ; un alcynyle ; un hétéroalkyle ; un hétéroalcényle ; et un hétéroalcynyle ;

dans lequel ledit alkyle, alcényle, alcynyle, hétéroalkyle, hétéroalcényle et hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, $-OCF_3$, un cyano, un nitro, -C(O)OH ou $NH_2$ ;

- $R^6$ est choisi parmi un hydrogène ; un alkyle ; un alcényle ; un alcynyle ; un hétéroalkyle ; un hétéroalcényle ; un hétéroalcynyle ; un aryle ; un hétérocycle ; un arylalkyle ; un arylalcényle ; un arylalcynyle ; un arylhétéroalkyle ; un arylhétéroalcényle ; un arylhétéroalcynyle ; un hétérocycle-alkyle ; un hétérocycle-alcényle ; un hétérocycle-alcynyle ; un hétérocycle-hétéroalkyle, un hétérocycle-hétéroalcényle ou un hétérocycle-hétéroalcynyle ;

dans lequel ledit alkyle, alcényle, alcynyle, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, $-OCF_3$, un cyano, un nitro, -C(O)OH ou $NH_2$ ;

- $R^7$ est non présent ou est choisi parmi un hydrogène ; un halogène ; un alkyle ; un alcényle ; un alcynyle ; un hétéroalkyle ; un hétéroalcényle ; un hétéroalcynyle ; un aryle ; un hétérocycle ; un arylalkyle ; un arylalcényle ; un arylalcynyle ; un arylhétéroalkyle ; un arylhétéroalcényle ; un arylhétéroalcynyle ; un hétérocycle-alkyle ; un hétérocycle-alcényle ; un hétérocycle-alcynyle ; un hétérocycle-hétéroalkyle, un hétérocycle-hétéroalcényle ou un hétérocycle-hétéroalcynyle ;

dans lequel ledit alkyle, alcényle, alcynyle, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, -$OCF_3$, un cyano, un nitro, -C(O)OH ou $NH_2$ ;

- chaque $R^{10}$ est choisi indépendamment dans le groupe consistant en un halogène ; -$OR^{11}$ ; =O ; -$SR^{11}$ ; =S ; -$S(O)R^{12}$ ; -$S(O)_2R^{12}$ ; -$S(O)_2NR^{13}R^{14}$ ; un trifluorométhyle ; un nitro ; -$NR^{13}R^{14}$ ; -$NR^{11}S(O)_2R^{12}$ ; un cyano ; -$C(O)OR^{11}$ ; -$C(O)NR^{13}R^{14}$ ; -$C(O)R^{12}$ ; un alkyle ; un alcényle ; un alcynyle ; un hétéroalkyle ; un hétéroalcényle ; un hétéroalcynyle ; un aryle ; un hétérocycle ; un arylalkyle ; un arylalcényle ; un arylalcynyle ; un arylhétéroalkyle ; un arylhétéroalcényle ; un arylhétéroalcynyle ; un hétérocycle-alkyle ; un hétérocycle-alcényle ; un hétérocycle-alcynyle ; un hétérocycle-hétéroalkyle, un hétérocycle-hétéroalcényle ou un hétérocycle-hétéroalcynyle ;

et dans lequel ledit alkyle, alcényle, alcynyle, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, -$OCF_3$, un cyano, un nitro, -C(O)OH ou $NH_2$ ;

- chaque $R^{11}$ est choisi indépendamment parmi un hydrogène ; un alkyle ; un alcényle ; un alcynyle ; un hétéroalkyle ; un hétéroalcényle ; un hétéroalcynyle ; un aryle ; un arylalkyle ; arylalcényle ; un arylalcynyle ; un arylhétéroalkyle ; un arylhétéroalcényle ; un arylhétéroalcynyle ; un hétérocycle ; hétérocycle-alkyle ; un hétérocycle-alcényle ; un hétérocycle-alcynyle ; un hétérocycle-hétéroalkyle ; un hétérocycle-hétéroalcényle ; et un hétérocycle-hétéroalcynyle ;

dans lequel ledit alkyle, alcényle, alcynyle, hétéroalkyle, hétéroalcényle, hétéroalcynyle, aryle, arylalkyle, arylalcényle, arylalcynyle, arylhétéroalkyle, arylhétéroalcényle, arylhétéroalcynyle, hétérocycle, hétérocycle-alkyle, hétérocycle-alcényle, hétérocycle-alcynyle, hétérocycle-hétéroalkyle, hétérocycle-hétéroalcényle et hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, -$OCF_3$, un cyano, un nitro, -C(O)OH ou $NH_2$ ;

- chaque $R^{12}$ est choisi indépendamment parmi un hydrogène ; un hydroxyle ; un alkyle ; un alcényle ; un alcynyle ; un hétéroalkyle ; hétéroalcényle ; un hétéroalcynyle ; un aryle ; un arylalkyle ; arylalcényle ; un arylalcynyle ; un arylhétéroalkyle ; un arylhétéroalcényle ; un arylhétéroalcynyle ; un hétérocycle ; hétérocycle-alkyle ; un hétérocycle-alcényle ; un hétérocycle-alcynyle ; un hétérocycle-hétéroalkyle ; un hétérocycle-hétéroalcényle ; et un hétérocycle-hétéroalcynyle ;

dans lequel ledit alkyle, alcényle, alcynyle, hétéroalkyle, hétéroalcényle, hétéroalcynyle, aryle, arylalkyle, arylalcényle, arylalcynyle, arylhétéroalkyle, arylhétéroalcényle, arylhétéroalcynyle, hétérocycle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, -$OCF_3$, un cyano, un nitro, -C(O)OH ou $NH_2$ ;

- chaque $R^{13}$ et $R^{14}$ est choisi indépendamment parmi un hydrogène ; un alkyle ; un alcényle ; un alcynyle ; un hétéroalkyle ; un hétéroalcényle ; un hétéroalcynyle ; un aryle ; un arylalkyle ; un arylalcényle ; un arylalcynyle ; un arylhétéroalkyle ; un arylhétéroalcényle ; un arylhétéroalcynyle ; un hétérocycle ; hétérocycle-alkyle ; un hétérocycle-alcényle ; un hétérocycle-alcynyle ; un hétérocycle-hétéroalkyle ; un hétérocycle-hétéroalcényle ; et un hétérocycle-hétéroalcynyle ;

et dans lequel ledit alkyle, alcényle, alcynyle, hétéroalkyle, hétéroalcényle, hétéroalcynyle, aryle, arylalkyle, arylalcényle, arylalcynyle, arylhétéroalkyle, arylhétéroalcényle, arylhétéroalcynyle, hétérocycle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, -$OCF_3$, un cyano, un nitro, -C(O)OH ou $NH_2$ ;

et dans lequel $R^{13}$ et $R^{14}$ peuvent être pris ensemble avec le N auquel ils sont liés afin de former un hétérocycle (à 5, 6 ou 7 chaînons) qui peut être non substitué ou substitué avec un ou plusieurs substituants choisis indépendamment parmi un alkyle, un alcényle, un alcynyle, un hétéroalkyle, un hétéroalcényle, un hétéroalcynyle, un hydroxyle, =O, un halogène, -SH, =S, un trifluorométhyle, -$OCF_3$, un cyano, un nitro, -C(O)OH ou $NH_2$ ;

ou un sel pharmaceutiquement acceptable de celui-ci.

**2.** Composé selon la revendication 1, dans lequel $R^3$ est H.

3. Composé selon la revendication 1 ou 2, dans lequel $R^1$ est choisi parmi un aryle ou un hétéroaryle, dans lequel ledit aryle ou hétéroaryle peut être non substitué ou substitué avec un ou plusieurs $R^{10}$.

4. Composé selon les revendications 1 à 3, dans lequel un parmi $R^{2a}$ et $R^{2b}$ est un hydrogène, et l'autre parmi $R^{2a}$ et $R^{2b}$ est choisi parmi un hydrogène ; un cyano ; un alkyle ; un alcényle ; un alcynyle ; un hétéroalkyle ; un hétéroalcényle ; un hétéroalcynyle ; un aryle ; un arylalkyle ; un arylalcényle ; un arylalcynyle ; un arylhétéroalkyle ; un arylhétéroalcényle ; un arylhétéroalcynyle ; un hétérocycle ; hétérocycle-alkyle ; un hétérocycle-alcényle ; un hétérocycle-alcynyle ; un hétérocycle-hétéroalkyle, un hétérocycle-hétéroalcényle ou un hétérocycle-hétéroalcynyle ; ou $R^{2a}$ et $R^{2b}$ peuvent être pris ensemble pour former un vinyle ou un vinylalkyle ;
et dans lequel ledit alkyle, alcényle, alcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle ou hétérocycle-hétéroalcynyle peut être non substitué ou substitué.

5. Composé selon les revendications 1 à 4, dans lequel le composé possède une structure selon la formule (C-I) ou (C-II),

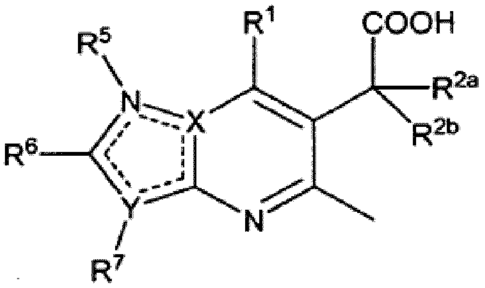
(C-I)

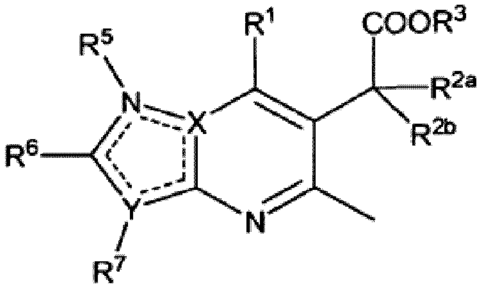
(C-II)

dans lesquelles X, Y, les lignes en pointillés, $R^1$, $R^{2a}$, $R^{2b}$, $R^5$, $R^6$ et $R^7$ sont chacun tels que définis dans les revendications 1 à 4.

6. Composé selon les revendications 1 à 5, dans lequel le composé possède une structure selon la formule (E), qui consiste en les formules (E-I), (E-II), (E-III) et (E-IV),

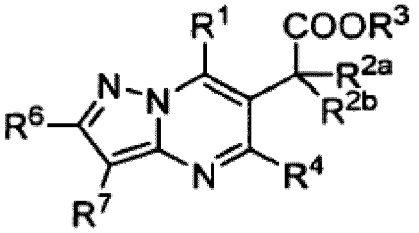
(E-I)

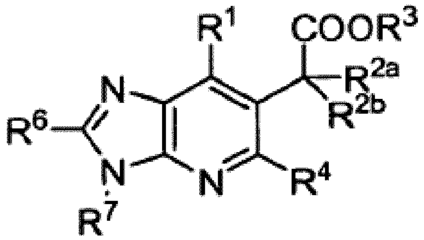
(E-II)

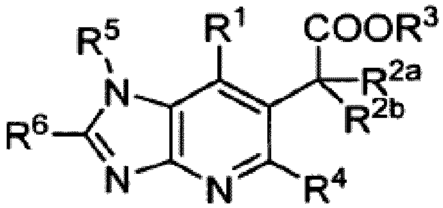
(E-III)

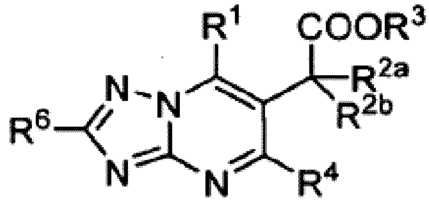
(E-IV)

dans lesquelles $R^1$, $R^{2a}$, $R^{2b}$, $R^3$, $R^4$, $R^5$, $R^6$ et $R^7$ sont chacun tels que définis dans les revendications 1 à 4.

7. Composé selon la revendication 1, qui est :

le 2-(7-((R)-3-(4-chlorophénylsulfonamido)pipéridin-1-yl)-5-méthylpyrazolo[1,5-a] pyrimidin-6-yl)pentanoate

d'éthyle ;
le 2-(7-((S)-3-(4-chlorophénylsulfonamido)pipéridin-1-yl)-5-méthylpyrazolo[1,5-a] pyrimidin-6-yl)pentanoate d'éthyle ;
le 2-(7-((R)-3-(4-chlorophénylsulfonamido)pyrrolidin-1-yl)-5-méthylpyrazolo[1,5-a] pyrimidin-6-yl)pentanoate d'éthyle ;
le 2-(7-((S)-3-(4-chlorophénylsulfonamido)pyrrolidin-1-yl)-5-méthylpyrazolo[1,5-a] pyrimidin-6-yl)pentanoate d'éthyle ;
le 2-(5-méthyl-7-phénylpyrazolo[1,5-a]pyrimidin-6-yl)pentanoate de méthyle ;
le 2-(5-méthyl-7-p-tolylpyrazolo[1,5-a]pyrimidin-6-yl)pentanoate de méthyle ;
le 2-(2-tert-butyl-5-méthyl-7-phénylpyrazolo[1,5-a]pyrimidin-6-yl)pentanoate de méthyle ;
le 2-(2-tert-butyl-5-méthyl-7-p-tolylpyrazolo[1,5-a]pyrimidin-6-yl)pentanoate de méthyle ;
le 2-(5-méthyl-2-phényl-7-p-tolylpyrazolo[1,5-a]pyrimidin-6-yl)pentanoate de méthyle ;
le 2-(2-tert-butyl-7-(3-hydroxyphényl)-5-méthylpyrazolo[1,5-a]pyrimidin-6-yl)-pentanoate de méthyle ;
le 2-(2-tert-butyl-7-(2-naphtyl)-5-méthylpyrazolo[1,5-a]pyrimidin-6-yl)pentanoate de méthyle ;
le 2-(2-tert-butyl-7-(1H-indol-5-yl)-5-méthylpyrazolo[1,5-a]pyrimidin-6-yl)-pentanoate de méthyle ;
le 2-(2-tert-butyl-7-(1H-indol-6-yl)-5-méthylpyrazolo[1,5-a]pyrimidin-6-yl)-pentanoate de méthyle ;
le 2-(2-tert-butyl-7-(1-benzofuran-5-yl)-5-méthylpyrazolo[1,5-a]pyrimidin-6-yl)-pentanoate de méthyle ;
le 2-(2-tert-butyl-7-(1-benzothiophèn-5-yl)-5-méthylpyrazolo[1,5-a]pyrimidin-6-yl)-pentanoate de méthyle ;
le 2-(2-tert-butyl-7-(2,3-dihydrobenzofuran-5-yl)-5-méthylpyrazolo[1,5-a]pyrimidin-6-yl)pentanoate de méthyle ;
le 2-(2-tert-butyl-7-(4-chlorophényl)-5-méthylpyrazolo[1,5-a]pyrimidin-6-yl)-pentanoate de méthyle ;
le 2-(2-tert-butyl-7-(3,4-diméthylphényl)-5-méthylpyrazolo[1,5-a]pyrimidin-6-yl)-pentanoate de méthyle ;
le 2-(2-tert-butyl-7-(4-éthylphényl)-5-méthylpyrazolo[1,5-a]pyrimidin-6-yl)-pentanoate de méthyle ;
le 2-(2-tert-butyl-7-(3,4-dihydro-2H-benzo[b][1,4]dioxépin-7-yl)-5-méthylpyrazolo [1,5-a]pyrimidin-6-yl)pentanoate de méthyle ;
le 2-(2-tert-butyl-7-(2-(7-(4-méthyl-3,4-dihydro-2H-benzo[b][1,4]oxazin-7-yl)-5-méthylpyrazolo[1,5-a]pyrimidin-6-yl)pentanoate de méthyle ;
le 2-(3-bromo-5-méthyl-7-p-tolylpyrazolo[1,5-a]pyrimidin-6-yl)pentanoate d'éthyle ;
le 2-(5-méthyl-3-phényl-7-p-tolylpyrazolo[1,5-a]pyrimidin-6-yl)pentanoate d'éthyle ;
le 2-(5-méthyl-3,7-di-p-tolylpyrazolo[1,5-a]pyrimidin-6-yl)pentanoate d'éthyle ;
le 2-(3-bromo-2-tert-butyl-5-méthyl-7-p-tolylpyrazolo[1,5-a]pyrimidin-6-yl)-pentanoate de méthyle ;
le 2-(2-tert-butyl-5-méthyl-3,7-di-p-tolylpyrazolo[1,5-a]pyrimidin-6-yl)pentanoate de méthyle ;
le 2-(5-méthyl-2-propyl-7-p-tolylpyrazolo[1,5-a]pyrimidin-6-yl)pentanoate de méthyle ;
le 2-(2-(furan-2-yl)-5-méthyl-7-p-tolylpyrazolo[1,5-a]pyrimidin-6-yl)pentanoate de méthyle ;
le 2-(2-tert-butyl-7-(4-chloro-2-fluorophényl)-5-méthylpyrazolo[1,5-a]pyrimidin-6-yl)pentanoate de méthyle ;
le 2-(2-tert-butyl-7-(2-fluoro-4-méthylphényl)-5-méthylpyrazolo[1,5-a]pyrimidin-6-yl)pentanoate de méthyle ;
le 2-(2-tert-butyl-7-p-tolyl-5-méthylpyrazolo[1,5-a]pyrimidin-6-yl)-6,6,6-trifluoro-hexanoate de méthyle ;
le 2-(2-tert-butyl-7-p-tolyl-5-méthylpyrazolo[1,5-a]pyrimidin-6-yl)-3-phényl-propanoate de méthyle ;
le 2-(2-tert-butyl-7-(1-méthylindol-5-yl)-5-méthylpyrazolo[1,5-a]pyrimidin-6-yl)-3-phénylpropanoate de méthyle ;
le 2-(2-tert-butyl-5-méthyl-7-(1-méthylindolin-5-yl)pyrazolo[1,5-a]pyrimidin-6-yl)-pentanoate de méthyle ;
le 2-(2-tert-butyl-5-méthyl-7-(1-méthyl-1H-indol-6-yl)pyrazolo[1,5-a]pyrimidin-6-yl)pentanoate de méthyle ;
le 2-(2-tert-butyl-7-(chroman-6-yl)-5-méthylpyrazolo[1,5-a]pyrimidin-6-yl)-pentanoate de méthyle ;
le 2-(2-tert-butyl-7-p-tolyl-5-méthylpyrazolo[1,5-a]pyrimidin-6-yl)-3-méthyl-pentanoate de méthyle ;
le 2-(2-tert-butyl-3-chloro-7-p-tolyl-5-méthylpyrazolo[1,5-a]pyrimidin-6-yl)-pentanoate de méthyle ;
le 2-(2-tert-butyl-7-p-tolyl-5-méthylpyrazolo[1,5-a]pyrimidin-6-yl)-4-méthoxy-butanoate de méthyle ;
le 2-(2-tert-butyl-5-méthyl-7-(4-iso-propylphényl)pyrazolo[1,5-a]pyrimidin-6-yl)pentanoate de méthyle ;
le 2-(2-tert-butyl-5-méthyl-7-(4-trifluorométhylphényl)pyrazolo[1,5-a]pyrimidin-6-yl)pentanoate de méthyle ;
le 2-(2-tert-butyl-7-(2,4-difluorophényl)-5-méthylpyrazolo[1,5-a]pyrimidin-6-yl)-pentanoate de méthyle ;
le 2-(2-tert-butyl-7-(2-chloro-4-méthylphényl)-5-méthylpyrazolo[1,5-a]pyrimidin-6-yl)pentanoate de méthyle ;
le 2-(7-(2-amino-4-méthylphényl)-2-tert-butyl-5-méthylpyrazolo[1,5-a]pyrimidin-6-yl)pentanoate de méthyle ;
le 2-(2-tert-butyl-7-(2-méthoxy-4-chlorophényl)-5-méthylpyrazolo[1,5-a]pyrimidin-6-yl)pentanoate de méthyle ;
le 2-(2-tert-butyl-7-(2-fluoro-4-méthoxyphényl)-5-méthylpyrazolo[1,5-a]pyrimidin-6-yl)pentanoate de méthyle ;
le 2-(2-tert-butyl-5-méthyl-7-(5-quinoline)pyrazolo[1,5-a]pyrimidin-6-yl)pentanoate de méthyle ;
le 2-(2-tert-butyl-5-méthyl-7-(8-quinoline)pyrazolo[1,5-a]pyrimidin-6-yl)pentanoate de méthyle ;
le 2-(2-tert-butyl-7-(2,4-diméthylphényl)-5-méthylpyrazolo[1,5-a]pyrimidin-6-yl)-pentanoate de méthyle ;
le 2-(2-tert-butyl-3,5-diméthyl-7-p-tolylpyrazolo[1,5-a]pyrimidin-6-yl)pentanoate de méthyle ;
le 2-(2-tert-butyl-5-méthyl-3-phényl-7-p-tolylpyrazolo[1,5-a]pyrimidin-6-yl)-pentanoate de méthyle ;

le 2-(2,3,5-triméthyl-7-p-tolyl-3H-imidazo[4,5-b]pyridin-6-yl)acétate de méthyle ;
le 2-(2,3,5-triméthyl-7-p-tolyl-3H-imidazo[4,5-b]pyridin-6-yl)pentanoate de méthyle ;
le 2-(1,2,5-triméthyl-7-p-tolyl-1H-imidazo[4,5-b]pyridin-6-yl)pentanoate d'éthyle ;
le 2-(3,5-diméthyl-2-propyl-7-p-tolyl-3H-imidazo[4,5-b]pyridin-6-yl)pentanoate d' éthyle ;
le 2-(3,5-diméthyl-2-isopropyl-7-p-tolyl-3H-imidazo[4,5-b]pyridin-6-yl)pentanoate d' éthyle ;
l'acide 2-(7-((R)-3-(4-chlorophénylsulfonamido)pipéridin-1-yl)-5-méthylpyrazolo [1,5-a]pyrimidin-6-yl)pentanoïque ;
l'acide 2-(7-((S)-3-(4-chlorophénylsulfonamido)pipéridin-1-yl)-5-méthylpyrazolo [1,5-a]pyrimidin-6-yl)pentanoïque ;
l'acide 2-(7-((R)-3-(4-chlorophénylsulfonamido)pyrrolidin-1-yl)-5-méthylpyrazolo [1,5-a]pyrimidin-6-yl)pentanoïque ;
l'acide 2-(7-((S)-3-(4-chlorophénylsulfonamido)pyrrolidin-1-yl)-5-méthylpyrazolo [1,5-a]pyrimidin-6-yl)pentanoïque ;
l'acide 2-(5-méthyl-7-phénylpyrazolo[1,5-a]pyrimidin-6-yl)pentanoïque ;
l'acide 2-(5-méthyl-7-p-tolylpyrazolo[1,5-a]pyrimidin-6-yl)pentanoïque ;
l'acide 2-(2-tert-butyl-5-méthyl-7-phénylpyrazolo[1,5-a]pyrimidin-6-yl)pentanoïque ;
l'acide 2-(2-tert-butyl-5-méthyl-7-p-tolylpyrazolo[1,5-a]pyrimidin-6-yl)pentanoïque ;
l'acide 2-(3-bromo-2-tert-butyl-5-méthyl-7-p-tolylpyrazolo[1,5-a]pyrimidin-6-yl)-pentanoïque ;
l'acide 2-(5-méthyl-2-phényl-7-p-tolylpyrazolo[1,5-a]pyrimidin-6-yl)pentanoïque ;
l'acide 2-(2-tert-butyl-7-(3-hydroxyphényl)-5-méthylpyrazolo[1,5-a]pyrimidin-6-yl)-pentanoïque ;
l'acide 2-(2-tert-butyl-5-méthyl-7-(2-naphtyl)pyrazolo[1,5-a]pyrimidin-6-yl)-pentanoïque ;
l'acide 2-(2-tert-butyl-7-(1H-indol-5-yl)-5-méthylpyrazolo[1,5-a]pyrimidin-6-yl)-pentanoïque ;
l'acide 2-(2-tert-butyl-7-(1H-indol-6-yl)-5-méthylpyrazolo[1,5-a]pyrimidin-6-yl)-pentanoïque ;
l'acide 2-(7-(benzofuran-5-yl)-2-tert-butyl-5-méthylpyrazolo[1,5-a]pyrimidin-6-yl)-pentanoïque ;
l'acide 2-(7-(benzo[b]thiophèn-5-yl)-2-tert-butyl-5-méthylpyrazolo[1,5-a]pyrimidin-6-yl)pentanoïque ;
l'acide 2-(2-tert-butyl-7-(2,3-dihydrobenzofuran-5-yl)-5-méthylpyrazolo[1,5-a] pyrimidin-6-yl)pentanoïque ;
l'acide 2-(2-tert-butyl-7-(4-chlorophényl)-5-méthylpyrazolo[1,5-a]pyrimidin-6-yl)-pentanoïque ;
l'acide 2-(2-tert-butyl-7-(3,4-diméthylphényl)-5-méthylpyrazolo[1,5-a]pyrimidin-6-yl)pentanoïque ;
l'acide 2-(2-tert-butyl-7-(4-éthylphényl)-5-méthylpyrazolo[1,5-a]pyrimidin-6-yl)-pentanoïque ;
l'acide 2-(2-tert-butyl-7-(3,4-dihydro-2H-benzo[b][1,4]dioxépin-7-yl)-5-méthyl-pyrazolo[1,5-a]pyrimidin-6-yl)pentanoïque ;
l'acide 2-(2-tert-butyl-7-(4-méthyl-3,4-dihydro-2H-benzo[b][1,4]oxazin-7-yl)-5-méthylpyrazolo[1,5-a]pyrimidin-6-yl)pentanoïque ;
l'acide 2-(5-méthyl-3-phényl-7-p-tolylpyrazolo[1,5-a]pyrimidin-6-yl)pentanoïque ;
l'acide 2-(3,7-di-p-tolyl-5-méthylpyrazolo[1,5-a]pyrimidin-6-yl)pentanoïque ;
l'acide 2-(2-tert-butyl-3,7-di-p-tolyl-5-méthylpyrazolo[1,5-a]pyrimidin-6-yl)-pentanoïque ;
l'acide 2-(5-méthyl-2-propyl-7-p-tolylpyrazolo[1,5-a]pyrimidin-6-yl)pentanoïque ;
l'acide 2-(2-(furan-2-yl)-5-méthyl-7-p-tolylpyrazolo[1,5-a]pyrimidin-6-yl)-pentanoïque ;
l'acide 2-(2-tert-butyl-7-(4-chloro-2-fluorophényl)-5-méthylpyrazolo[1,5-a] pyrimidin-6-yl)pentanoïque ;
l'acide 2-(2-tert-butyl-7-(2-fluoro-4-méthylphényl)-5-méthylpyrazolo[1,5-a] pyrimidin-6-yl)pentanoïque ;
l'acide 2-(2-tert-butyl-7-p-tolyl-5-méthylpyrazolo[1,5-a]pyrimidin-6-yl)-6,6,6-trifluorohexanoïque ;
l'acide 2-(2-tert-butyl-7-p-tolyl-5-méthylpyrazolo[1,5-a]pyrimidin-6-yl)-3-phényl-propanoïque ;
l'acide 2-(2-tert-butyl-5-méthyl-7-(1-méthylindolin-5-yl)pyrazolo[1,5-a]pyrimidin-6-yl)pentanoïque ;
l'acide 2-(2-tert-butyl-5-méthyl-7-(1-méthyl-1H-indol-6-yl)pyrazolo[1,5-a] pyrimidin-6-yl)pentanoïque ;
l'acide 2-(2-tert-butyl-7-(chroman-6-yl)-5-méthylpyrazolo[1,5-a]pyrimidin-6-yl)-pentanoïque ;
l'acide 2-(2-tert-butyl-7-p-tolyl-5-méthylpyrazolo[1,5-a]pyrimidin-6-yl)-3-méthyl-pentanoïque ;
l'acide 2-(2-tert-butyl-3-chloro-7-p-tolyl-5-méthylpyrazolo[1,5-a]pyrimidin-6-yl)-pentanoïque ;
l'acide 2-(2-tert-butyl-7-p-tolyl-5-méthylpyrazolo[1,5-a]pyrimidin-6-yl)-4-méthoxy-butanoïque ;
l'acide 2-(2-tert-butyl-5-méthyl-7-(4-iso-propylphényl)pyrazolo[1,5-a]pyrimidin-6-yl)pentanoïque ;
l'acide 2-(2-tert-butyl-5-méthyl-7-(4-trifluorométhylphényl)pyrazolo[1,5-a] pyrimidin-6-yl)pentanoïque ;
l'acide 2-(2-tert-butyl-7-(2,4-difluorophényl)-5-méthylpyrazolo[1,5-a]pyrimidin-6-yl)pentanoïque ;
l'acide 2-(2-tert-butyl-7-(2-chloro-4-méthylphényl)-5-méthylpyrazolo[1,5-a] pyrimidin-6-yl)pentanoïque ;
l'acide 2-(7-(2-amino-4-méthylphényl)-2-tert-butyl-5-méthylpyrazolo[1,5-a] pyrimidin-6-yl)pentanoïque ;
l'acide 2-(2-tert-butyl-7-(2-méthoxy-4-chlorophényl)-5-méthylpyrazolo[1,5-a] pyrimidin-6-yl)pentanoïque ;
l'acide 2-(2-tert-butyl-7-(2-fluoro-4-méthoxyphényl)-5-méthylpyrazolo[1,5-a] pyrimidin-6-yl)pentanoïque ;
l'acide 2-(2-tert-butyl-5-méthyl-7-(5-quinoline)pyrazolo[1,5-a]pyrimidin-6-yl)-pentanoïque ;
l'acide 2-(2-tert-butyl-5-méthyl-7-(8-quinoline)pyrazolo[1,5-a]pyrimidin-6-yl)-pentanoïque ;
l'acide 2-(2-tert-butyl-7-(2,4-diméthylphényl)-5-méthylpyrazolo[1,5-a]pyrimidin-6-yl)pentanoïque ;

l'acide 2-(2-tert-butyl-3,5-diméthyl-7-p-tolylpyrazolo[1,5-a]pyrimidin-6-yl)-pentanoïque ;
l'acide 2-(2-tert-butyl-5-méthyl-3-phényl-7-p-tolylpyrazolo[1,5-a]pyrimidin-6-yl)-pentanoïque ;
l'acide 2-(2,3,5-triméthyl-7-p-tolyl-3H-imidazo[4,5-b]pyridin-6-yl)pentanoïque ;
l'acide 2-(1,2,5-triméthyl-7-p-tolyl-1H-imidazo[4,5-b]pyridin-6-yl)pentanoïque ;
l'acide 2-(2-propyl-3,5-diméthyl-7-p-tolyl-3H-imidazo[4,5-b]pyridin-6-yl)-pentanoïque ;
le 2-(2-isopropyl-3,5-diméthyl-7-p-tolyl-3H-imidazo[4,5-b]pyridin-6-yl)pentanoate ;
le 2-(2-isopropyl-5-méthyl-7-p-tolyl-[1,2,4]-triazolo[1,5-a]pyrimidin-6-yl)acétate de méthyle ;
l'acide 2-(2-isopropyl-5-méthyl-7-p-tolyl-[1,2,4]-triazolo[1,5-a]pyrimidin-6-yl)-acétique ;
le 2-[2-tert-butyl-7-(1,2-dihydroacénaphtylèn-5-yl)-5-méthylpyrazolo[1,5-a] pyrimidin-6-yl]acétate de méthyle ;
l'acide 2-[2-tert-butyl-7-(1,2-dihydroacénaphtylèn-5-yl)-5-méthylpyrazolo[1,5-a] pyrimidin-6-yl]acétique ;
le 2-[2-tert-butyl-7-(2-méthoxy-4-méthylphényl)-5-méthylpyrazolo[1,5-a]pyrimidin-6-yl]acétate de méthyle ;
l'acide 2-[2-tert-butyl-7-(2-méthoxy-4-méthylphényl)-5-méthylpyrazolo[1,5-a] pyrimidin-6-yl]acétique ;
l'acide 2-[2-tert-butyl-7-(2-hydroxy-4-méthylphényl)-5-méthylpyrazolo[1,5-a] pyrimidin-6-yl]acétique ;
le 2-(2-isopropyl-5-méthyl-7-p-tolyl-[1,2,4]-triazolo[1,5-a]pyrimidin-6-yl)pentanoate de méthyle ;
l'acide 2-(2-isopropyl-5-méthyl-7-p-tolyl-[1,2,4]-triazolo[1,5-a]pyrimidin-6-yl)-pentanoïque ;
l'acide 2-(2-benzyl-5-méthyl-7-p-tolyl-[1,2,4]-triazolo[1,5-a]pyrimidin-6-yl)-pentanoïque ; ou
le 2-(2-benzyl-5-méthyl-7-p-tolyl-[1,2,4]-triazolo[1,5-a]pyrimidin-6-yl)pentanoate d' éthyle ;

ou un sel pharmaceutiquement acceptable de ceux-ci.

**8.** Composés selon les revendications 1 à 7, pour une utilisation comme médicament.

**9.** Composés selon la revendication 8, pour une utilisation comme médicament pour la prévention ou le traitement d'une infection virale chez un animal.

**10.** Composés selon la revendication 9, dans lesquels ladite infection virale est une infection par le VIH.

**11.** Composés selon la revendication 9 ou 10, dans lesquels ledit animal est un humain.

**12.** Utilisation de composés selon les revendications 1 à 7, pour la préparation d'un médicament destiné à prévenir ou à traiter une infection virale chez un animal.

**13.** Utilisation selon la revendication 12, dans laquelle l'infection virale est une infection par le VIH.

**14.** Composition pharmaceutique comprenant les composés selon les revendications 1 à 7 en tant que principe actif en mélange avec au moins un support pharmaceutiquement acceptable.

**15.** Composition pharmaceutique selon la revendication 14, comprenant en outre au moins un autre composé ayant une activité antivirale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03062238 A
- WO 03062204 A
- WO 03047564 A
- WO 03049690 A
- WO 03035076 A
- WO L000810810 A
- JP 2003171381 B
- JP 2003119137 B
- WO 9615111 A, Hoye, T.
- US 20060094706 A1

### Non-patent literature cited in the description


- **VANDAMME et al.** *Antiviral Chem. Chemother.,* 1998, vol. 9, 187-203
- **PERELSON et al.** *Nature,* 1997, vol. 387, 123-124
- **SCHMIT et al.** *J. Infect. Dis.,* 1996, vol. 174, 962-968
- **THEODORA W. GREENE.** Protective Groups in Organic Chemistry. John Wiley & Sons, Inc, 1991
- **PAQUETTE, LEO A.** Principles of Modem Heterocyclic Chemistry. 1968
- The Chemistry of Heterocyclic Compounds, A series of Monographs. John Wiley & Sons, 1950
- **KATRITZKY, ALAN R ; REES, C.W. ; SCRIVEN, E.** Comprehensive Heterocyclic Chemistry. Pergamon Press, 1996
- *J. Am. Chem. Soc.,* 1960, vol. 82, 5566
- **CHOU et al.** *Adv. Enzyme Reg.,* 1984, vol. 22, 27
- **ELION et al.** *J. Biol. Chem.,* 1954, vol. 208, 477-488
- **BABA et al.** *Antimicrob. Agents Chemother,* 1984, vol. 25, 515-517
- **E. L. ELIEL.** Stereochemistry of Carbon Compounds. McGraw Hill, 1962
- **LOCHMULLER, C. H.** *J. Chromatogr.,* 1975, vol. 113 (3), 283-302
- **ELIEL, E. ; WILEN, S.** Stereochemistry of Organic Compounds. John Wiley & Sons, Inc, 1994, 322
- **JACOB III.** *J. Org. Chem.,* 1982, vol. 47, 4165
- Chiral Liquid Chromatography. Chapman and Hall, 1989
- **OKAMOTO.** Optical resolution of dihydropyridine enantiomers by High-performance liquid chromatography using phenylcarbamates of polysaccharides as a chiral stationary phase. *J. of Chromatogr.,* 1990, vol. 513, 375-378
- McCutcheon's Detergents and Emulsifiers Annual. MC Publishing Crop, 1981
- Tensid-Taschenbucw. Hanser Verlag, 1981
- Encyclopaedia of Surfactants. Chemical Publishing Co, 1981
- **RAUTIO J. et al.** Prodrugs: design and clinical applications. *Nature Reviews Drug Discovery,* 2008
- **ADACHI, A. ; GENDELMAN, H. ; KOENIG, S. ; FOLKS, T. ; WILLEY, R. ; RABSON, A. ; MARTIN, M.** Production of acquired immunodeficiency syndrome-associated retrovirus in human and nonhuman cells transfected with an infectious molecular clone. *J. Virol.,* 1986, vol. 59, 284-291
- **BARR-SINOUSSI, F. ; CHERMANN, J.C. ; REY, F ; NUGEYRE, M.T. ; CHAMARET, S. ; GRUEST, J. ; DAUGUET, C. ; AXLER-BLIN, C. ; V,ZINET-BRUN, F. ; ROUZIOUX, C.** Isolation of a T-lymphotropic retrovirus from patient at risk for AIDS. *Science,* 1983, vol. 220, 868-871
- **MIYOSHI, I. ; TAGUCHI, H ; KOBONISHI, I ; YOSHIMOTO, S ; OHTSUKI, Y. ; SHIRAISHI, Y ; AKAGI,T.** Type C virus-producing cell lines derived from adult T cell leukemia. *Gann mongr,* 1982, vol. 28, 219-228
- **BARTHOLOMEEUSEN, K. et al.** Differential interaction of HIV-1 integrase and JPO2 with the C terminus of LEDGF/p75. *J. Mol. Biol.,* 2007, vol. 372, 407-421
- **BARTHOLOMEEUSEN, K. et al.** Lens Epithelium Derived Growth Factor/p75 interacts with the transposase derived DDE domain of pogZ. *J. Biol. Chem.,* 2009
- **BUSSCHOTS, K. et al.** The interaction of LEDGF/p75 with integrase is lentivirus-specific and promotes DNA binding. *J. Biol. Chem.,* 2005, vol. 280, 17841-17847